(19) Europäisches Patentamt
European Patent Office
Office européen des brevets

(11) EP 4 729 529 A1

(12) **EUROPEAN PATENT APPLICATION**
published in accordance with Art. 153(4) EPC

(43) Date of publication:
**22.04.2026 Bulletin 2026/17**

(21) Application number: **24799894.1**

(22) Date of filing: **30.04.2024**

(51) International Patent Classification (IPC):
***C07K 7/08*** $^{(2006.01)}$ ***C07K 7/04*** $^{(2006.01)}$
***C07K 7/02*** $^{(2006.01)}$ ***A61K 38/00*** $^{(2006.01)}$

(52) Cooperative Patent Classification (CPC):
**A61K 38/00; C07K 7/02; C07K 7/04; C07K 7/08**

(86) International application number:
**PCT/CN2024/090789**

(87) International publication number:
**WO 2024/227437 (07.11.2024 Gazette 2024/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**04.05.2023 CN 202310522881**
**14.08.2023 CN 202311022217**
**15.09.2023 CN 202311193280**
**14.11.2023 CN 202311512882**
**13.12.2023 CN 202311710528**
**28.12.2023 CN 202311834661**
**11.01.2024 CN 202410040688**
**04.02.2024 CN 202410158092**
**13.03.2024 CN 202410285970**

(71) Applicant: **Xizang Haisco Pharmaceutical Co., Ltd.**
**Lhoka, Tibet 856099 (CN)**

(72) Inventors:
- **LI, Yao**
  **Chengdu, Sichuan 611130 (CN)**
- **CHEN, Lei**
  **Chengdu, Sichuan 611130 (CN)**
- **LIU, Lei**
  **Chengdu, Sichuan 611130 (CN)**
- **HUANG, Haitao**
  **Chengdu, Sichuan 611130 (CN)**
- **TANG, Pingming**
  **Chengdu, Sichuan 611130 (CN)**
- **ZHANG, Chen**
  **Chengdu, Sichuan 611130 (CN)**
- **YAN, Pangke**
  **Chengdu, Sichuan 611130 (CN)**

(74) Representative: **AWA Denmark A/S**
**Strandgade 56**
**1401 Copenhagen K (DK)**

Remarks:
The complete document including Reference Table(s) and the Sequence Listing(s) can be downloaded from the EPO website

# (54) PREPARATION OF PEPTIDE INHIBITOR OF INTERLEUKIN-23 RECEPTOR AND USE THEREOF

(57) Provided are a new peptide inhibitor of an interleukin-23 receptor, a stereoisomer or pharmaceutically acceptable salt or solvate thereof, or a pharmaceutical composition containing same, and the use of the peptide inhibitor in the treatment or prevention of diseases or conditions including inflammatory bowel disease, Crohn's disease, psoriasis, etc.

EP 4 729 529 A1

## Description

### Technical Field

The present disclosure relates to a new peptide inhibitor of an interleukin-23 receptor and the use thereof in the treatment or prevention of various diseases and conditions, including inflammatory bowel disease, Crohn's disease and psoriasis.

### Background Art

As a cytokine, Interleukin-23 (IL-23) has been thought to play a crucial role in the pathogenesis of autoimmune diseases, such as multiple sclerosis, asthma, rheumatoid arthritis, psoriasis, and inflammatory bowel disease (IBD). Model studies with acute and chronic IBD mice have shown that IL-23R and downstream effector cytokines play a major role in the pathogenesis of the disease. IL-23R is expressed on a variety of adaptive and innate immune cells, including Th17 cells, $\gamma\delta$ T cells, natural killer (NK) cells, dendritic cells, macrophages, and innate lymphocytes, which are abundant in the intestine. IL-23R gene expression and protein levels have been found to be elevated on the surface of the intestinal mucosa in IBD patients. Studies have suggested that this effect is mediated by IL-23 through promoting the development of a population of pathogenic CD4+ T cells that produce IL-6, IL-17, and tumour necrosis factor (TNF).

The produced IL-23 is enriched in the intestine, and plays a key role in regulating the balance between tolerance and immunity through T cell-dependent and T cell-independent enteritis pathways via effects on T helper cell 1 (Th1)- and Th17-related cytokines and inhibition of regulatory T cell responses in the intestine (which facilitates inflammation). In addition, polymorphism of IL-23 receptor (IL-23R) has been associated with susceptibility to inflammatory bowel disease (IBD), further establishing the critical role of the IL-23 pathway in intestinal homeostasis.

Psoriasis, a chronic skin disease affecting 2% to 3% of the total population, has been shown to be mediated by the inflammatory response mechanism of T cells in the body. IL-23 is one of several interleukins, which is believed to be a key player in the pathogenesis of psoriasis, and which is said to maintain chronic autoimmune inflammation by inducing interleukin-17, regulating memory T cells, and activating macrophages. It has been shown that the expression of IL-23 and IL-23R is increased in tissues of psoriasis patients, and antibodies that neutralize IL-23 have exhibited IL-23-dependent inhibition of psoriasis development in animal models of psoriasis.

IL-23 is a heterodimer consisting of a unique p19 subunit and the p40 subunit of IL-12, and is a cytokine involved in the development of T helper cell 1 (TH1) that produces interferon-$\gamma$ (IFN-$\gamma$). Although both IL-23 and IL-12 contain the p40 subunit, they have different phenotypic properties. For example, IL-12-deficient animals are susceptible to inflammatory autoimmune diseases, whereas IL-23-deficient animals are resistant, presumably because IL-23-deficient animals have a reduced number of CD4+ T cells, which produce IL-6, IL-17, and TNF, in the CNS. IL-23 binds to IL-23R, which is a heterodimeric receptor consisting of IL-12R$\beta$1 and IL-23R subunits. Binding of IL-23 to IL-23R activates Jak-Stat signalling molecules, Jak2, Tyk2, as well as Stat1, Stat3, Stat4, and Stat5, among which Stat4 activation is substantially weaker and results in the formation of a different DNA-binding Stat complex in response to IL-23 compared to IL-12. IL-23R constitutively binds to Jak2 and binds to Stat3 in a ligand-dependent manner. Compared to IL-12, which predominantly acts on naive CD4 (+) T cells, IL-23 preferentially acts on memory CD4 (+) T cells.

Therapeutic moieties that inhibit the IL-23 pathway have been identified for use in the treatment of IL-23-related diseases. A large number of antibodies that bind to IL-23 or IL-23R have been identified, including ustekinumab (a humanized antibody that binds to IL-23), which has been approved for the treatment of psoriasis. Polypeptide inhibitors that bind to IL-23R and inhibit the binding of IL-23 to IL-23R have recently been identified. Clinical trials in Crohn's disease or psoriasis with ustekinumab and briakinumab (which target the common p40 subunit), as well as tildrakizumab, guselkumab, MEDI2070, and BI-655066 (which target the unique p19 subunit of IL-23), have highlighted the potential of blockade of IL-23 signalling in the treatment of human inflammatory diseases. While these findings are promising, it remains challenging to identify stable and selective agents that preferentially target the IL-23 pathway in the intestine, which can be used to treat enteritis (including Crohn's disease, ulcerative colitis, and related conditions).

Therefore, there remains a need in the art for novel therapies targeting the IL-23 pathway, which can be used to treat and prevent IL-23-related diseases, including diseases associated with autoimmunity. Furthermore, compounds and methods that specifically target IL-23R from the cavity side of the intestine can provide therapeutic benefits to IBD patients. The present disclosure addresses these needs by providing new peptide inhibitors that bind IL-23R to inhibit IL-23 binding and signal transduction and are suitable for oral use.

### Summary of the Invention

The present disclosure discloses a new peptide inhibitor of an interleukin-23 receptor, or a stereoisomer, a pharmaceutically acceptable salt or a solvate thereof, or a pharmaceutical composition containing same, and the use of

the peptide inhibitor in the treatment or prevention of diseases or conditions including inflammatory bowel disease, Crohn's disease, psoriasis, etc.

The peptide compounds of the present disclosure have protein stability and are stable against plasma proteases, epithelial proteases, gastric and intestinal proteases, lung surface proteases, intracellular proteases, etc. The peptide compounds have specific targeting for IL-23, and have a relatively-long plasma half-life and good pharmacokinetic and pharmacodynamic properties.

The present disclosure relates to a cyclic peptide compound, or a stereoisomer, a pharmaceutically acceptable salt, a solvate or a dimer thereof, wherein the peptide compound has an amino acid sequence of formula (I), formula (II), formula (II-1), formula (III), formula (III-1), formula (IV), formula (IV-1) or formula (V):

$Xa_1$-$Xa_2$-$Xa_3$-$Xa_4$-$Xa_5$-$Xa_6$-$Xa_7$-$Xa_8$-$Xa_9$-$Xa_{10}$-$Xa_{11}$-$Xa_{12}$-$Xa_{13}$ (I)

(Ac)$Xa_1$-$Xa_2$-Thr-$Xa_4$-$Xa_5$-$Xa_6$-$Xa_7$-$Xa_8$-$Xa_9$-$Xa_{10}$-Asn-(3-Pal)-Sarc($NH_2$) (II)

(Ac)$Xa_1$-$Xa_2$-Thr-$Xa_4$-Lys(Ac)-$Xa_6$-$Xa_7$-$Xa_8$-$Xa_9$-Glu-Asn-(3-Pal)-Sarc($NH_2$) (II-1)

(SEQ ID NO.1)

(Ac)$Xa_1$-$Xa_2$-Thr-Trp($R_1$)-$Xa_5$-$Xa_6$-$Xa_7$-$Xa_8$-$Xa_9$-$Xa_{10}$-Asn-(3-Pal)-Sarc($NH_2$) (III)

(Ac)$Xa_1$-$Xa_2$-Thr-Trp($CH_3$)-Lys(Ac)-$Xa_6$-$Xa_7$-$Xa_8$-$Xa_9$-Glu-Asn-(3-Pal)-Sarc($NH_2$) (III-1)

(SEQ ID NO.2)

(N-terminus)$Xa_1$-Asn-Thr-Trp($R_1$)-$Xa_5$-$Xa_6$-$Xa_7$-(2-Nal)-$Xa_9$-$Xa_{10}$-$Xa_{11}$-$Xa_{12}$-Sarc(C-terminus)

(IV)

(N-terminus)$Xa_1$-Asn-Thr-Trp($CH_3$)-Lys(Ac)-$Xa_6$-$Xa_7$-(2-Nal)-$Xa_9$-Glu-$Xa_{11}$-$Xa_{12}$-Sarc(C-terminus)

(IV-1) (SEQ ID NO.3)

(N-terminus)$Xa_1$-His-Thr-Trp($R_1$)-$Xa_5$-$Xa_6$-$Xa_7$-(2-Nal)-$Xa_9$-$Xa_{10}$-$Xa_{11}$-$Xa_{12}$-Sarc(C-terminus)

(V)

alternatively, the peptide compound is a dimer compound, and the dimer compound is formed by linking the peptide compound to amino acid residues in the peptide compound via a polyethylene glycol chain; the polyethylene glycol chain is

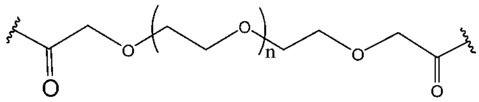,

n is any integer selected from 0-99;

alternatively, the peptide compound is optionally conjugated to a modifying group at $Xa_1$ or Xas;

or alternatively, the peptide compound is optionally conjugated to a modifying group at $Xa_1$, $Xa_5$, or $Xa_7$;

in some embodiments, the modifying group is

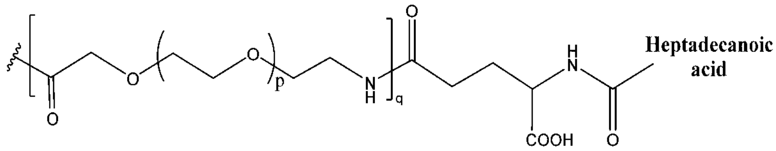,

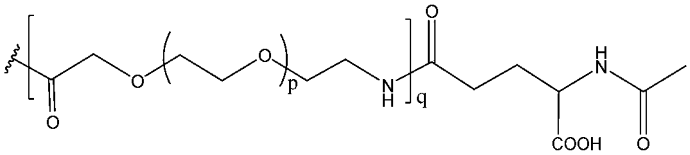,

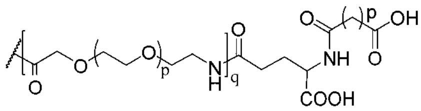 or 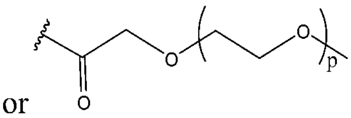

, in which p is any integer selected from 0-50, and q is any integer selected from 0-50;

in some embodiments, the modifying group is

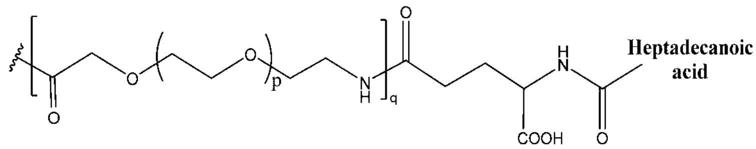,

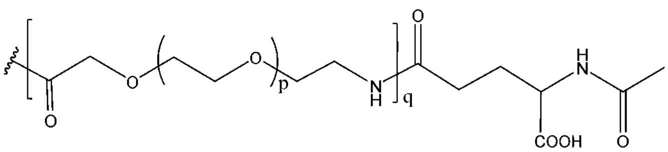 or 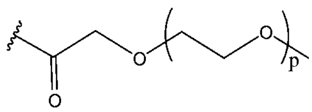,

in which p is any integer selected from 0-50, and q is any integer selected from 0-50;

in some embodiments, the modifying group is

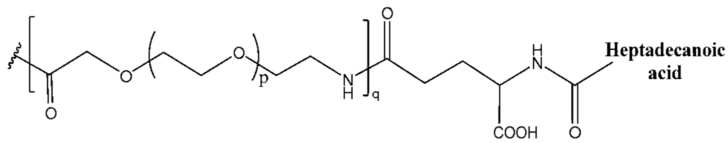

, in which p is any integer selected from 0-5, and q is any integer selected from 0-5;

in some embodiments, the modifying group is

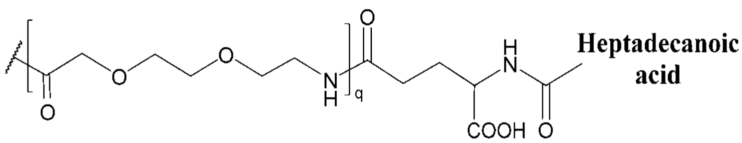,

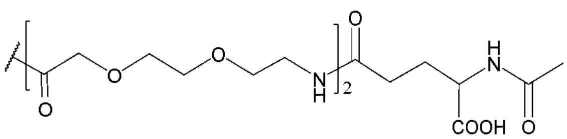, 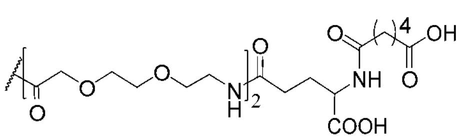,

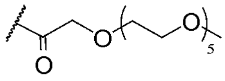, 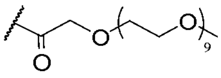 or 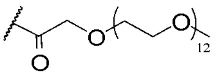;

in some embodiments, p is 1, and q is 2;

in which $Xa_1$ and $Xa_6$ are each independently selected from Pen, Pcn, Asn, Ala, Ala(3-amino), Ala(2-ethyne), Ala(3-azido), Ala(2-ethene), Val(2-ethene), Asp, 2,4-diaminobutyric acid, Ser, Cys, Hcys, or Glu, and the residues of $Xa_1$ and $Xa_6$ react to form a peptide ring or form a cyclic peptide via $L_1$;

in some embodiments, $Xa_1$ and $Xa_6$ are each independently selected from Pen, Asn, Ala, Ala(3-amino), Ala(2-ethyne), Ala(3-azido), Ala(2-ethene), Val(2-ethene), Asp, 2,4-diaminobutyric acid, Ser, Cys, Hcys, or Glu, and the residues of $Xa_1$ and $Xa_6$ react to form a peptide ring;

in some embodiments, $Xa_1$ and $Xa_6$ are each independently selected from Pen, Asn, Ala, Ala(3-amino), Ala(2-ethyne), Ala(3-azido), Ala(2-ethene), or Val(2-ethene), and the residues of $Xa_1$ and $Xa_6$ react to form a peptide ring; the residues of $Xa_1$ and $Xa_6$ preferably undergo a condensation reaction, an RCM ring-closing reaction, etc. to form a peptide ring;

in some embodiments, $Xa_1$ and $Xa_6$ are selected from Pen;

in some embodiments, $Xa_1$ and $Xa_6$ are each independently selected from Asn or Ala(3-amino);

in some embodiments, $Xa_1$ is selected from Asn, and $Xa_6$ is selected from Ala(3-amino);

in some embodiments, $Xa_1$ and $Xa_6$ are each independently selected from Ala(2-ethyne) or Ala(3-azido);

in some embodiments, $Xa_1$ is selected from Ala(3-azido), and $Xa_6$ is selected from Ala(2-ethyne);

in some embodiments, $Xa_1$ and $Xa_6$ are selected from Val(2-ethene);

in some embodiments, $Xa_1$ and $Xa_6$ are selected from Ala(2-ethene);

in some embodiments, $Xa_1$ and $Xa_6$ are each independently selected from Asp or Ala(3-amino);

in some embodiments, $Xa_1$ and $Xa_6$ are selected from 2,4-diaminobutyric acid;

in some embodiments, $Xa_1$ and $Xa_6$ are each independently selected from 2,4-diaminobutyric acid or Asp;

in some embodiments, $Xa_1$ and $Xa_6$ are each independently selected from Ser or Ala(3-amino);

in some embodiments, $Xa_1$ and $Xa_6$ are each independently selected from 2,4-diaminobutyric acid or Ser;

in some embodiments, $Xa_1$ and $Xa_6$ are selected from Ala(3-amino);

in some embodiments, $Xa_1$ and $Xa_6$ are each independently selected from Cys or Asp;

in some embodiments, $Xa_1$ and $Xa_6$ are each independently selected from Glu or Ala(3-amino);

in some embodiments, $Xa_1$ and $Xa_6$ are each independently selected from Hcys or Asp;

in some embodiments, the residues of $Xa_1$ and $Xa_6$ react to form the following structure:

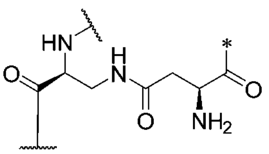, 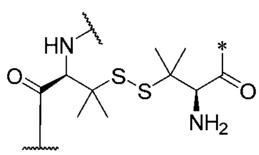, 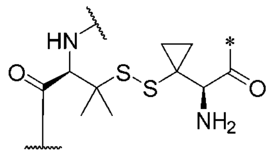,

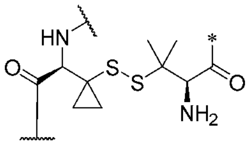, 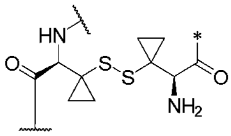, 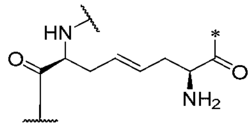, 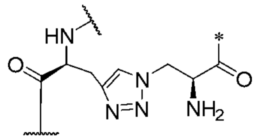,

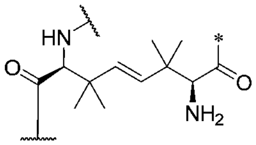, 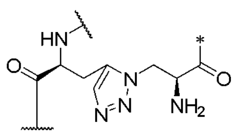, 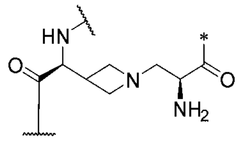, 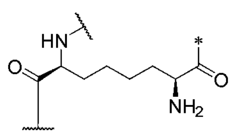,

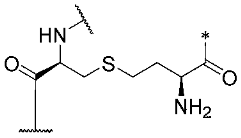, 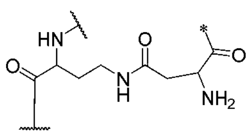, 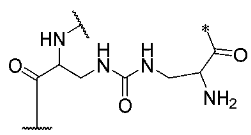, 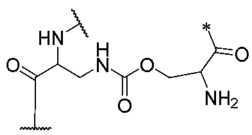,

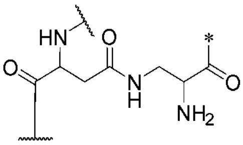, 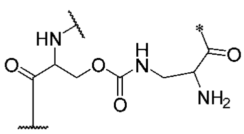, 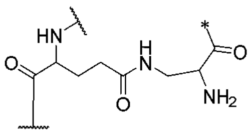, or 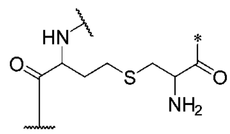;

in some embodiments, the residues of $Xa_1$ and $Xa_6$ react to form the following structure:

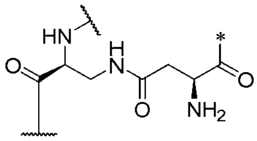, 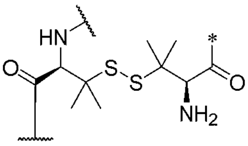, 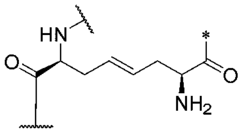,

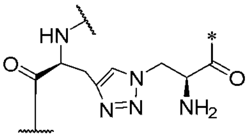, 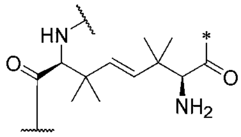, 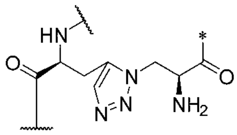, 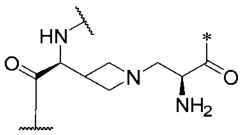,

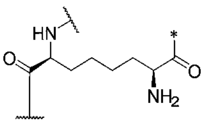, 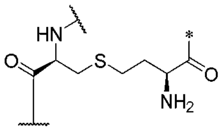, 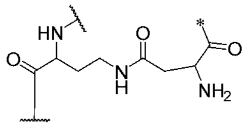, 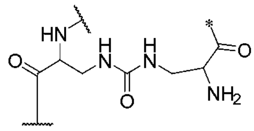,

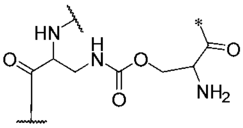, 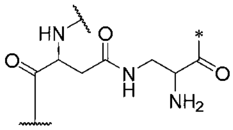, 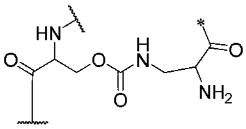, 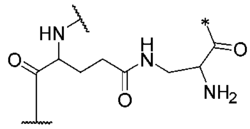,

or

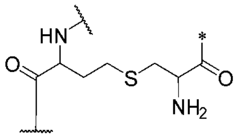;

in some embodiments, the residues of $Xa_1$ and $Xa_6$ react to form the following structure:

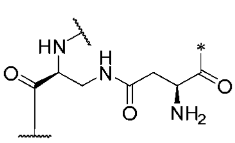, 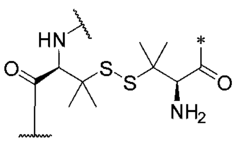, 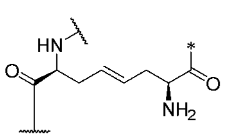,

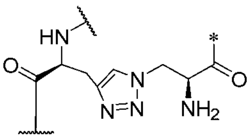, 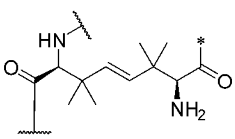, 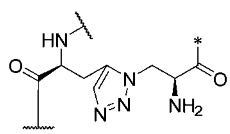, 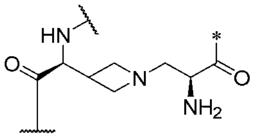,

or

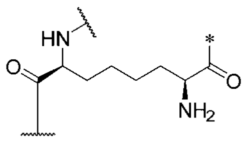;

in which the * end is an end of $Xa_1$, $Xa_1$ is linked to $Xa_2$ via the * position, and the $NH_2$ terminal end is linked to a protecting group;
or the * end is an end of $Xa_1$, $Xa_1$ is linked to $Xa_2$ via the * position, and the $NH_2$ terminal end is conjugated to a modifying group;
in some embodiments, the modifying group conjugated to the $NH_2$ terminal end is selected from

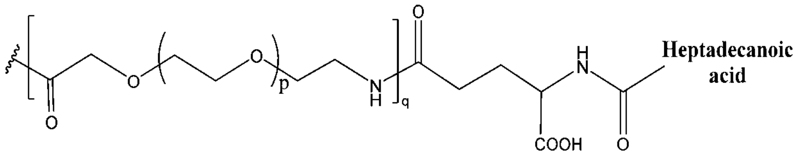,

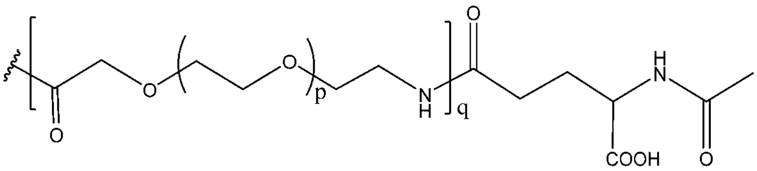,

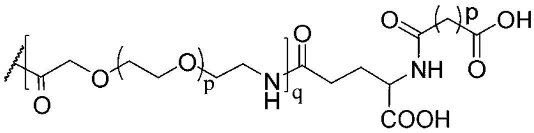 or 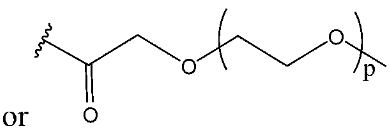;

in some embodiments, the modifying group conjugated to the $NH_2$ terminal end is selected from

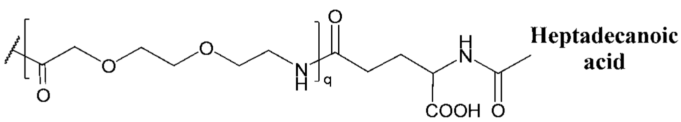,

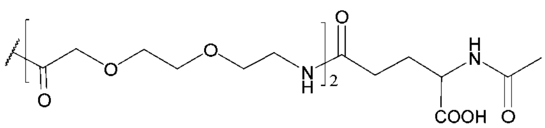, 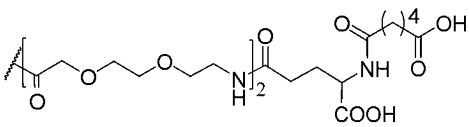,

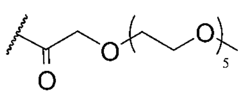, 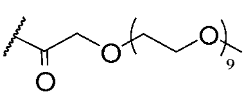 or 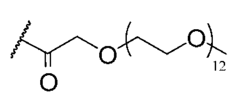;

$Xa_2$ is selected from Asn, His, or an analogue of Asn and His;
in some embodiments, $Xa_2$ is selected from Asn;
in some embodiments, $Xa_2$ is selected from Asn, His, or an analogue of His, and the analogue of His is selected from

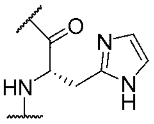 or 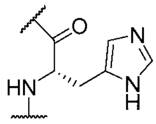;

in some embodiments, $Xa_2$ is selected from Asn, His, or an analogue of His, and the analogue of His is selected from

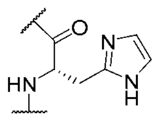 ;

in some embodiments, $Xa_2$ is selected from Asn, or an analogue of His, and the analogue of His is selected from

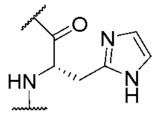 ;

$Xa_3$ is selected from Thr or an analogue of Thr;
in some embodiments, $Xa_3$ is selected from Thr;
$Xa_4$ is selected from Trp or an analogue of Trp;
in some embodiments, $Xa_4$ is an analogue of Trp, and the analogue of Trp is selected from

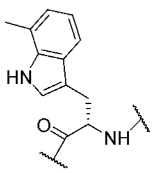 , 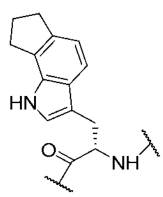 , 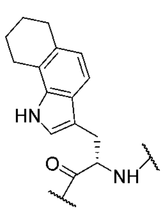 , 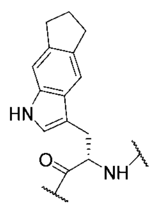 , 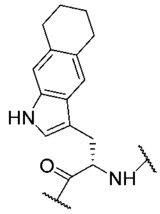 ,

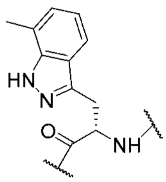 , 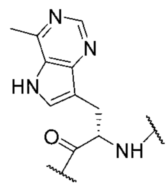 , 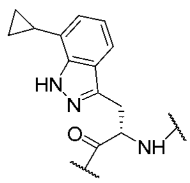 , or 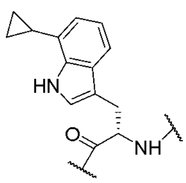 ;

$Xa_5$ is selected from Lys, Gln, Arg, Cit, or an analogue of Lys, Gln, Cit, and Arg; alternatively, a residue of $Xa_5$ is conjugated to a modifying group;
$Xa_5$ is selected from Lys, Gln, Arg, Cit, or an analogue of Lys, Gln, Cit, and Arg;
in some embodiments, $Xa_5$ is selected from Lys, Gln, Arg, Cit, or an analogue of Arg, and the analogue of Arg is selected from

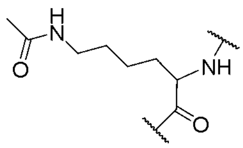 ;

in some embodiments, $Xa_5$ is selected from Lys, Gln, Arg, or an analogue of Lys, Gln and Arg;
in some embodiments, $Xa_5$ is selected from Lys, Gln, Arg, or an analogue of Lys and Arg;
in some embodiments, $Xa_5$ is selected from Lys, or an analogue of Lys;
in some embodiments, $Xa_5$ is selected from Lys;
in some embodiments, $Xa_5$ is selected from Lys, Gln, Arg, or an analogue of Lys and Arg; the analogue of Lys and Arg is selected from

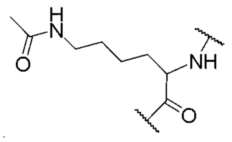

or

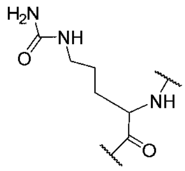

; in some embodiments, $Xa_5$ is selected from Lys, Gln, Arg or an analogue of Arg, and the analogue of Arg is selected from

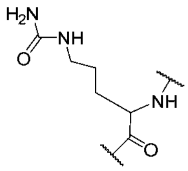

;

in some embodiments, the modifying group conjugated to the residue of $Xa_5$ is selected from

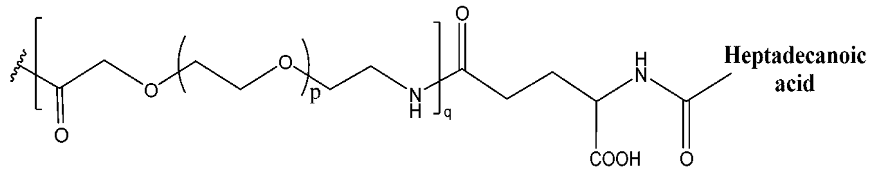

,

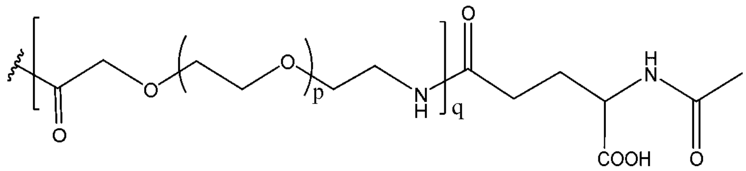

,

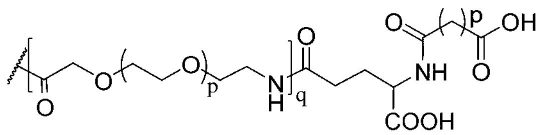

or

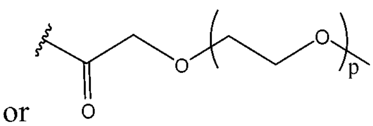

;

in some embodiments, the modifying group conjugated to the residue of $Xa_5$ is selected from

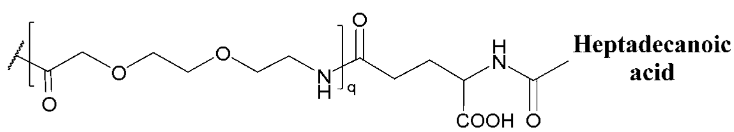

,

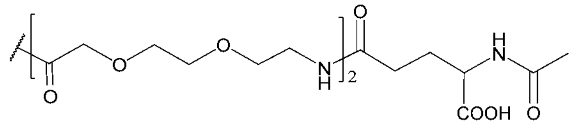

,

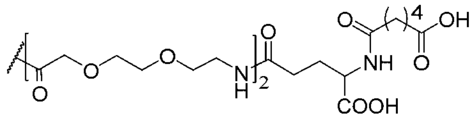

,

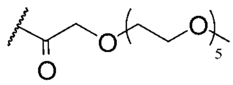, 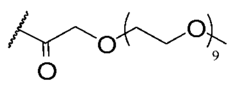 or 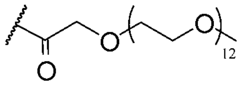;

$Xa_7$ is selected from Phe or an analogue of Phe; alternatively, a residue of $Xa_7$ is conjugated to a modifying group; $Xa_7$ is selected from Phe or an analogue of Phe;

in some embodiments, $Xa_7$ is selected from Phe or an analogue of Phe, and the analogue of Phe is selected from

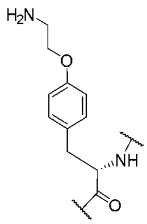, 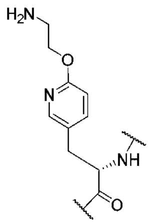, 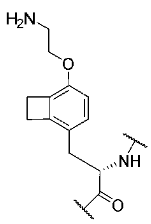, 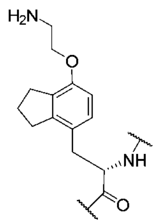,

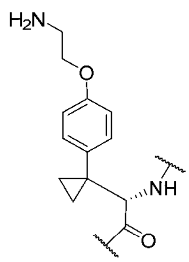, 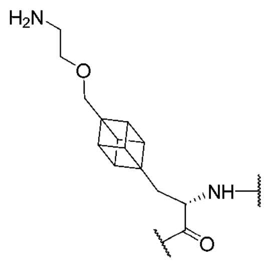 or 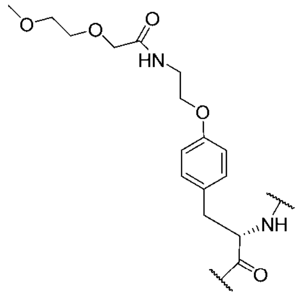;

in some embodiments, $Xa_7$ is selected from Phe or an analogue of Phe, and the analogue of Phe is selected from

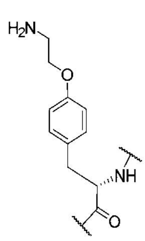, 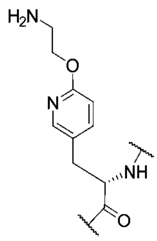, 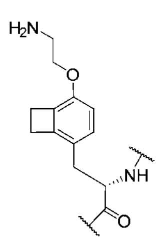, 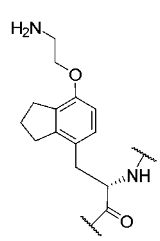,

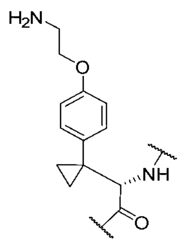 or 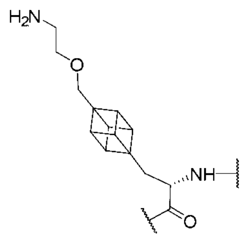;

in some embodiments, $Xa_7$ is selected from Phe or an analogue of Phe, and the analogue of Phe is selected from

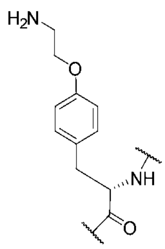, 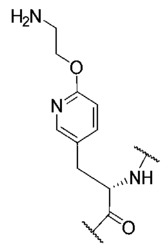, 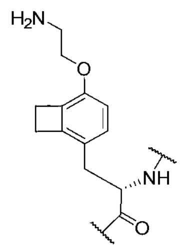,

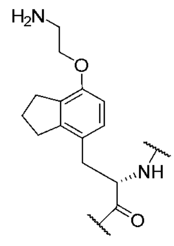, or 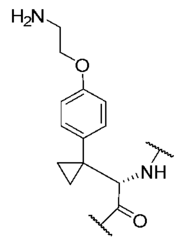;

in some embodiments, the modifying group conjugated to the residue of $Xa_7$ is selected from

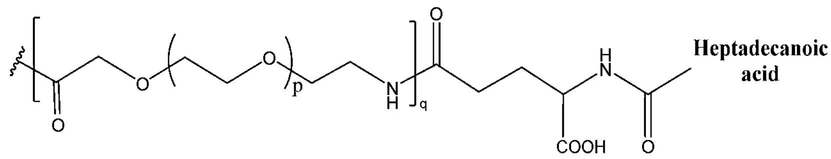,

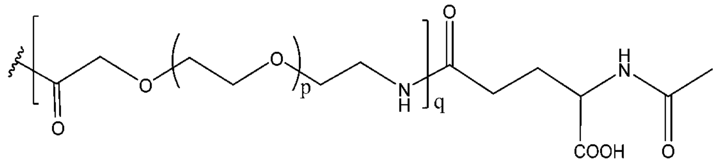,

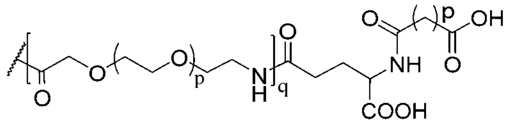 or 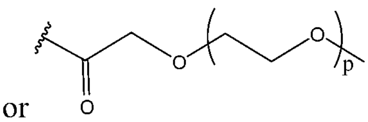;

$Xa_8$ is selected from Phe, Trp, 2-Nal, or an analogue of Phe, Trp, and 2-Nal, and
in some embodiments, $Xa_8$ is selected from Phe, Trp, 2-Nal, or an analogue of Phe, Trp, and 2-Nal, and the analogue of Phe, Trp, and 2-Nal is selected from

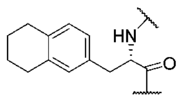, 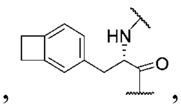, 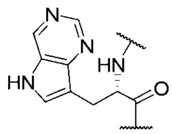, 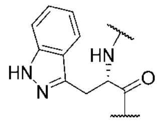, 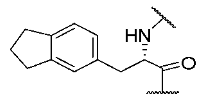, 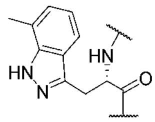,

or

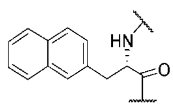;

in some embodiments, $Xa_8$ is selected from Phe, Trp, 2-Nal, or an analogue of Phe, Trp, and 2-Nal, and the analogue of Phe, Trp, and 2-Nal is selected from

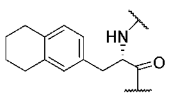, 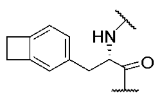, 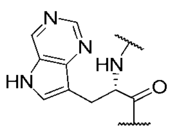, 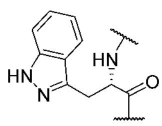, 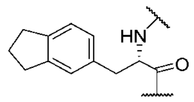,

or

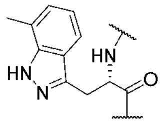;

in some embodiments, $Xa_8$ is selected from Phe, Trp, 2-Nal, or an analogue of Phe, Trp, and 2-Nal, and the analogue of Phe, Trp, and 2-Nal is selected from

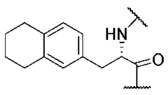, 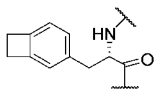, 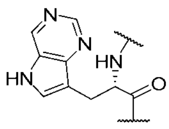, 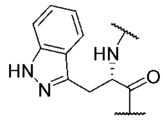, or 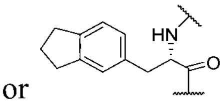;

$Xa_9$ is selected from Thp or an analogue of Thp;
in some embodiments, $Xa_9$ is selected from Thp or an analogue of Thp, and the analogue of Thp is selected from

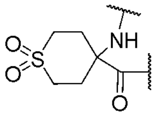, 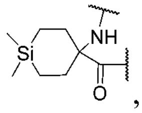, 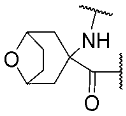, or 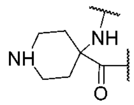;

in some embodiments, $Xa_9$ is selected from Thp or an analogue of Thp, and the analogue of Thp is selected from

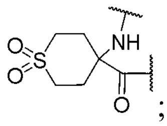;

$Xa_{10}$ is selected from Glu, Cys, or an analogue of Glu and Cys;
in some embodiments, $Xa_{10}$ is selected from Glu or an analogue of Glu;
in some embodiments, $Xa_{10}$ is selected from Glu;
in some embodiments, $Xa_{10}$ is selected from Glu or Cys;
$Xa_{11}$ is selected from Asn, Lys, or an analogue of Asn and Lys;
in some embodiments, $Xa_{11}$ is selected from Asn or Lys;
in some embodiments, $Xa_{11}$ is selected from Asn;
in some embodiments, $Xa_{11}$ is selected from Lys;
$Xa_{12}$ is selected from 3-Pal, Phe, Asp, or an analogue of 3-Pal, Phe, and Asp;
in some embodiments, $Xa_{12}$ is selected from 3-Pal or Phe;
$Xa_{13}$ is selected from Sarc or an analogue of Sarc;
in some embodiments, $Xa_{13}$ is selected from Sarc;
alternatively, the residues of any amino acid in $Xa_1$, $Xa_2$, $Xa_3$, $Xa_4$, $Xa_5$, $Xa_7$, $Xa_8$, $Xa_9$, $Xa_{10}$, $Xa_{11}$, $Xa_{12}$, and $Xa_{13}$ are directly condensed or are linked via $L_1$ to form one or more peptide rings;
in some embodiments, the residues of any amino acid in $Xa_1$, $Xa_2$, $Xa_3$, $Xa_4$, $Xa_5$, $Xa_7$, $Xa_8$, $Xa_9$, $Xa_{10}$, $Xa_{11}$, $Xa_{12}$ and $Xa_{13}$ are linked via $L_1$ to form a peptide ring;
in some embodiments, the residues of any amino acid in $Xa_2$, $Xa_3$, $Xa_4$, $Xa_5$, $Xa_7$, $Xa_8$, $Xa_9$, $Xa_{10}$, $Xa_{11}$, $Xa_{12}$ and $Xa_{13}$ are linked via $L_1$ to form a peptide ring;
in some embodiments, the residues of any two amino acids in $Xa_2$, $Xa_3$, $Xa_4$, $Xa_5$, $Xa_7$, $Xa_8$, $Xa_9$, $Xa_{10}$, $Xa_{11}$, $Xa_{12}$ and $Xa_{13}$ are linked via $L_1$ to form a peptide ring;
in some embodiments, the residues of any three amino acids in $Xa_2$, $Xa_3$, $Xa_4$, $Xa_5$, $Xa_7$, $Xa_8$, $Xa_9$, $Xa_{10}$, $Xa_{11}$, $Xa_{12}$ and $Xa_{13}$ are linked via $L_1$ to form a peptide ring;
alternatively, a polypeptide is separated by covalent bonds formed between reactive groups $Xa_1$, $Xa_6$ and $Xa_{10}$ and a molecular scaffold to form a polypeptide loop containing at least two rings;
in some embodiments, the residues of 2-Nal and $Xa_{12}$ are linked via $L_1$ to form a peptide ring; and/or
the residues of $Xa_9$ and $Xa_{12}$ are linked via $L_1$ to form a peptide ring; and/or
the residues of Lys(Ac) and $Xa_7$ are linked via $L_1$ to form a peptide ring; and/or
the residues of $Xa_7$ and $Xa_{11}$ are linked via $L_1$ to form a peptide ring; and/or
the residues of 2-Nal and Glu are linked via $L_1$ to form a peptide ring; and/or

the residues of $Xa_1$ and Glu are linked via $L_1$ to form a peptide ring; and/or
the residues of $Xa_1$ and $Xa_{10}$ are linked via $L_1$ to form a peptide ring; and/or
the residues of $Xa_1$ and $Xa_{11}$ are linked via $L_1$ to form a peptide ring; and/or
the residues of 2-Nal and Sarc are linked via $L_1$ to form a peptide ring; and/or
the residues of $Xa_9$ and Sarc are linked via $L_1$ to form a peptide ring; and/or
the residues of $Xa_{11}$ and Sarc are linked via $L_1$ to form a peptide ring; and/or
the residues of $Xa_1$, Sarc and Glu are linked via $L_1$ to form a peptide ring;
in some embodiments, the residues of $Xa_1$ and Glu are linked via $L_1$ to form a peptide ring; the residues of $Xa_{11}$ and Sarc are linked via $L_1$ to form a peptide ring;
in some embodiments, the residues of Trp($R_1$) and $Xa_5$ are linked via $L_1$ to form a peptide ring;
$R_1$ is selected from H, $C_{1-4}$ alkyl, $C_{3-6}$ cycloalkyl or 4- to 8-membered heterocycloalkyl, wherein the alkyl, cycloalkyl or heterocycloalkyl is optionally further substituted with 1-4 substituents selected from halogen, =O, $C_{1-4}$ alkyl, halo$C_{1-4}$ alkyl, CN, OH and $NH_2$;
in some embodiments, $R_1$ is selected from $C_{1-2}$ alkyl, cyclopropyl, cyclobutyl, cyclopentyl or cyclohexyl, wherein the alkyl, cyclopropyl, cyclobutyl, cyclopentyl or cyclohexyl is optionally further substituted with 1-4 substituents selected from F, Cl, Br, =O, methyl, ethyl, $-CH_2CH_2F$, $-CH_2CHF_2$, $-CH_2CF_3$, $-CH_2F$, $-CHF_2$, $-CF_3$, CN, OH and $NH_2$;
in some embodiments, the residues of Trp($CH_3$) and Lys(Ac) are linked via $L_1$ to form a peptide ring;
in some embodiments, the residues of 2-Nal and $Xa_{12}$ are linked via $L_1$ to form a peptide ring;
in some embodiments, the residues of $Xa_9$ and $Xa_{12}$ are linked via $L_1$ to form a peptide ring;
in some embodiments, the residues of Lys(Ac) and $Xa_7$ are linked via $L_1$ to form a peptide ring;
in some embodiments, the residues of $Xa_7$ and $Xa_{11}$ are linked via $L_1$ to form a peptide ring;
in some embodiments, the residues of $Xa_1$ and Glu are linked via $L_1$ to form a peptide ring;
in some embodiments, the residues of $Xa_1$ and $Xa_{10}$ are linked via $L_1$ to form a peptide ring;
in some embodiments, the residues of $Xa_1$ and $Xa_{11}$ are linked via $L_1$ to form a peptide ring;
in some embodiments, the residues of 2-Nal and Sarc are linked via $L_1$ to form a peptide ring;
in some embodiments, the residues of $Xa_9$ and Sarc are linked via $L_1$ to form a peptide ring;
in some embodiments, the residues of $Xa_{11}$ and Sarc are linked via $L_1$ to form a peptide ring;
in some embodiments, the residues of $Xa_1$, Sarc and Glu are linked via $L_1$ to form a peptide ring;
in some embodiments, the residues of $Xa_1$ and $Xa_6$ react to form a peptide ring; and the residues of Trp($R_1$) and $Xa_5$ are directly condensed or are linked via $L_1$ to form a peptide ring;
in some embodiments, the residues of Trp($CH_3$) and Lys(Ac) are directly condensed or are linked via $L_1$ to form a peptide ring;
$L_1$ is $W_1$-$R^L$-$W_2$;
$R^L$ is selected from a bond, $C_{1-6}$ alkylene, $C_{2-4}$ alkenylene, $C_{2-4}$ alkynylene, 3-to 6-membered cycloalkyl, 4- to 6-membered heterocycloalkyl, 5- to 6-membered heteroaryl, 6- to 10-membered aryl, or $-(OCH_2CH_2)_a-$, wherein the alkylene, alkenylene, alkynylene, cycloalkyl, heterocycloalkyl, heteroaryl, or aryl is optionally further substituted with 1-4 $R^{L1}$;
in some embodiments, RL is selected from a bond, C1-6 alkylene, C2-4 alkenylene, C2-4 alkynylene, 3- to 6-membered cycloalkyl, 4- to 6-membered heterocycloalkyl, 5- to 6-membered heteroaryl, or 6- to 10-membered aryl, wherein the alkylene, alkenylene, alkynylene, cycloalkyl, heterocycloalkyl, heteroaryl, or aryl is optionally further substituted with 1-4 $R^{L1}$;
each $R^{L1}$ is independently selected from halogen, =O, $C_{1-4}$ alkyl, $C_{2-4}$ alkenyl, $C_{1-4}$ alkoxy, 3- to 6-membered cycloalkyl, COOH, $NH_2$, or $-NH-C(=O)-C_{1-4}$ alkyl, wherein the alkyl, alkoxy, or cycloalkyl is optionally further substituted with 1-4 substituents selected from halogen, CN, OH, and $NH_2$;
in some embodiments, each $R^{L1}$ is independently selected from halogen, =O, $C_{1-4}$ alkyl, $C_{2-4}$ alkenyl, $C_{1-4}$ alkoxy, 3- to 6-membered cycloalkyl, COOH, or $NH_2$, wherein the alkyl, alkoxy, or cycloalkyl is optionally further substituted with 1-4 substituents selected from halogen, CN, OH, and $NH_2$;
$W_1$ and $W_2$ are each independently selected from a bond, $C_{1-6}$ alkylene, -O-, - S-, $-NR^{W1}-$, $-CONR^{W1}-$, $-NR^{W1}CO-$, -C(=O)O-, or -OC(=O)-, wherein one or more $-CH_2-$ in the alkylene are optionally replaced by 1-4 groups selected from -O-, -S-, - $NR^{W1}$-, or -CO-, and the alkylene is optionally further substituted with 1-4 substituents selected from halogen, =O, $C_{1-4}$ alkyl, halo$C_{1-4}$ alkyl, CN, OH, and $NH_2$; and
$R^{W1}$ is selected from H, $C_{1-4}$ alkyl, or halogen;
in some embodiments, $L_1$ is selected from a bond, vinyl, propenyl, butenyl, $-O-(CH_2)_r-O-(CH_2)_r-NH-C(=O)-$, $-O-(CH_2)_r-O-(CH_2)_r-$, $-O-(CH_2)_r-O-(CH_2)_r-NH-$, - $C(=O)-(CH_2)_r-O-(CH_2)_r-O-(CH_2)_r-$, $-C(=O)-(CH_2)_r-O-(CH_2)_r-O-(CH_2)_r-NH-$, - $C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, $-(CH_2)_r-O-(CH_2)_r-$, $-O-(CH_2)_r-NH-$, $C_{1-6}$ alkylene, - C(=O)-, $-C(=O)-(CH_2)_r-NH-$,

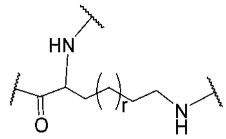, 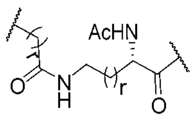

, $-(CH_2)_r-O-(CH_2)_r-NH-$, $-O-(CH_2)_r-O-(CH_2)_r-O-(CH_2)_r-O-(CH_2)_r-NH-$, $-(CH_2)_r-NH-$, $-C(=O)-(CH_2)_r-O-(CH_2)_r-NH-$, $-(CH_2)_r-NH-C(=O)-(CH_2)_r-$, or $-C(=O)-(CH_2)-(OCH_2CH_2)_a-NH-$;

in some embodiments, $L_1$ is selected from a bond, vinyl, propenyl, butenyl, $-O-(CH_2)_r-O-(CH_2)_r-NH-C(=O)-$, $-O-(CH_2)_r-O-(CH_2)_r-$, $-O-(CH_2)_r-O-(CH_2)_r-NH-$, - $C(=O)-(CH_2)_r-O-(CH_2)_r-O-(CH_2)_r-$, $-C(=O)-(CH_2)_r-O-(CH_2)_r-O-(CH_2)_r-NH-$, - $C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, -(CH2)r-O-(CH2)r-, $-O-(CH_2)_r-NH-$, $C_{1-2}$ alkylene, - C(=O)-, $-C(=O)-(CH_2)_r-NH-$,

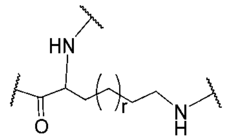, 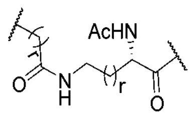

, $-(CH_2)_r-O-(CH_2)_r-NH-$, $-O-(CH_2)_r-O-(CH_2)_r-O-(CH_2)_r-O-(CH_2)_r-NH-$, $-(CH_2)_r-NH-$, or $-C(=O)-(CH_2)_r-O-(CH_2)_r-NH-$;

in some embodiments, $L_1$ is selected from a bond, vinyl, propenyl, butenyl, $-O-(CH_2)_r-O-(CH_2)_r-NH-C(=O)-$, $-O-(CH_2)_r-O-(CH_2)_r-$, $-O-(CH_2)_r-O-(CH_2)_r-NH-$, - $C(=O)-(CH_2)_r-O-(CH_2)_r-O-(CH_2)_r-$, $-C(=O)-(CH_2)_r-O-(CH_2)_r-O-(CH_2)_r-NH-$, - $C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, $-(CH_2)_r-O-(CH_2)_r-$, $-O-(CH_2)_r-NH-$, $C_{1-2}$ alkylene, - C(=O)-, $-C(=O)-(CH_2)_r-NH-$,

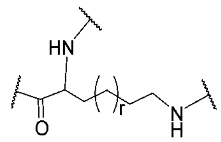, 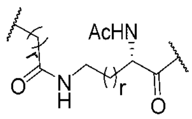

, $-(CH_2)_r-O-(CH_2)_r-NH-$, $-O-(CH_2)_r-O-(CH_2)_r-O-(CH_2)_r-O-(CH_2)_r-NH-$, or $-(CH_2)_r-NH-$;

in some embodiments, $L_1$ is selected from a bond, vinyl, propenyl, butenyl, $-O-(CH_2)_r-O-(CH_2)_r-NH-C(=O)-$, $-O-(CH_2)_r-O-(CH_2)_r-$, $-O-(CH_2)_r-O-(CH_2)_r-NH-$, - $C(=O)-(CH_2)_r-O-(CH_2)_r-O-(CH_2)_r-$, $-C(=O)-(CH_2)_r-O-(CH_2)_r-O-(CH_2)_r-NH-$, - $C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, $-(CH_2)_r-O-(CH_2)_r-$, $-O-(CH_2)_r-NH-$, $C_{1-2}$ alkylene, - C(=O)-, $-C(=O)-(CH_2)_r-NH-$,

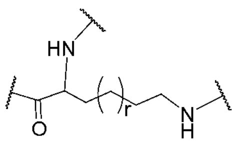, or 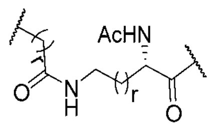;

in some embodiments, $L_1$ is selected from a bond, vinyl, propenyl, butenyl, $-O-(CH_2)_r-O-(CH_2)_r-NH-C(=O)-$, $-O-(CH_2)_r-O-(CH_2)_r-$, $-O-(CH_2)_r-O-(CH_2)_r-NH-$, - $C(=O)-(CH_2)_r-O-(CH_2)_r-O-(CH_2)_r-$, $-C(=O)-(CH_2)_r-O-(CH_2)_r-O-(CH_2)_r-NH-$, - $C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, $-(CH_2)_r-O-(CH_2)_r-$, $-O-(CH_2)_r-NH-$, $C_{1-2}$ alkylene, - $C(=O)-(CH_2)_r-NH-$,

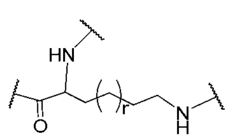, or 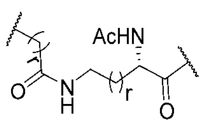;

in some embodiments, $L_1$ is selected from a bond, vinyl, propenyl, butenyl, $-O-(CH_2)_r-O-(CH_2)_r-NH-C(=O)-$,

$-O-(CH_2)_r-O-(CH_2)_r-$, $-O-(CH_2)_r-O-(CH_2)_r-NH-$, $-C(=O)-(CH_2)_r-O-(CH_2)_r-O-(CH_2)_r-$, $-C(=O)-(CH_2)_r-O-(CH_2)_r-O-(CH_2)_r-NH-$, $-C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, $-(CH_2)_r-O-(CH_2)_r-$, $-O-(CH_2)_r-NH-$, $C_{1-2}$ alkylene, $-C(=O)-(CH_2)_r-NH-$, or

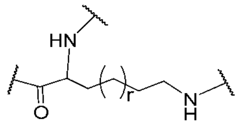;

r is selected from 0, 1, 2, 3, 4, 5, 6, 7, 8, 9, or 10;
in some embodiments, r is selected from 0, 1, 2, 3, 4 or 5;
in some embodiments, r is selected from 0, 1, 2 or 3;
a is any integer selected from 0-10;
in some embodiments, a is selected from 1, 2, 3, 4, 5 or 6;
in some embodiments, a is selected from 3, 4, 5 or 6;
and the peptide compound is optionally linked to a protecting group;
the protecting group is selected from Ac, glutaryl, succinyl, $NH_2$, or OH;
Unless otherwise specified in the present disclosure, the protecting groups Ac, glutaryl and succinyl are linked to the N-terminus of an amino acid; the protecting groups $NH_2$ and OH are linked to the C-terminus of an amino acid;
in some embodiments, at the N-terminus and the C-terminus, a protecting group is present or absent;
in some embodiments, a protecting group is present at the N-terminus and the C-terminus, and the protecting group is selected from Ac or $NH_2$;
in some embodiments, a protecting group is absent at the N-terminus and the C-terminus, provided that: (1) when $Xa_1$ at the N-terminus is linked to the residues of other amino acids via $L_1$, a protecting group at the N-terminus is absent; (2) when Sarc at the C-terminus is linked to the residues of other amino acids via $L_1$, a protecting group at the C-terminus is absent; alternatively, a polypeptide is separated by covalent bonds formed between reactive groups $Xa_1$, $Xa_6$ and $Xa_{10}$ and a molecular scaffold to form a polypeptide loop containing at least two rings;
in some embodiments, the molecular scaffold is selected from:

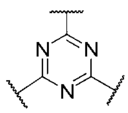,

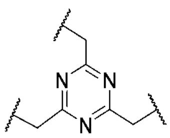, 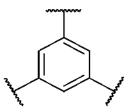, 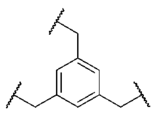, 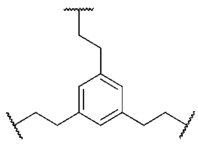, 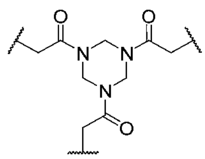,

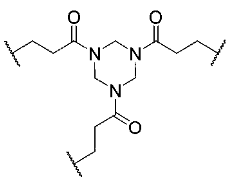, or 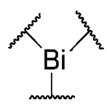;

in some embodiments, the molecular scaffold is selected from:

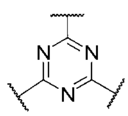,

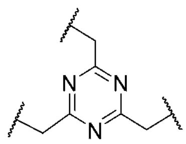, 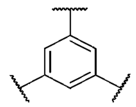, 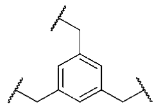, 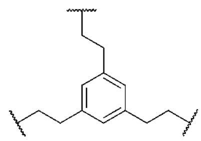, 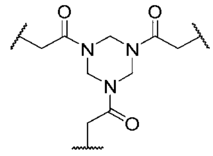

or

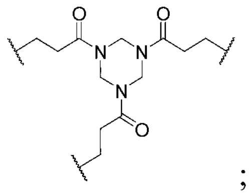;

provided that: the peptide compound is not of the following structure: (Ac)Pen-Asn-Thr-Trp($CH_3$)-Lys(Ac)-Pen-Phe [4-(2-aminoethoxy)]-[2-Nal]-Thp-Glu-Asn-[3-Pal]-Sarc($NH_2$), wherein a disulphide bond is formed between Pen and Pen.

As a more specific first technical solution of the present disclosure, the present disclosure provides a cyclic peptide compound, or a stereoisomer, a pharmaceutically acceptable salt, a solvate or a dimer thereof, wherein the peptide compound has an amino acid sequence of formula (I):

$$Xa_1\text{-}Xa_2\text{-}Xa_3\text{-}Xa_4\text{-}Xa_5\text{-}Xa_6\text{-}Xa_7\text{-}Xa_8\text{-}Xa_9\text{-}Xa_{10}\text{-}Xa_{11}\text{-}Xa_{12}\text{-}Xa_{13} \quad (I)$$

in which $Xa_1$ and $Xa_6$ are each independently selected from Pen, Pcn, Asn, Ala, Ala(3-amino), Ala(2-ethyne), Ala(3-azido), Ala(2-ethene), Val(2-ethene), Asp, 2,4-diaminobutyric acid, Ser, Cys, Hcys, or Glu, and the residues of $Xa_1$ and $Xa_6$ react to form a peptide ring or form a cyclic peptide via $L_1$; in some embodiments, $Xa_1$ and $Xa_6$ are each independently selected from Pen, Asn, Ala, Ala(3-amino), Ala(2-ethyne), Ala(3-azido), Ala(2-ethene), Val(2-ethene), Asp, 2,4-diaminobutyric acid, Ser, Cys, Hcys, or Glu, and the residues of $Xa_1$ and $Xa_6$ react to form a peptide ring;
$Xa_2$ is selected from Asn, His, or an analogue of Asn and His;
$Xa_3$ is selected from Thr or an analogue of Thr;
$Xa_4$ is selected from Trp or an analogue of Trp;
$Xa_5$ is selected from Lys, Gln, Arg, Cit, or an analogue of Lys, Gln, Cit, and Arg;
$Xa_7$ is selected from Phe or an analogue of Phe;
$Xa_8$ is selected from Phe, Trp, 2-Nal, or an analogue of Phe, Trp, and 2-Nal;
$Xa_9$ is selected from Thp or an analogue of Thp;
$Xa_{10}$ is selected from Glu, Cys, or an analogue of Glu and Cys;
$Xa_{11}$ is selected from Asn, Lys, or an analogue of Asn and Lys;
$Xa_{12}$ is selected from 3-Pal, Phe, Asp, or an analogue of 3-Pal, Phe, and Asp;
$Xa_{13}$ is selected from Sarc or an analogue of Sarc;
alternatively, the residues of any amino acid in $Xa_1$, $Xa_2$, $Xa_3$, $Xa_4$, $Xa_5$, $Xa_7$, $Xa_8$, $Xa_9$, $Xa_{10}$, $Xa_{11}$, $Xa_{12}$, and $Xa_{13}$ are directly condensed or are linked via $L_1$ to form one or more peptide rings;
$L_1$ is $W_1$-$R^L$-$W_2$;
$R^L$ is selected from a bond, $C_{1-6}$ alkylene, $C_{2-4}$ alkenylene, $C_{2-4}$ alkynylene, 3-to 6-membered cycloalkyl, 4- to 6-membered heterocycloalkyl, 5- to 6-membered heteroaryl, 6- to 10-membered aryl, or -($OCH_2CH_2$)$_a$-, wherein the alkylene, alkenylene, alkynylene, cycloalkyl, heterocycloalkyl, heteroaryl, or aryl is optionally further substituted with 1-4 $R^{L1}$; in some embodiments, RL is selected from a bond, C1-6 alkylene, C2-4 alkenylene, C2-4 alkynylene, 3- to 6-membered cycloalkyl, 4- to 6-membered heterocycloalkyl, 5- to 6-membered heteroaryl, or 6-to 10-membered aryl, wherein the alkylene, alkenylene, alkynylene, cycloalkyl, heterocycloalkyl, heteroaryl, or aryl is optionally further substituted with 1-4 $R^{L1}$;
a is any integer selected from 0-10;
each $R^{L1}$ is independently selected from halogen, =O, $C_{1-4}$ alkyl, $C_{2-4}$ alkenyl, $C_{1-4}$ alkoxy, 3- to 6-membered cycloalkyl, COOH, $NH_2$, or -NH-C(=O)-$C_{1-4}$ alkyl, wherein the alkyl, alkoxy, or cycloalkyl is optionally further substituted with 1-4 substituents selected from halogen, CN, OH, and $NH_2$;
$W_1$ and $W_2$ are each independently selected from a bond, $C_{1-6}$ alkylene, -O-, - S-, -$NR^{W1}$- , -$CONR^{W1}$-, -$NR^{W1}$CO-, -C(=O)O-, or -OC(=O)-, wherein one or more -$CH_2$- in the alkylene are optionally replaced by 1-4 groups selected from

-O-, -S-, - $NR^{W1}$-, or -CO-, and the alkylene is optionally further substituted with 1-4 substituents selected from halogen, =O, $C_{1-4}$ alkyl, halo$C_{1-4}$ alkyl, CN, OH, and $NH_2$; and

$R^{W1}$ is selected from H, $C_{1-4}$ alkyl, or halogen;

and the peptide compound is optionally linked to a protecting group;

the protecting group is selected from Ac, glutaryl, succinyl, $NH_2$, or OH;

alternatively, a polypeptide is separated by covalent bonds formed between reactive groups $Xa_1$, $Xa_6$ and $Xa_{10}$ and a molecular scaffold to form a polypeptide loop containing at least two rings;

alternatively, the peptide compound is optionally conjugated to a modifying group at $Xa_1$ or Xas; or alternatively, the peptide compound is optionally conjugated to a modifying group at $Xa_1$, $Xa_5$, or $Xa_7$;

provided that: the peptide compound is not of the following structure: (Ac)Pen-Asn-Thr-Trp($CH_3$)-Lys(Ac)-Pen-Phe [4-(2-aminoethoxy)]-[2-Nal]-Thp-Glu-Asn-[3-Pal]-Sarc($NH_2$), wherein a disulphide bond is formed between Pen and Pen.

Further, the peptide compound has an amino acid sequence of formula (I):

$$Xa_1\text{-}Xa_2\text{-}Xa_3\text{-}Xa_4\text{-}Xa_5\text{-}Xa_6\text{-}Xa_7\text{-}Xa_8\text{-}Xa_9\text{-}Xa_{10}\text{-}Xa_{11}\text{-}Xa_{12}\text{-}Xa_{13} \quad (I)$$

wherein $Xa_1$ and $Xa_6$ are each independently selected from Pen, Asn, Ala, Ala(3-amino), Ala(2-ethyne), Ala(3-azido), Ala(2-ethene), Val(2-ethene), Asp, 2,4-diaminobutyric acid, Ser, Cys, Hcys, or Glu, and the residues of $Xa_1$ and $Xa_6$ react to form a peptide ring;

$Xa_2$ is selected from Asn, His, or an analogue of Asn and His;

$Xa_3$ is selected from Thr or an analogue of Thr;

$Xa_4$ is selected from Trp or an analogue of Trp;

$Xa_5$ is selected from Lys, Gln, Arg, Cit, or an analogue of Lys, Gln, Cit, and Arg; in some embodiments, $Xa_5$ is selected from Lys, Gln, Arg, or an analogue of Lys, Gln and Arg;

$Xa_7$ is selected from Phe or an analogue of Phe;

$Xa_8$ is selected from Phe, Trp, 2-Nal, or an analogue of Phe, Trp and 2-Nal;

$Xa_9$ is selected from Thp or an analogue of Thp;

$Xa_{10}$ is selected from Glu, Cys, or an analogue of Glu and Cys;

$Xa_{11}$ is selected from Asn, Lys, or an analogue of Asn and Lys;

$Xa_{12}$ is selected from 3-Pal, Phe, Asp, or an analogue of 3-Pal, Phe, and Asp;

$Xa_{13}$ is selected from Sarc or an analogue of Sarc;

alternatively, the residues of any amino acid in $Xa_1$, $Xa_2$, $Xa_3$, $Xa_4$, $Xa_5$, $Xa_7$, $Xa_8$, $Xa_9$, $Xa_{10}$, $Xa_{11}$, $Xa_{12}$, and $Xa_{13}$ are directly condensed or are linked via $L_1$ to form one or more peptide rings; in some embodiments, the residues of any amino acid in $Xa_1$, $Xa_2$, $Xa_3$, $Xa_4$, $Xa_5$, $Xa_7$, $Xa_8$, $Xa_9$, $Xa_{10}$, $Xa_{11}$, $Xa_{12}$ and $Xa_{13}$ are linked via $L_1$ to form one or more peptide rings;

$L_1$ is $W_1$-$R^L$-$W_2$;

$R^L$ is selected from a bond, $C_{1-6}$ alkylene, $C_{2-4}$ alkenylene, $C_{2-4}$ alkynylene, 3-to 6-membered cycloalkyl, 4- to 6-membered heterocycloalkyl, 5- to 6-membered heteroaryl, or 6- to 10-membered aryl, wherein the alkylene, alkenylene, alkynylene, cycloalkyl, heterocycloalkyl, heteroaryl, or aryl is optionally further substituted with 1-4 $R^{L1}$;

each $R^{L1}$ is independently selected from halogen, =O, $C_{1-4}$ alkyl, $C_{2-4}$ alkenyl, $C_{1-4}$ alkoxy, 3- to 6-membered cycloalkyl, COOH, $NH_2$, or -NH-C(=O)-$C_{1-4}$ alkyl, wherein the alkyl, alkoxy, or cycloalkyl is optionally further substituted with 1-4 substituents selected from halogen, CN, OH, and $NH_2$;

$W_1$ and $W_2$ are each independently selected from a bond, $C_{1-6}$ alkylene, -O-, - S-, -$NR^{W1}$-, -CON$R^{W1}$-, -$NR^{W1}$CO-, -C(=O)O-, or -OC(=O)-, wherein one or more -$CH_2$- in the alkylene are optionally replaced by 1-4 groups selected from -O-, -S-, - $NR^{W1}$-, or -CO-, and the alkylene is optionally further substituted with 1-4 substituents selected from halogen, =O, $C_{1-4}$ alkyl, halo$C_{1-4}$ alkyl, CN, OH, and $NH_2$; and

$R^{W1}$ is selected from H, $C_{1-4}$ alkyl, or halogen;

and the peptide compound is optionally linked to a protecting group;

the protecting group is selected from Ac, glutaryl, succinyl, $NH_2$, or OH;

alternatively, a polypeptide is separated by covalent bonds formed between reactive groups $Xa_1$, $Xa_6$ and $Xa_{10}$ and a molecular scaffold to form a polypeptide loop containing at least two rings;

provided that: the peptide compound is not of the following structure: (Ac)Pen-Asn-Thr-Trp($CH_3$)-Lys(Ac)-Pen-Phe [4-(2-aminoethoxy)]-[2-Nal]-Thp-Glu-Asn-[3-Pal]-Sarc($NH_2$), wherein a disulphide bond is formed between Pen and Pen.

Further, the peptide compound has an amino acid sequence of formula (I):

$$Xa_1\text{-}Xa_2\text{-}Xa_3\text{-}Xa_4\text{-}Xa_5\text{-}Xa_6\text{-}Xa_7\text{-}Xa_8\text{-}Xa_9\text{-}Xa_{10}\text{-}Xa_{11}\text{-}Xa_{12}\text{-}Xa_{13} \quad (I)$$

wherein $Xa_1$ and $Xa_6$ are each independently selected from Pen, Asn, Ala, Ala(3-amino), Ala(2-ethyne), Ala(3-azido), Ala(2-ethene), Val(2-ethene), Asp, 2,4-diaminobutyric acid, Ser, Cys, Hcys, or Glu, and the residues of $Xa_1$ and $Xa_6$ react to form a peptide ring;
$Xa_2$ is selected from Asn, His, or an analogue of Asn and His;
$Xa_3$ is selected from Thr or an analogue of Thr;
$Xa_4$ is selected from Trp or an analogue of Trp;
$Xa_5$ is selected from Lys, Gln, Arg, or an analogue of Lys, Gln, and Arg;
$Xa_7$ is selected from Phe or an analogue of Phe;
$Xa_8$ is selected from Phe, Trp, 2-Nal, or an analogue of Phe, Trp, and 2-Nal, and
$Xa_9$ is selected from Thp or an analogue of Thp;
$Xa_{10}$ is selected from Glu, Cys, or an analogue of Glu and Cys;
$Xa_{11}$ is selected from Asn, Lys, or an analogue of Asn and Lys;
$Xa_{12}$ is selected from 3-Pal, Phe, Asp, or an analogue of 3-Pal, Phe, and Asp;
$Xa_{13}$ is selected from Sarc or an analogue of Sarc;
alternatively, the residues of any amino acid in $Xa_1$, $Xa_2$, $Xa_3$, $Xa_4$, $Xa_5$, $Xa_7$, $Xa_8$, $Xa_9$, $Xa_{10}$, $Xa_{11}$, $Xa_{12}$, and $Xa_{13}$ are linked via $L_1$ to form one or more peptide rings;
$L_1$ is $W_1$-$R^L$-$W_2$;
$R^L$ is selected from a bond, $C_{1-6}$ alkylene, $C_{2-4}$ alkenylene, $C_{2-4}$ alkynylene, 3-to 6-membered cycloalkyl, 4- to 6-membered heterocycloalkyl, 5- to 6-membered heteroaryl, or 6- to 10-membered aryl, wherein the alkylene, alkenylene, alkynylene, cycloalkyl, heterocycloalkyl, heteroaryl, or aryl is optionally further substituted with 1-4 $R^{L1}$;
each $R^{L1}$ is independently selected from halogen, =O, $C_{1-4}$ alkyl, $C_{2-4}$ alkenyl, $C_{1-4}$ alkoxy, 3- to 6-membered cycloalkyl, COOH, $NH_2$, or -NH-C(=O)-$C_{1-4}$ alkyl, wherein the alkyl, alkoxy, or cycloalkyl is optionally further substituted with 1-4 substituents selected from halogen, CN, OH, and $NH_2$;
$W_1$ and $W_2$ are each independently selected from a bond, $C_{1-6}$ alkylene, -O-, - S-, -$NR^{W1}$-, -$CONR^{W1}$-, -$NR^{W1}CO$-, -C(=O)O-, or -OC(=O)-, wherein one or more -$CH_2$- in the alkylene are optionally replaced by 1-4 groups selected from -O-, -S-, - $NR^{W1}$-, or -CO-, and the alkylene is optionally further substituted with 1-4 substituents selected from halogen, =O, $C_{1-4}$ alkyl, halo$C_{1-4}$ alkyl, CN, OH, and $NH_2$; and
$R^{W1}$ is selected from H, $C_{1-4}$ alkyl, or halogen;
and the peptide compound is optionally linked to a protecting group;
the protecting group is selected from Ac, glutaryl, succinyl, $NH_2$, or OH;
provided that: the peptide compound is not of the following structure: (Ac)Pen-Asn-Thr-Trp($CH_3$)-Lys(Ac)-Pen-Phe [4-(2-aminoethoxy)]-[2-Nal]-Thp-Glu-Asn-[3-Pal]-Sarc($NH_2$), wherein a disulphide bond is formed between Pen and Pen.

Further, the peptide compound has an amino acid sequence of formula (I):

$$Xa_1\text{-}Xa_2\text{-}Xa_3\text{-}Xa_4\text{-}Xa_5\text{-}Xa_6\text{-}Xa_7\text{-}Xa_8\text{-}Xa_9\text{-}Xa_{10}\text{-}Xa_{11}\text{-}Xa_{12}\text{-}Xa_{13} \quad (I)$$

in which $Xa_1$ and $Xa_6$ are each independently selected from Pen, Asn, Ala, Ala(3-amino), Ala(2-ethyne), Ala(3-azido), Ala(2-ethene), or Val(2-ethene), and the residues of $Xa_1$ and $Xa_6$ react to form a peptide ring;
$Xa_2$ is selected from Asn, His, or an analogue of Asn and His;
$Xa_3$ is selected from Thr or an analogue of Thr;
$Xa_4$ is selected from Trp or an analogue of Trp;
$Xa_5$ is selected from Lys or an analogue of Lys;
$Xa_7$ is selected from Phe or an analogue of Phe;
$Xa_8$ is selected from Phe, Trp, 2-Nal, or an analogue of Phe, Trp, and 2-Nal, and
$Xa_9$ is selected from Thp or an analogue of Thp;
$Xa_{10}$ is selected from Glu or an analogue of Glu;
$Xa_{11}$ is selected from Asn, Lys, or an analogue of Asn and Lys;
$Xa_{12}$ is selected from 3-Pal, Phe, Asp, or an analogue of 3-Pal, Phe, and Asp;
$Xa_{13}$ is selected from Sarc or an analogue of Sarc;
alternatively, the residues of any amino acid in $Xa_2$, $Xa_3$, $Xa_4$, $Xa_5$, $Xa_7$, $Xa_8$, $Xa_9$, $Xa_{10}$, $Xa_{11}$, $Xa_{12}$, and $Xa_{13}$ are linked via $L_1$ to form a peptide ring;
$L_1$ is $W_1$-$R^L$-$W_2$;
$R^L$ is selected from a bond, $C_{1-6}$ alkylene, $C_{2-4}$ alkenylene, $C_{2-4}$ alkynylene, 3-to 6-membered cycloalkyl, 4- to 6-membered heterocycloalkyl, 5- to 6-membered heteroaryl, or 6- to 10-membered aryl, wherein the alkylene,

alkenylene, alkynylene, cycloalkyl, heterocycloalkyl, heteroaryl, or aryl is optionally further substituted with 1-4 $R^{L1}$;
each $R^{L1}$ is independently selected from halogen, =O, $C_{1-4}$ alkyl, $C_{2-4}$ alkenyl, $C_{1-4}$ alkoxy, 3- to 6-membered cycloalkyl, COOH, or $NH_2$, wherein the alkyl, alkoxy, or cycloalkyl is optionally further substituted with 1-4 substituents selected from halogen, CN, OH, and $NH_2$;
$W_1$ and $W_2$ are each independently selected from a bond, $C_{1-6}$ alkylene, -O-, - S-, -$NR^{W1}$-, -$CONR^{W1}$-, -$NR^{W1}CO$-, -C(=O)O-, or -OC(=O)-, wherein one or more -$CH_2$- in the alkylene are optionally replaced by 1-4 groups selected from -O-, -S-, - $NR^{W1}$-, or -CO-, and the alkylene is optionally further substituted with 1-4 substituents selected from halogen, =O, $C_{1-4}$ alkyl, halo$C_{1-4}$ alkyl, CN, OH, and $NH_2$; and
$R^{W1}$ is selected from H, $C_{1-4}$ alkyl, or halogen;
and the peptide compound is optionally linked to a protecting group;
the protecting group is selected from Ac, glutaryl, succinyl, $NH_2$, or OH;
provided that: the peptide compound is not of the following structure: (Ac)Pen-Asn-Thr-Trp($CH_3$)-Lys(Ac)-Pen-Phe [4-(2-aminoethoxy)]-[2-Nal]-Thp-Glu-Asn-[3-Pal]-Sarc($NH_2$), wherein a disulphide bond is formed between Pen and Pen.

As a more specific second technical solution of the present disclosure, provided is the peptide compound, or the stereoisomer, the pharmaceutically acceptable salt, the solvate or the dimer thereof, wherein the peptide compound has an amino acid sequence of formula (II) or formula (II-1):

(Ac)$Xa_1$-$Xa_2$-Thr-$Xa_4$-$Xa_5$-$Xa_6$-$Xa_7$-$Xa_8$-$Xa_9$-$Xa_{10}$-Asn-(3-Pal)-Sarc($NH_2$) (II)

(Ac)$Xa_1$-$Xa_2$-Thr-$Xa_4$-Lys(Ac)-$Xa_6$-$Xa_7$-$Xa_8$-$Xa_9$-Glu-Asn-(3-Pal)-Sarc($NH_2$) (II-1)

wherein the residues of $Xa_1$ and $Xa_6$ react to form a peptide ring; or the residues of $Xa_1$ and $Xa_6$ are linked via $L_1$ to form a peptide ring;
other groups are as defined in any of the preceding technical solutions.

Further, the peptide compound has an amino acid sequence of formula (II-1):

(Ac)$Xa_1$-$Xa_2$-Thr-$Xa_4$-Lys(Ac)-$Xa_6$-$Xa_7$-$Xa_8$-$Xa_9$-Glu-Asn-(3-Pal)-Sarc($NH_2$) (II-1)

wherein the residues of $Xa_1$ and $Xa_6$ react to form a peptide ring;
other groups are as defined in any of the preceding technical solutions.

As a more specific third technical solution of the present disclosure, provided is the peptide compound, or the stereoisomer, the pharmaceutically acceptable salt, the solvate or the dimer thereof, wherein the peptide compound has an amino acid sequence of formula (III) or formula (III-1):

(Ac)$Xa_1$-$Xa_2$-Thr-Trp($R_1$)-$Xa_3$-$Xa_6$-$Xa_7$-$Xa_8$-$Xa_9$-$Xa_{10}$-Asn-(3-Pal)-Sarc($NH_2$) (III)

(Ac)$Xa_1$-$Xa_2$-Thr-Trp($CH_3$)-Lys(Ac)-$Xa_6$-$Xa_7$-$Xa_8$-$Xa_9$-Glu-Asn-(3-Pal)-Sarc($NH_2$) (III-1)

wherein the residues of $Xa_1$ and $Xa_6$ react to form a peptide ring; or the residues of $Xa_1$ and $Xa_6$ are linked via $L_1$ to form a peptide ring;
and the residues of Trp($R_1$) and $Xa_5$ are directly condensed or are linked via $L_1$ to form a peptide ring;
or the residues of Trp($CH_3$) and Lys(Ac) are directly condensed or are linked via $L_1$ to form a peptide ring;
or $Xa_1$, $Xa_6$ and $Xa_{10}$ and the molecular scaffold form a bicyclic peptide;
$R_1$ is selected from H, $C_{1-4}$ alkyl, $C_{3-6}$ cycloalkyl or 4- to 8-membered heterocycloalkyl, wherein the alkyl, cycloalkyl or heterocycloalkyl is optionally further substituted with 1-4 substituents selected from halogen, =O, $C_{1-4}$ alkyl, halo$C_{1-4}$ alkyl, CN, OH and $NH_2$;

Further, the peptide compound has an amino acid sequence of formula (III) or formula (III-1):

(Ac)$Xa_1$-$Xa_2$-Thr-Trp($R_1$)-$Xa_5$-$Xa_6$-$Xa_7$-$Xa_8$-$Xa_9$-$Xa_{10}$-Asn-(3-Pal)-Sarc($NH_2$) (III)

(Ac)$Xa_1$-$Xa_2$-Thr-Trp($CH_3$)-Lys(Ac)-$Xa_6$-$Xa_7$-$Xa_8$-$Xa_9$-Glu-Asn-(3-Pal)-Sarc($NH_2$) (III-1)

wherein the residues of $Xa_1$ and $Xa_6$ react to form a peptide ring;

and the residues of $Trp(R_1)$ and $Xa_5$ are linked via $L_1$ to form a peptide ring;
or the residues of $Trp(CH_3)$ and Lys(Ac) are linked via $L_1$ to form a peptide ring;
or $Xa_1$, $Xa_6$ and $Xa_{10}$ and the molecular scaffold form a bicyclic peptide;
$R_1$ is selected from H, $C_{1-4}$ alkyl, $C_{3-6}$ cycloalkyl or 4- to 8-membered heterocycloalkyl, wherein the alkyl, cycloalkyl or heterocycloalkyl is optionally further substituted with 1-4 substituents selected from halogen, =O, $C_{1-4}$ alkyl, halo$C_{1-4}$ alkyl, CN, OH and $NH_2$;
other groups are as defined in any of the preceding technical solutions.

Further, the peptide compound has an amino acid sequence of formula (III-1):

(Ac)$Xa_1$-$Xa_2$-Thr-$Trp(R_1)$-$Xa_5$-$Xa_6$-$Xa_7$-$Xa_8$-$Xa_9$-$Xa_{10}$-Asn-(3-Pal)-Sarc($NH_2$) (III)

(Ac)$Xa_1$-$Xa_2$-Thr-$Trp(CH_3)$-Lys(Ac)-$Xa_6$-$Xa_7$-$Xa_8$-$Xa_9$-Glu-Asn-(3-Pal)-Sarc($NH_2$) (III-1)

wherein the residues of $Xa_1$ and $Xa_6$ react to form a peptide ring;
and the residues of $Trp(R_1)$ and $Xa_5$ are linked via $L_1$ to form a peptide ring;
or the residues of $Trp(CH_3)$ and Lys(Ac) are linked via $L_1$ to form a peptide ring;
$R_1$ is selected from H, $C_{1-4}$ alkyl, $C_{3-6}$ cycloalkyl or 4- to 8-membered heterocycloalkyl, wherein the alkyl, cycloalkyl or heterocycloalkyl is optionally further substituted with 1-4 substituents selected from halogen, =O, $C_{1-4}$ alkyl, halo$C_{1-4}$ alkyl, CN, OH and $NH_2$;
other groups are as defined in any of the preceding technical solutions.

Further, the peptide compound has an amino acid sequence of formula (III-1):

(Ac)$Xa_1$-$Xa_2$-Thr-$Trp(CH_3)$-Lys(Ac)-$Xa_6$-$Xa_7$-$Xa_8$-$Xa_9$-Glu-Asn-(3-Pal)-Sarc($NH_2$) (III-1)

wherein the residues of $Xa_1$ and $Xa_6$ react to form a peptide ring;
and the residues of $Trp(CH_3)$ and Lys(Ac) are linked via $L_1$ to form a peptide ring;
other groups are as defined in any of the preceding technical solutions.

As a more specific fourth technical solution of the present disclosure, provided is the peptide compound, or the stereoisomer, the pharmaceutically acceptable salt, the solvate or the dimer thereof, wherein the peptide compound has an amino acid sequence of formula (IV), formula (IV-1) or formula (V):

(N-tenninus)$Xa_1$-Asn-Thr-$Trp(R_1)$-$Xa_5$-$Xa_6$-$Xa_7$-(2-Nal)-$Xa_9$-$Xa_{10}$-$Xa_{11}$-$Xa_{12}$-Sarc(C-terminus) (IV)

(N-terminus)$Xa_1$-Asn-Thr-$Trp(CH_3)$-Lys(Ac)-$Xa_6$-$Xa_7$-(2-Nal)-$Xa_9$-Glu-$Xa_{11}$-$Xa_{12}$-Sarc(C-terminus) (IV-1)

or

(N-terminus)$Xa_1$-His-Thr-$Trp(R_1)$-$Xa_5$-$Xa_6$-$Xa_7$-(2-Nal)-$Xa_9$-$Xa_{10}$-$Xa_{11}$-$Xa_{12}$-Sarc(C-terminus) (V)

wherein the residues of $Xa_1$ and $Xa_6$ react to form a peptide ring; or the residues of $Xa_1$ and $Xa_6$ are linked via $L_1$ to form a peptide ring;
at the N-terminus and the C-terminus, a protecting group is present or absent;
or the N-terminus is conjugated to a modifying group;
the residues of 2-Nal and $Xa_{12}$ are linked via $L_1$ to form a peptide ring; and/or
the residues of $Xa_9$ and $Xa_{12}$ are linked via $L_1$ to form a peptide ring; and/or
the residues of Lys(Ac) and $Xa_7$ are linked via $L_1$ to form a peptide ring; and/or
the residues of $Xa_7$ and $Xa_{11}$ are linked via $L_1$ to form a peptide ring; and/or
the residues of 2-Nal and Glu are linked via $L_1$ to form a peptide ring; and/or
the residues of $Xa_1$ and Glu are linked via $L_1$ to form a peptide ring; and/or
the residues of $Xa_1$ and $Xa_{11}$ are linked via $L_1$ to form a peptide ring; and/or
the residues of $Xa_1$ and $Xa_{10}$ are linked via $L_1$ to form a peptide ring; and/or
the residues of 2-Nal and Sarc are linked via $L_1$ to form a peptide ring; and/or

the residues of $Xa_9$ and Sarc are linked via $L_1$ to form a peptide ring; and/or
the residues of $Xa_{11}$ and Sarc are linked via $L_1$ to form a peptide ring; and/or
the residues of $Xa_1$, Sarc and Glu are linked via $L_1$ to form a peptide ring; and/or
or $Xa_1$, $Xa_6$ and $Xa_{10}$ and the molecular scaffold form a bicyclic peptide;
$R_1$ is selected from $C_{1-2}$ alkyl, cyclopropyl, cyclobutyl, cyclopentyl or cyclohexyl, wherein the alkyl, cyclopropyl, cyclobutyl, cyclopentyl or cyclohexyl is optionally further substituted with 1-4 substituents selected from F, Cl, Br, =O, methyl, ethyl, $-CH_2CH_2F$, $-CH_2CHF_2$, $-CH_2CF_3$, $-CH_2F$, $-CHF_2$, $-CF_3$, CN, OH and $NH_2$;
provided that: (1) when $Xa_1$ at the N-terminus is linked to the residues of other amino acids via $L_1$, a protecting group at the N-terminus is absent;
(2) when Sarc at the C-terminus is linked to the residues of other amino acids via $L_1$, a protecting group at the C-terminus is absent;
other groups are as defined in any of the preceding technical solutions.

Further, the peptide compound has an amino acid sequence of formula (IV) or formula (IV-1):

(N-terminus)$Xa_1$-Asn-Thr-Trp($R_1$)-$Xa_5$-$Xa_6$-$Xa_7$-(2-Nal)-$Xa_9$-$Xa_{10}$-$Xa_{11}$-$Xa_{12}$-Sarc(C-terminus) (IV)

(N-terminus)$Xa_1$-Asn-Thr-Trp($CH_3$)-Lys(Ac)-$Xa_6$-$Xa_7$-(2-Nal)-$Xa_9$-Glu-$Xa_{11}$-$Xa_{12}$-Sarc(C-terminus) (IV-1), wherein the residues of $Xa_1$ and $Xa_6$ react to form a peptide ring;
at the N-terminus and the C-terminus, a protecting group is present or absent; the residues of 2-Nal and $Xa_{12}$ are linked via $L_1$ to form a peptide ring; and/or
the residues of $Xa_9$ and $Xa_{12}$ are linked via $L_1$ to form a peptide ring; and/or
the residues of Lys(Ac) and $Xa_7$ are linked via $L_1$ to form a peptide ring; and/or
the residues of $Xa_7$ and $Xa_{11}$ are linked via $L_1$ to form a peptide ring; and/or
the residues of 2-Nal and Glu are linked via $L_1$ to form a peptide ring; and/or
the residues of $Xa_1$ and Glu are linked via $L_1$ to form a peptide ring; and/or
the residues of $Xa_1$ and $Xa_{11}$ are linked via $L_1$ to form a peptide ring; and/or
the residues of $Xa_1$ and $Xa_{10}$ are linked via $L_1$ to form a peptide ring; and/or
the residues of 2-Nal and Sarc are linked via $L_1$ to form a peptide ring; and/or
the residues of $Xa_9$ and Sarc are linked via $L_1$ to form a peptide ring; and/or
the residues of $Xa_{11}$ and Sarc are linked via $L_1$ to form a peptide ring; and/or
the residues of $Xa_1$, Sarc and Glu are linked via $L_1$ to form a peptide ring;
$R_1$ is selected from $C_{1-2}$ alkyl, cyclopropyl, cyclobutyl, cyclopentyl or cyclohexyl, wherein the alkyl, cyclopropyl, cyclobutyl, cyclopentyl or cyclohexyl is optionally further substituted with 1-4 substituents selected from F, Cl, Br, =O, methyl, ethyl, $-CH_2CH_2F$, $-CH_2CHF_2$, $-CH_2CF_3$, $-CH_2F$, $-CHF_2$, $-CF_3$, CN, OH and $NH_2$;
provided that: (1) when $Xa_1$ at the N-terminus is linked to the residues of other amino acids via $L_1$, a protecting group at the N-terminus is absent;
(2) when Sarc at the C-terminus is linked to the residues of other amino acids via $L_1$, a protecting group at the C-terminus is absent;
other groups are as defined in any of the preceding technical solutions.

Further, the peptide compound has an amino acid sequence of formula (IV-1):

(N-terminus)$Xa_1$-Asn-Thr-Trp($CH_3$)-Lys(Ac)-$Xa_6$-$Xa_7$-(2-Nal)-$Xa_9$-Glu-$Xa_{11}$-$Xa_{12}$-Sarc(C-terminus) (IV-1)

wherein the residues of $Xa_1$ and $Xa_6$ react to form a peptide ring;
at the N-terminus and the C-terminus, a protecting group is present or absent;
the residues of 2-Nal and $Xa_{12}$ are linked via $L_1$ to form a peptide ring; and/or
the residues of $Xa_9$ and $Xa_{12}$ are linked via $L_1$ to form a peptide ring; and/or
the residues of Lys(Ac) and $Xa_7$ are linked via $L_1$ to form a peptide ring; and/or
the residues of $Xa_7$ and $Xa_{11}$ are linked via $L_1$ to form a peptide ring; and/or
the residues of 2-Nal and Glu are linked via $L_1$ to form a peptide ring; and/or
the residues of $Xa_1$ and Glu are linked via $L_1$ to form a peptide ring; and/or
the residues of $Xa_1$ and $Xa_{11}$ are linked via $L_1$ to form a peptide ring; and/or
the residues of 2-Nal and Sarc are linked via $L_1$ to form a peptide ring; and/or
the residues of $Xa_9$ and Sarc are linked via $L_1$ to form a peptide ring; and/or

the residues of $Xa_{11}$ and Sarc are linked via $L_1$ to form a peptide ring; and/or
the residues of $Xa_1$, Sarc and Glu are linked via $L_1$ to form a peptide ring;
provided that: (1) when $Xa_1$ at the N-terminus is linked to the residues of other amino acids via $L_1$, a protecting group at the N-terminus is absent;
(2) when Sarc at the C-terminus is linked to the residues of other amino acids via $L_1$, a protecting group at the C-terminus is absent;
other groups are as defined in any of the preceding technical solutions. As a more specific fifth technical solution of the present disclosure, provided is the peptide compound, or the stereoisomer, the pharmaceutically acceptable salt, the solvate or the dimer thereof, wherein
the residues of $Xa_1$ and $Xa_6$ react to form a peptide ring or form a cyclic peptide via $L_1$; and the residues of one or two groups of $Xa_4$ and $Xa_5$, $Xa_1$ and $Xa_{10}$, $Xa_8$ and $Xa_{10}$, $Xa_8$ and $Xa_{12}$, $Xa_8$ and $Xa_{13}$, $Xa_9$ and $Xa_{13}$, $Xa_5$ and $Xa_7$, $Xa_{11}$ and $Xa_{13}$, $Xa_1$ and $Xa_{11}$, $Xa_7$ and $Xa_{11}$ are directly condensed or are linked via $L_1$ to form a peptide ring;
or $Xa_1$, $Xa_6$ and $Xa_{10}$ and the molecular scaffold form a bicyclic peptide;
other groups are as defined in any of the preceding technical solutions.

Further, in the peptide compound, or the stereoisomer, the pharmaceutically acceptable salt, the solvate or the dimer thereof, the residues of $Xa_1$ and $Xa_6$ react to form a peptide ring; and the residues of one or two groups of $Xa_4$ and $Xa_5$, $Xa_1$ and $Xa_{10}$, $Xa_8$ and $Xa_{10}$, $Xa_8$ and $Xa_{12}$, $Xa_8$ and $Xa_{13}$, $Xa_9$ and $Xa_{13}$, $Xa_5$ and $Xa_7$, $Xa_{11}$ and $Xa_{13}$, $Xa_1$ and $Xa_{11}$, $Xa_7$ and $Xa_{11}$ are directly condensed or are linked via $L_1$ to form a peptide ring;

or $Xa_1$, $Xa_6$ and $Xa_{10}$ and the molecular scaffold form a bicyclic peptide;
other groups are as defined in any of the preceding technical solutions.

As a more specific sixth technical solution of the present disclosure, provided is the peptide compound, or the stereoisomer, the pharmaceutically acceptable salt, the solvate or the dimer thereof, wherein the peptide compound is linked to amino acid residues in the peptide compound via a polyethylene glycol chain to form a dimer compound; the polyethylene glycol chain is

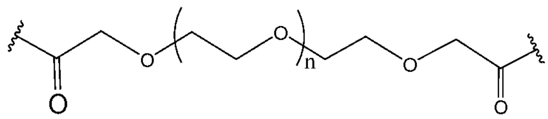,

n is any integer selected from 0-99;
other groups are as defined in any of the preceding technical solutions. As a more specific seventh technical solution of the present disclosure, provided is the peptide compound, or the stereoisomer, the pharmaceutically acceptable salt, the solvate or the dimer thereof, wherein the modifying group is

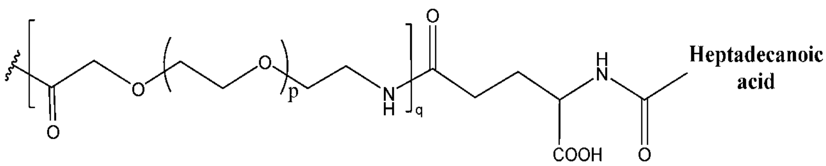,

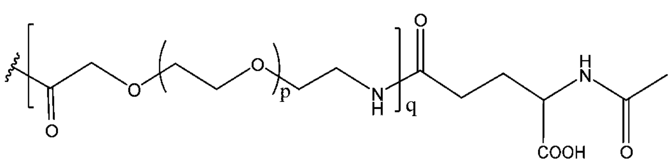,

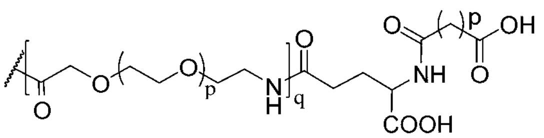 or 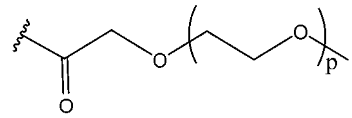,

in which p is any integer selected from 0-50, and q is any integer selected from 0-50; in some embodiments, the

modifying group is

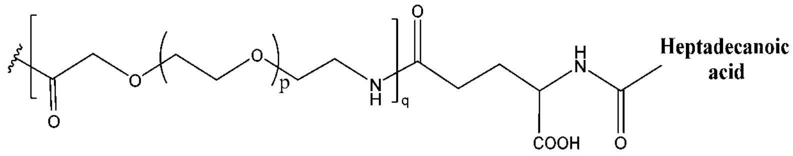

,

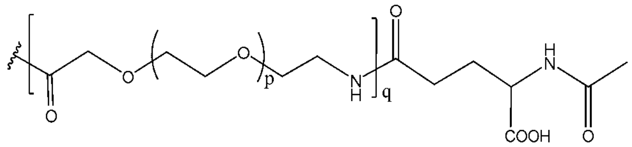

or

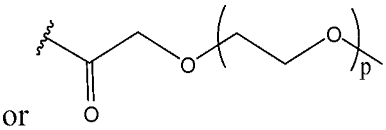

,

in which p is any integer selected from 0-50, and q is any integer selected from 0-50;
other groups are as defined in any of the preceding technical solutions.

Further, the modifying group is

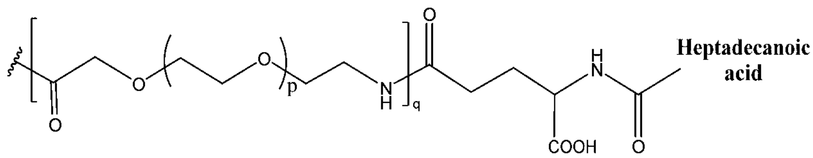

,

in which p is any integer selected from 0-5, and q is any integer selected from 0-5; and other groups are as defined in any of the preceding technical solutions.

Further, the modifying group is

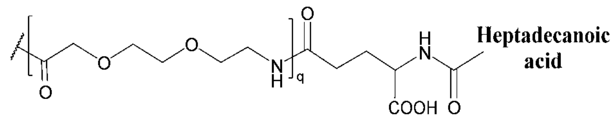

,

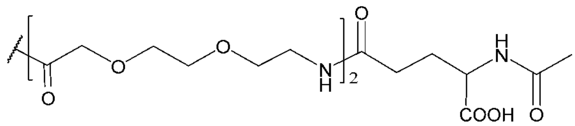

,

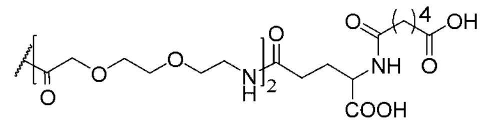

,

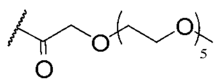

,

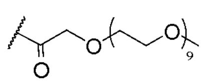

, or

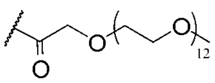

;

and
other groups are as defined in any of the preceding technical solutions.

As a more specific eighth technical solution of the present disclosure, provided is the peptide compound, or the stereoisomer, the pharmaceutically acceptable salt, the solvate or the dimer thereof, wherein

the residues of $Xa_1$ and $Xa_6$ react to form the following structure:

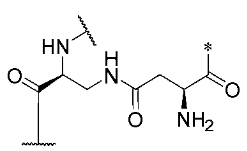

,

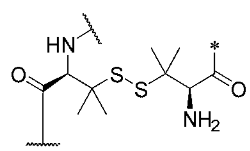

,

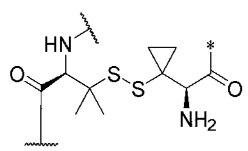

,

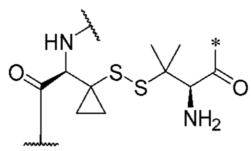

,

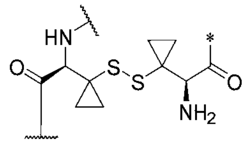, 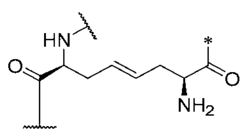, 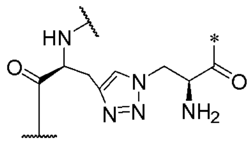, 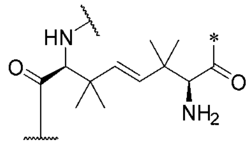,

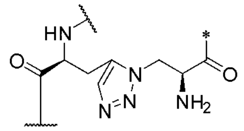, 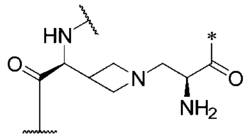, 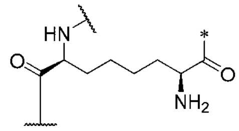, 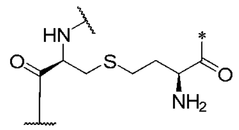,

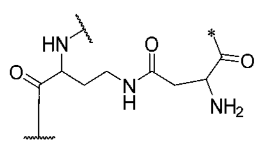, 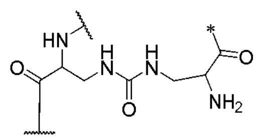, 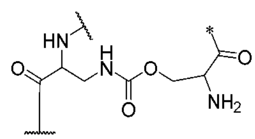, 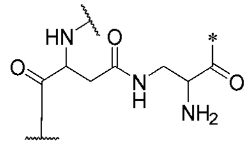,

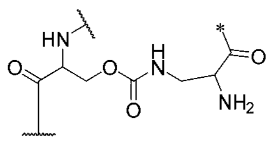, 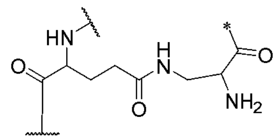, or 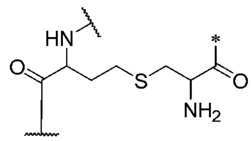;

in which the * end is an end of $Xa_1$, $Xa_1$ is linked to $Xa_2$ via the * position, and the $NH_2$ terminal end is linked to a protecting group; or the * end is an end of $Xa_1$, $Xa_1$ is linked to $Xa_2$ via the * position, and the NH2 terminal end is linked to a protecting group or the $NH_2$ terminal end is conjugated to a modifying group;

$Xa_2$ is selected from Asn, His, or an analogue of His, and the analogue of His is selected from

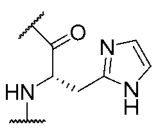 or 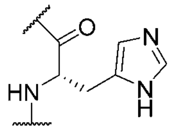;

or the analogue of His is

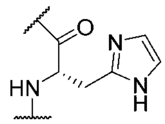;

or the analogue of His is

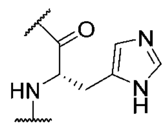;

$Xa_3$ is Thr;

$Xa_4$ is an analogue of Trp, and the analogue of Trp is selected from

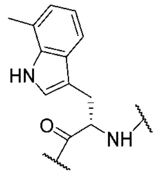,

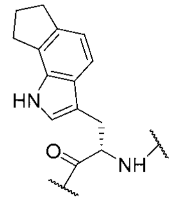, 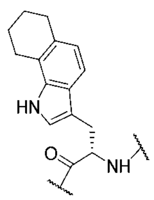, 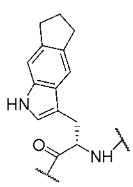, 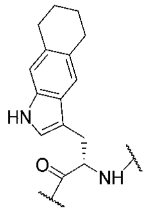, 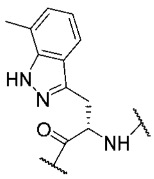, 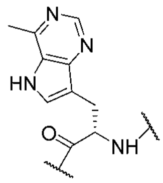,

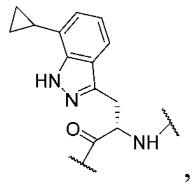, or 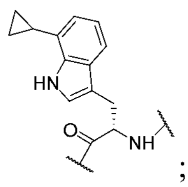;

$Xa_5$ is selected from Lys, Gln, Arg, Cit, or an analogue Arg and Lys, and the analogue of Arg and Lys is selected from

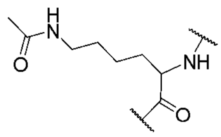 or 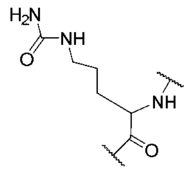;

or alternatively, a residue of $Xa_5$ is conjugated to a modifying group;

$Xa_7$ is selected from Phe or an analogue of Phe, and the analogue of Phe is selected from

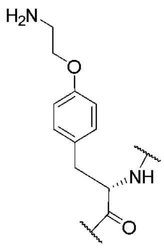, 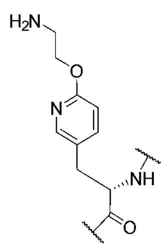, 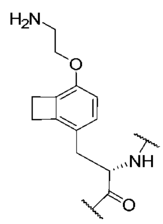, 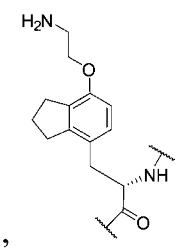, 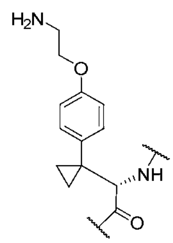,

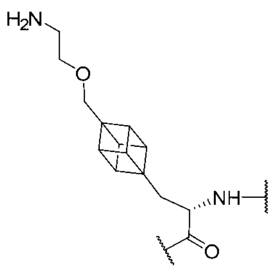, or 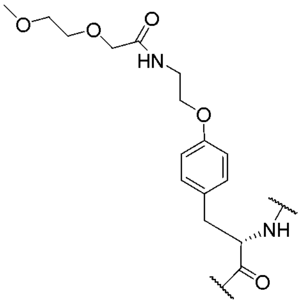;

or alternatively, a residue of $Xa_7$ is conjugated to a modifying group;

$Xa_8$ is selected from Phe, Trp, 2-Nal, or an analogue of Phe, Trp, and 2-Nal, and the analogue of Phe, Trp, and 2-Nal is selected from

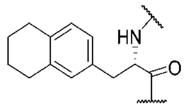, 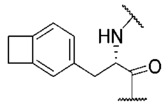,

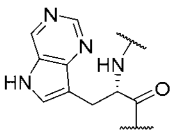, 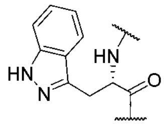, 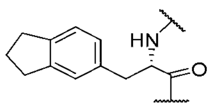, or 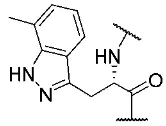; or is 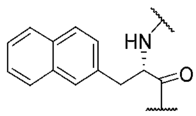;

$Xa_9$ is selected from Thp or an analogue of Thp, and the analogue of Thp is selected from

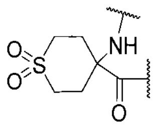, 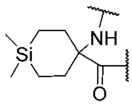, 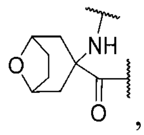, or 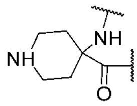;

$Xa_{10}$ is selected from Glu or Cys;
$Xa_{11}$ is selected from Asn or Lys; and
$Xa_{12}$ is selected from 3-Pal or Phe;
the molecular scaffold is selected from:

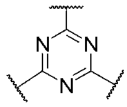, 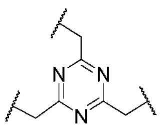, 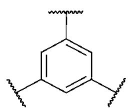,

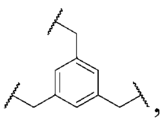, 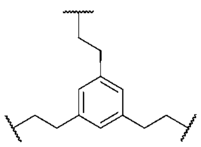, 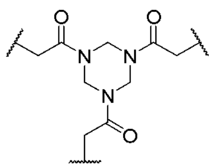 or 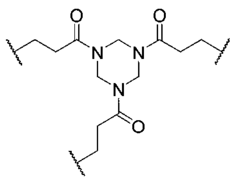;

or is

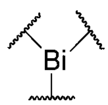;

other groups are as defined in any of the preceding technical solutions.

Further, in the peptide compound, or the stereoisomer, the pharmaceutically acceptable salt, the solvate, or the dimer thereof, the residues of Xa1 and $Xa_6$ react to form the following structure:

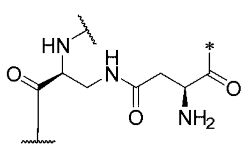, 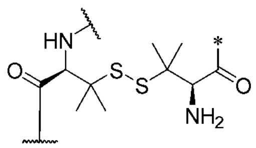,

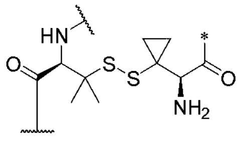, 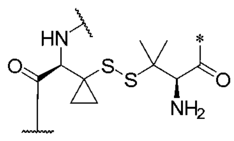, 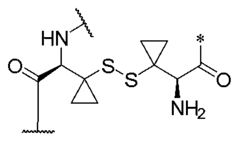, 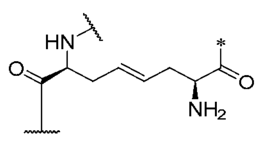,

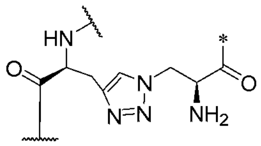, 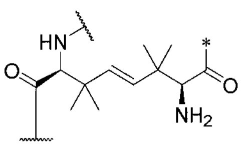, 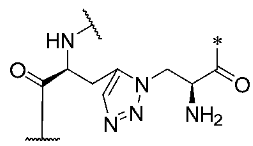, 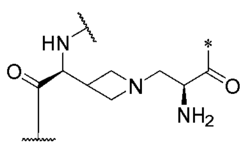,

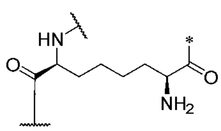, 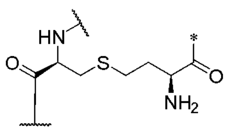, 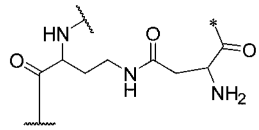, 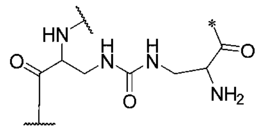,

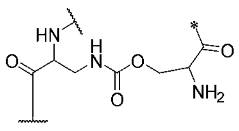, 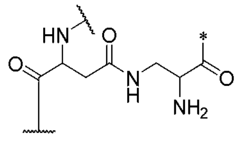, 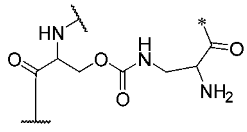, 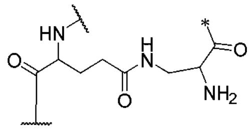,

or

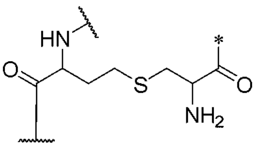;

in which the * end is an end of $Xa_1$, $Xa_1$ is linked to $Xa_2$ via the * position, and the $NH_2$ terminal end is linked to a protecting group;

$Xa_2$ is selected from Asn, His, or an analogue of His, and the analogue of His is

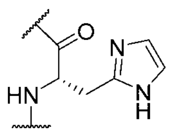;

$Xa_4$ is an analogue of Trp, and the analogue of Trp is selected from

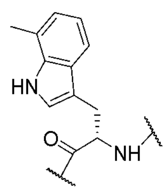,

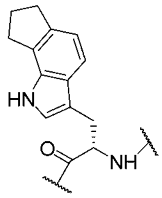, 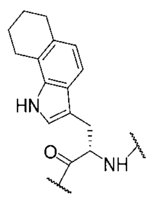, 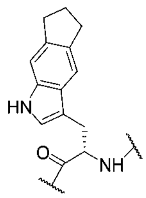, 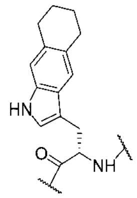, 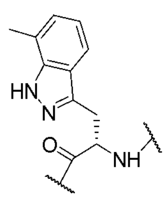, 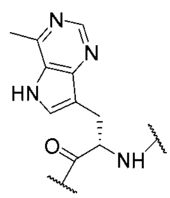,

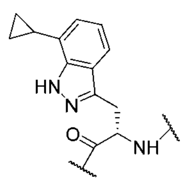, or 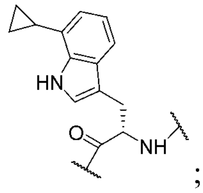;

$Xa_5$ is selected from Lys, Gln, Arg, or an analogue Arg, and the analogue of Arg is

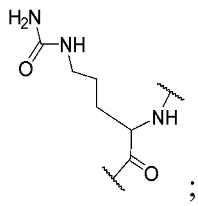;

$Xa_7$ is selected from Phe or an analogue of Phe, and the analogue of Phe is selected from

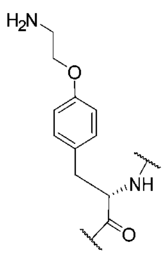, 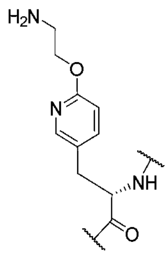, 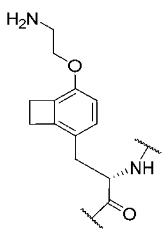, 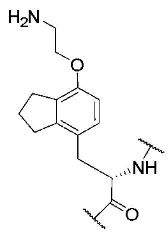, 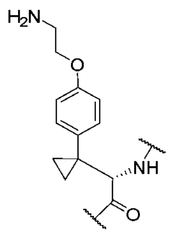,

or

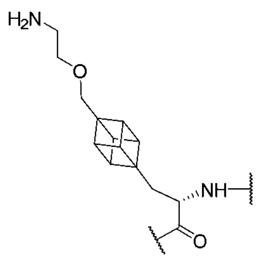;

and

$Xa_8$ is selected from Phe, Trp, 2-Nal, or an analogue of Phe, Trp, and 2-Nal, and the analogue of Phe, Trp, and 2-Nal is selected from

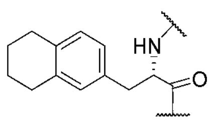,

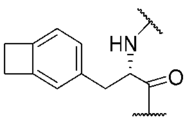, 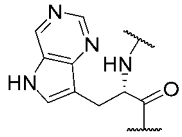, 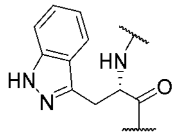, 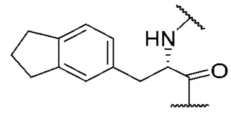, or 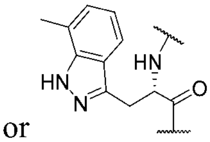;

$Xa_9$ is selected from Thp or an analogue of Thp, and the analogue of Thp is selected from

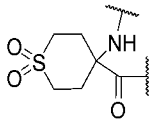, 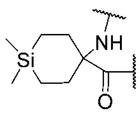, 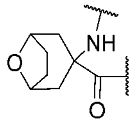, or 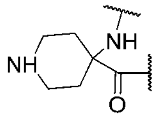;

$Xa_{10}$ is selected from Glu or Cys;
$Xa_{11}$ is selected from Asn or Lys; and
$Xa_{12}$ is selected from 3-Pal or Phe;
the molecular scaffold is selected from:

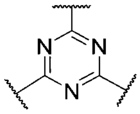, 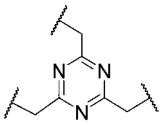, 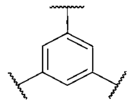,

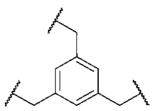, 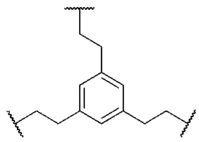, 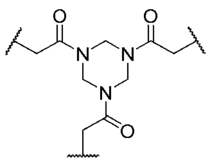 or 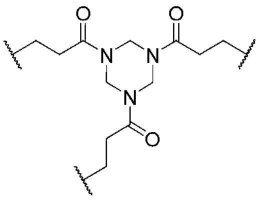;

other groups are as defined in any of the preceding technical solutions.

Further, in the peptide compound, or the stereoisomer, the pharmaceutically acceptable salt, the solvate or the dimer thereof,

the residues of $Xa_1$ and $Xa_6$ react to form the following structure:

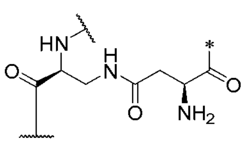, 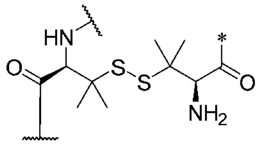, 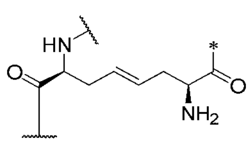, 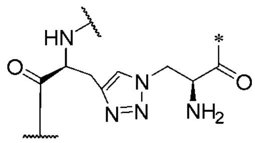,

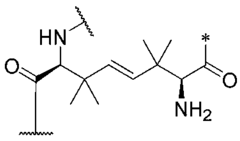, 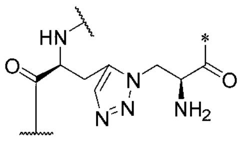, 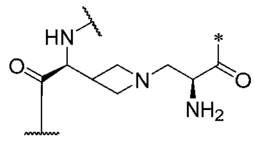, or 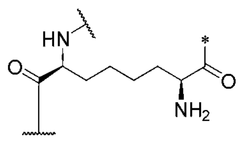;

or the residues of $Xa_1$ and $Xa_6$ react to form the following structure:

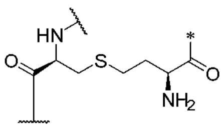, 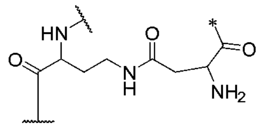, 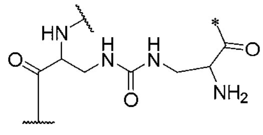, 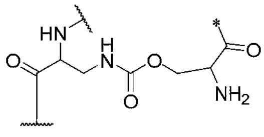,

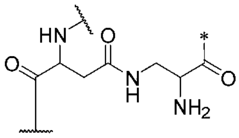, 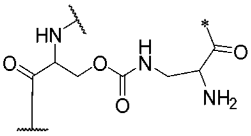, 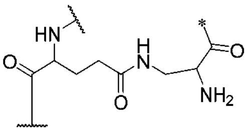, or 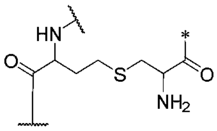;

or the residues of $Xa_1$ and $Xa_6$ react to form the following structure:

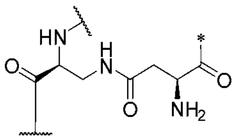, 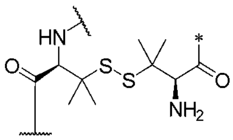, 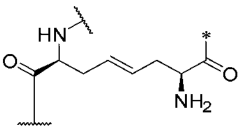, 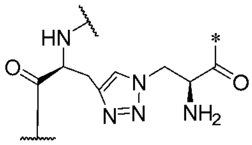,

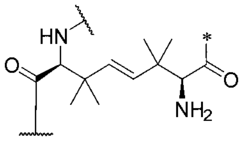, 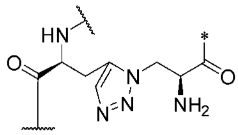, 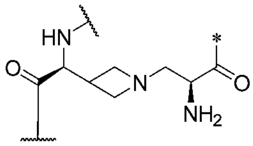, 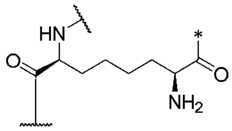,

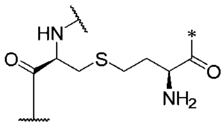, 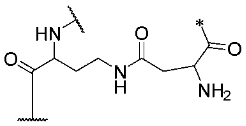, 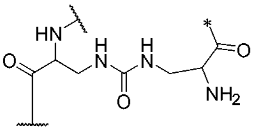, 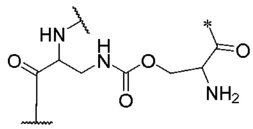,

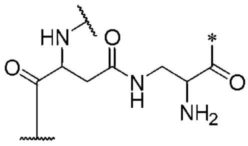, 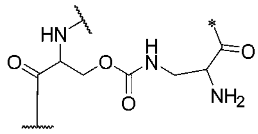, 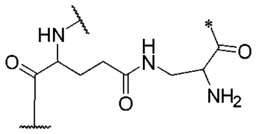, or 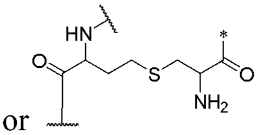;

in which the * end is an end of $Xa_1$, $Xa_1$ is linked to $Xa_2$ via the * position, and the $NH_2$ terminal end is linked to a protecting group;

$Xa_2$ is selected from Asn, His, or an analogue of His, and the analogue of His is selected from

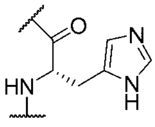 or 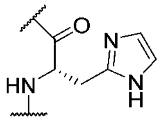;

$Xa_4$ is an analogue of Trp, and the analogue of Trp is selected from

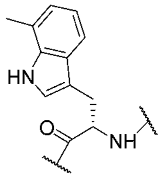,

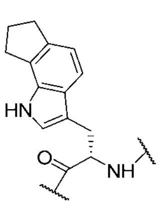, 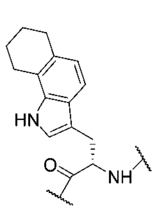, 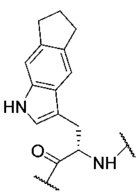, 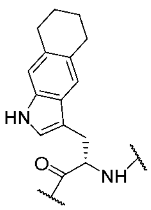, 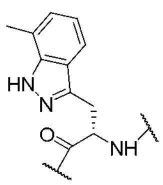, 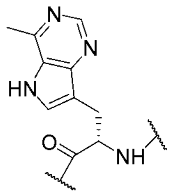,

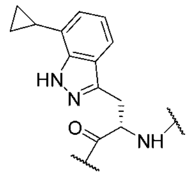, or 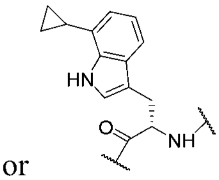;

$Xa_5$ is selected from Lys, Gln, Arg, or an analogue Arg, and the analogue of Arg is

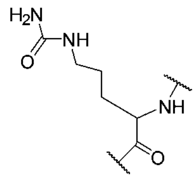;

$Xa_7$ is selected from Phe or an analogue of Phe, and the analogue of Phe is selected from

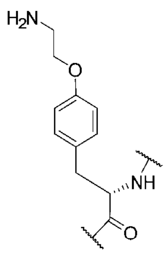, 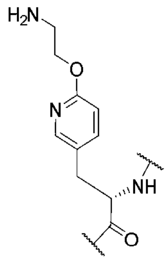, 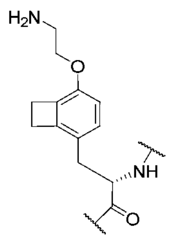, 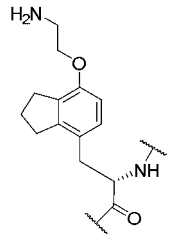, 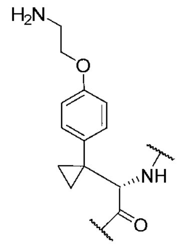,

or

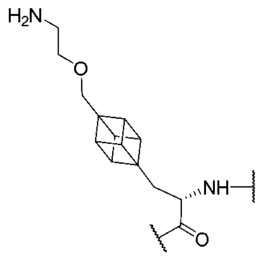

; and in some embodiments, $Xa_7$ is selected from Phe or an analogue of Phe, and the analogue of Phe is selected from

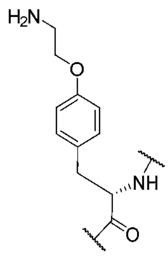, 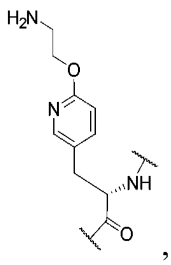,

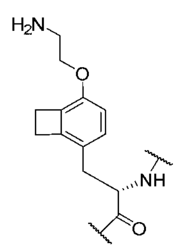, 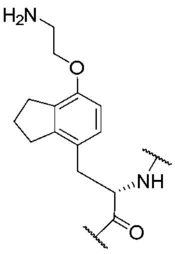, or 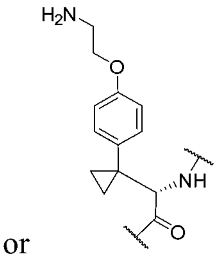;

$Xa_8$ is selected from Phe, Trp, 2-Nal, or an analogue of Phe, Trp, and 2-Nal, and the analogue of Phe, Trp, and 2-Nal is selected from

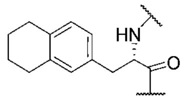, 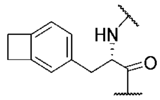,

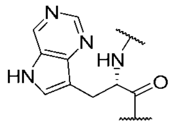, 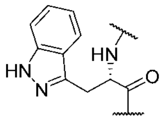, or 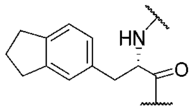;

or the analogue of Trp is

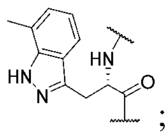;

$Xa_9$ is selected from Thp or an analogue of Thp, and the analogue of Thp is selected from

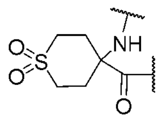, 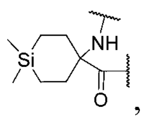, 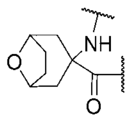, or 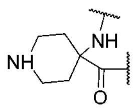;

$Xa_{10}$ is selected from Glu or Cys;
$Xa_{11}$ is selected from Asn or Lys; and
$Xa_{12}$ is selected from 3-Pal or Phe;
other groups are as defined in any of the preceding technical solutions.

As a more specific ninth technical solution of the present disclosure, provided is the peptide compound, or the stereoisomer, the pharmaceutically acceptable salt, the solvate or the dimer thereof, wherein the molecular scaffold is selected from:

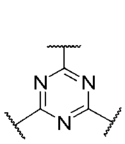, 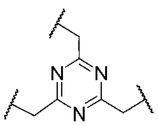, 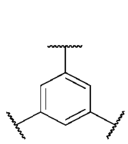, 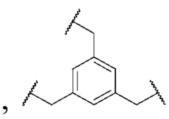, 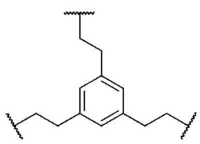, 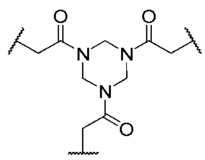,

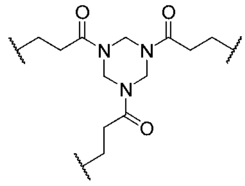

, or

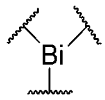

; in some embodiments, the molecular scaffold is selected from:

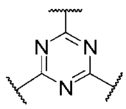

,

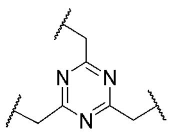

,

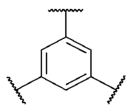

,

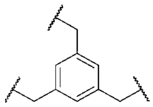

,

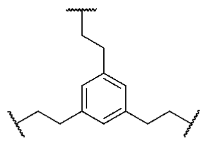

,

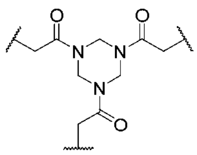

or

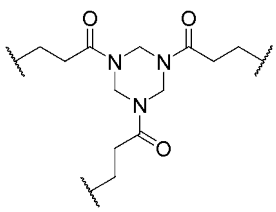

;

other groups are as defined in any of the preceding technical solutions.

As a more specific tenth technical solution of the present disclosure, provided is the peptide compound, or the stereoisomer, the pharmaceutically acceptable salt, the solvate or the dimer thereof, wherein

$L_1$ is selected from a bond, vinyl, propenyl, butenyl, $-O-(CH_2)_r-O-(CH_2)_r-NH-C(=O)-$, $-O-(CH_2)_r-O-(CH_2)_r-$, $-O-(CH_2)_r-O-(CH_2)_r-NH-$, $-C(=O)-(CH_2)_r-O-(CH_2)_r-O-(CH_2)_r-$, $-C(=O)-(CH_2)_r-O-(CH_2)_r-O-(CH_2)_r-NH-$, $-C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, $-(CH_2)_r-O-(CH_2)_r-$, $-O-(CH_2)_r-NH-$, $C_{1-6}$ alkylene, $-C(=O)-$, $-C(=O)-(CH_2)_r-NH-$,

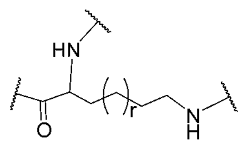

,

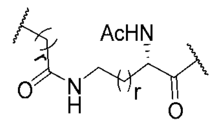

, $-(CH_2)_r-O-(CH_2)_r-NH-$, $-O-(CH_2)_r-O-(CH_2)_r-O-(CH_2)_r-O-(CH_2)_r-NH-$, $-(CH_2)_r-NH-$, $-C(=O)-(CH_2)_r-O-(CH_2)_r-NH-$, $-(CH_2)_r-NH-C(=O)-(CH_2)_r-$, or $-C(=O)-(CH_2)-(OCH_2CH_2)_a-NH-$;

Further, $L_1$ is selected from a bond, vinyl, propenyl, butenyl, $-O-(CH_2)_r-O-(CH_2)_r-NH-C(=O)-$, $-O-(CH_2)_r-O-(CH_2)_r-$, $-O-(CH_2)_r-O-(CH_2)_r-NH-$, $-C(=O)-(CH_2)_r-O-(CH_2)_r-O-(CH_2)_r-$, $-C(=O)-(CH_2)_r-O-(CH_2)_r-O-(CH_2)_r-NH-$, $-C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, -(CH2) r-O-(CH2)r-, -O-(CH2)r-NH-, $C_{1-2}$ alkylene, $-C(=O)-$, - $C(=O)-(CH_2)_r-NH-$,

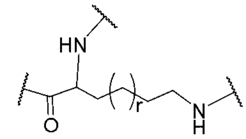

,

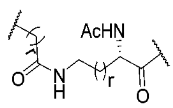

, $-(CH_2)_r-O-(CH_2)_r-NH-$, $-O-(CH_2)_r-O-(CH_2)_r-O-(CH_2)_r-O-(CH_2)_r-NH-$, $-(CH_2)_r-NH-$, or $-C(=O)-(CH_2)_r-O-(CH_2)_r-NH-$;

Further, $L_1$ is selected from a bond, vinyl, propenyl, butenyl, $-O-(CH_2)_r-O-(CH_2)_r-NH-C(=O)-$, $-O-(CH_2)_r-O-(CH_2)_r-$, $-O-(CH_2)_r-O-(CH_2)_r-NH-$, $-C(=O)-(CH_2)_r-O-(CH_2)_r-O-(CH_2)_r-$, $-C(=O)-(CH_2)_r-O-(CH_2)_r-O-(CH_2)_r-NH-$, $-C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, -(CH2) r-O-(CH2)r-, -O-(CH2)r-NH-, $C_{1-2}$ alkylene, $-C(=O)-$, - $C(=O)-(CH_2)_r-NH-$,

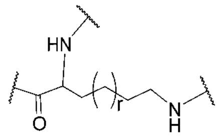 or 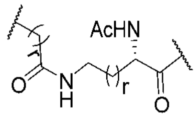

, or is selected from $-(CH_2)_r-O-(CH_2)_r-NH-$, $-O-(CH_2)_r-O-(CH_2)_r-O-(CH_2)_r-O-(CH_2)_r-NH-$, or $-(CH_2)_r-NH-$;

further, in the peptide compound, or the stereoisomer, the pharmaceutically acceptable salt, the solvate, or the dimer thereof, $L_1$ is selected from a bond, vinyl, propenyl, butenyl, $-O-(CH_2)_r-O-(CH_2)_r-NH-C(=O)-$, $-O-(CH_2)_r-O-(CH_2)_r-$, $-O-(CH_2)_r-O-(CH_2)_r-NH-$, $-C(=O)-(CH_2)_r-O-(CH_2)_r-O-(CH_2)_r-$, $-C(=O)-(CH_2)_r-O-(CH_2)_r-O-(CH_2)_r-NH-$, $-C(=O)-(CH_2)_r-O-(CH_2)_r-NH-C(=O)-$, $-NH-C(=O)-$, $-C(=O)-(CH_2)_r-O-(CH_2)_r-$, $-O-(CH_2)_r-NH-C(=O)-(CH_2)_r-$, $-(CH_2)_r-O-(CH_2)_r-$, $-O-(CH_2)_r-NH-$, $C_{1-2}$ alkylene, $-C(=O)-(CH_2)_r-NH-$, or

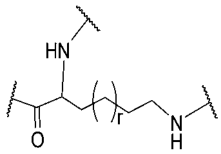 ;

or $L_1$ is

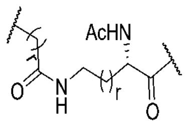 ;

r is selected from 0, 1, 2, 3, 4, 5, 6, 7, 8, 9, or 10;

a is selected from 3, 4, 5, or 6;

other groups are as defined in any of the preceding technical solutions.

As a more specific eleventh technical solution of the present disclosure, provided is the peptide compound, or the stereoisomer, the pharmaceutically acceptable salt, the solvate or the dimer thereof, wherein the peptide compound is of a structure selected from one of the structures in Table 1.

Table 1:

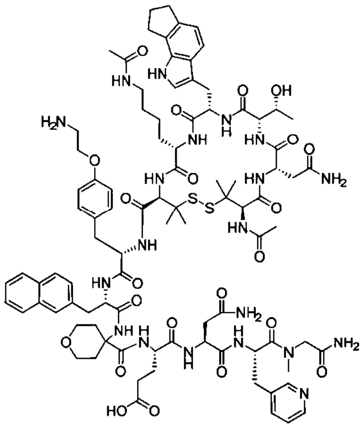

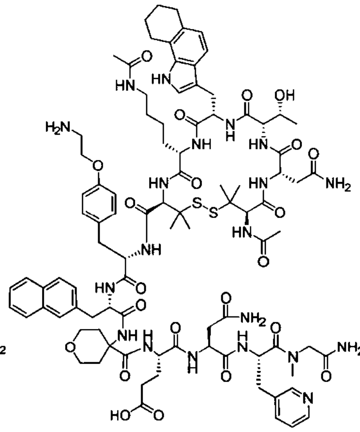

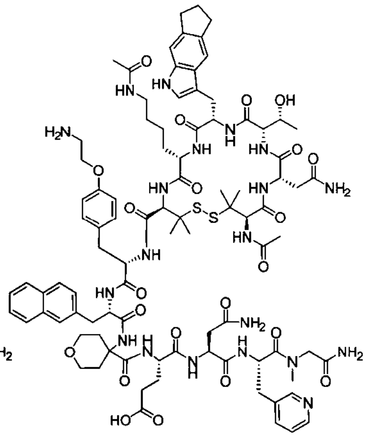

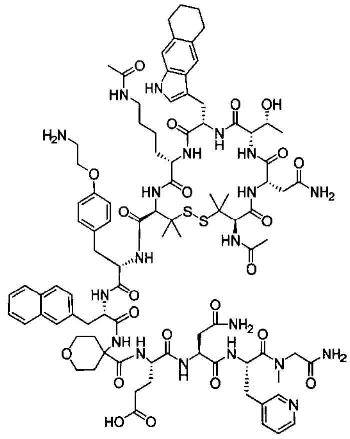

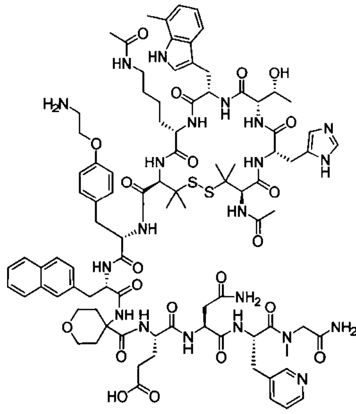

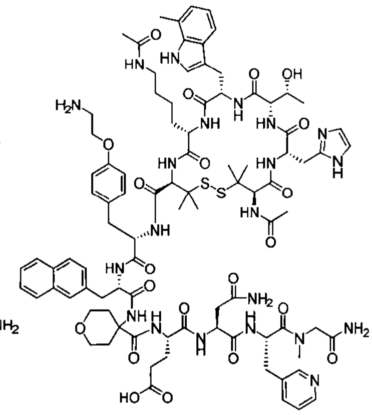

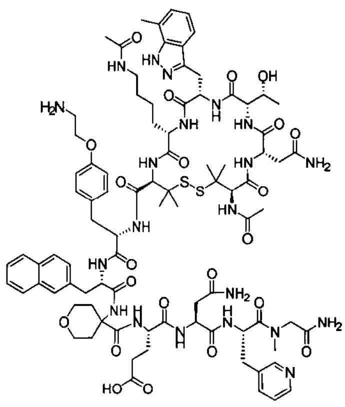
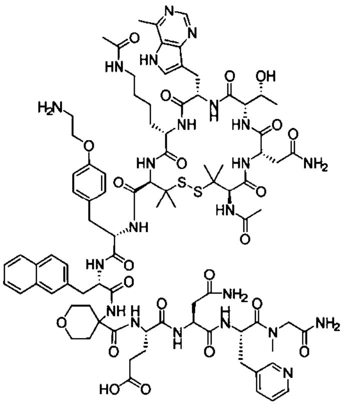
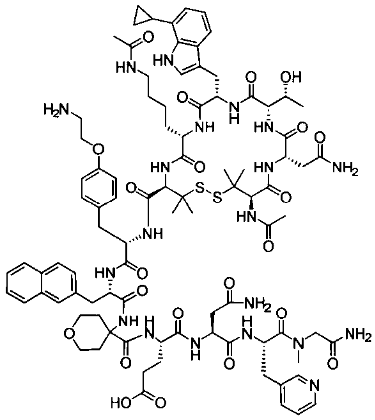

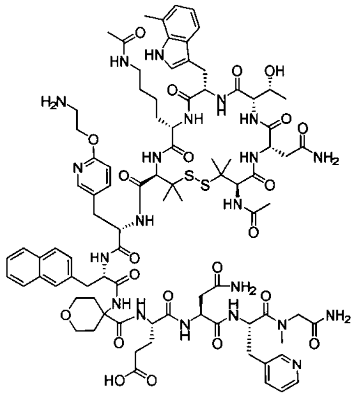
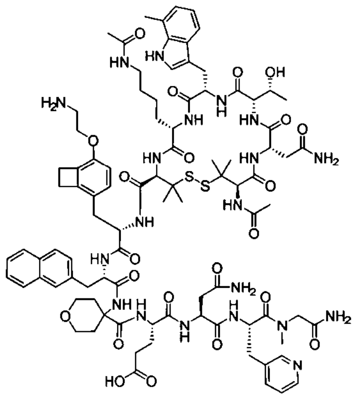
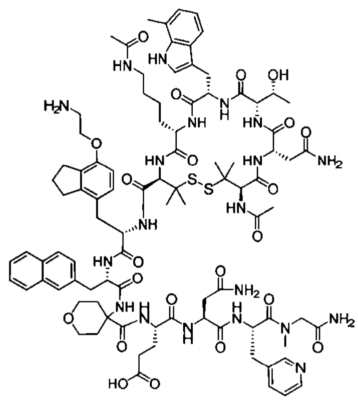

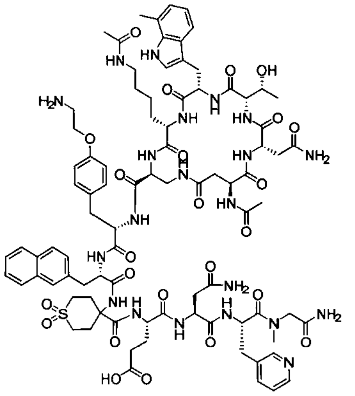
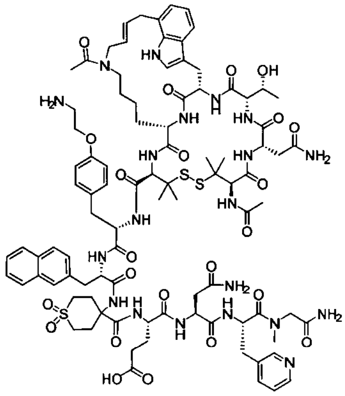
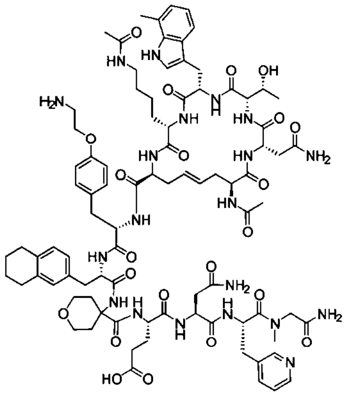

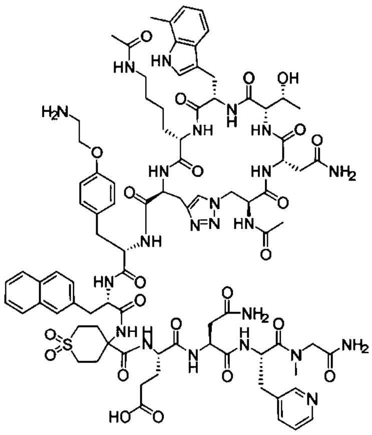
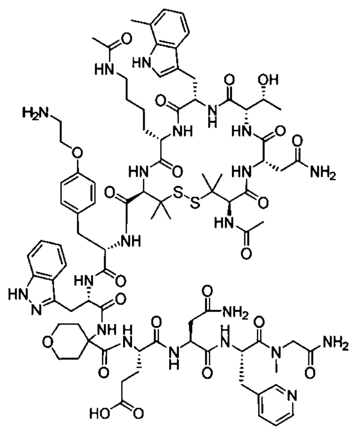
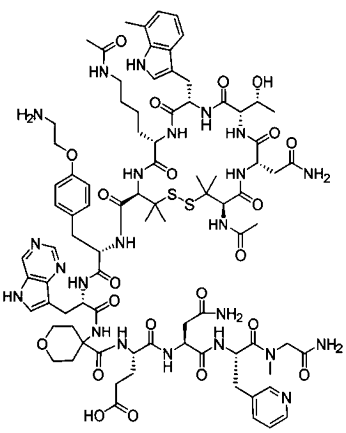

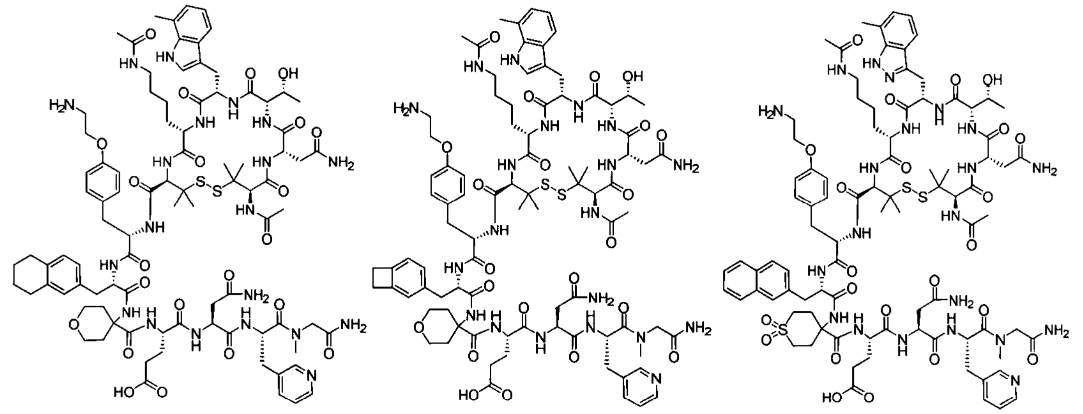

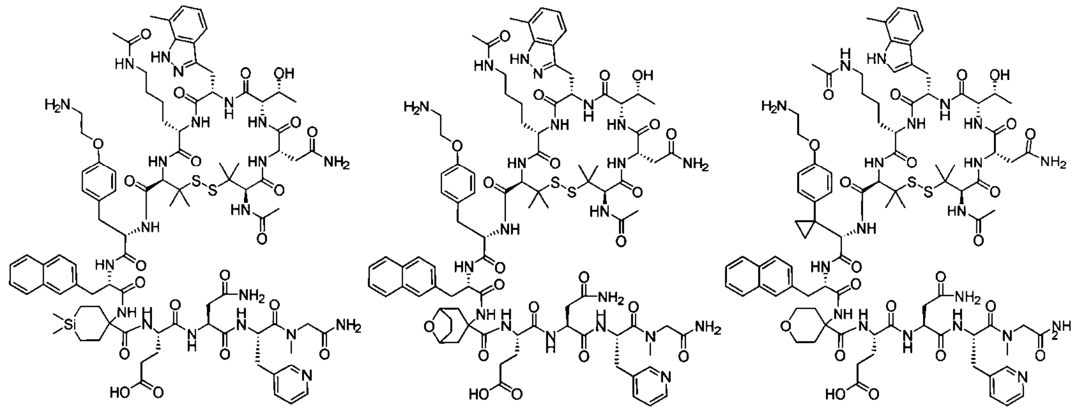

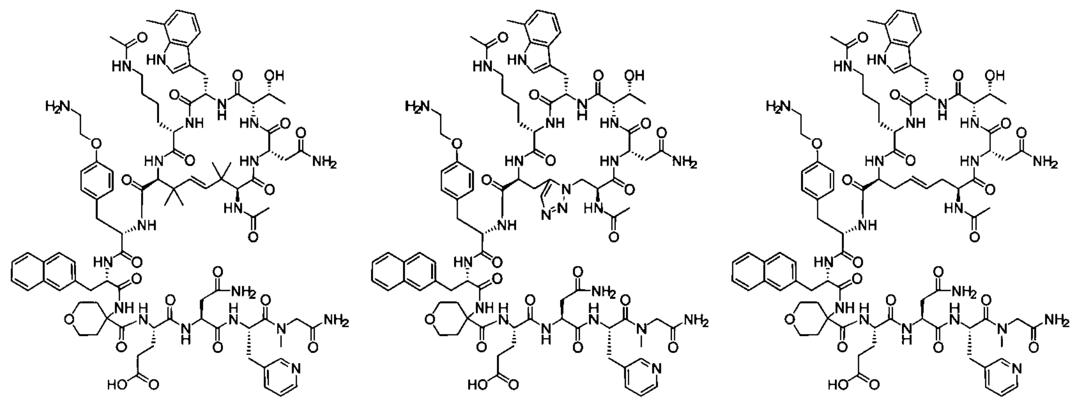

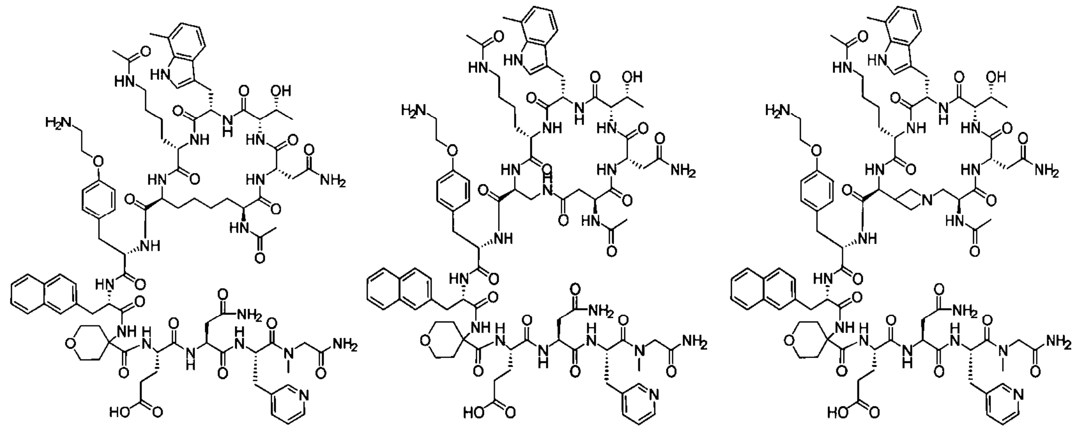

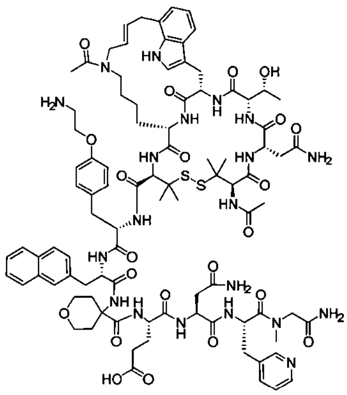
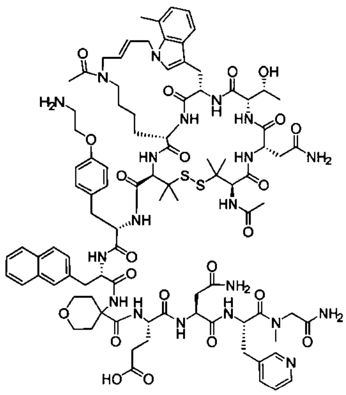
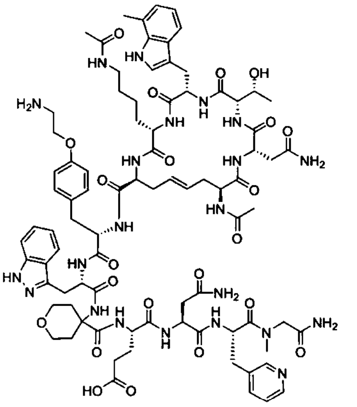

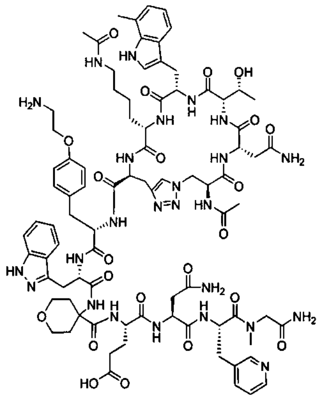
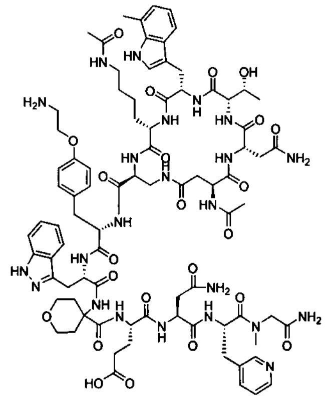
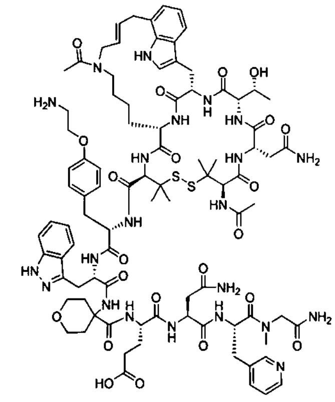

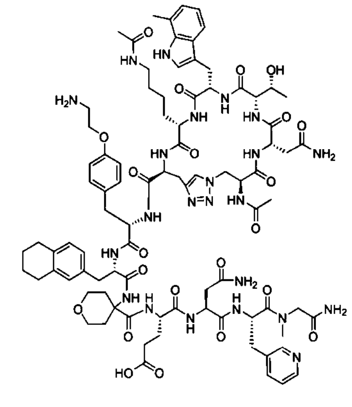
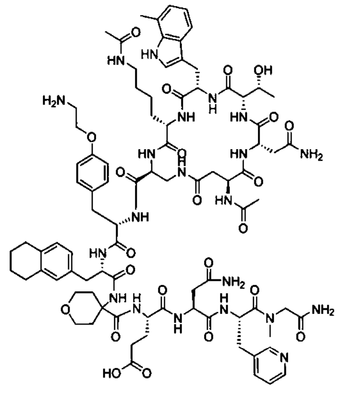
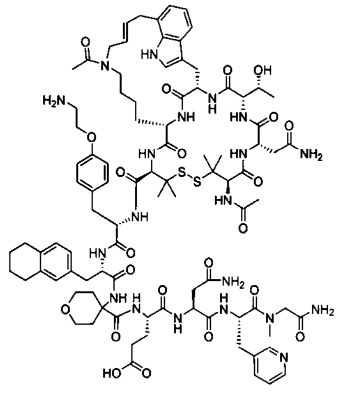

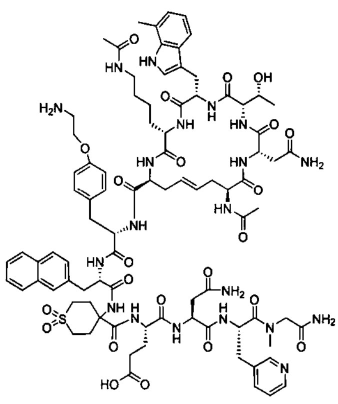
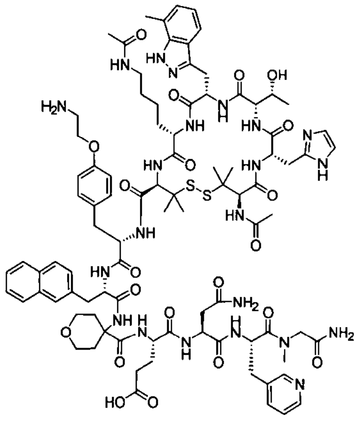
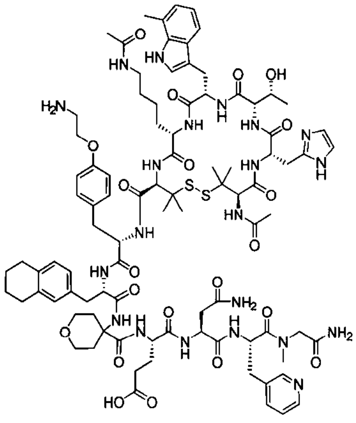

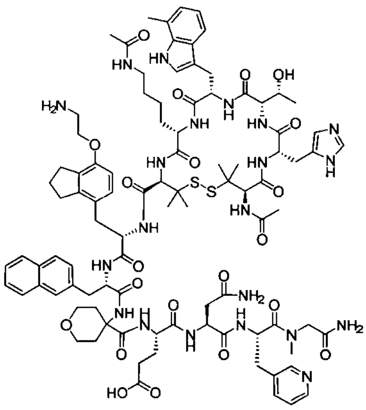
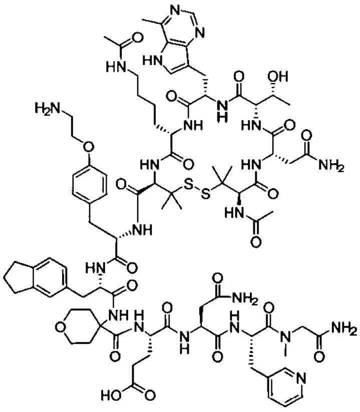
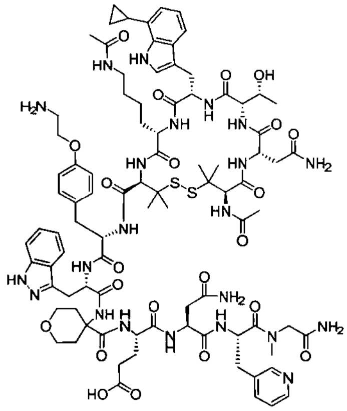
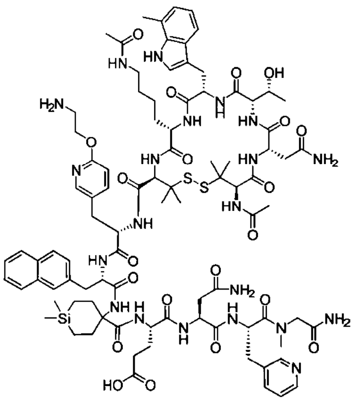
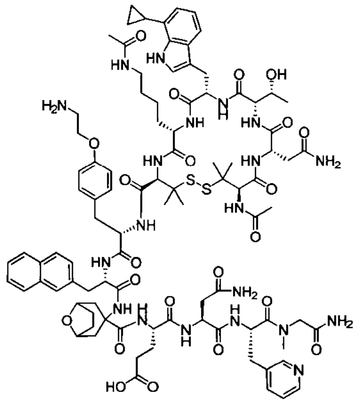
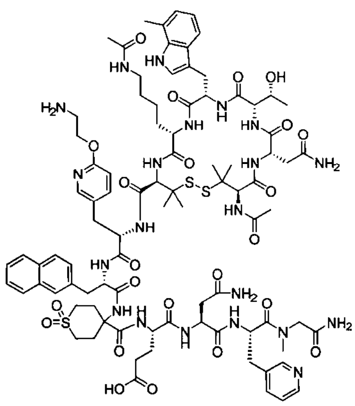
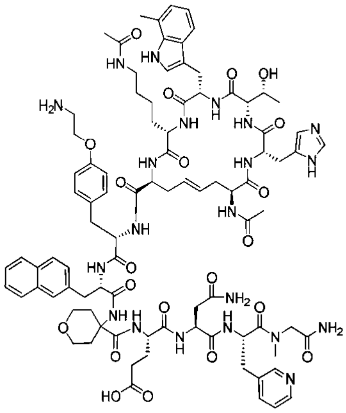
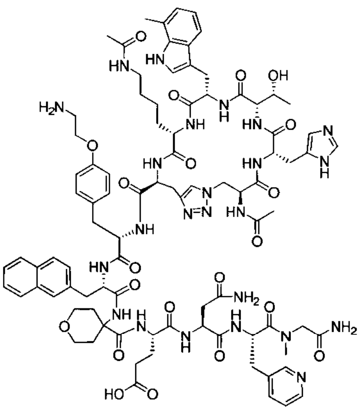
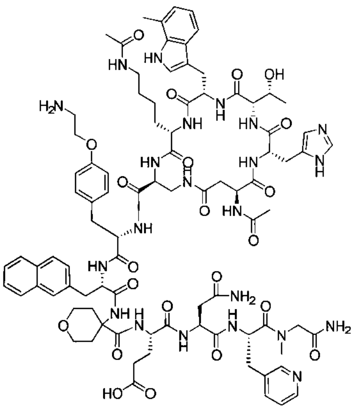
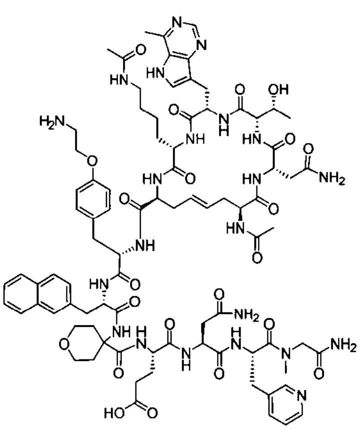
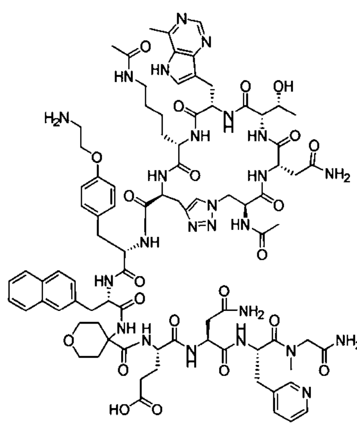
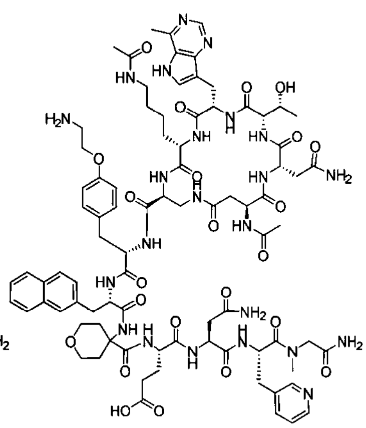

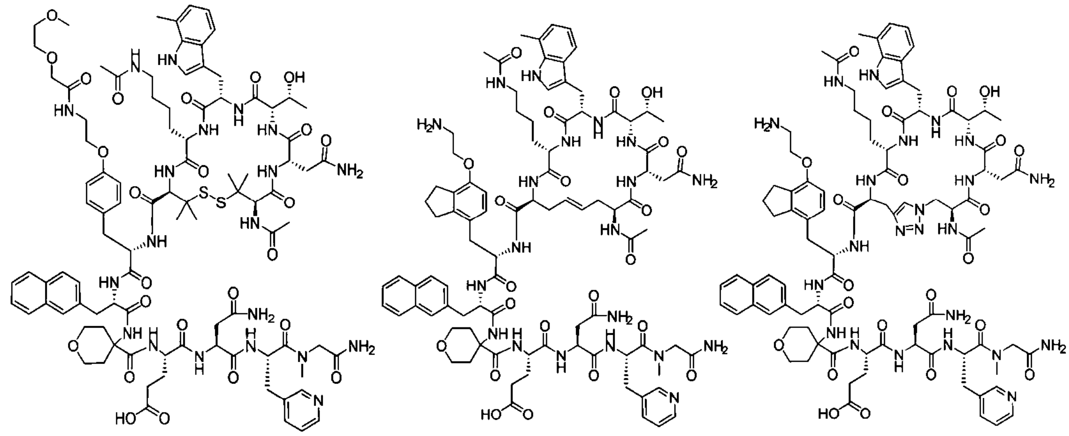

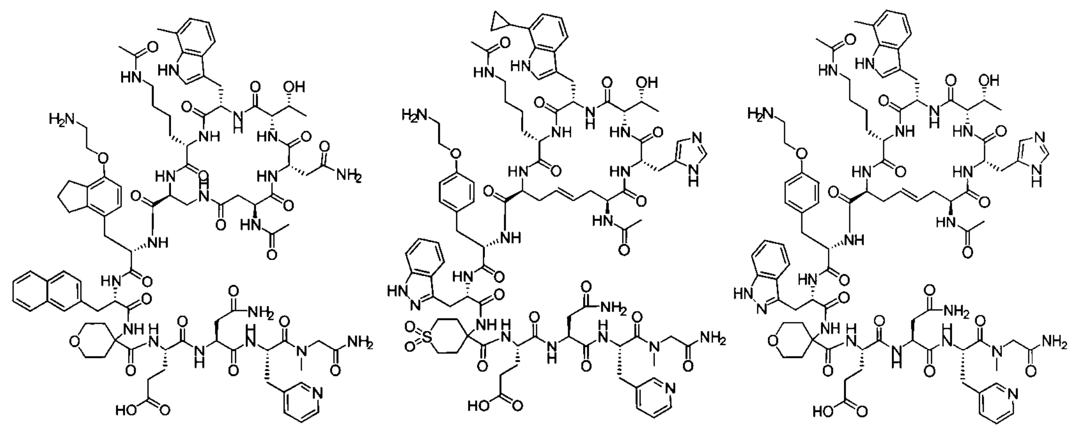

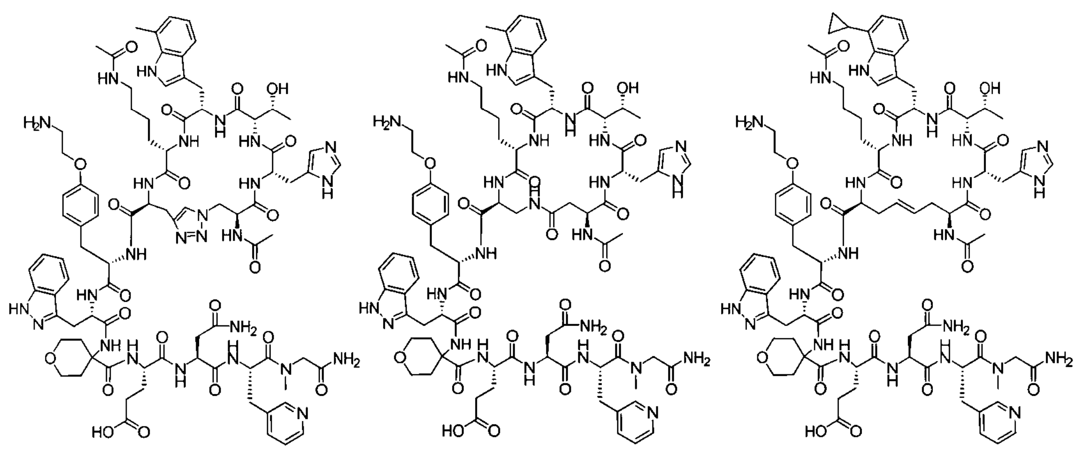

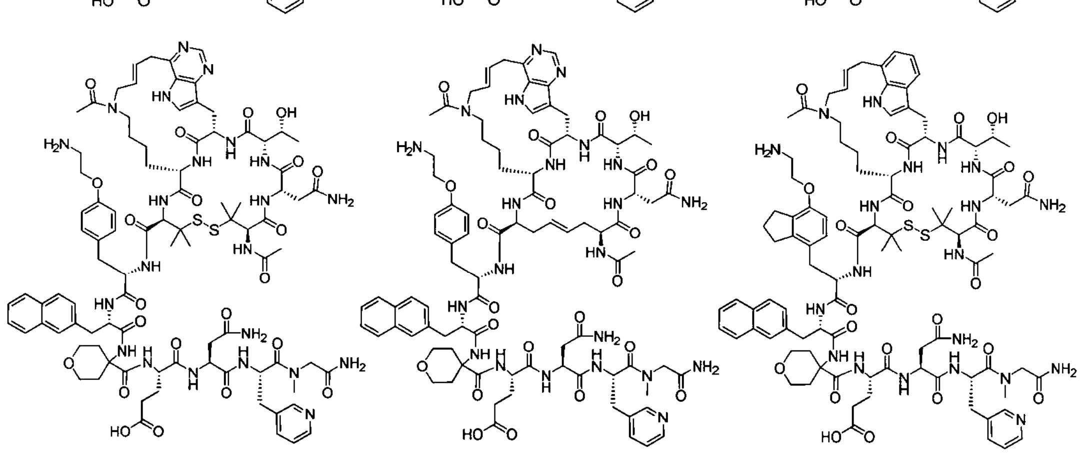

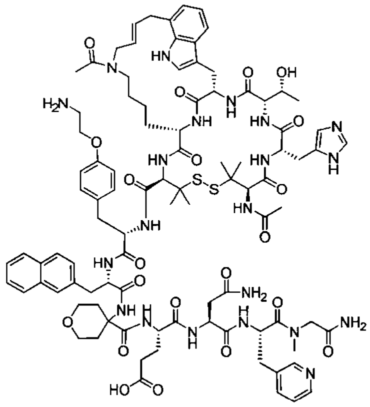
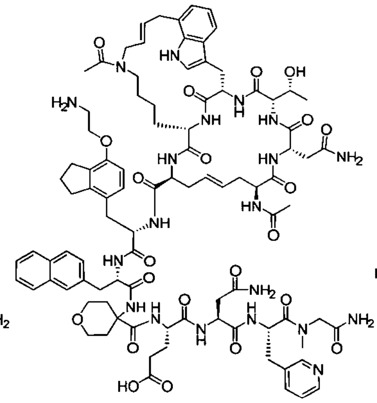
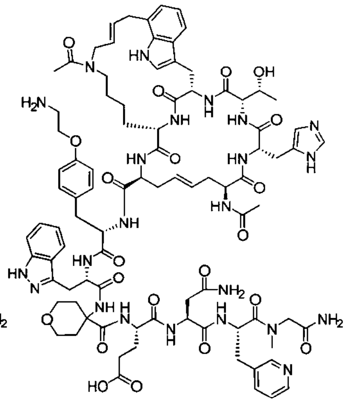

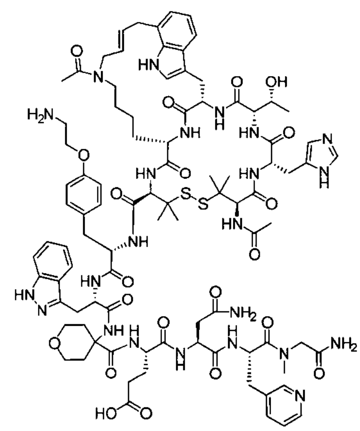
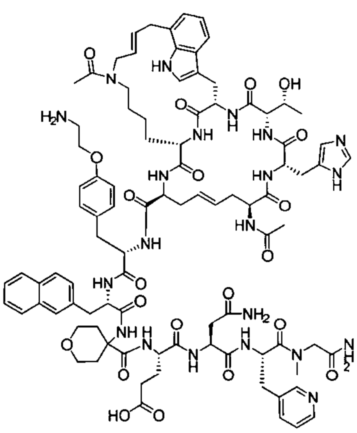
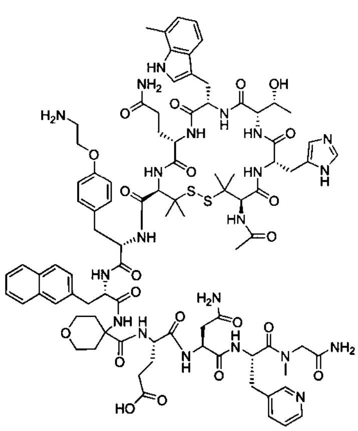

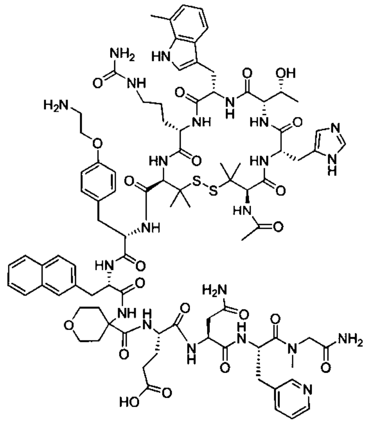
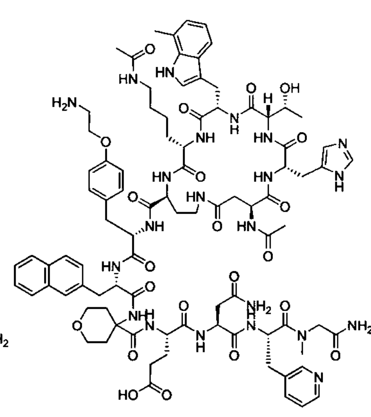
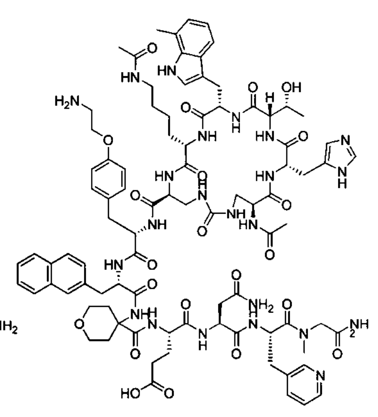

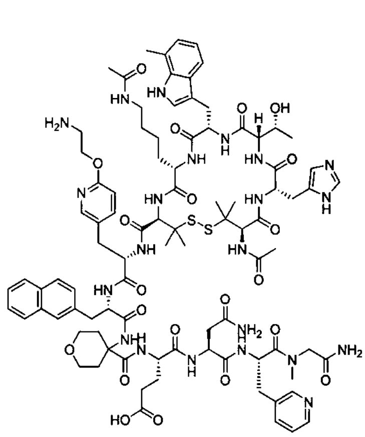
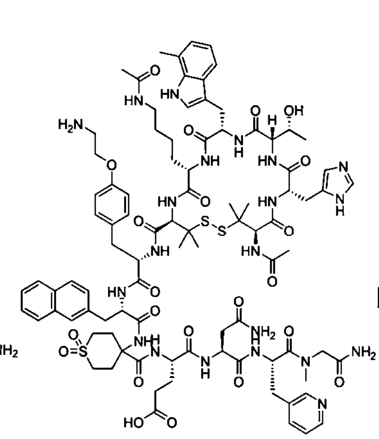
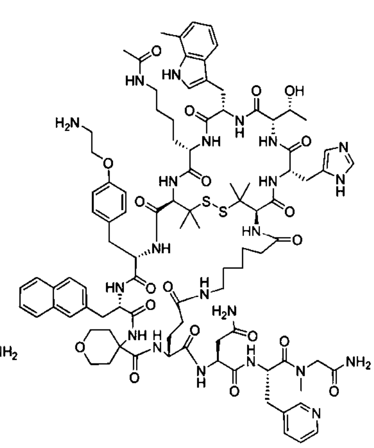

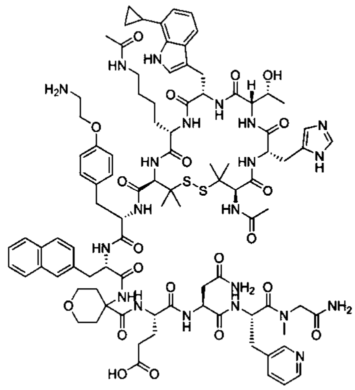

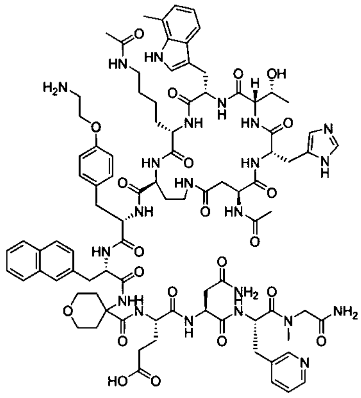

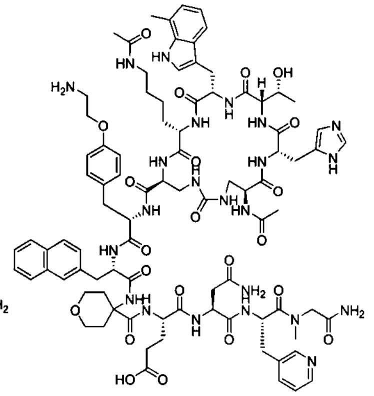

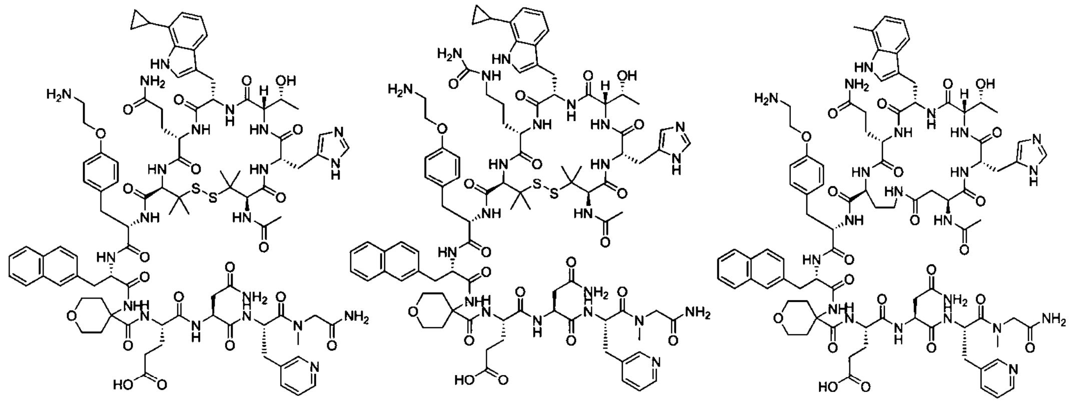

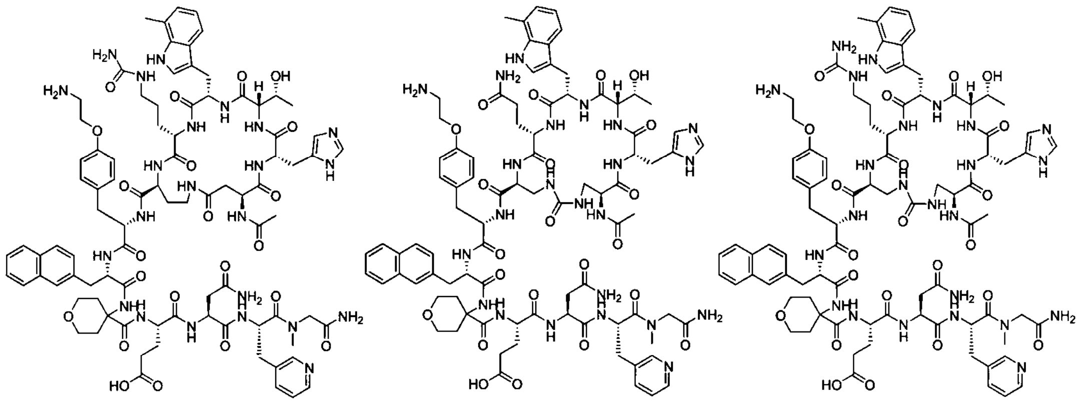

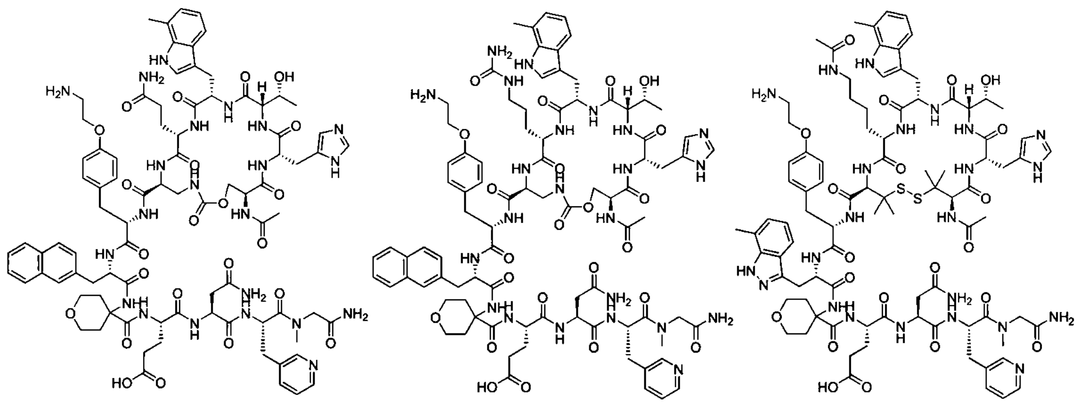

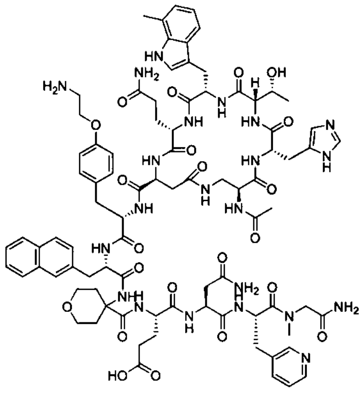
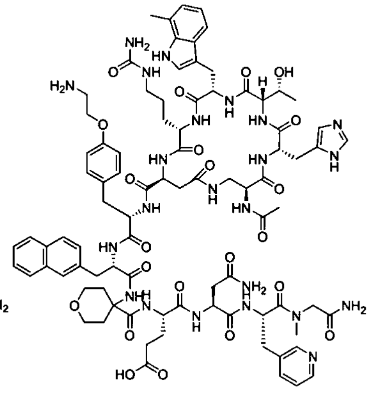
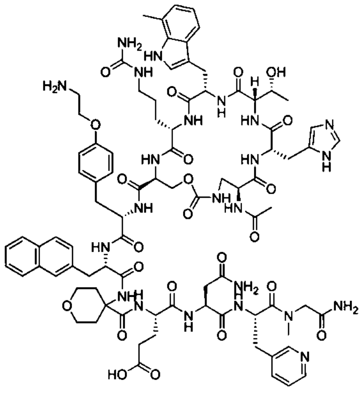

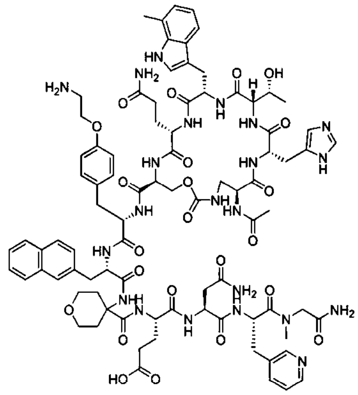
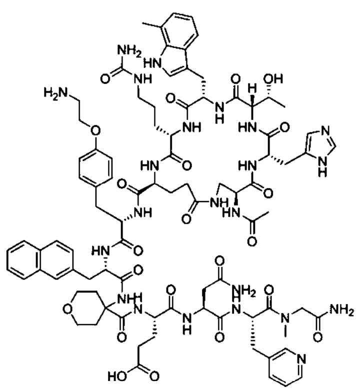
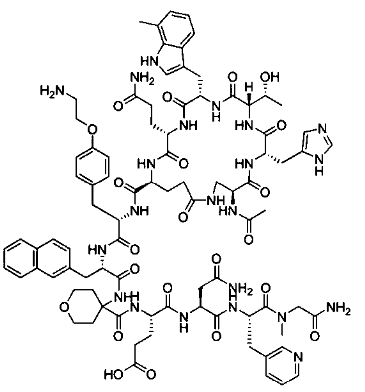

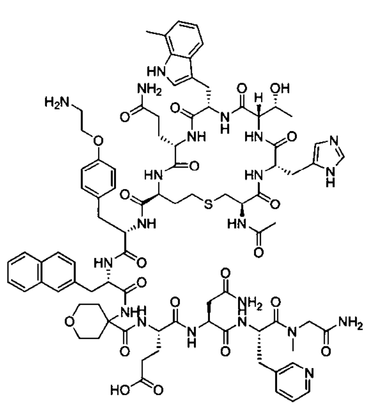
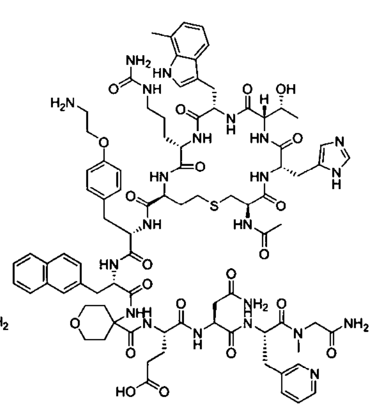
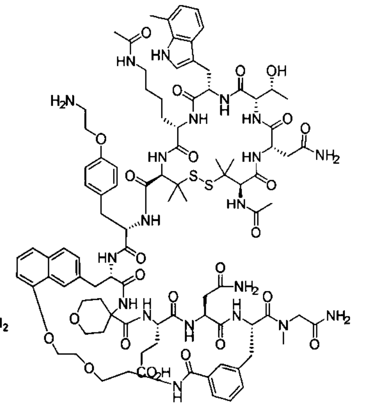

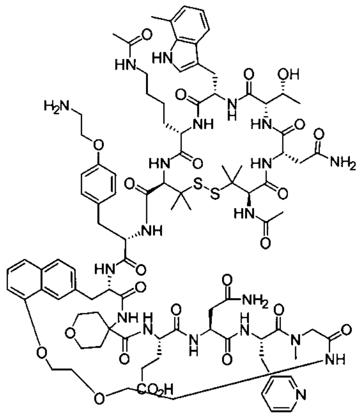
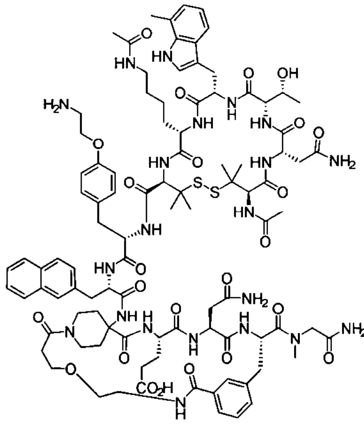
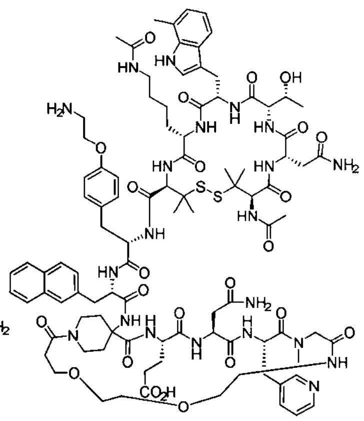

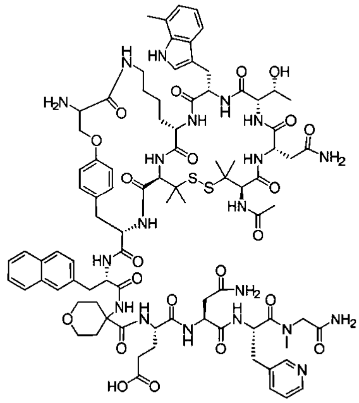

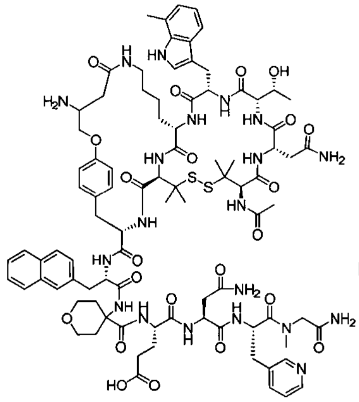

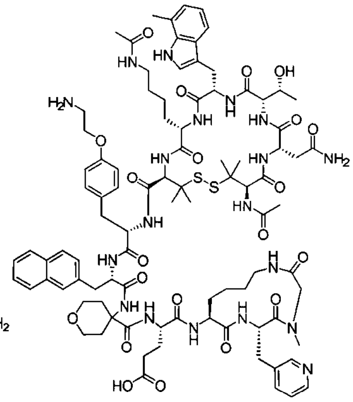

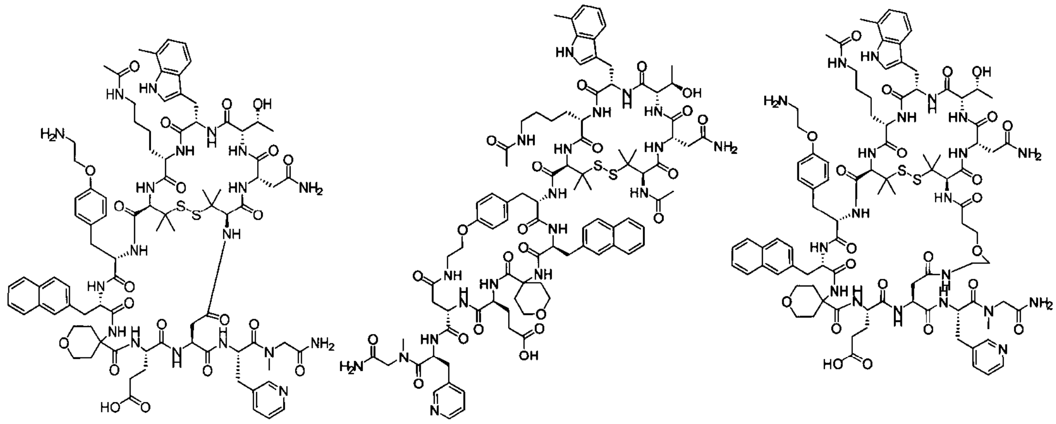

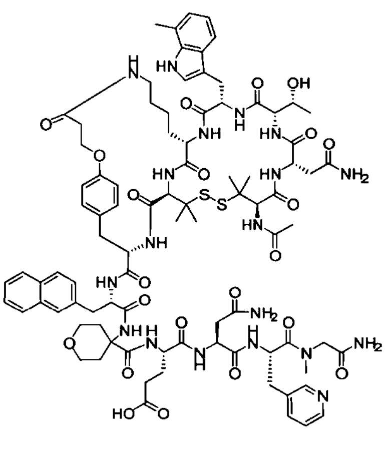

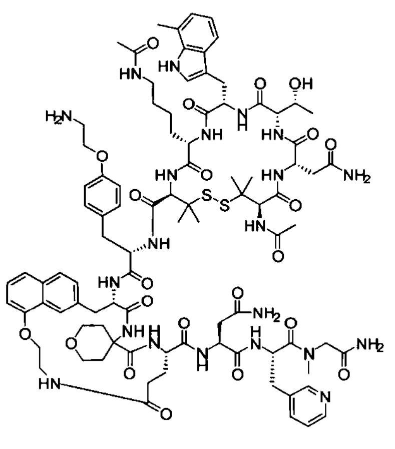

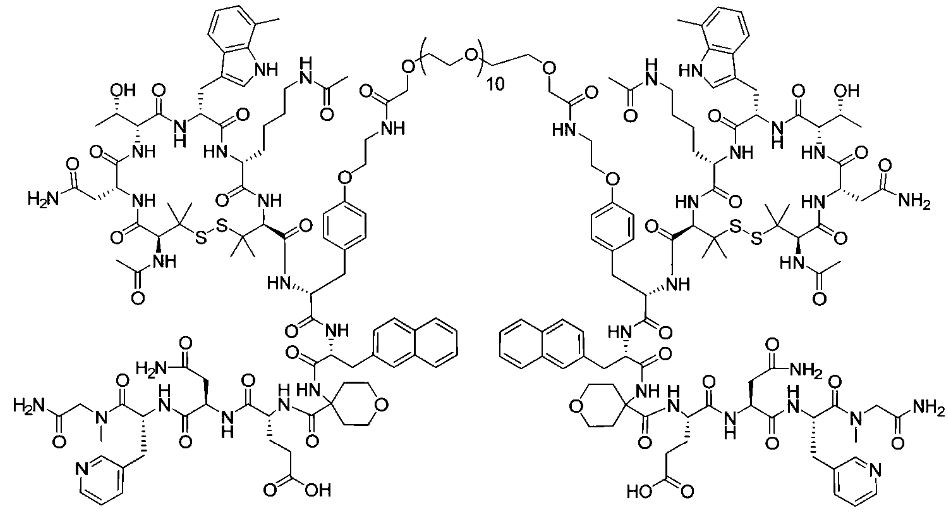

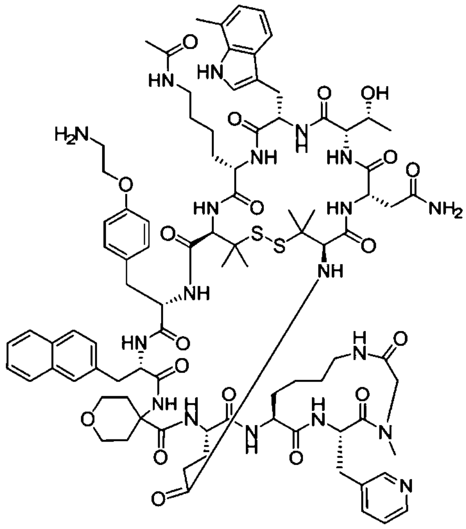

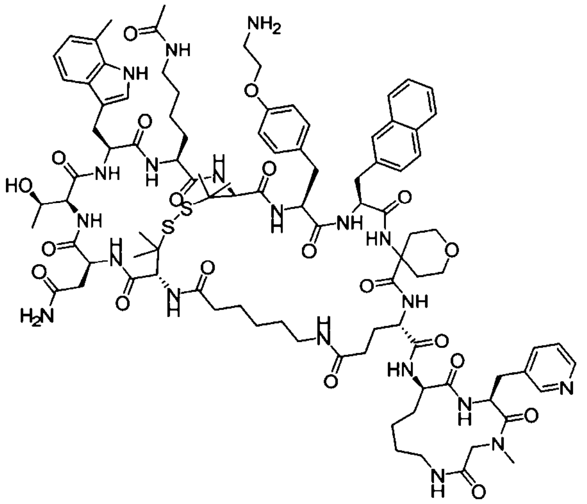

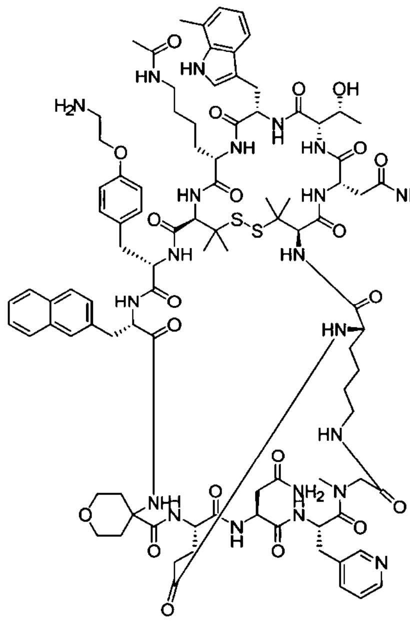

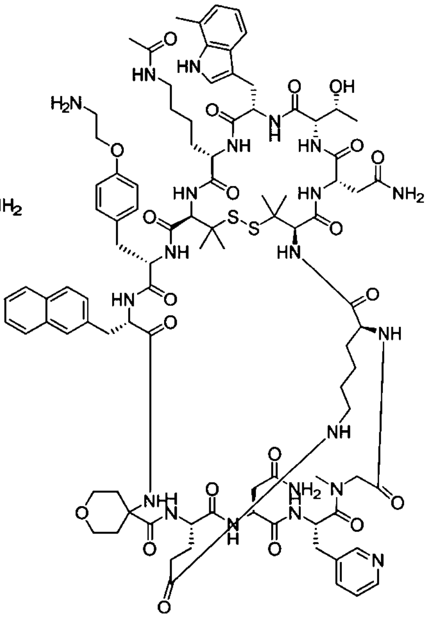

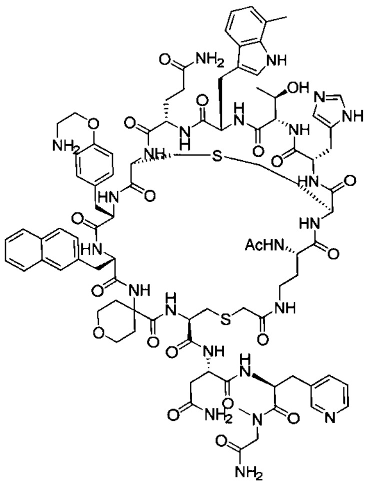

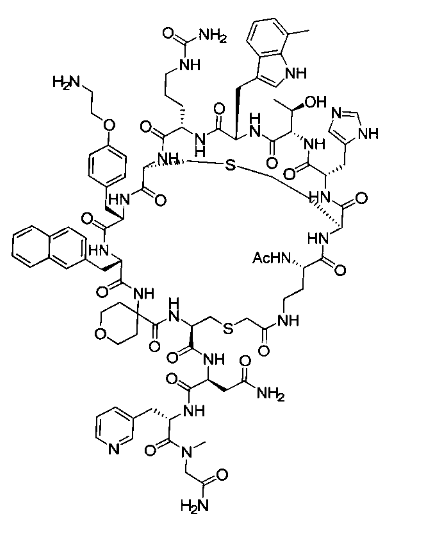

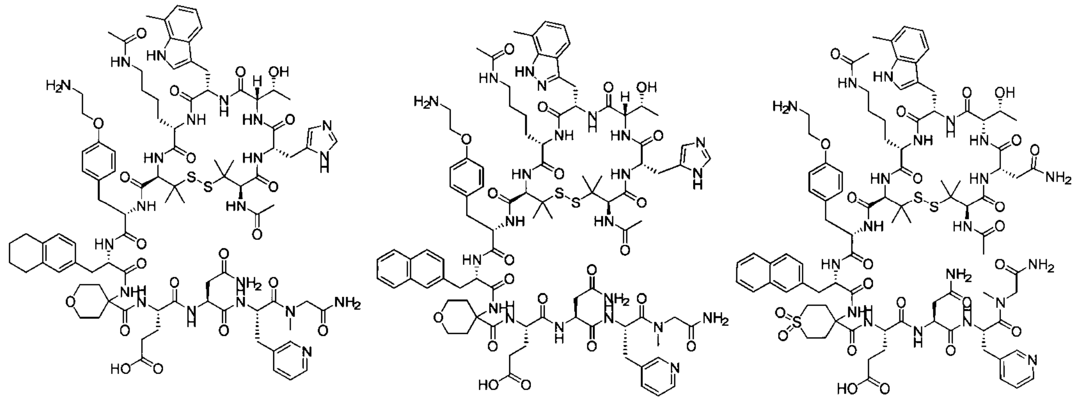

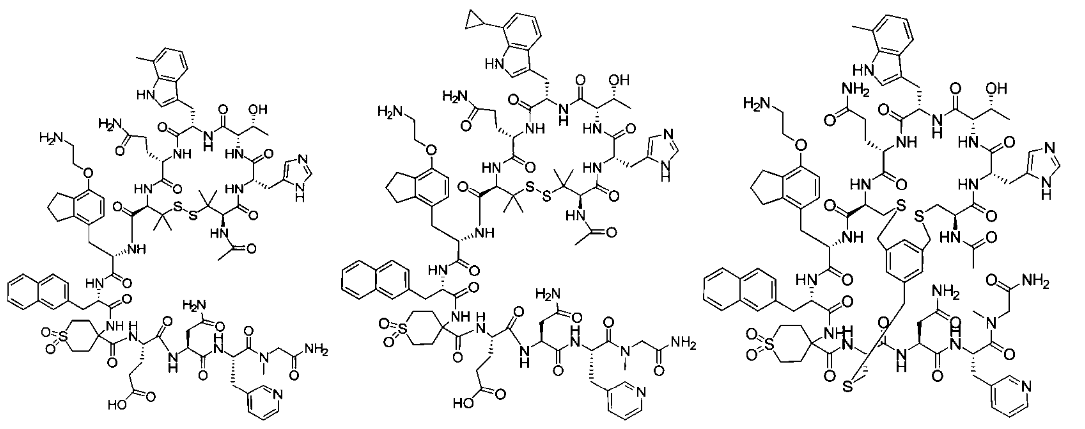

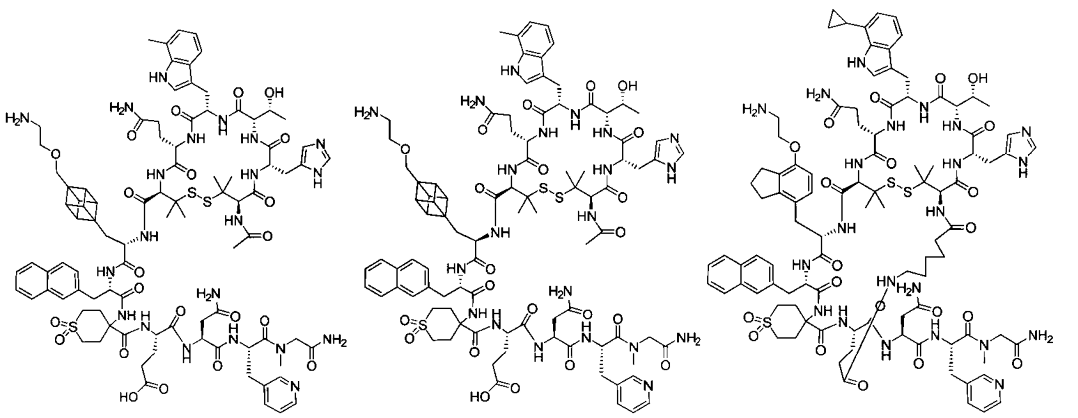

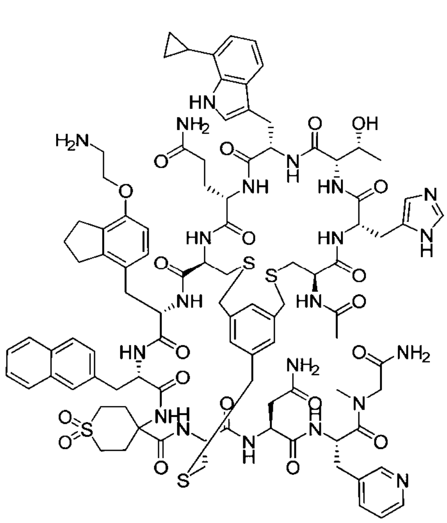

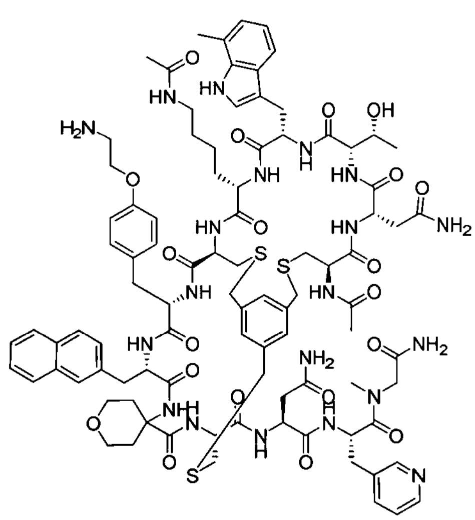

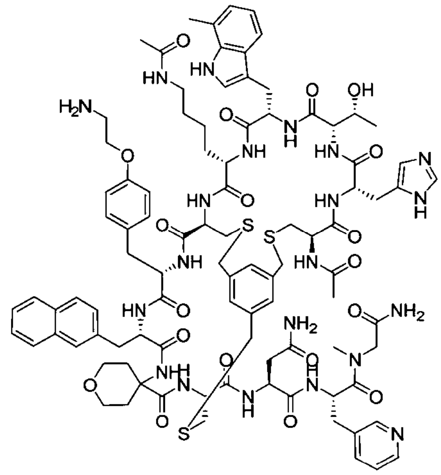

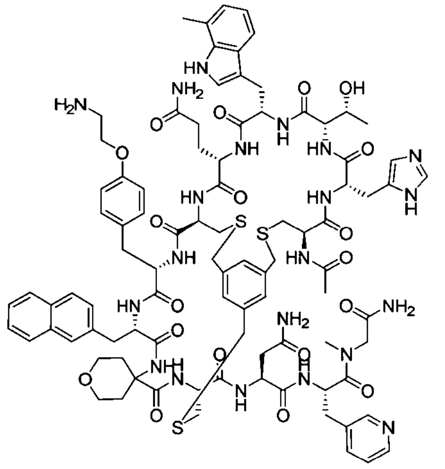

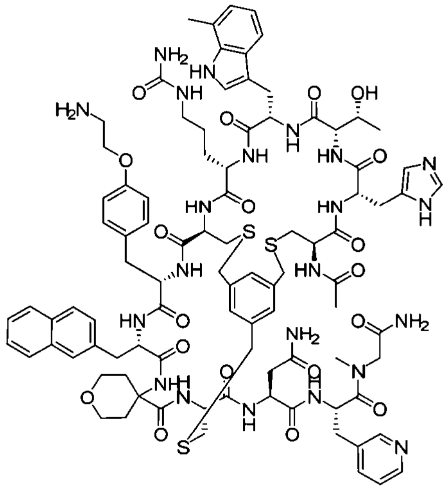

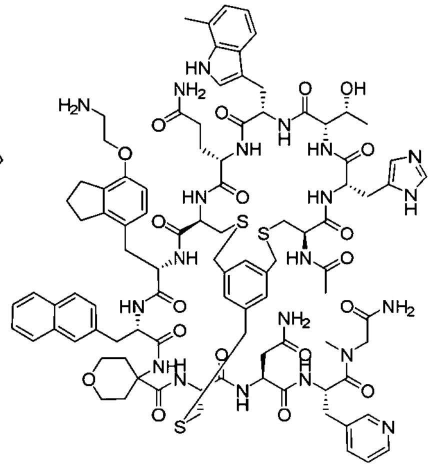

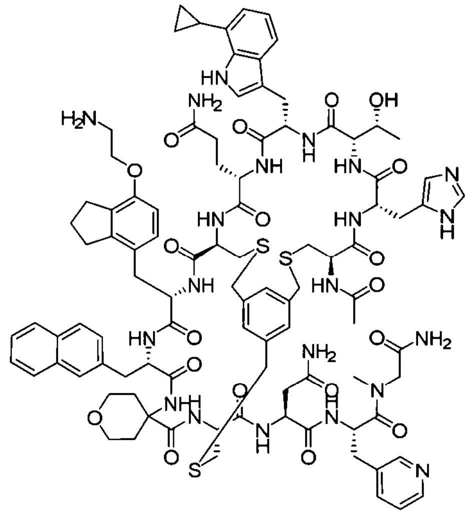

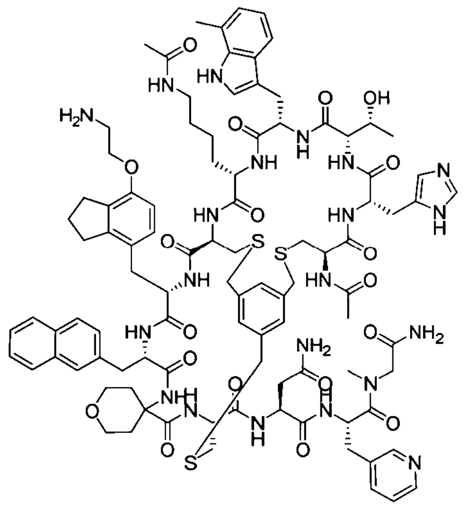

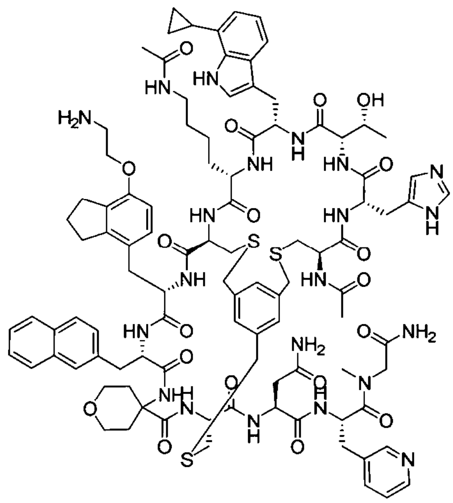

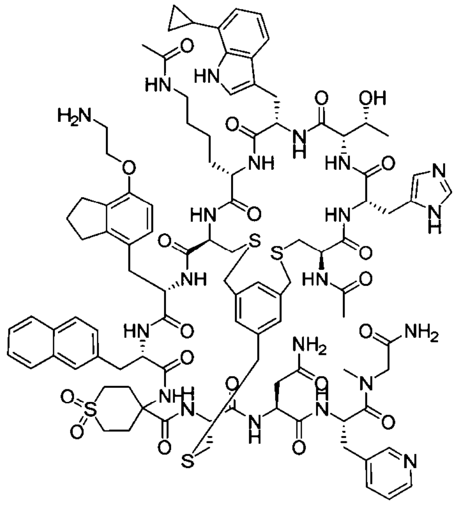

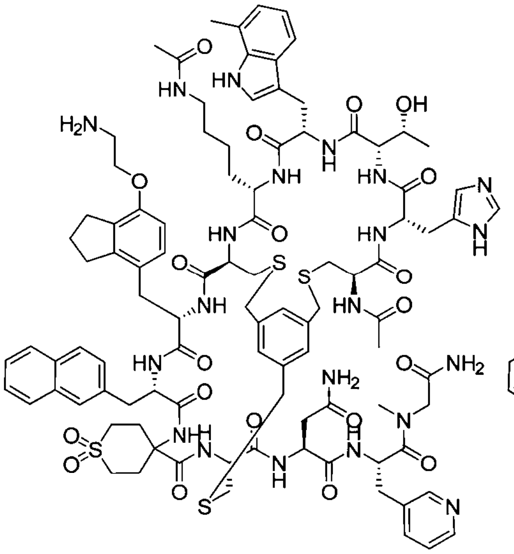

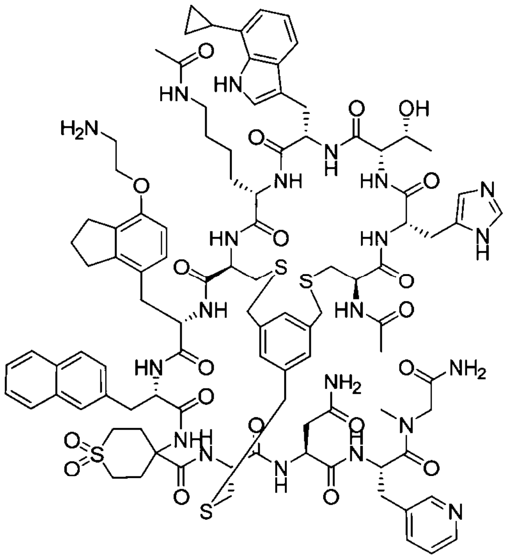

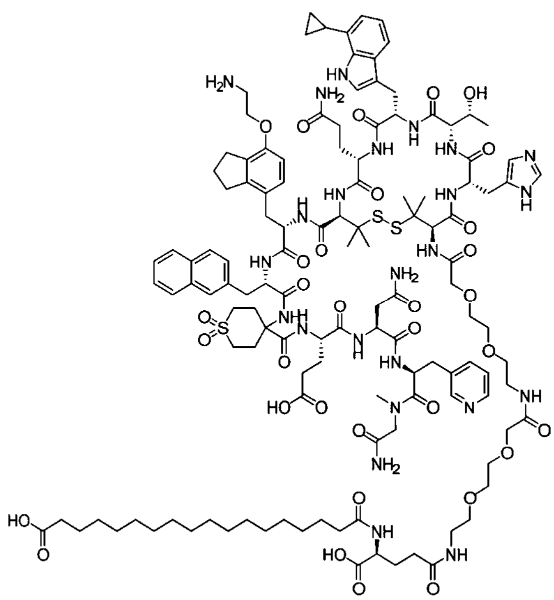

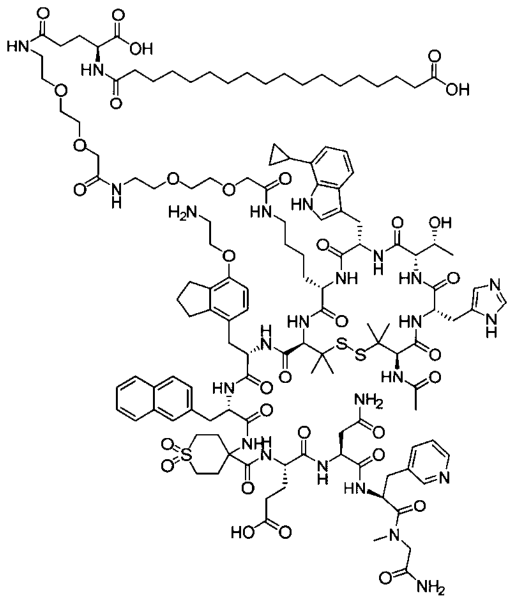

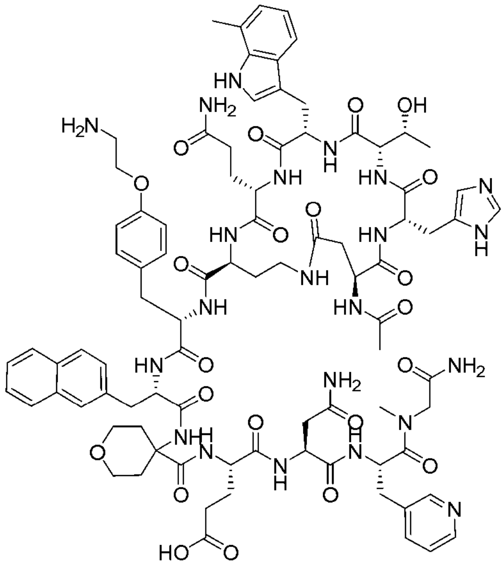

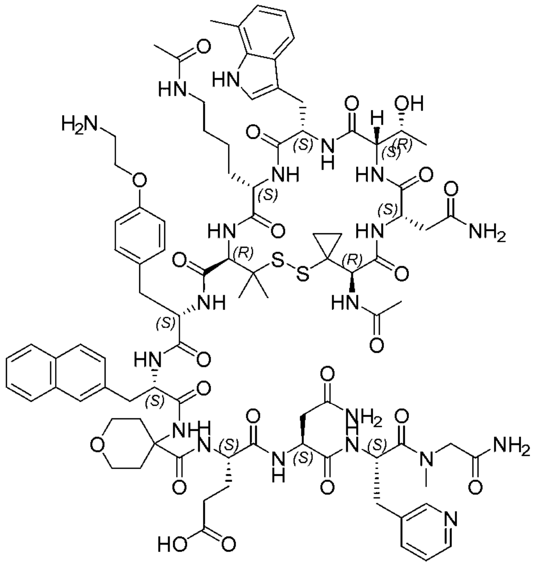

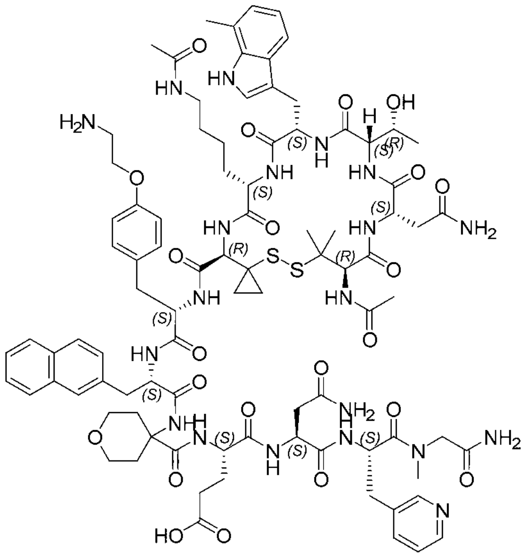

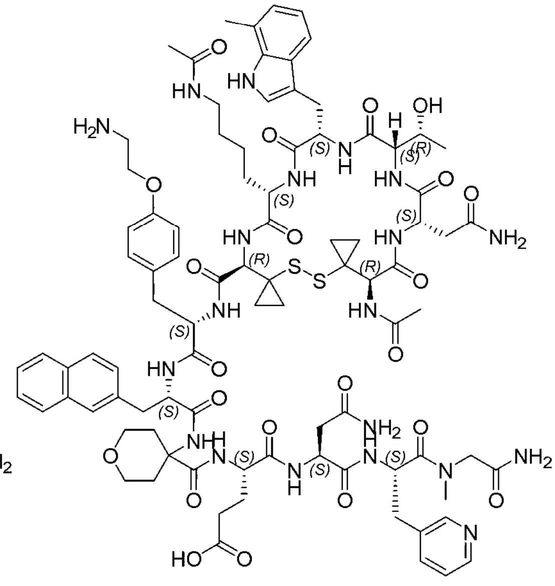

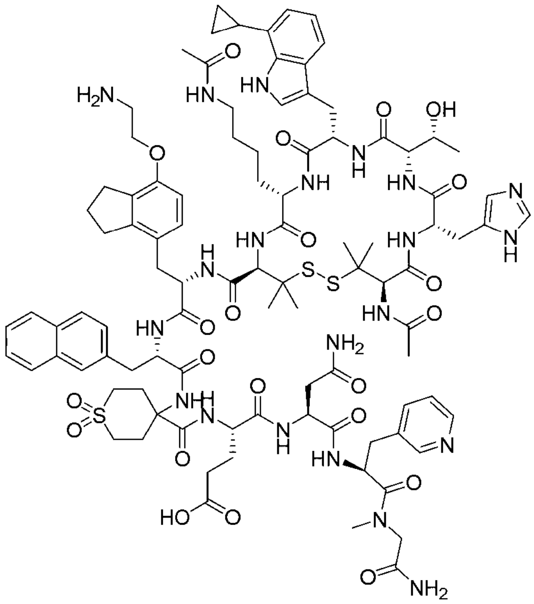

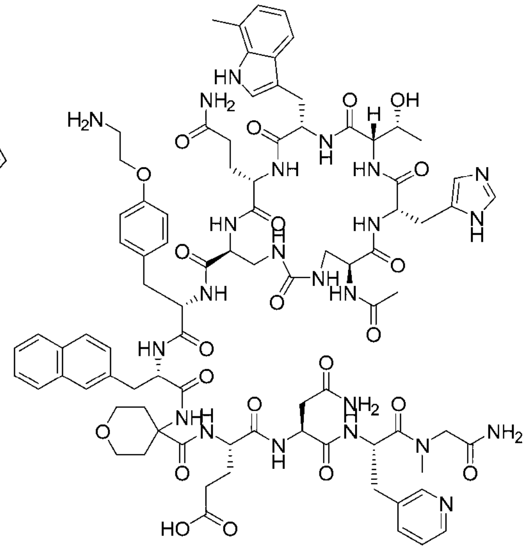

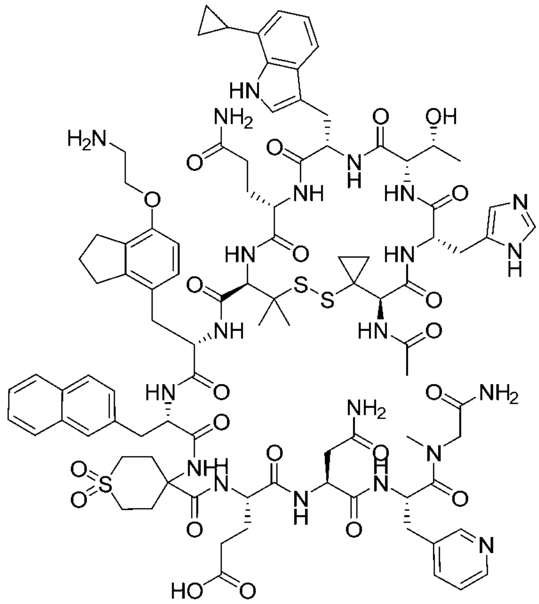

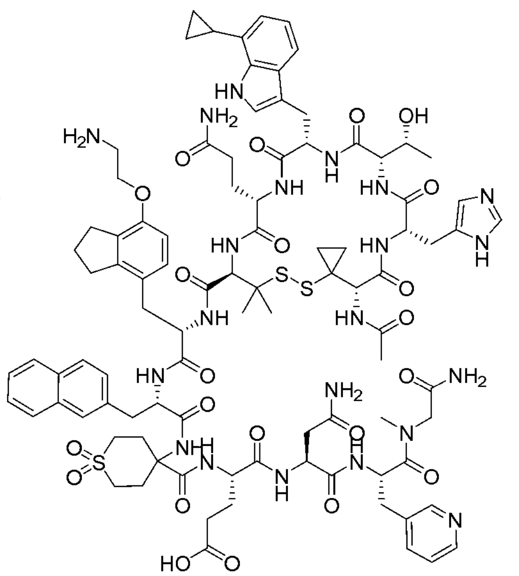

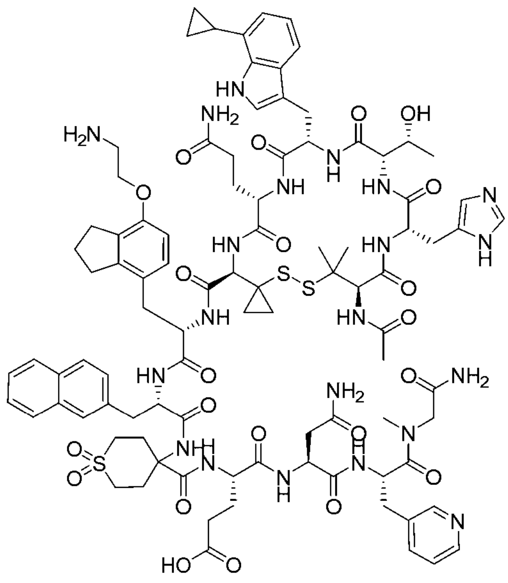

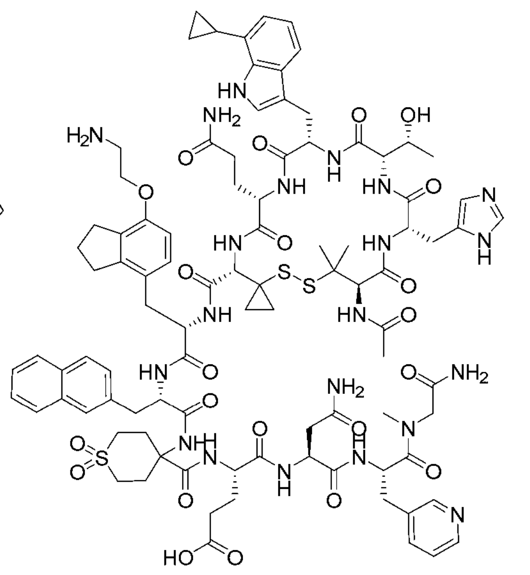

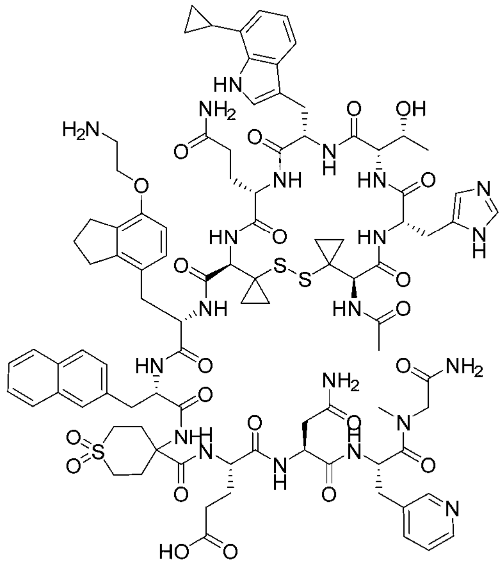

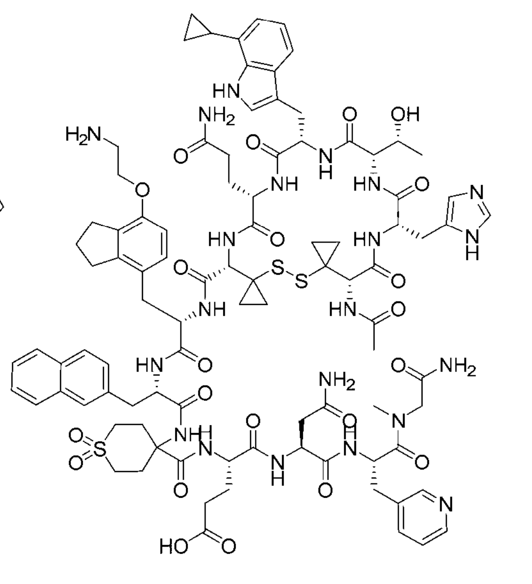

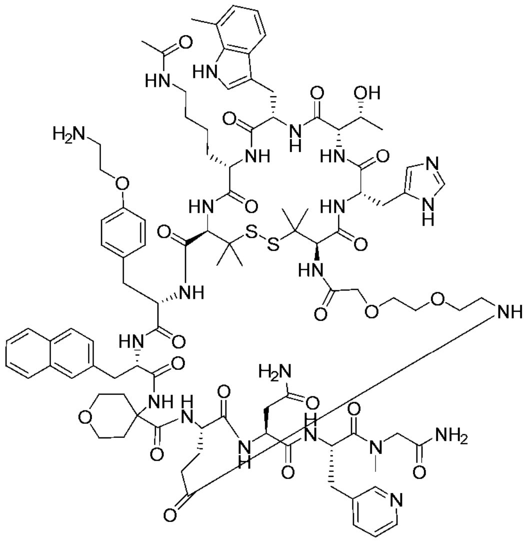

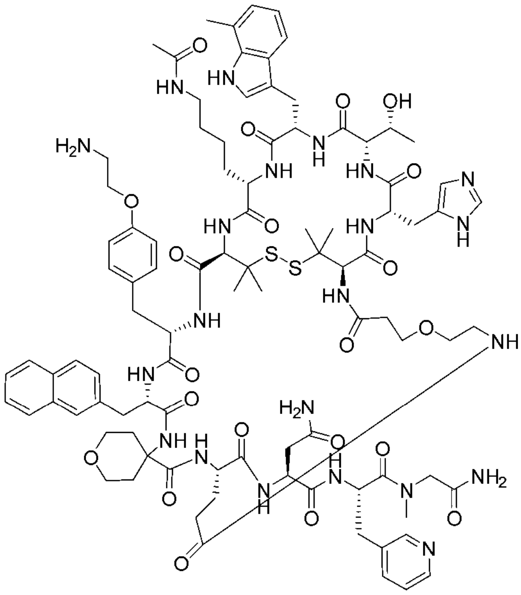

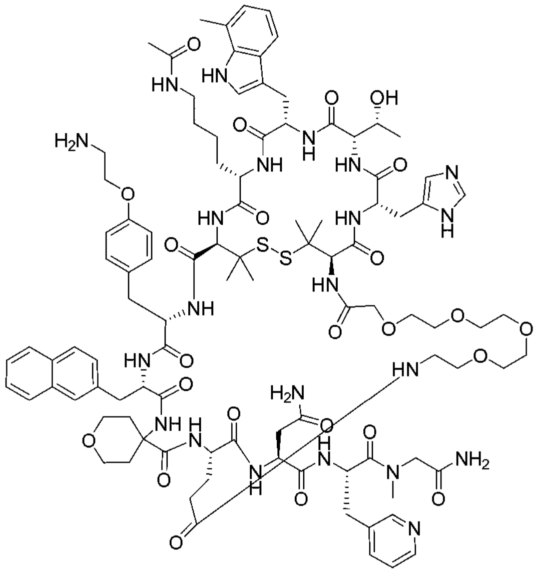

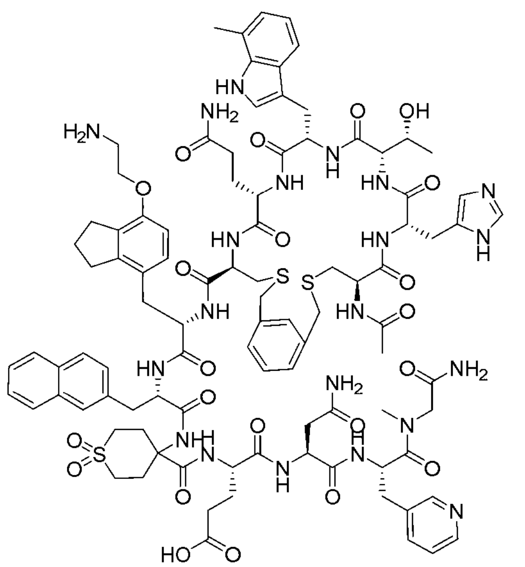

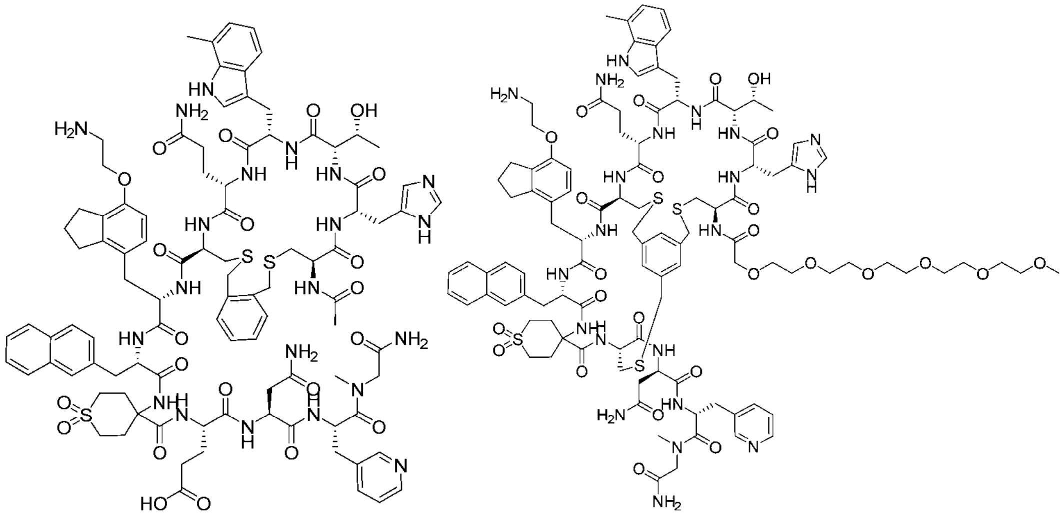

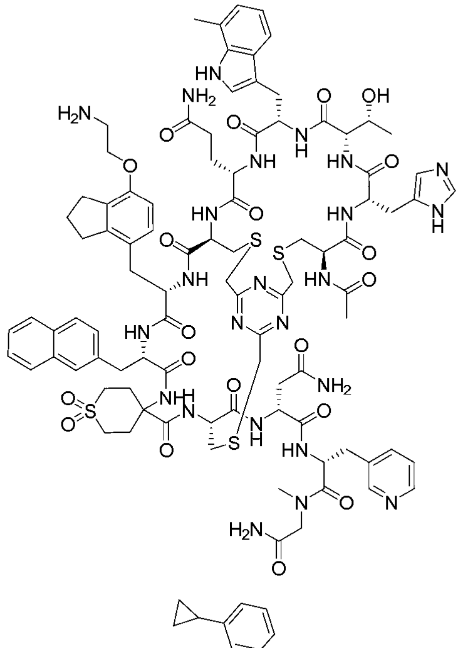
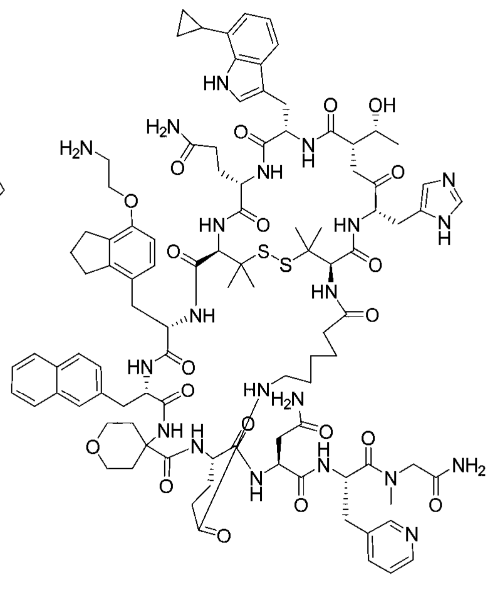

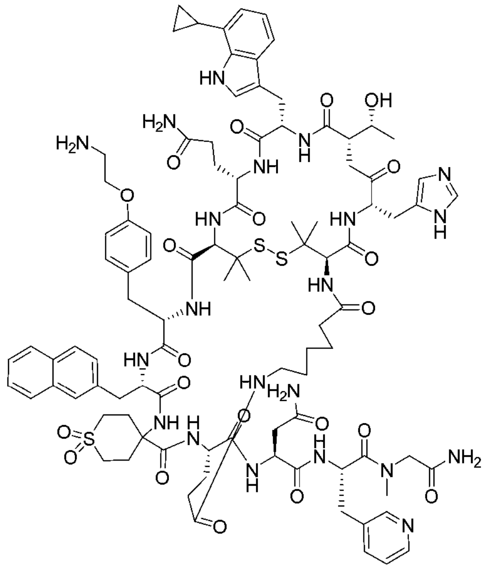
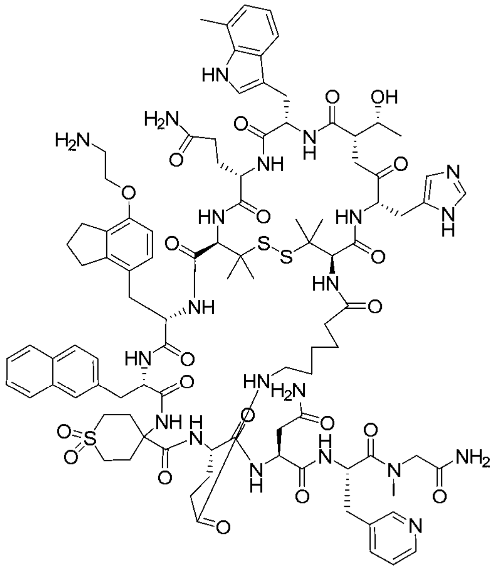

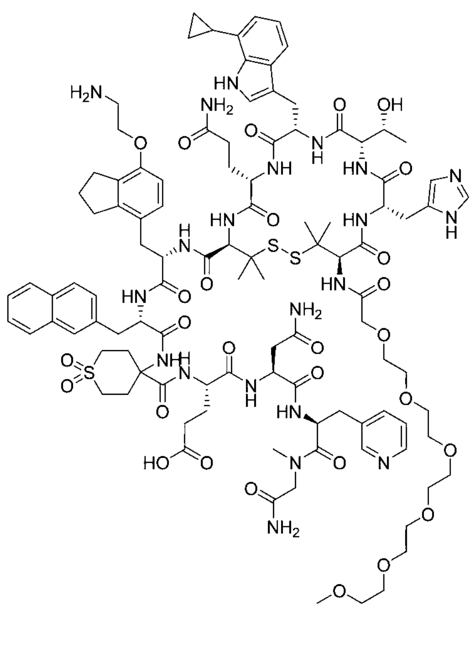
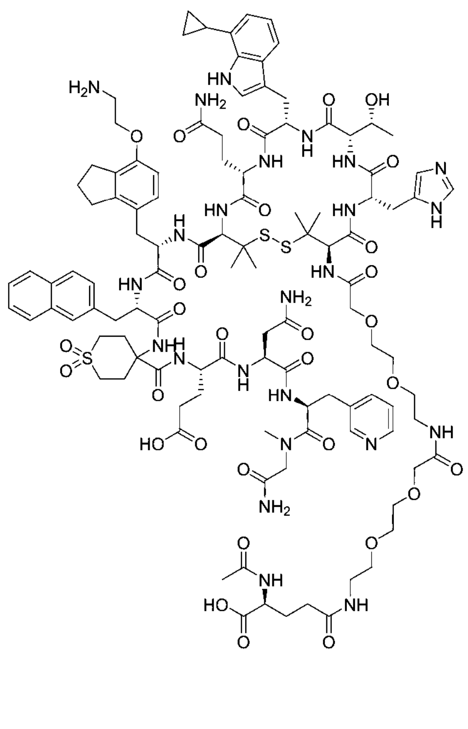

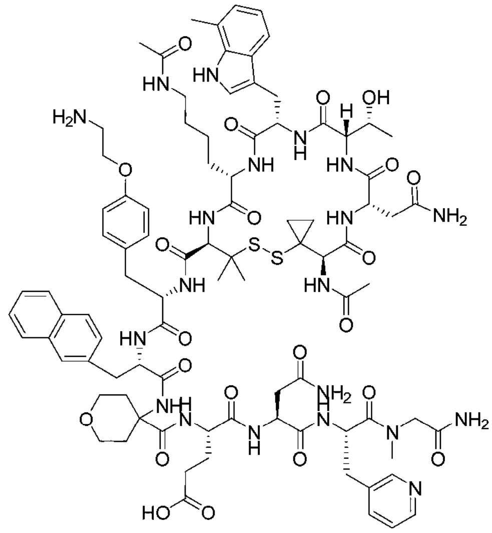
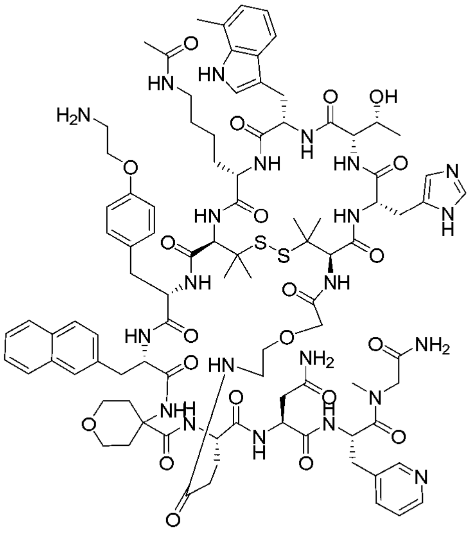

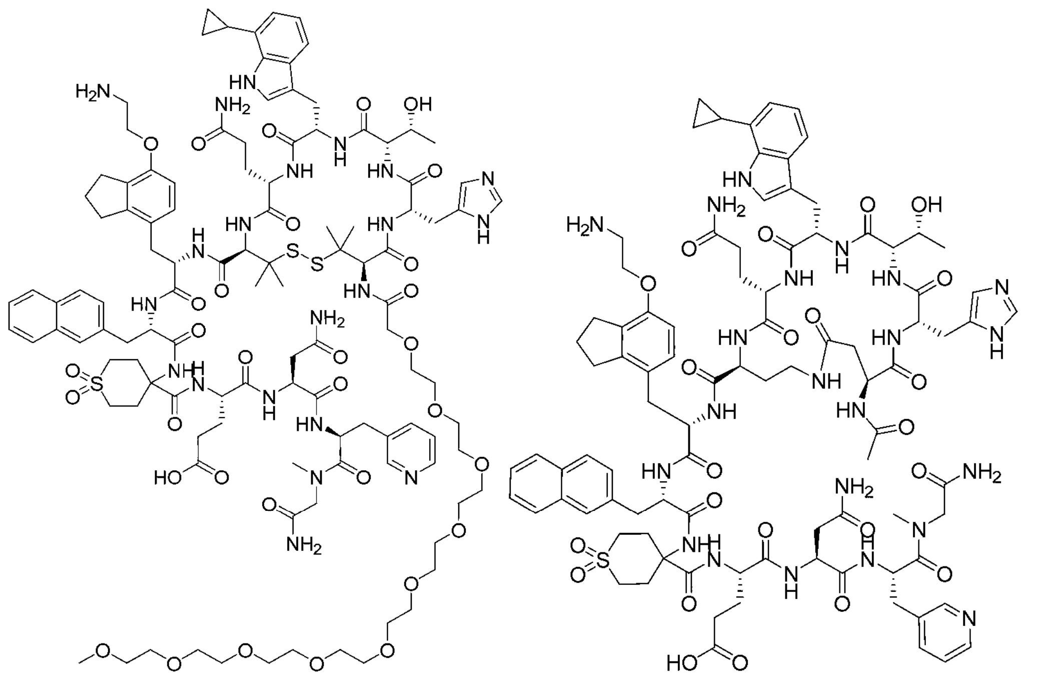

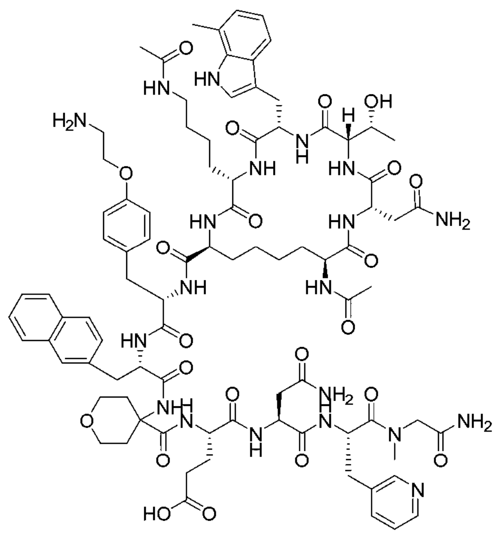
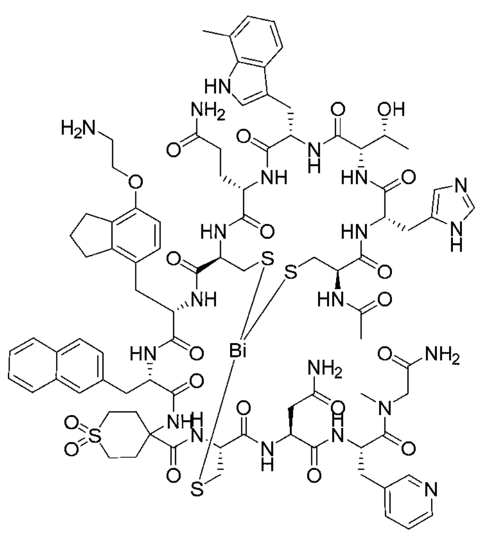

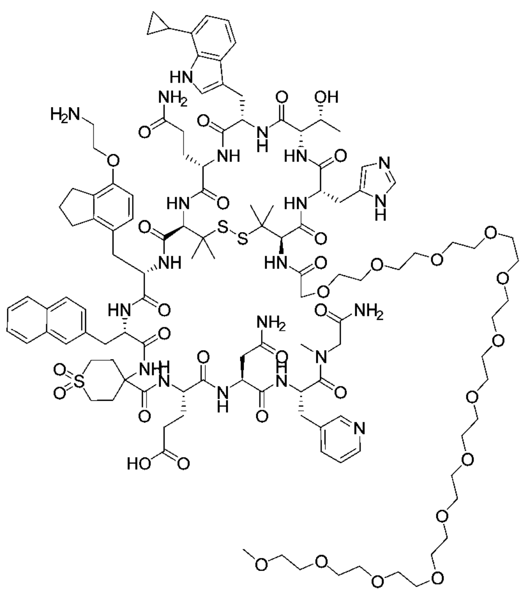

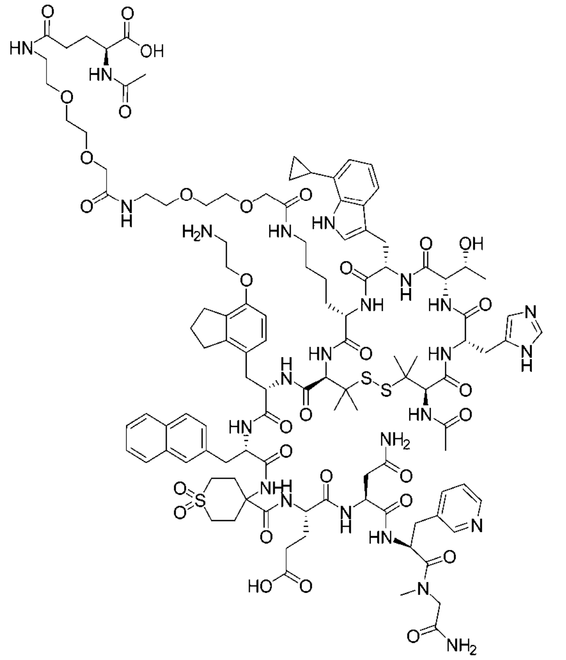

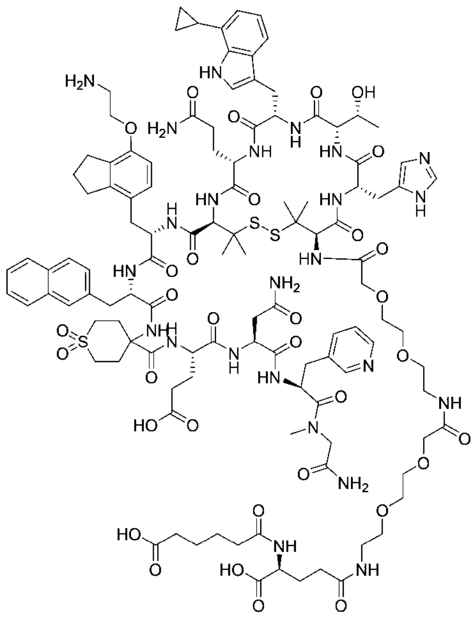

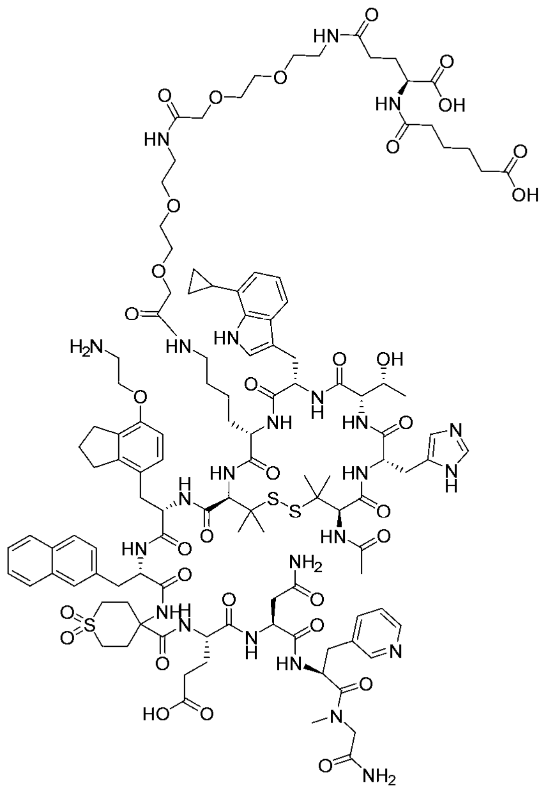

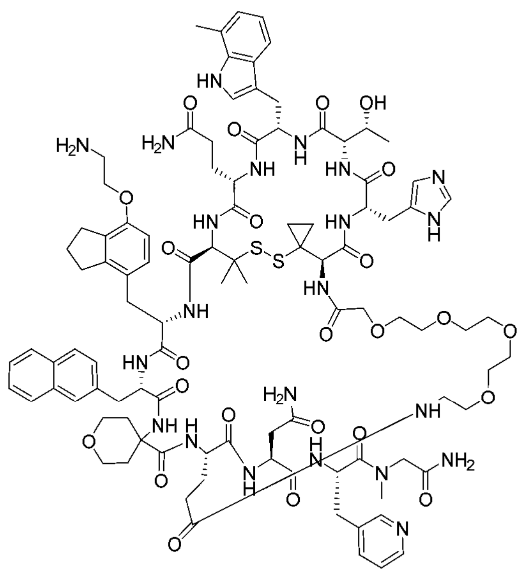 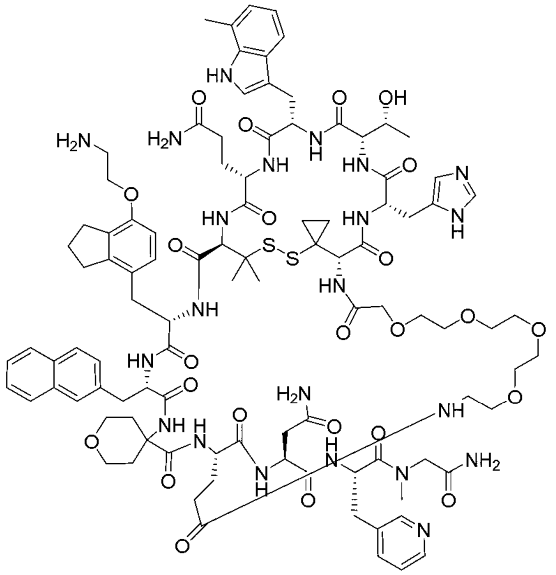

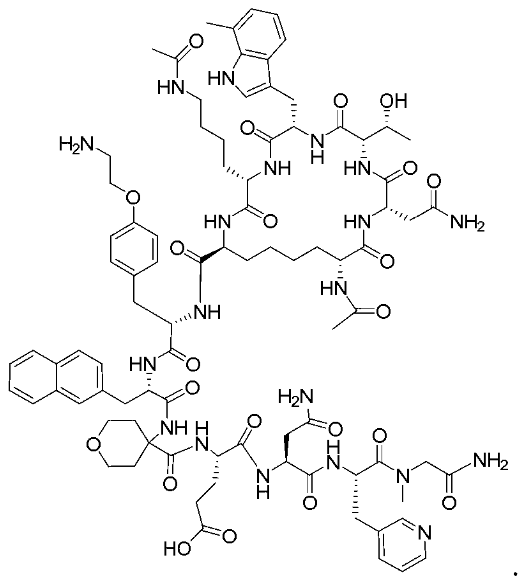.

The present disclosure also relates to a pharmaceutical composition, comprising the peptide compound or the pharmaceutically acceptable salt thereof according to any one of the first to eleventh technical solutions, and a pharmaceutically acceptable carrier and/or excipient.

The present disclosure also relates to use of the peptide compound or the pharmaceutically acceptable salt thereof according to any one of the first to eleventh technical solutions, or the pharmaceutical composition in the preparation of a medicament for preventing and treating an IL-23-overexpressing disease or condition in a diseased tissue of a subject.

Further, the IL-23-overexpressing disease or condition includes inflammatory bowel disease, Crohn's disease, and psoriasis.

The present disclosure also relates to a pharmaceutical composition or a pharmaceutical preparation, comprising 1-1500 mg of the peptide compound or the pharmaceutically acceptable salt thereof according to any one of the first to eleventh technical solutions, and a pharmaceutically acceptable carrier and/or excipient.

The present disclosure also relates to a method for treating a disease in a mammal or a human, comprising administering to a subject a therapeutically effective amount of the peptide compound or the pharmaceutically acceptable salt thereof according to any one of the first to eleventh technical solutions, wherein the therapeutically effective amount is preferably 1-1500 mg, and the disease is preferably inflammatory bowel disease, Crohn's disease and psoriasis.

The present disclosure also provides a composition or a pharmaceutical preparation, containing the peptide compound or the pharmaceutically acceptable salt thereof according to any one of the preceding solutions, and a pharmaceutically acceptable carrier and/or excipient. The pharmaceutical composition can be in a unit preparation form (the unit preparation is also referred to as "preparation specification").

Further, the composition or pharmaceutical preparation of the present disclosure contains 1-1500 mg of the peptide compound or the pharmaceutically acceptable salt thereof according to any one of the preceding solutions, and a pharmaceutically acceptable carrier and/or excipient.

The present disclosure also provides the use of the peptide compound or the pharmaceutically acceptable salt thereof according to any one of the preceding solutions in the preparation of a medicament for preventing and treating an IL-23-overexpressing disease or condition in a diseased tissue of a subject. Further, the IL-23-overexpressing disease or condition includes inflammatory bowel disease, Crohn's disease, and psoriasis.

The present disclosure also provides a method for treating a disease in a mammal or a human, comprising administering to a subject a therapeutically effective amount of the peptide compound or the pharmaceutically acceptable salt thereof according to any one of the preceding solutions, wherein the disease is preferably inflammatory bowel disease, Crohn's disease and psoriasis, and the therapeutically effective amount is preferably 1-1500 mg. In some embodiments, the mammal described in the present disclosure does not include human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the peptide compound or the pharmaceutically acceptable salt thereof disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, and 100-200 mg;

in some embodiments, the pharmaceutical composition or preparation of the present disclosure contains the above therapeutically effective amount of the peptide compound or the pharmaceutically acceptable salt thereof according to the present disclosure;

the present disclosure relates to a pharmaceutical composition or pharmaceutical preparation, comprising a therapeutically effective amount of the peptide compound or the pharmaceutically acceptable salt thereof according to the present disclosure, and a carrier and/or excipient. The pharmaceutical composition can be in a unit preparation form (the amount of the active drug in the unit preparation is also referred to as the "preparation specification"). In some embodiments, the pharmaceutical composition comprises, without limitation, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg or 1500 mg of the peptide compound or the pharmaceutically acceptable salt thereof according to the present disclosure.

Provided is a method for treating a disease in a mammal or a human, comprising administering to a subject a therapeutically effective amount of the peptide compound or the pharmaceutically acceptable salt thereof according to the present disclosure, and a pharmaceutically acceptable carrier and/or excipient, wherein the therapeutically effective amount is preferably 1-1500 mg, and the disease is preferably inflammatory bowel disease, Crohn's disease and psoriasis.

Provided is a method for treating a disease in a mammal or a human, comprising administering to a subject the peptide compound or the pharmaceutically acceptable salt thereof according to the present disclosure, as a drug, and a pharmaceutically acceptable carrier and/or excipient at a daily dose of 1-1500 mg/day, wherein the daily dose may be a single dose or divided doses. In some embodiments, the daily dose includes, but is not limited to, 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800

mg/day, 50-800 mg/day, 100-800 mg/day, 200-800 mg/day, 25-400 mg/day, 50-400 mg/day, 100-400 mg/day, and 200-400 mg/day. In some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 300 mg/day, 400 mg/day, 600 mg/day, 800 mg/day, 1000 mg/day, 1200 mg/day, 1400 mg/day, and 1500 mg/day.

The present disclosure relates to a kit, which may comprise a composition in a single-dose or multi-dose form, wherein the kit comprises the peptide compound or the pharmaceutically acceptable salt thereof according to the present disclosure, and the amount of the compound, or the stereoisomer, or the pharmaceutically acceptable salt thereof according to the present disclosure is the same as that in the above pharmaceutical composition.

In the present disclosure, the amount of the compound, or the stereoisomer, or the pharmaceutically acceptable salt thereof according to the present disclosure is calculated in the form of a free base in each case.

The term "preparation specification" refers to the weight of the active drug contained in each vial, tablet or other unit preparation.

**Terms**

Unless otherwise specified in the present disclosure, the terms of the present disclosure have the following meanings.

The term "peptide" of the present disclosure broadly refers to a sequence in which two or more amino acids are linked together by a peptide bond. It is to be understood that the term neither implies a polymer of amino acids of a particular length, nor is it intended to imply or distinguish whether a polypeptide is produced using recombinant techniques, chemical synthesis, or enzymatic synthesis, or whether it is naturally occurring.

The carbon, hydrogen, oxygen, sulphur, nitrogen and halogen involved in the groups and compounds of the present disclosure all include isotopes thereof, and are optionally further replaced by one or more of the corresponding isotopes thereof, wherein the isotopes of carbon include $^{12}C$, $^{13}C$ and $^{14}C$; the isotopes of hydrogen include protium (H), deuterium (D, also known as heavy hydrogen) and tritium (T, also known as superheavy hydrogen); the isotopes of oxygen include $^{16}O$, $^{17}O$ and $^{18}O$; the isotopes of sulphur include $^{32}S$, $^{33}S$, $^{34}S$ and $^{36}S$; the isotopes of nitrogen include $^{14}N$ and $^{15}N$; the isotope of fluorine includes $^{19}F$; the isotopes of chlorine include $^{35}Cl$ and $^{37}Cl$; and the isotopes of bromine include $^{79}Br$ and $^{81}Br$. Radiolabelled compounds of the compounds disclosed in the present disclosure can be prepared using standard methods known in the art.

The term "dimer" of the present disclosure broadly refers to a peptide comprising two or more monomeric subunits. Certain dimers comprise two DRPs. The dimers of the present disclosure include homodimers and heterodimers. The monomeric subunits of the dimer may be linked at their C-terminus or N-terminus, or they may be linked via internal amino acid residues. The individual monomeric subunits of the dimer may be linked through the same site, or each monomeric subunit may be linked through different sites (e.g., C-terminus, N-terminus, or internal sites).

The term "cyclization" or "formation of a peptide ring" as used herein refers to a reaction in which a portion of a polypeptide molecule is linked to another portion of the polypeptide molecule to form a closed ring, or a portion of a polypeptide molecule is linked to other several portions of the polypeptide molecule to form a plurality of closed rings, such as by forming a disulphide bridge or other similar bond, or linking through a linker.

The term "Ac" of the present disclosure refers to "acetyl".

The term "derivative" or "analogue" herein refers to a product derived from the substitution of hydrogen atoms or atomic groups in a compound by other atoms or atomic groups. It is to be understood that amino acid analogues of peptide compounds as defined herein are within the scope of the present disclosure. Examples of such suitable modified amino acid derivatives comprise one or more modifications selected from: modification to the N-terminus and/or C-terminus; replacement of one or more amino acid residues with one or more non-natural amino acid residues (e.g., replacement of one or more polar amino acid residues with one or more isosteric or isoelectronic amino acids; and replacement of one or more nonpolar amino acid residues with other non-natural isosteric or isoelectronic amino acids); addition of a spacer group; replacement of one or more oxidation-sensitive amino acid residues with one or more oxidation-tolerant amino acid residues; replacement of one or more amino acid residues with alanine, and replacement of one or more L-amino acid residues with one or more D-amino acid residues; N-alkylation of one or more amide bonds in a bicyclic peptide ligand; replacement of one or more peptide bonds with a surrogate bond; modification to the peptide backbone length; replacement or substitution of hydrogen on the $\alpha$-carbon of one or more amino acid residues with another chemical group; modification of amino acids such as glycine, alanine, phenylalanine, cysteine, lysine, glutamic/aspartic acid, tyrosine, etc. with suitable amine-, thiol-, carboxylic acid-, and phenol-reactive reagents to functionalize the amino acid; and introduction or replacement of amino acids to introduce orthogonal reactivity suitable for functionalization, for example, amino acids carrying an azide or alkyne group can be functionalized with a moiety carrying an alkyne or azide, respectively.

Unless otherwise specified, all the amino acids are used in the L-configuration.

The names of some common amino acids, along with their three-letter abbreviations and single-letter abbreviations, are shown in the table below.

| Name of amino acid | Three-letter abbreviation | Single-letter abbreviation | Name of amino acid | Three-letter abbreviation | Single-letter abbreviation |
|---|---|---|---|---|---|
| Cysteine | Cys | C | Naphthylalanine | 1Nal | / |
| Homocysteine | Heys | / | Aspartic acid | Asp | D |
| Hydroxyproline | Hyp | / | Glutamic acid | Glu | E |
| Proline | Pro | P | Tryptophan | Trp | W |
| Alanine | Ala | A | Arginine | Arg | R |
| β-alanine | β-Ala | / | Serine | Ser | S |
| Sarcosine | Sarc | / | Threonine | Thr | T |
| Methionine | Met | M | Phenylalanine | Phe | F |
| Asparagine | Asn | N | Tyrosine | Tyr | Y |
| Leucine | Leu | L | Histidine | His | H |
| Valine | Val | V | Isoleucine | Ile | I |
| Lysine | Lys | K | Glycine | Gly | G |
| Citrulline | Cit | / | Glutamine | Gln | Q |

2-Nal :

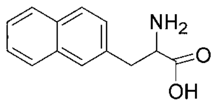
;

3-Pal :

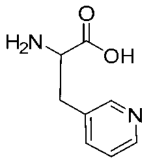
;

Thp:

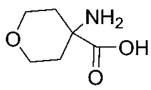
;

Pen:

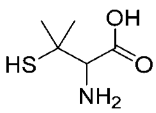
;

Pcn:

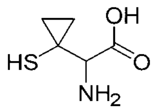
;

Ala(3-amino):

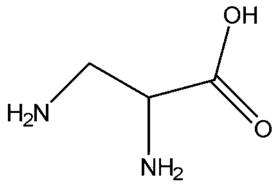
;

Ala(2-ethyne):

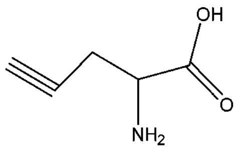
;

Ala(3-azido):

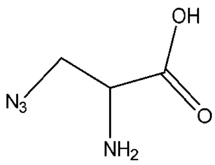
;

Ala(2-ethene):

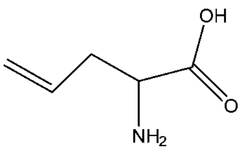
;

Val(2-ethene):

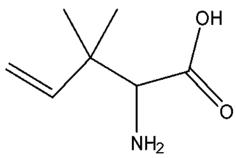
;

2,4-diaminobutyric acid:

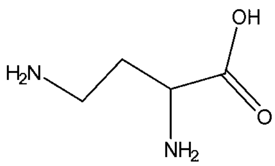
;

The term "pharmaceutically acceptable salt" refers to a salt of the compound of the present disclosure, which salt maintains the biological effectiveness and characteristics of a free acid or a free base and is obtained by reacting the free

acid with a non-toxic inorganic base or organic base, or reacting the free base with a non-toxic inorganic acid or organic acid.

The term "pharmaceutical composition" represents a mixture of one or more compounds or stereoisomers, solvates, pharmaceutically acceptable salts or eutectics thereof as described herein and other components including physiologically/pharmaceutically acceptable carriers and/or excipients.

The term "carrier" refers to a system that does not cause significant irritation to the organism and does not eliminate the biological activity and characteristics of the administered compound and can change the way the drug enters the human body and the distribution of the drug in the body, control the release rate of the drug and deliver the drug to targeted organs. Non-limiting examples of the carrier include microcapsule, microsphere, nanoparticle, liposome, etc.

The term "excipient" refers to a substance that is not a therapeutic agent per se, but used as a diluent, adjuvant, binder and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, pharmaceutically acceptable excipients can provide various functions and can be described as a wetting agent, a buffer, a suspending agent, a lubricant, an emulsifier, a disintegrant, an absorbent, a preservative, a surfactant, a colourant, a flavouring agent, and a sweetening agent. Examples of pharmaceutically acceptable excipients include, but are not limited to: (1) sugars, such as lactose, glucose, and sucrose; (2) starches, such as corn starch and potato starch; (3) cellulose and derivatives thereof, such as sodium carboxymethyl cellulose, ethyl cellulose, cellulose acetate, hydroxypropyl methylcellulose, hydroxypropyl cellulose, microcrystalline cellulose, and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffer solutions; (21) polyesters, polycarbonates and/or polyanhydrides; and (22) other non-toxic compatible substances used in a pharmaceutical preparation.

## Detailed Description of Embodiments

The content of the present disclosure is described in detail by means of the following examples. In examples in which no specific conditions are indicated, experimental methods are carried out under conventional conditions. The listed examples are intended to better illustrate the content of the present disclosure but should not be construed as limiting the content of the present disclosure. Non-essential improvements and adjustments made to the embodiments by a person of ordinary skill in the art according to the above Summary of the Invention still fall within the scope of protection of the present disclosure.

### Detection method

The structures of compounds are determined by mass spectrometry (MS).

MS is determined with Agilent 6120B (ESI) and Agilent 6120B (APCI); and
HPLC is determined with Agilent 1260DAD high pressure liquid chromatograph (Zorbax SB-C18 100 × 4.6 mm, 3.5 μM).

### Description of abbreviations:

DCM: dichloromethane
DMF: N,N-dimethylformamide
DIEA: N,N-diisopropylethylamine
MeOH: methanol
TFA: trifluoroacetic acid
DMSO: dimethylsulfoxide
DIC: N,N'-diisopropylcarbodiimide
HOBT: 1-hydroxybenzotriazole
HOAT: N-hydroxy-7-azabenzotriazole

## Intermediate 1:

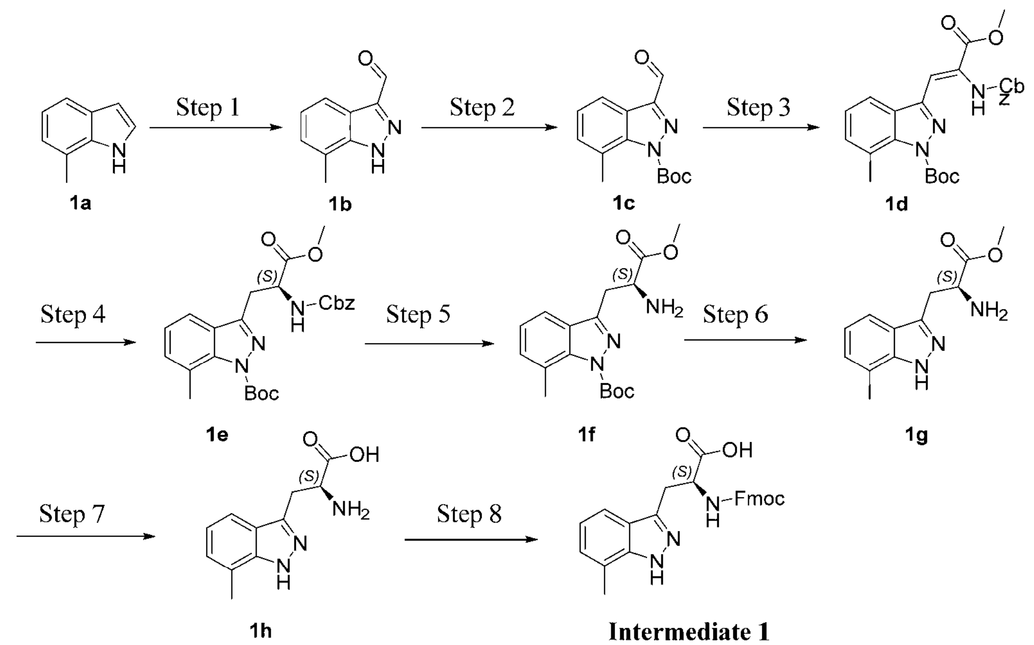


Step 1: Sodium nitrite (42.14 g, 610.7 mmol) was added to DMF (300 mL) and water (400 mL), and hydrochloric acid (2 mol/L, 103 mL) was added dropwise at 0°C after nitrogen replacement. Then, **Compound 1a** (10 g, 76.3 mmol) was dissolved in DMF (300 mL), and the mixture was added dropwise to the reaction solution over 2 h. The reaction was moved to room temperature, and stirred overnight. The starting materials were completely consumed as detected by thin-layer chromatography. Water (2 L) was added, and the mixture was extracted with ethyl acetate (500 mL x 3). The organic phases were combined, spin-dried, and separated and purified by a silica gel chromatography column (EA : PE = 5 : 1) to give **Compound 1b** (7.4 g, 60.6%).

LC-MS (ESI): m/z= 161.0 $[M+H]^+$.

Step 2: **Compound 1b** (7.4 g, 45.9 mmol) was dissolved in dichloromethane (100 mL), and triethylamine (7 g, 68.9 mmol) and di-tert-butyl dicarbonate (12 g, 55.2 mmol) were added. The reaction was stirred overnight, then diluted with dichloromethane (100 mL), and washed with water (100 mL) three times. Then, the organic phase was concentrated to give **Compound 1c** (10 g, 83.2%).

LC-MS (ESI): m/z= 261.0 $[M+H]^+$.

Step 3: (±)-benzyloxycarbonyl-a-phosphonoglycine trimethyl ester (14 g, 42.3 mmol) was dissolved in dichloromethane (100 mL), DBU (6.43 g, 42.3 mmol) was added after nitrogen replacement, and the mixture was stirred for 30 min. Then, **Compound 1c** (10 g, 38.4 mmol) was dissolved in dichloromethane (100 mL), and the mixture was added dropwise to the reaction. The reaction was continued to be stirred overnight. The reaction mixture was diluted with dichloromethane (100 mL), washed with 5% aqueous citric acid solution (100 mL) and saturated brine (100 mL), then dried over anhydrous sodium sulphate, filtered, and spin-dried. Separation and purification by a silica gel chromatography column (EA : PE = 3 : 1) afforded **Compound 1d** (12 g, 67.1%).

LC-MS (ESI): m/z= 466.2 $[M+H]^+$.

Step 4: **Compound 1d** (5 g, 10.74 mmol) was dissolved in a mixed solution of methanol (50 mL) and dichloromethane (20 mL), and (+)-1,2-bis(2S,5S)-2,5-diethylphospholanobenzene(cyclooctadiene)rhodium trifluoromethanesulfonate (0.5 g, 0.69 mmol) was added. The pressure was increased to about 4.0 bar by filling with hydrogen using an autoclave, and the mixture was stirred at room temperature for 3 h. The solid was filtered off, and the reaction solution was concentrated to give **Compound 1e** (5 g, 99.8%).

LC-MS (ESI): m/z= 468.2 $[M+H]^+$.

Step 5: **Compound 1e** (5 g, 10.69 mmol) was dissolved in a mixed solution of methanol (50 mL) and dichloromethane (20 mL), and palladium on carbon (10%, 1 g) was added. The mixture was stirred overnight under hydrogen atmosphere. Then, the solid was filtered off, and the reaction solution was spin-dried to give **Compound 1f** (3.2 g, 89.7%).

LC-MS (ESI): m/z= 334.2 $[M+H]^+$.

Step 6: **Compound 1f** (3.2 g, 9.60 mmol) was added to dichloromethane (30 mL), and trifluoroacetic acid (10 mL) was added dropwise. The reaction was stirred at room temperature for 2 h. The starting materials were completely consumed as monitored by LCMS. The resultant was spin-dried directly to give crude **Compound 1g,** which was used

directly in the next reaction without purification.

LC-MS (ESI): m/z= 234.1 $[M+H]^+$.

Step 7: **Compound 1g** (2.2 g, 9.43 mmol) was dissolved in tetrahydrofuran (20 mL), and lithium hydroxide monohydrate (1.58 g, 37.7 mmol) and water (20 mL) were added. The reaction was stirred overnight at room temperature, and extracted with ethyl acetate (10 mL x 3) to remove impurities. Then, the pH of the aqueous phase was adjusted to neutral with dilute hydrochloric acid (1N), and the product was precipitated, filtered, and dried to give **Compound 1h** (2 g, 96.7%).

LC-MS (ESI): m/z= 220.1 $[M+H]^+$.

Step 8: **Compound 1h** (2 g, 9.12 mmol) was dissolved in a mixed solution of acetonitrile (20 mL) and water (20 mL), and sodium bicarbonate (3.83 g, 45.6 mmol) and 9-fluorenylmethyl N-succinimidyl carbonate (4.6 g, 13.7 mmol) were added. The reaction was stirred overnight at room temperature. After the reaction was completed, the pH was adjusted to neutral by dropwise adding dilute hydrochloric acid, and most acetonitrile was spun off by vacuum concentration at 40°C. The solid was collected by filtration to give the crude product, which was separated and purified by a silica gel chromatography column (DCM : MeOH = 10 : 1) to give **Intermediate 1** (2.5 g, 62.1%).

LC-MS (ESI): m/z= 442.2 $[M+H]^+$.

$^1H$ NMR (400 MHz, DMSO-$d_6$) δ 12.81 (s, 1H), 7.87 (d, 2H), 7.68 - 7.57 (m, 3H), 7.54 - 7.43 (m, 1H), 7.42 - 7.35 (m, 2H), 7.32 - 7.22 (m, 2H), 7.06 (d, 1H), 6.99 - 6.90 (m, 1H), 4.44 - 4.34 (m, 1H), 4.21 - 3.93 (m, 3H), 3.43 - 3.25 (m, 2H), 2.47 (s, 3H).

## Intermediate 2:

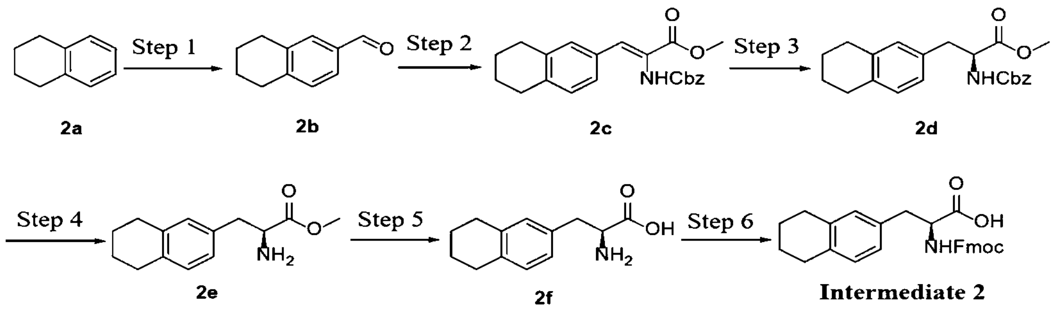


2a 2b 2c 2d

2e 2f Intermediate 2

Step 1: **Compound 2a** (10.0 g, 75.65 mmol) was dissolved in dichloromethane (100 mL), $SnCl_4$ (23.7 g, 90.78 mmol) was added dropwise to the above reaction solution, and the temperature was lowered to 0°C. After 5 min, 1,1-dichlorodimethyl ether (9.6 g, 83.22 mmol) was added dropwise, and the mixture was stirred at 0°C for 3 h. After the reaction was completed, 200 mL of water was added to the reaction system. The resulting solution was extracted with dichloromethane, dried over anhydrous sodium sulphate, concentrated under reduced pressure, and subjected to column chromatography (EA : PE = 1 : 50) to give **Compound 2b** (4.1 g, 33.8%).

LC-MS (ESI): m/z= 161.1 $[M+H]^+$.

Step 2: (±)-benzyloxycarbonyl-a-phosphonoglycine trimethyl ester (9.9 g, 29.93 mmol) was dissolved in dichloromethane (100 mL), DBU (4.9 g, 32.42 mmol) was added after nitrogen replacement, and the mixture was stirred for 30 min. Then, **Compound 2b** (4.0 g, 24.94 mmol) was dissolved in dichloromethane (100 mL), and the mixture was added dropwise to the reaction. The reaction was stirred overnight. The reaction mixture was diluted with dichloromethane (100 mL), washed with 5% aqueous citric acid solution (100 mL) and saturated brine (100 mL), then dried over anhydrous sodium sulphate, filtered, and spin-dried. Separation and purification by a silica gel chromatography column (EA : PE = 5 : 1) afforded **Compound 2c** (6.1 g, 66.9%).

LC-MS (ESI): m/z= 366.2 $[M+H]^+$.

Step 3: **Compound 2c** (6.0 g, 16.42 mmol) was dissolved in a mixed solution of methanol (50 mL) and dichloromethane (20 mL), and (+)-1,2-bis(2S,5S)-2,5-diethylphospholanobenzene(cyclooctadiene)rhodium trifluoromethanesulfonate (0.6 g, 0.84 mmol) was added. The pressure was increased to about 4.0 bar by filling with hydrogen using an autoclave, and the mixture was stirred at room temperature for 3 h. The solid was filtered off, and the reaction solution was concentrated to give **Compound 2d** (5.9 g, 98%).

LC-MS (ESI): m/z= 368.2 $[M+H]^+$.

Step 4: **Compound 2d** (5.9 g, 9.08 mmol) was dissolved in a mixed solution of methanol (50 mL) and dichloromethane (20 mL), and palladium on carbon (10%, 1 g) was added. The mixture was stirred overnight under hydrogen atmosphere. Then, the solid was filtered off, and the reaction solution was spin-dried to give **Compound 2e** (3.5 g, 93.4%).

LC-MS (ESI): m/z= 234.1 $[M+H]^+$.

Step 5: **Compound 2e** (3.5 g, 15.00 mmol) was dissolved in tetrahydrofuran (35 mL), and lithium hydroxide monohydrate (1.4 g, 59.71 mmol) and water (25 mL) were added. The reaction was stirred overnight at room temperature, and extracted with ethyl acetate (20 mL x 3) to remove impurities. Then, the pH of the aqueous phase was adjusted to neutral with dilute hydrochloric acid (1N), and the product was precipitated, filtered, and dried to give **Compound 2f** (2.8 g, 85.1%).

LC-MS (ESI): m/z= 220.1 $[M+H]^+$.

Step 6: **Compound 2f** (2.7 g, 12.31 mmol) was dissolved in a mixed solution of acetonitrile (25 mL) and water (25 mL), and sodium bicarbonate (10.3 g, 123.10 mmol) and 9-fluorenylmethyl N-succinimidyl carbonate (5.0 g, 14.77 mmol) were added. The reaction was stirred overnight at room temperature. After the reaction was completed, the pH was adjusted to neutral by dropwise adding dilute hydrochloric acid, and most acetonitrile was spun off by vacuum concentration at 40°C. The solid was collected by filtration to give the crude product, which was separated and purified by a silica gel chromatography column (DCM : MeOH = 12 : 1) to give **Intermediate 2** (1.3 g, 23.9%).

LC-MS (ESI): m/z= 442.2 $[M+H]^+$.

$^1$H NMR (400 MHz, MeOD) δ 7.77 (d, 2H), 7.60 - 7.48 (m, 2H), 7.37 (t, 2H), 7.26 (q, 2H), 6.94 - 6.78 (m, 3H), 4.46 - 4.21 (m, 2H), 4.14 (dt, 2H), 3.13 (dd, 1H), 2.86 (dd, 1H), 2.67 (s, 4H), 1.71 (s, 4H).

## Intermediate 3:

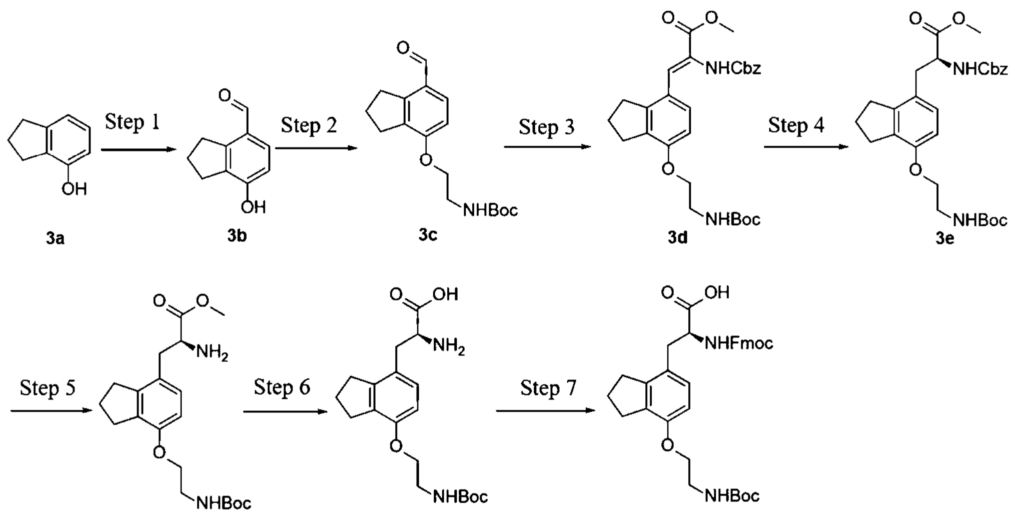


3a 3b 3c 3d 3e

3f 3g Intermediate 3

Step 1: **Compound 3a** (10.0 g, 73.43 mmol) was dissolved in dichloromethane (100 mL), $TiCl_4$ (25.5 g, 134.38 mmol) was added dropwise to the above reaction solution, and the temperature was lowered to 0°C. After 5 min, 1,1-dichlorodimethyl ether (9.4 g, 81.51 mmol) was added dropwise, and the mixture was stirred at 0°C for 3 h. After the reaction was completed, 200 mL of water was added to the reaction system. The resulting solution was extracted with dichloromethane, dried over anhydrous sodium sulphate, concentrated under reduced pressure, and subjected to column chromatography (EA : PE = 1 : 4) to give **Compound 3b** (5.58 g, 46.3%).

LC-MS (ESI): m/z= 163.2 $[M+H]^+$.

Step 2: **Compound 3b** (3.0 g, 18.27 mmol), tert-butyl (2-bromoethyl)carbamate (4.9 g, 21.91 mmol), potassium carbonate (5.1 g, 36.54 mmol), and sodium iodide (0.8 g, 5.47 mmol) were dissolved in N,N-dimethylformamide (20 mL), and the mixture was stirred at 25°C for 16 h. After the reaction was completed, 100 mL of water was added. The reaction solution was extracted with ethyl acetate three times (50 mL × 3), washed with saturated sodium chloride solution, dried over anhydrous sodium sulphate, concentrated under reduced pressure, and subjected to column chromatography (EA : PE = 4 : 1) to give Compound **3c** (4.9 g, 87%).

LC-MS (ESI): m/z= 306.1 $[M+H]^+$.

Step 3: (±)-benzyloxycarbonyl-a-phosphonoglycine trimethyl ester (6.2 g, 18.74 mmol) was dissolved in dichloromethane (100 mL), DBU (3.1 g, 20.31 mmol) was added after nitrogen replacement, and the mixture was stirred for 30 min. Then, **Compound 3c** (4.8 g, 15.62 mmol) was dissolved in dichloromethane (100 mL), and the mixture was added dropwise to the reaction. The reaction was stirred overnight. The reaction mixture was diluted with dichloromethane (100 mL), washed with 5% aqueous citric acid solution (100 mL) and saturated brine (100 mL), then dried over anhydrous sodium sulphate, filtered, and spin-dried. Separation and purification by a silica gel chromatography column (EA : PE = 3 : 1) afforded **Compound 3d** (6.1 g, 76.2%).

LC-MS (ESI): m/z= 511.1 $[M+H]^+$.

Step 4: **Compound 3d** (6.0 g, 11.71 mmol) was dissolved in a mixed solution of methanol (50 mL) and dichloromethane (20 mL), and (+)-1,2-bis(2S,5S)-2,5-diethylphospholanobenzene(cyclooctadiene)rhodium trifluoromethanesulfonate (0.6 g, 0.83 mmol) was added. The pressure was increased to about 4.0 bar by filling with hydrogen using an autoclave, and the mixture was stirred at room temperature for 3 h. The solid was filtered off, and the reaction solution was concentrated to give **Compound 3e** (5.9 g, 98%).

LC-MS (ESI): m/z= 513.2 $[M+H]^+$.

Step 5: **Compound 3e** (5.9 g, 11.46 mmol) was dissolved in a mixed solution of methanol (50 mL) and dichloromethane (20 mL), and palladium on carbon (10%, 1 g) was added. The mixture was stirred overnight under hydrogen atmosphere. Then, the solid was filtered off, and the reaction solution was spin-dried to give **Compound 3f** (3.7 g, 84.5%).

LC-MS (ESI): m/z= 379.4 $[M+H]^+$.

Step 6: **Compound 3f** (3.7 g, 9.72 mmol) was dissolved in tetrahydrofuran (25 mL), and lithium hydroxide monohydrate (1.2 g, 48.6 mmol) and water (25 mL) were added. The reaction was stirred overnight at room temperature, and extracted with ethyl acetate (15 mL x 3) to remove impurities. Then, the pH of the aqueous phase was adjusted to neutral with dilute hydrochloric acid (1N), and the product was precipitated, filtered, and dried to give **Compound 3g** (3.2 g, 89.8%).

LC-MS (ESI): m/z= 365.2 $[M+H]^+$.

Step 7: **Compound 3g** (2.7 g, 7.41 mmol) was dissolved in a mixed solution of acetonitrile (25 mL) and water (25 mL), and sodium bicarbonate (6.2 g, 74.16 mmol) and 9-fluorenylmethyl N-succinimidyl carbonate (3.3 g, 9.63 mmol) were added. The reaction was stirred overnight at room temperature. After the reaction was completed, the pH was adjusted to neutral by dropwise adding dilute hydrochloric acid, and most acetonitrile was spun off by vacuum concentration at 40°C. The solid was collected by filtration to give the crude product, which was separated and purified by a silica gel chromatography column (DCM : MeOH = 10 : 1) to give **Intermediate 3** (2.7 g, 62.1%).

LC-MS (ESI): m/z= 587.2 $[M+H]^+$.

$^1H$ NMR (400 MHz, DMSO-d6) δ 12.65 (s, 1H), 7.88 (d, 2H), 7.67 (s, 2H), 7.41 (d, 2H), 7.36 - 7.24 (m, 2H), 7.03 - 6.88 (m, 2H), 6.65 (d, 1H), 4.26 - 4.09 (m, 4H), 3.91 (s, 2H), 3.28 (s, 2H), 2.99 (d, 1H), 2.90 - 2.68(m, 5H), 2.51 (s, 1H), 1.98 (s, 2H), 1.39 (s, 9H).

## Intermediate 4:

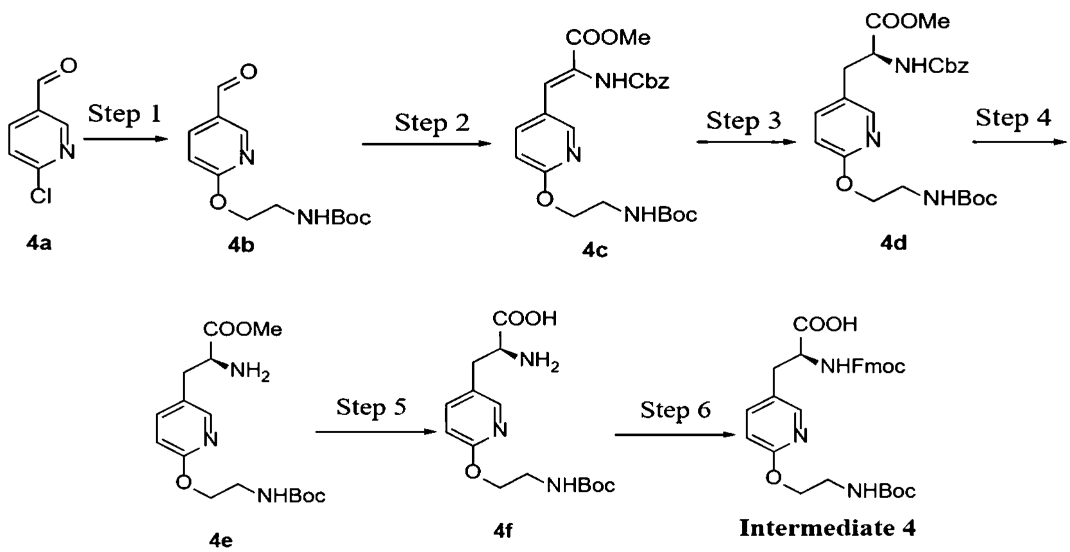


4a 4b 4c 4d 4e 4f Intermediate 4

Step 1: **Compound 4a** (2.0 g, 14.09 mmol) and N-(tert-butoxycarbonyl)ethanolamine (4.5 g, 28.18 mmol) were dissolved in N,N-dimethylacetamide (20 mL), and potassium carbonate (3.9 g, 28.18 mmol) was added. The mixture was stirred at 80°C under microwave irradiation for 2 h. After the reaction was completed, 20 mL of water was added to the reaction system. The resulting solution was extracted with ethyl acetate three times (40 mL × 3), dried over anhydrous sodium sulphate, concentrated under reduced pressure, and subjected to column chromatography (PE : PE = 4 : 1) to give **Compound 4b** (0.9 g, 24.0%).

LC-MS (ESI): m/z= 267.2 $[M+H]^+$.

Step 2: (±)-benzyloxycarbonyl-a-phosphonoglycine trimethyl ester (6.1 g, 18.47 mmol) was dissolved in dichloromethane (100 mL), DBU (3.1 g, 20.02 mmol) was added after nitrogen replacement, and the mixture was stirred for 30 min. Then, **Compound 4b** (4.1 g, 15.40 mmol) was dissolved in dichloromethane (100 mL), and the mixture was

added dropwise to the reaction. The reaction was stirred overnight. The reaction mixture was diluted with dichloromethane (100 mL), washed with 5% aqueous citric acid solution (100 mL) and saturated brine (100 mL), then dried over anhydrous sodium sulphate, filtered, and spin-dried. Separation and purification by a silica gel chromatography column (EA : PE = 3 : 1) afforded **Compound 4c** (4.5 g, 62.0%).

LC-MS (ESI): m/z= 472.2 $[M+H]^+$.

Step 3: **Compound 4c** (4.4 g, 9.33 mmol) was dissolved in a mixed solution of methanol (40 mL) and dichloromethane (20 mL), and (+)-1,2-bis(2S,5S)-2,5-diethylphospholanobenzene(cyclooctadiene)rhodium trifluoromethanesulfonate (0.6 g, 0.83 mmol) was added. The pressure was increased to about 4.0 bar by filling with hydrogen using an autoclave, and the mixture was stirred at room temperature for 3 h. The solid was filtered off, and the reaction solution was concentrated to give **Compound 4d** (4.3 g, 97%).

LC-MS (ESI): m/z= 474.2 $[M+H]^+$.

Step 4: **Compound 4d** (4.3 g, 9.08 mmol) was dissolved in a mixed solution of methanol (50 mL) and dichloromethane (20 mL), and palladium on carbon (10%, 1 g) was added. The mixture was stirred overnight under hydrogen atmosphere. Then, the solid was filtered off, and the reaction solution was spin-dried to give **Compound 4e** (2.9 g, 94.1%).

LC-MS (ESI): m/z= 340.3 $[M+H]^+$.

Step 5: **Compound 4e** (3.0 g, 8.84 mmol) was dissolved in tetrahydrofuran (25 mL), and lithium hydroxide monohydrate (0.9 g, 37.6 mmol) and water (25 mL) were added. The reaction was stirred overnight at room temperature, and extracted with ethyl acetate (20 mL x 3) to remove impurities. Then, the pH of the aqueous phase was adjusted to neutral with dilute hydrochloric acid (1N), and the product was precipitated, filtered, and dried to give **Compound 4f** (2.4 g, 83.4%).

LC-MS (ESI): m/z= 326.2 $[M+H]^+$.

Step 6: **Compound 4f** (2.4 g, 7.38 mmol) was dissolved in a mixed solution of acetonitrile (20 mL) and water (20 mL), and sodium bicarbonate (6.2 g, 73.8 mmol) and 9-fluorenylmethyl N-succinimidyl carbonate (3.2 g, 9.59 mmol) were added. The reaction was stirred overnight at room temperature. After the reaction was completed, the pH was adjusted to neutral by dropwise adding dilute hydrochloric acid, and most acetonitrile was spun off by vacuum concentration at 40°C. The solid was collected by filtration to give the crude product, which was separated and purified by a silica gel chromatography column (DCM : MeOH = 10 : 1) to give **Intermediate** 4 (1.9 g, 47.0%).

LC-MS (ESI): m/z= 548.2 $[M+H]^+$.

$^1$H NMR (400 MHz, DMSO-d6) δ 12.80 (s, 1H), 8.02 (d, 1H), 7.88 (d, 2H), 7.73 (d, 1H), 7.66 - 7.58 (m, 3H), 7.41 (t, 2H), 7.30 (dd, 2H), 6.93 (s, 1H), 6.70 (d, 1H), 4.24 - 4.09 (m, 6H), 3.26 (q, 2H), 3.02 (dd, 1H), 2.87 - 2.75 (m, 1H), 1.37 (s, 9H).

## Intermediate 5:

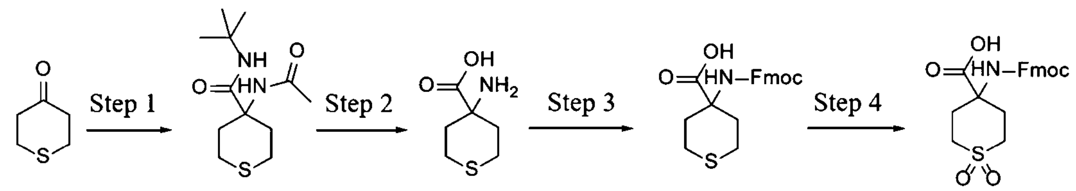


5a 5b 5c 5d Intermediate 5

Step 1: The known **Compound 5a** (5 g, 0.043 mol) was added to trifluoroethanol (50 mL), followed by tert-butyl isocyanide (7.15 g, 0.086 mol) and ammonium acetate (13.3 g, 0.172 mol). The mixture was reacted at room temperature for 3 d. TLC showed that the reaction was completed. The reaction solution was concentrated to dryness, and separated and purified by a C18 reverse phase column (composition of mobile phases A and B: mobile phase A: acetonitrile; and mobile phase B: water (with 0.1% TFA), (A/B = 35/65)) to give **Compound 5b** (9.5 g, yield: 85%).

LCMS m/z =259.2 $[M+1]^+$.

Step 2: **Compound 5b** (7.5 g, 0.029 mol) was added to 6N HCl (75 mL), and the mixture was reacted overnight at 100°C. LCMS showed that the starting materials were completely reacted. The resultant was concentrated to dryness, and separated and purified by a C18 reverse phase column (composition of mobile phases A and B: mobile phase A: acetonitrile; and mobile phase B: water (with 0.1% TFA), (A/B = 5/95)) to give **Compound 5c** (4.6 g, yield: 98%).

LCMS m/z =162.1 $[M+1]^+$.

Step 3: **Compound 5c** (4.6 g, 0.028 mol) was added to acetonitrile (46 mL) and water (46 mL), followed by 9-fluorenylmethyl N-succinimidyl carbonate (Fmoc-Osu) (10.5 g, 0.03 mol) and sodium bicarbonate (24.4 g, 0.28 mol). The mixture was reacted overnight at room temperature. The reaction was completed as monitored by LC-MS. The pH was adjusted to 5-6 with 1N HCl, and acetonitrile was removed by concentration. The resultant was extracted with ethyl acetate

(200 mL), and the ethyl acetate phase was concentrated to dryness, and purified by column chromatography (DCM : MeOH = 10 : 1) to give **Compound 5d** (6.3 g, yield: 58%).

LCMS m/z =384.2 $[M+1]^+$.

Step 4: **Compound 5d** (5.0 g, 0.013 mol) was added to dichloromethane (50 mL), followed by 85% m-chloroperoxybenzoic acid (m-CPBA) (7.9 g, 0.039 mol) portionwise. The mixture was reacted overnight at room temperature. The reaction was completed as monitored by LC-MS. The reaction was quenched with sodium thiosulphate solution (100 mL), stirred for 30 min, and extracted with ethyl acetate (200 mL). The ethyl acetate phase was concentrated to dryness. Separation and purification by a preparative liquid chromatography column (preparative liquid chromatography conditions: preparative C18 reverse phase column; mobile phases: deionized water with 0.1% trifluoroacetic acid (A), and acetonitrile with 0.1% trifluoroacetic acid (B); gradient elution: B = 5%-70%; elution time: 15 min; flow rate: 12 mL/min; column temperature: 30°C; and retention time: 8.54 min) afforded **Intermediate 5** (3.5 g, yield: 64%).

$^1H$ NMR (400 MHz, $CDCl_3$) δ 7.78-7.76 (d, 2H), 7.60-7.58 (d, 2H), 7.43-7.40 (t, 2H), 7.34-7.31 (t, 2H), 4.52-4.51 (d, 2H), 4.22-4.19 (t, 1H), 3.03 (s, 4H), 2.60-2.50 (m, 4H).

LCMS m/z =433.1 $[M+H_2O]^+$.

## Intermediate 6:

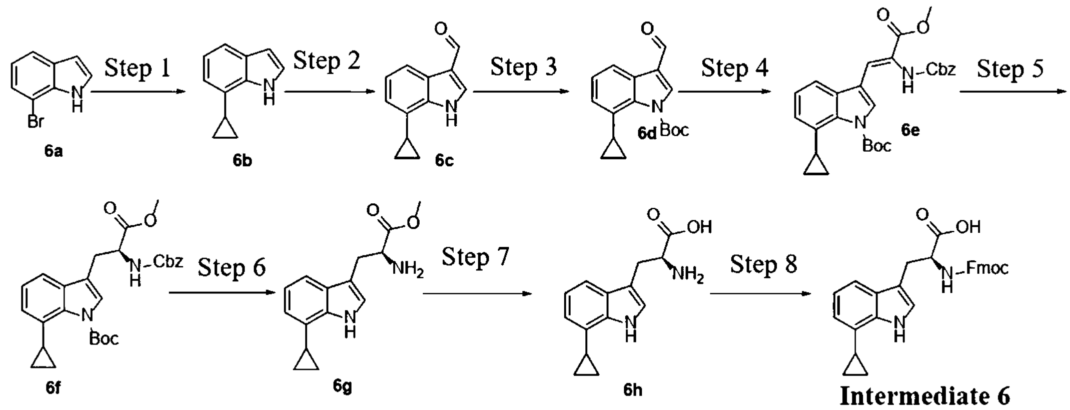


6a 6b 6c 6d 6e 6f 6g 6h Intermediate 6

Step 1: **Compound 6a** (10.0 g, 51.28 mmol), cyclopropylboronic acid (8.8 g, 102.56 mmol), and caesium carbonate (50.46 g, 153.84 mmol) were added to dioxane (200 mL) and water (40 mL), followed by 1,1'-bis(diphenylphosphino)ferrocene]palladium dichloride (3.8 g, 5.13 mmol). The mixture was reacted overnight at 80°C after nitrogen replacement, concentrated, and then separated and purified by a silica gel chromatography column (EA : PE = 5 : 2) to give Compound **6b** (7.0 g, 86.9%).

LC-MS (ESI): m/z= 158.1 $[M+H]^+$.

Step 2: DMF (6.5 g, 89.18 mmol) was added dropwise to phosphorus oxychloride (70 mL) at 0°C. After ten minutes, **Compound 6b** (7.0 g, 44.59 mmol) was dissolved in phosphorus oxychloride (30 mL) at 0°C, and the mixture was reacted at room temperature for 1 h, neutralized with saturated sodium bicarbonate in an ice bath to pH = 7-8, and extracted with dichloromethane (100 mL × 3). The organic phases were combined, spin-dried, concentrated, and then separated and purified by a silica gel chromatography column (EA : PE = 5 : 2) to give **Compound 6c** (4.5 g, 54.5%).

LC-MS (ESI): m/z= 186.2 $[M+H]^+$.

Step 3: **Compound 6c** (4.5 g, 24.32 mmol) was dissolved in acetonitrile (50 mL), and di-tert-butyl dicarbonate (10.6 g, 48.64 mmol) was added, followed by 4-dimethylaminopyridine (3.6 g, 29.18 mmol). The mixture was reacted at room temperature for 2 h, and extracted with dichloromethane (100 mL × 3). The organic phases were combined, spin-dried, concentrated, and then separated and purified by a silica gel chromatography column (EA : PE = 5 : 1) to give **Compound 6d** (6.0 g, 86.9%).

LC-MS (ESI): m/z= 230.2 $[M-56+H]^+$.

Step 4: (±)-benzyloxycarbonyl-a-phosphonoglycine trimethyl ester (7.0 g, 21.05 mmol) was dissolved in dichloromethane (100 mL), DBU (6.43 g, 42.3 mmol) was added after nitrogen replacement, and the mixture was stirred for 30 min. Then, **Compound 6d** (6.0 g, 21.05 mmol) was dissolved in dichloromethane (100 mL), and the mixture was added dropwise to the reaction. The reaction was continued to be stirred overnight. The reaction mixture was diluted with dichloromethane (100 mL), washed with 5% aqueous citric acid solution (100 mL) and saturated brine (100 mL), then dried over anhydrous sodium sulphate, filtered, and spin-dried. Separation and purification by a silica gel chromatography column (EA : PE = 3 : 1) afforded **Compound 6e** (5.7 g, 55.3%).

LC-MS (ESI): m/z= 491.3 $[M+H]^+$.

Step 5: **Compound 6e** (5.7 g, 11.6 mmol) was dissolved in methanol (50 mL), and (+)-1,2-bis(2S,5S)-2,5-diethylphospholanobenzene(cyclooctadiene)rhodium trifluoromethanesulfonate (420 mg, 0.58 mmol) was added. The pressure was increased to about 2.5 MPa by filling with hydrogen using an autoclave, and the mixture was stirred overnight at room temperature. The solid was filtered off, and the reaction solution was concentrated to give **Compound 6f** (5.7 g, 99.9%).

LC-MS (ESI): m/z= 493.2 $[M+H]^+$.

Step 6: **Compound 6f** (3.0 g, 6.1 mmol) was dissolved in dichloromethane (50 mL), and in an ice bath, triethylamine (3.1 g, 30.5 mmol) was added, followed by iodotrimethylsilane (6.1 g, 30.5 mmol). The mixture was stirred at room temperature for 16 h, and concentrated at room temperature. Then, methanol (5 mL) was added to quench the reaction. Separation and purification by a C18 reverse phase column (water with 0.5% TFA : acetonitrile = 3 : 10) afforded **Compound 6g** (1.5 g, 95.5%).

LC-MS (ESI): m/z= 259.1 $[M+H]^+$.

Step 7: **Compound 6g** (1.5 g, 5.8 mmol) was dissolved in tetrahydrofuran (20 mL), and lithium hydroxide monohydrate (1.58 g, 37.7 mmol) and water (20 mL) were added. The reaction was stirred at room temperature for 2 h. Then, the pH of the aqueous phase was adjusted to neutral with dilute hydrochloric acid (1N). The next step was carried out directly.

LC-MS (ESI): m/z= 245.1 $[M+H]^+$.

Step 8: Sodium bicarbonate (4.9 g, 58.0 mmol) and 9-fluorenylmethyl N-succinimidyl carbonate (2.9 g, 8.7 mmol) were added to the reaction solution obtained in the previous step. The reaction was stirred overnight at room temperature. After the reaction was completed, the pH was adjusted to 5-6 by dropwise adding dilute hydrochloric acid, and most acetonitrile was spun off by vacuum concentration at 40°C. The resultant was extracted with dichloromethane (100 mL × 3). The organic phases were combined, spin-dried, and separated and purified by a silica gel chromatography column (DCM : MeOH = 10 : 1) to give **Intermediate 6** (1.2 g, two-step yield: 44.4%).

LC-MS (ESI): m/z= 467.2 $[M+H]^+$.

$^1H$ NMR (400 MHz, DMSO-d6) δ 10.83 (s, 1H), 7.89 - 7.85 (m, 2H), 7.63 (d, 2H), 7.42 - 7.34 (m, 3H), 7.32 - 7.24 (m, 2H), 7.16 - 6.98 (m, 2H), 6.88 - 6.80 (m, 1H), 6.60 (d, 1H), 4.22 - 4.14 (m, 3H), 4.11 - 4.05 (m, 1H), 3.26 - 3.19 (m, 1H), 3.07 - 2.98 (m, 1H), 2.25 - 2.15 (m, 1H), 1.00 - 0.92 (m, 2H), 0.72 - 0.62 (m, 2H).

**Intermediate 7** and **Intermediate 8**:

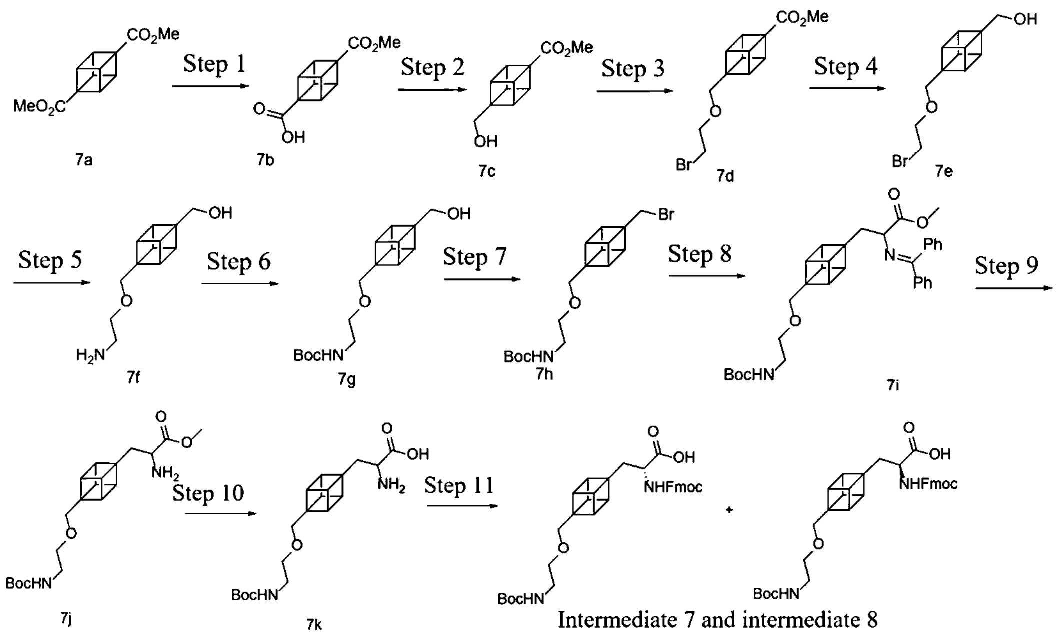

Intermediate 7 and intermediate 8

Step 1: The known **Compound 7a** (20 g, 0.09 mol) was added to methanol (46 mL) and tetrahydrofuran (460 mL), followed by sodium hydroxide (3.6 g, 0.09 mol). The mixture was reacted at room temperature for 18 h. LCMS showed that the starting materials were completely reacted. The reaction solution was concentrated to dryness, and diluted with water (300 mL). The pH was adjusted to 5-6 with 1N HCl. The resultant was extracted with ethyl acetate (300 mL), and the ethyl acetate phase was concentrated to dryness, and purified by column chromatography (DCM : MeOH = 10 : 1) to give **Compound 7b** (17.8 g, yield: 95%).

LCMS m/z =207.1 $[M+1]^+$.

Step 2: **Compound 7b** (17.8 g, 0.086 mol) was added to tetrahydrofuran (1 L). The temperature was lowered to

0°C. A solution of borane in dimethyl sulphide (10 mol/L, 10 mL) was added dropwise. After the dropwise addition was completed, the mixture was reacted for 2 h. LCMS showed that the starting materials were completely reacted. Methanol (30 mL) was added dropwise to quench the excess borane. The resultant was concentrated to dryness, and extracted with ethyl acetate (500 mL) and water (500 mL). The ethyl acetate phase was concentrated to dryness, and purified by column chromatography (DCM : MeOH = 10 : 1) to give **Compound 7c** (15 g, yield: 91%).

LCMS m/z =215.2 $[M+23]^+$.

Step 3: Pyridine (27.84 g, 0.352 mol) was added to dichloromethane (800 mL), and the temperature was lowered to -20°C. Trifluoromethanesulfonic anhydride (94.8 g, 0.336 mol) was added dropwise. After the dropwise addition was completed, 2-bromoethanol (40 g, 0.32 mol) was added to the system, and the mixture was stirred at -20°C for 30 minutes. Dichloromethane was removed by concentration at a low temperature of 30°C. The residue was dissolved in MTBE, and the mixture was filtered. The mother solution was concentrated to dryness at a low temperature of 25°C. A portion (59.2 g, 0.231 mol) was added to a solution of **7c** (14.8 g, 0.077 mol) in toluene (440 mL), followed by DIEA (29.6 g, 0.231 mol). After the addition was completed, the mixture was stirred overnight at 90°C. TLC showed that the starting materials were completely reacted. The resultant was concentrated to dryness, and extracted with ethyl acetate (500 mL) and water (500 mL). The ethyl acetate phase was concentrated to dryness, and purified by column chromatography (PE: EA = 2 : 1) to give **Compound 7d** (21.6 g, yield: 94%).

Step 4: **Compound 7d** (21.6 g, 0.072 mol) was added to tetrahydrofuran (430 mL), followed by lithium aluminium hydride (5.47 g, 0.144 mol) portionwise. The mixture was reacted at room temperature for 2 h. The reaction was completed as monitored by TLC. 5.5 mL of water was slowly added dropwise, 5.5 mL of 15% sodium hydroxide solution was then slowly added dropwise, and finally 16.5 mL of water was added dropwise, followed by anhydrous sodium sulphate. The mixture was stirred for 30 min, and filtered. The mother solution was concentrated to dryness to give **7e** (15.9 g, yield: 81%).

LCMS m/z = 271.2 $[M+1]^+$.

Step 5: **Compound 7e** (12.5 g, 0.046 mol) was added to aqueous ammonia (250 mL), and the mixture was reacted at room temperature for 18 h. The starting materials were not completely reacted as monitored by TLC. The resultant was concentrated directly to dryness to give crude **7f,** which was used in the next reaction.

LCMS m/z = 208.2 $[M+1]^+$.

Step 6: The crude **Compound 7f** was added to acetonitrile (120 mL) and water (120 mL), followed by di-tert-butyl dicarbonate (10.0 g, 0.046 mol) and sodium carbonate (9.75 g, 0.092 mol). The mixture was stirred at room temperature for 18 h, concentrated to remove acetonitrile, and extracted with ethyl acetate (200 mL). The resultant was concentrated to dryness, and purified by column chromatography (PE : EA = 1 : 1) to give Compound **7g** (5.4 g, yield: 38%).

LCMS m/z = 252.2 $[M+1-56]^+$.

Step 7: **Compound 7g** (5.4 g, 0.017 mol) was added to dichloromethane (54 mL), and the temperature was lowered to 0°C. Carbon tetrabromide (9.7 g, 0.28 mol) and triphenylphosphine (7.7 g, 0.28 mol) were added. The mixture was reacted at room temperature for 18 h. The reaction was completed as monitored by LCMS. The resultant was concentrated to dryness, and purified by column chromatography (PE : EA = 2 : 1) to give **7h** (2.4 g, yield: 35%).

LCMS m/z = 314.2 $[M+1-56]^+$.

Step 8: **Compound 7h** (2.4 g, 0.006 mol) was added to DMF (24 mL), followed by diphenylmethyleneglycine methyl ester (3.15 g, 0.012 mol) and potassium tert-butoxide (1.74 g, 0.015 mol). The mixture was reacted at room temperature for 18 h. The starting materials were not completely reacted as monitored by LCMS. The resultant was extracted with ethyl acetate (100 mL) and water (100 mL). The ethyl acetate phase was back-washed with water twice, concentrated to dryness, and purified by column chromatography (PE : EA = 1 : 1) to give 7i (1.8 g, yield: 51%).

LCMS m/z = 543.2 $[M+1]^+$.

Step 9: **Compound 7i** (1.8 g, 3.3 mmol) was added to tetrahydrofuran (32 mL), followed by 1N HCl (16 mL). The mixture was reacted at room temperature for 1 h. The starting materials were completely reacted as monitored by LCMS. The pH was adjusted to 8-9 with aqueous sodium carbonate solution. The resultant was extracted with ethyl acetate (100 mL) and water (50 mL). The ethyl acetate phase was concentrated to dryness, and purified by column chromatography (DCM : MeOH = 10 : 1) to give **7j** (1.25 g, yield: 100%).

LCMS m/z = 323.2 $[M+1-56]^+$.

Step 10: **Compound 7j** (1.25 g, 3.3 mmol) was added to methanol (13 mL) and water (4 mL), followed by lithium hydroxide (554 mg, 13.2 mmol). The mixture was reacted at room temperature for 4 h. The starting materials were completely reacted as monitored by LCMS. The reaction solution was used directly in the next reaction.

LCMS m/z = 309.2 $[M+1-56]^+$.

Step 11: The pH of the reaction solution from the previous step was adjusted to 5-6 with 1N HCl, and then adjusted to 8-9 with aqueous sodium bicarbonate solution. Then, 9-fluorenylmethyl N-succinimidyl carbonate (1.3 g, 3.96 mmol) and sodium bicarbonate (2.77 g, 33 mmol) were added. The mixture was stirred at room temperature for 2 h. LCMS showed that the starting materials were completely reacted. The pH was adjusted to 7 with 1N HCl. The resultant was extracted with ethyl acetate (100 mL) and water (100 mL). The ethyl acetate phase was concentrated to dryness, and purified by column chromatography (DCM : MeOH = 10 : 1) to give 1.8 g of racemate, which was subjected to chiral

resolution (instrument: SFC Prep 150 AP; and chromatographic column: Daicel AD-H (19 mm × 250 mm). The sample was dissolved in methanol, and filtered with a 0.45 μm filter head to prepare a sample solution. Preparative chromatography conditions: composition of mobile phases A and B: mobile phase A: CO2; and mobile phase B: methanol; isocratic elution: mobile phase B = 25%; and flow rate: 40 ml/min) to give **Intermediate 7** (P1) (730 mg, yield: 38%) at 4.1 min and **Intermediate 8** (P2) (750 mg, yield: 39%) at 4.6 min.

**Intermediate 7:** $^1$H NMR (400 MHz, $CDCl_3$) δ 7.77 - 7.75 (d, 2H), 7.60 - 7.57 (t, 2H), 7.41 - 7.37 (t, 2H), 7.32 - 7.29 (t, 2H), 4.49 - 4.41 (m, 3H), 4.23 - 4.20 (t, 1H), 3.73 -3.68 (m, 6H), 3.59 - 3.55 (m, 2H), 3.51 - 3.49 (m, 2H), 3.31 - 3.27 (m, 2H), 2.22 - 2.04 (m, 2H), 1.45 (s, 9H).

LCMS m/z =487.2 $[M+1-100]^+$.

**Intermediate 8:** $^1$H NMR (400 MHz, $CDCl_3$) δ 7.75 - 7.73 (d, 2H), 7.59 - 7.56 (t, 2H), 7.39 - 7.36 (t, 2H), 7.30 - 7.27 (t, 2H), 4.47 - 4.39 (m, 3H), 4.22 - 4.19 (t, 1H), 3.70 - 3.64 (m, 6H), 3.57 - 3.53 (m, 2H), 3.51 - 3.49 (m, 2H), 3.31 - 3.27 (m, 2H), 2.20 - 2.03 (m, 2H), 1.44 (s, 9H).

LCMS m/z =487.2 $[M+1-100]^+$.

## Intermediate 9 and Intermediate 10:

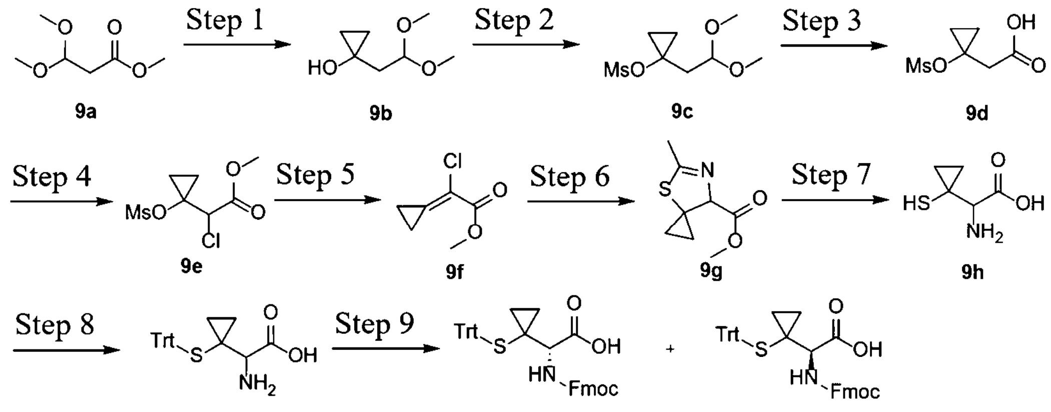


9a 9b 9c 9d 9e 9f 9g 9h 9i

**Intermediate 9 and intermediate 10**

Step 1: **Compound 9a** (10.4 g, 70.5 mmol) was dissolved in tetrahydrofuran (200 mL), tetraisopropyl titanate (4 ml, 13.6 mmol) was added at 0°C after nitrogen replacement, and ethylmagnesium bromide (58.67 ml, 176 mmol) was added dropwise. The mixture was warmed to room temperature, and reacted overnight. After the reaction was completed, the reaction was quenched with water at 0°C, and extracted with ethyl acetate. The organic phases were combined, spin-dried, concentrated, and then separated and purified by a silica gel chromatography column (EA : PE = 1 : 5) to give Compound 9b (7.0 g, 71%).

$^1$H NMR (400 MHz, CDCl3) δ = 4.68 (t, 1H), 3.47 (s, 1H), 3.39 (s, 6H), 1.88 (d, 2H), 0.77 (t, 2H), 0.45 (t, 2H).

Step 2: **Compound 9b** (7.0 g, 47.95 mmol) was dissolved in dichloromethane (50 ml), triethylamine (14.53 g, 143.85 mmol) was added, and methanesulfonyl chloride (6.59 g, 57.54 mmol) was added dropwise at 0°C. The mixture was reacted at room temperature for 1 h, quenched with water in an ice bath, and extracted with dichloromethane. The organic phases were combined, spin-dried, and then concentrated to give crude Compound 9c.

$^1$H NMR (400 MHz, CDC13) δ = 4.69 (t, 1H), 3.37 (s, 6H), 3.01 (s, 3H), 2.15 (d, 2H), 1.30-1.26 (m, 2H), 0.81 (d, 2H).

Step 3: The crude **Compound 9c** from the previous step was dissolved in tetrahydrofuran/water (60 mL/30 ml), and potassium peroxymonosulfonate (24.91 g, 71.93 mmol) was added at 0°C. The mixture was reacted overnight at room temperature, and extracted with ethyl acetate. The organic phases were combined, spin-dried, and recrystallised from methyl tert-butyl ether to give **Compound 9d** (4.8 g, two-step yield: 52%).

LC-MS (ESI): m/z= 195.1 $[M+H]^+$.

Step 4: **Compound 9d** (7.0 g, 36.08 mmol) was dissolved in 1,2-dichloroethane (200 mL), and thionyl chloride (3.26 ml, 45 mmol) was added after nitrogen replacement. The mixture was refluxed for 1 hour, and cooled to room temperature. NCS (7.2 g, 54.12 mmol) and 10 drops of 4M hydrogen chloride in 1,4-dioxane were added. The mixture was refluxed overnight, and cooled to room temperature. Methanol (25 ml) was added. The mixture was continued to be reacted 1 hour. After the reaction was completed, the resultant was concentrated and spin-dried directly. Chloroform was added at 0°C. The liquid was collected by filtration, and concentrated to give crude **Compound 9e.**

LC-MS (ESI): m/z= 243.1 $[M+H]^+$

Step 5: The crude **Compound 9e** was dissolved in dichloromethane (20 mL), triethylamine (5.47 g, 54.12 mmol) was added at 0°C, and the mixture was continued to be stirred at this temperature for 3 h, quenched with water, and

extracted with dichloromethane. The organic phases were combined, concentrated, and separated and purified by a silica gel chromatography column (EA : PE = 1 : 5) to give Compound **9f** (3.5 g, two-step yield: 66%).

LC-MS (ESI): m/z= 147.1 $[M+H]^+$

Step 6: **Compound 9f** (3.0 g, 20.55 mmol) was dissolved in acetonitrile (50 mL), and sodium bicarbonate (17.26 g, 205.5 mmol) was added, followed by thioacetamide (1.54 g, 20.55 mmol). The mixture was stirred at 80°C for 5 h, filtered, concentrated, and separated and purified by a silica gel chromatography column (EA : PE = 1 : 1) to give Compound 9g (3 g, 79%).

LC-MS (ESI): m/z= 186.2 $[M+H]^+$.

Step 7: **Compound 9g** (1 g, 5.38 mmol) was placed in a 50 ml round-bottom flask, 3M hydrochloric acid solution (10 mL) was added, and the mixture was stirred at 100°C for 5 h. The reaction was completed, and extracted with methyl tert-butyl ether. The aqueous phase was concentrated directly to give **Compound 9h** hydrochloride.

LC-MS (ESI): m/z= 148.2 $[M+H]^+$.

Step 8: **Compound 9h** (1 g, 6.8 mmol) was placed in a 50 ml round-bottom flask, trifluoroacetic acid (5 ml) and triphenylmethanol (2.12 g, 8.16 mmol) were added, and the mixture was stirred at room temperature for 10 min. The resultant was spin-dried to give the target **Compound 9i.** The next step was carried out directly.

LC-MS (ESI): m/z= 388.5 $[M-H]^+$.

Step 9: The crude **Compound 9i** from the previous step was dissolved in acetonitrile and water (50 ml/10 ml), and sodium bicarbonate (5.7 g, 68 mmol) and 9-fluorenylmethyl N-succinimidyl carbonate (3.44 g, 10.2 mmol) were added. The mixture was stirred at room temperature for 3 h, filtered, added with saturated brine (30 ml), and extracted with ethyl acetate. The organic phases were combined, concentrated, and separated and purified by a silica gel chromatography column (DCM : MeOH = 10 : 1) to give 2.2 g of racemate (two-step yield: 43%). The racemate was subjected to chiral resolution (instrument: SFC Prep 150 AP; and chromatographic column: Daicel AD-H (19 mm × 250 mm). The sample was dissolved in methanol, and filtered with a 0.45 μm filter head to prepare a sample solution. Preparative chromatography conditions: composition of mobile phases A and B: mobile phase A: CO2; and mobile phase B: methanol; isocratic elution: mobile phase B = 25%; and flow rate: 40 ml/min) to give **Intermediate 9** (P1) (890 mg) at 3.8 min and **Intermediate 10** (P2) (910 mg) at 4.2 min.

**Intermediate 9:**

$^1H$ NMR (400 MHz, CDCl3) δ = 7.78 (d, 2H), 7.61 - 7.49 (m, 8H), 7.42 - 7.39 (m, 2H), 7.34 - 7.30 (m, 2H), 7.28 - 7.22 (m, 6H), 7.22 - 7.14 (m, 3H), 5.00 (d, 1H), 4.34 (d, 2H), 4.19 (t, 1H), 3.88 (d, 1H), 1.06 - 0.92 (m, 1H), 0.85 - 0.75 (m, 1H), 0.75 - 0.63 (m, 1H), 0.28 - 0.19 (m, 1H).

**Intermediate 10:**

$^1H$ NMR (400 MHz, CDCl3) δ = 7.77 (d, 2H), 7.59 - 7.54 (m, 8H), 7.42 - 7.39 (m, 2H), 7.33 - 7.30 (m, 2H), 7.26 - 7.22 (m, 6H), 7.21 - 7.13 (m, 3H), 5.00 (d, 1H), 4.34 (d, 2H), 4.19 (t, 1H), 3.89 (d, 1H), 1.02 - 0.97 (m, 1H), 0.86 - 0.74 (m, 1H), 0.73 - 0.62 (m, 1H), 0.26 - 0.20 (m, 1H).

**Intermediate 11:**

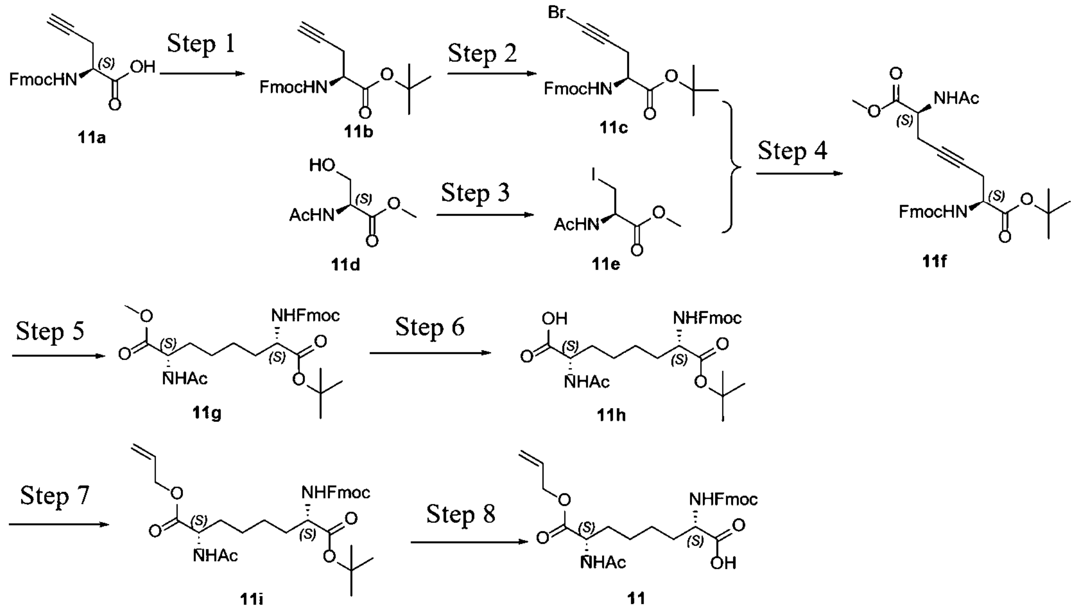

11a
11b
11c
11d
11e
11f
11g
11h
11i
11

Step 1: **Compound 11a** (20.0 g, 59.6 mmol) was dissolved in ethyl acetate (150 mL), cyclohexane (75 ml) and tert-butyl trichloroacetimidate (32.5 g, 149 mmol) were added at room temperature, and the mixture was warmed to 30°C, and reacted for 48 hours. After the reaction was completed, the resultant was concentrated directly under reduced pressure, and the crude product was separated and purified by a silica gel chromatography column (EA : PE = 10 : 90) to give **Compound 11b** (23.2 g, 99.4%).

$^1$H NMR (400 MHz, Chloroform-d) $\delta$ 7.76 (d, 2H), 7.61 (d, 2H), 7.39 (t, 2H), 7.31 (t, 2H), 5.67 (d, 1H), 4.40 (ddd, 3H), 4.24 (t, 1H), 2.76 (t, 2H), 2.04 (d, 1H), 1.50 (s, 9H).

Step 2: **Compound 11b** (10.0 g, 25.5 mmol) was dissolved in dry N,N-dimethylformamide (200 ml), and silver nitrate (434 mg, 2.55 mmol) and N-bromosuccinimide (5.46 g, 30.7 mmol) were added. After the addition was completed, the mixture was reacted overnight at room temperature. After the reaction was completed, the reaction was quenched with water (500 mL), and extracted with ethyl acetate (200 mL × 5). The organic phases were combined, dried over anhydrous sodium sulphate, and then concentrated to give the crude product, which was purified by silica gel column chromatography (EA : PE = 10 : 90) to give **11c** (10.5 g, 87.4%).

LC-MS (ESI): m/z= 470.1 $[M+H]^+$.

Step 3: At 0°C, **Compound 11d** (9.00 g, 55.8 mmol) was dissolved in dichloromethane (400 ml), and imidazole (4.94 g, 72.6 mmol), iodine (18.4 g, 72.6 mmol), and triphenylphosphine (19.0 g, 72.6 mmol) were added. After the addition was completed, the mixture was warmed to room temperature, and reacted overnight. After the reaction was completed, the reaction was quenched with saturated aqueous sodium thiosulphate solution (100 mL) and water (500 mL), and extracted with ethyl acetate (200 mL × 5). The organic phases were combined, dried over anhydrous sodium sulphate, and then concentrated to give the crude product, which was purified by silica gel column chromatography (EA : PE = 50 : 50) to give **11e** (8.90 g, 63.6%).

LC-MS (ESI): m/z= 272.0 $[M+H]^+$.

Step 4: Zinc powder (2.43 g, 37.2 mmol) and iodine (94.5 mg, 0.372 mmol) were added to a round-bottom flask, and the mixture was heated with a heating gun for 5 minutes under nitrogen protection, cooled to room temperature, and then protected with nitrogen three times. After cooling to 0°C, a solution of **Compound 11e** (3.17 g, 11.7 mmol) in DMF (50 mL) was added by injection. The mixture was warmed to room temperature, and reacted for about 2 hours. In addition, cuprous cyanide (950 mg, 10.6 mmol) and lithium chloride (900 mg, 21.2 mmol) were added into a round-bottom flask, and the mixture was warmed to 150°C under nitrogen protection, and reacted for 2 hours. After cooling to room temperature, DMF (10 mL) was added by injection, and the mixture was continued to be stirred for 10 minutes. After cooling to -15°C, the zinc powder-activated solution of **Compound 11e** in DMF was added by injection to the resultant. After 5 minutes, a solution of **Compound 11c** (5.00 g, 10.6 mmol) in DMF (40 mL) was added by injection to the resultant. Subsequently, the mixture was warmed to room temperature, and the reaction was stirred overnight. After the reaction was completed, the reaction was quenched with water (500 mL), and extracted with ethyl acetate (200 mL × 5). The organic phases were combined, dried over anhydrous sodium sulphate, and then concentrated to give the crude product, which was purified by silica gel column chromatography (EA : PE = 70 : 30) to give **11f** (3.47 g, 61.1%).

LC-MS (ESI): m/z= 535.3 $[M+H]^+$.

Step 5: **Compound 11f** (3.47 g, 6.49 mmol) was dissolved in ethyl acetate (200 ml), and Pd/C (2.0 g) was added.

The mixture was reacted overnight at room temperature after a hydrogen balloon was connected. After the starting materials disappeared as monitored by TLC, the solid was removed directly by filtration, and the resultant was washed with ethyl acetate. The organic phases were combined, and then concentrated directly to give crude **Compound 11g** (3.21 g).

LC-MS (ESI): m/z=539.3 $[M+H]^+$.

Step 6: The crude **Compound 11g** (3.21 g) was dissolved in 1,2-dichloroethane (200 mL), and trimethyltin hydroxide (4.31 g, 23.8 mmol) was added. The mixture was warmed to 70°C, and reacted overnight. After the reaction was completed, the reaction was cooled to room temperature, and concentrated directly under reduced pressure. The crude product was separated and purified by a silica gel chromatography column (DCM : MeOH = 80 : 20) to give Compound 11h (2.90 g, two-step yield: 85.2%).

LC-MS (ESI): m/z=525.3 $[M+H]^+$.

Step 7: **Compound 11h** (2.90 g, 5.53 mmol) was dissolved in dry N,N-dimethylformamide (150 ml), and sodium carbonate (2.34 g, 22.1 mmol) and 3-bromopropene (2.01 g, 16.6 mmol) were added. After the addition was completed, the mixture was reacted at room temperature for 3 days. After the reaction was completed, the reaction was quenched with water (500 mL), and extracted with ethyl acetate (200 mL × 5). The organic phases were combined, dried over anhydrous sodium sulphate, and then concentrated to give the crude product, which was purified by silica gel column chromatography (EA : PE = 70 : 30) to give **11i** (2.41 g, 77.2%).

LC-MS (ESI): m/z= 565.3 $[M+H]^+$.

Step 8: **Compound 11i** (2.41 g, 4.27 mmol) was dissolved in a mixed solvent of dichloromethane (50 ml) and trifluoroacetic acid (50 ml), and the mixture was reacted at room temperature. After the reaction was completed as monitored by TLC, the resultant was concentrated directly under reduced pressure. The resulting crude product was re-dissolved in dichloromethane (100 ml), and the pH was adjusted to about 7 with saturated aqueous sodium bicarbonate solution. Water was added, and then the mixture was extracted with dichloromethane (100 mL × 5). The organic phases were combined, dried over anhydrous sodium sulphate, and then concentrated to give the crude product, which was treated with dichloromethane to form a slurry so as to give **Intermediate 11** (1.69 g, 77.9%).

$^1H$ NMR (400 MHz, Methanol-$d_4$) δ 7.79 (d, 2H), 7.67 (t, 2H), 7.39 (t, 2H), 7.31 (td, 2H), 6.01 - 5.88 (m, 1H), 5.33 (dq, 1H), 5.22 (dq, 1H), 4.62 (dq, 2H), 4.42 - 4.33 (m, 3H), 4.23 (t, 1H), 4.13 (dd, 1H), 3.26 - 3.19 (m, 1H), 1.98 (s, 3H), 1.89 - 1.77 (m, 2H), 1.75 - 1.62 (m, 2H), 1.46 - 1.37 (m, 4H).

LC-MS (ESI): m/z= 509.2 $[M+H]^+$.

Example 1:

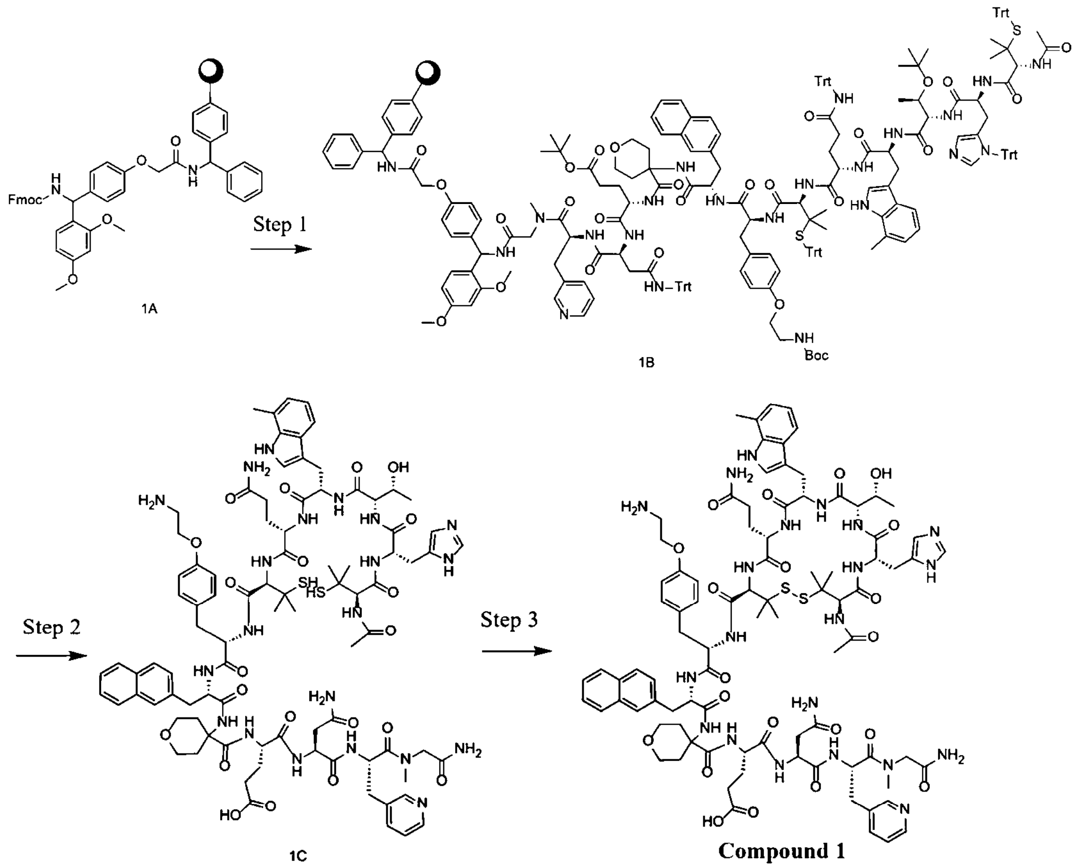


1A

1B

1C

**Compound 1**

Step 1: **1B** was synthesised using standard Fmoc chemistry.

1. Rink Amide MBHA Resin (1 mmol, 1.4 g, sub: 0.7 mmol/g) and dichloromethane as the solvent were added to a reactor, and the resin was swollen for 30 min. 20% piperidine/DMF was added, and the mixture was mixed for 30 minutes.
2. The solvent was pumped off, and the resin was rinsed with DMF five times.
3. Fmoc-Sar-OH-protected amino acid solution was added, and the mixture was mixed for 30 seconds. Then, a coupling reagent was added, and the mixture was bubbled with nitrogen for 1.5 hours. The reaction was monitored with ninhydrin.
4. The solvent was pumped off, and the resin was rinsed with DMF three times.
5. 20% piperidine/DMF was added, and the mixture was mixed for 30 minutes.
6. The solvent was pumped off, and the resin was rinsed with DMF five times.
7. Fmoc-protected amino acid solution was added, and the mixture was mixed for 30 seconds. Then, a coupling reagent was added, and the mixture was bubbled with nitrogen for 1.5 hours. The reaction was monitored with ninhydrin.
8. The solvent was pumped off, and the resin was rinsed with DMF three times.
9. Steps 5-8 were repeated for the coupling of the subsequent amino acid.
10. In the last step, the resin was rinsed with MeOH twice, DCM once, and MeOH twice. The solvent was pumped off under vacuum to give the peptide resin 1B (6.5 g), which was used directly in the next reaction.

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |

(continued)

| Serial No. | Starting material | Coupling reagent |
|---|---|---|
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

Step 2: 60 ml of cleavage solution (91% trifluoroacetic acid + 4% triisopropylsilane + 3% 1,2-ethanedithiol + 2% water) was added to a 100 ml reaction flask, and stirred evenly. Then, the peptide resin **1B** (6.5 g) was added, and the mixture was stirred at room temperature for 2 hours. The resin was filtered to obtain the filtrate, which was added to 500 ml of methyl tert-butyl ether (which was cooled to 0°C in advance). White floccules were precipitated, and centrifuged (3 min at 3000 rpm). The white precipitate was washed with methyl tert-butyl ether three times, and dried in vacuo to give the crude peptide **1C** as an off-white solid (2.4 g), which was used directly in the next reaction.

LCMS m/z = 627.6 $[M/3+H]^+$,940.9$[M/2+H]^+$

Step 3: To a 2 L reaction flask, water (750 ml), acetonitrile (250 ml), and **1C** (2 g, 1.06 mmol) were added successively, and stirred evenly. Then, iodine/acetonitrile solution (0.1 mol/L) was slowly added dropwise until the reaction solution turned light yellow. Ascorbic acid was added to quench the reaction, and the resultant was purified by preparative HPLC. Separation method: 1. Instrument: Waters 2767 (preparative liquid chromatographic instrument); and chromatography column: SunFire@PrepC18 (19 mm × 250 mm). 2. The sample was filtered with a 0.45 μm filter head to prepare a sample solution. 3. Preparative chromatography conditions: a. composition of mobile phases A and B: A: 0.1% trifluoroacetic acid/$H_2O$, and B: $CH_3CN$; b. gradient elution: mobile phase = 5%-45%; c. flow rate: 12 ml/min; d. elution time: 30 min; and retention time: 16 min. Lyophilization was carried out to afford **Compound 1** (400 mg, purity: 99%).

LCMS m/z = 626.9 $[M/3+H]^+$,939.9$[M/2+H]^+$

Example 2:

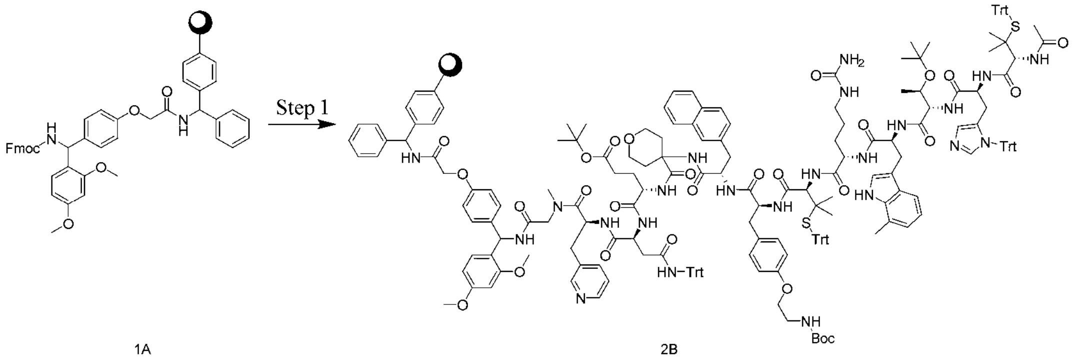


1A 2B

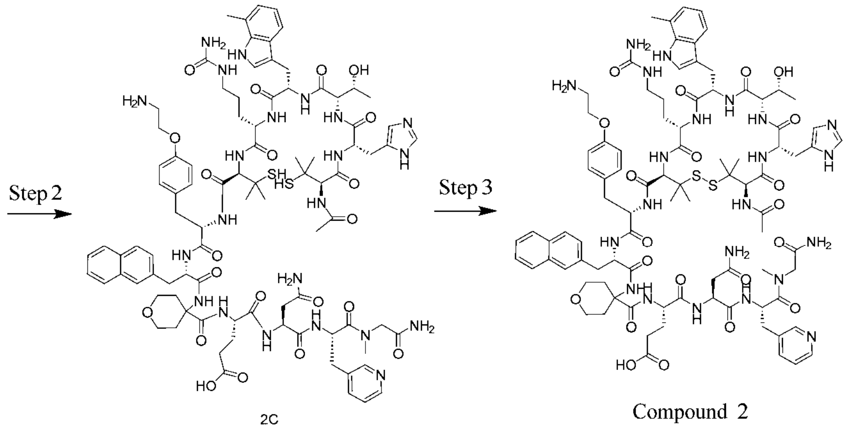


2C Compound 2

Step 1: **2B** was synthesised using standard Fmoc chemistry.

1. Rink Amide MBHA Resin (1 mmol, 1.4 g, sub: 0.7 mmol/g) and dichloromethane as the solvent were added to a reactor, and the resin was swollen for 30 min. 20% piperidine/DMF was added, and the mixture was mixed for 30 minutes.
2. The solvent was pumped off, and the resin was rinsed with DMF five times.
3. Fmoc-Sar-OH-protected amino acid solution was added, and the mixture was mixed for 30 seconds. Then, a coupling reagent was added, and the mixture was bubbled with nitrogen for 1.5 hours. The reaction was monitored with ninhydrin.
4. The solvent was pumped off, and the resin was rinsed with DMF three times.
5. 20% piperidine/DMF was added, and the mixture was mixed for 30 minutes.
6. The solvent was pumped off, and the resin was rinsed with DMF five times.
7. Fmoc-protected amino acid solution was added, and the mixture was mixed for 30 seconds. Then, a coupling reagent was added, and the mixture was bubbled with nitrogen for 1.5 hours. The reaction was monitored with ninhydrin.
8. The solvent was pumped off, and the resin was rinsed with DMF three times.
9. Steps 5-8 were repeated for the coupling of the subsequent amino acid.
10. In the last step, the resin was rinsed with MeOH twice, DCM once, and MeOH twice. The solvent was pumped off under vacuum to give the peptide resin **2B** (6.3 g), which was used directly in the next reaction.

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Cit-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |

(continued)

| Serial No. | Starting material | Coupling reagent |
|---|---|---|
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

Step 2: 60 ml of cleavage solution (91% trifluoroacetic acid + 4% triisopropylsilane + 3% 1,2-ethanedithiol + 2% water) was added to a 100 ml reaction flask, and stirred evenly. Then, the peptide resin **2B** (6.3 g) was added, and the mixture was stirred at room temperature for 2 hours. The resin was filtered to obtain the filtrate, which was added to 500 ml of methyl tert-butyl ether (which was cooled to 0°C in advance). White floccules were precipitated, and centrifuged (3 min at 3000 rpm). The white precipitate was washed with methyl tert-butyl ether three times, and dried in vacuo to give the crude peptide **2C** as an off-white solid (2.5 g), which was used directly in the next reaction.
LCMS m/z = 637.3 $[M/3+H]^+$,955.4$[M/2+H]^+$

Step 3: To a 2 L reaction flask, water (750 ml), acetonitrile (250 ml), and 2C (2 g, 1.06 mmol) were added successively, and stirred evenly. Then, iodine/acetonitrile solution (0.1 mol/L) was slowly added dropwise until the reaction solution turned light yellow. Ascorbic acid was added to quench the reaction, and the resultant was purified by preparative HPLC. Separation method: 1. Instrument: Waters 2767 (preparative liquid chromatographic instrument); and chromatography column: SunFire@PrepC18 (19 mm × 250 mm). 2. The sample was filtered with a 0.45 μm filter head to prepare a sample solution. 3. Preparative chromatography conditions: a. composition of mobile phases A and B: A: 0.1% trifluoroacetic acid/$H_2O$, and B: $CH_3CN$; b. gradient elution: mobile phase = 5%-45%; c. flow rate: 12 ml/min; d. elution time: 30 min; and retention time: 16 min. Lyophilization was carried out to afford **Compound 2** (410 mg, purity: 99%).
LCMS m/z = 636.6 $[M/3+H]^+$,954.4$[M/2+H]^+$

Example 3:

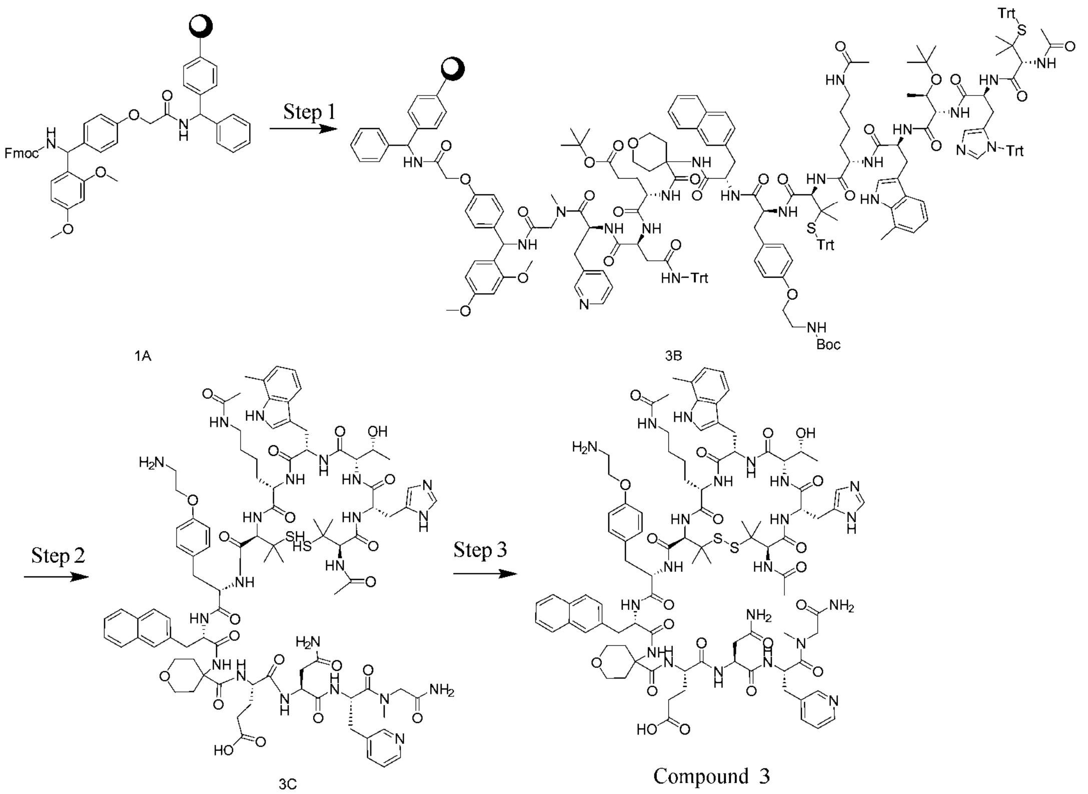


1A

3B

3C

Compound 3

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | FMOC-4-[2-(Boc-amido-ethoxy)]-L-phenylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 3** (50 mg, purity: 95%) was obtained by using the materials in the above table as starting materials and adopting a synthetic method for **Compound 1.**
LCMS m/z = 961.0 $[M/2+H]^+$

Example 4:

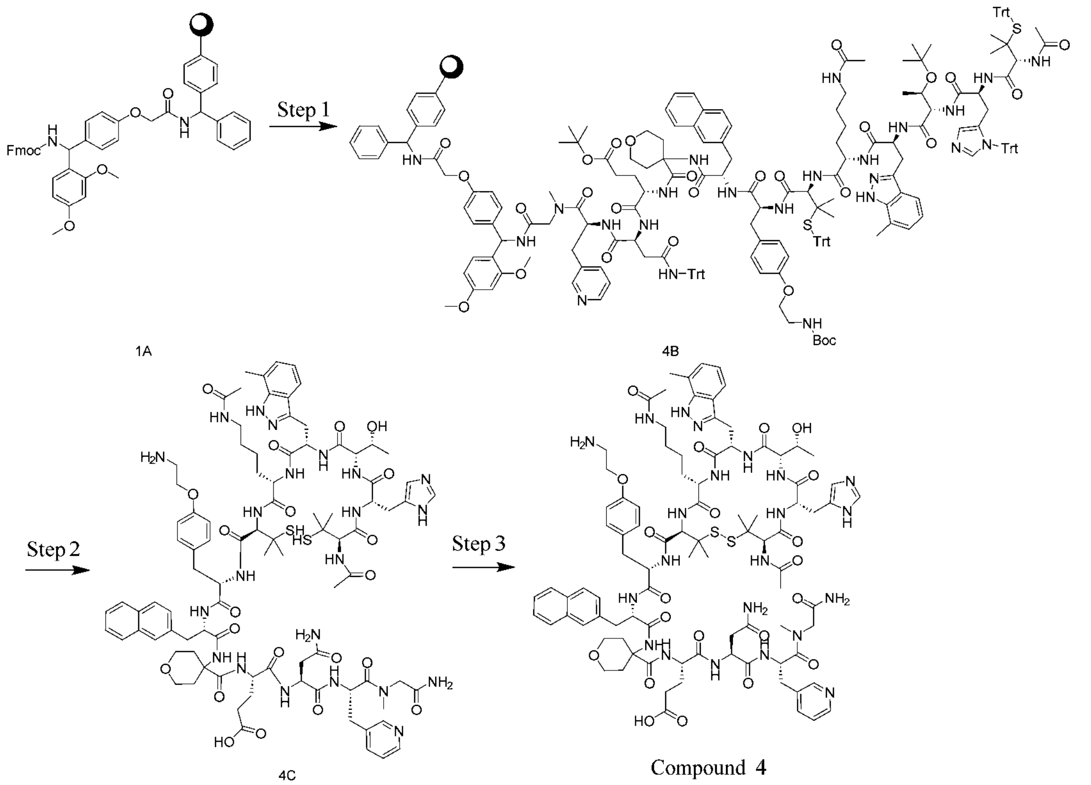


1A

4B

4C

Compound 4

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | **Intermediate 1** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 4** (50 mg, purity: 99%) was obtained by using the materials in the above table as starting materials and adopting a method for **Compound 1.**

LCMS m/z =961.5 [M/2+H]$^+$.

Example 5:

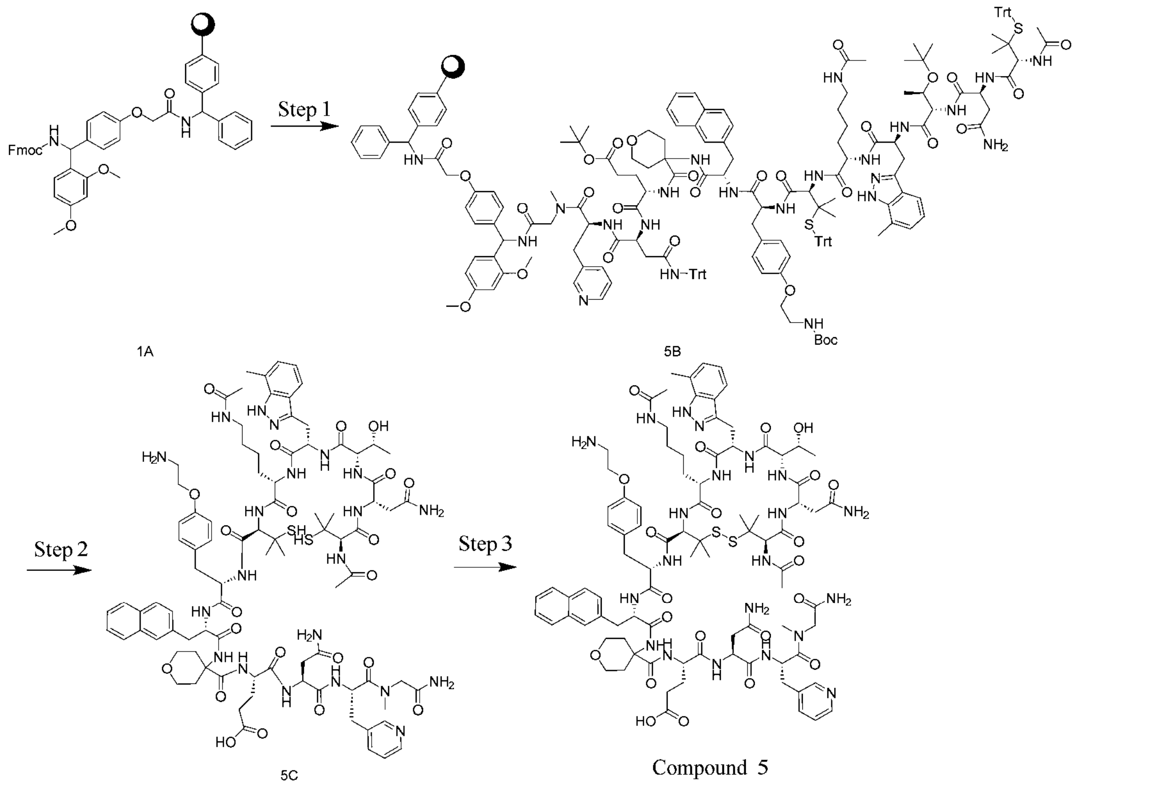


1A

5B

5C

Compound 5

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | **Intermediate 1** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-Asn(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 5** (50 mg, purity: 95%) was obtained by using the materials in the above table as starting materials and adopting a method for **Compound 1.**

LCMS m/z =950.3 $[M/2+H]^+$.

Example 6:

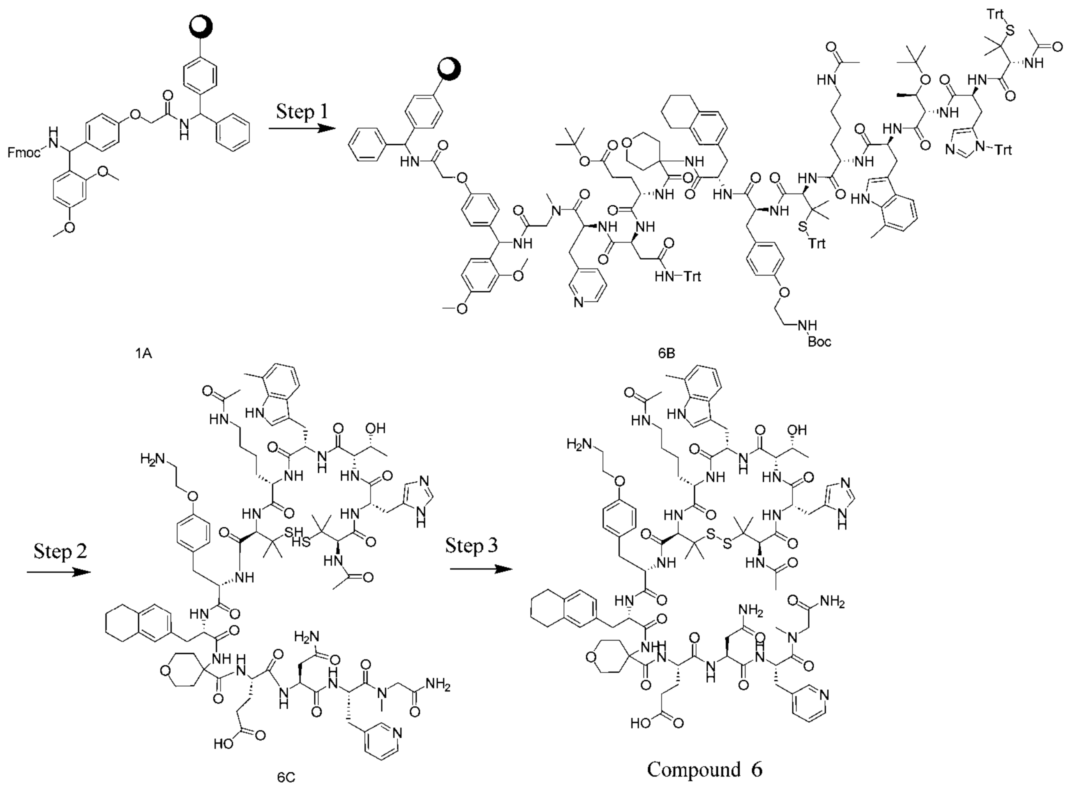


1A

6B

6C

Compound 6

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | **Intermediate 2** (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 6** (50 mg, purity: 94%) was obtained by using the materials in the above table as starting materials and adopting a method for **Compound 1.**

LCMS m/z =963.4 [M/2+H]$^+$.

Example 7:

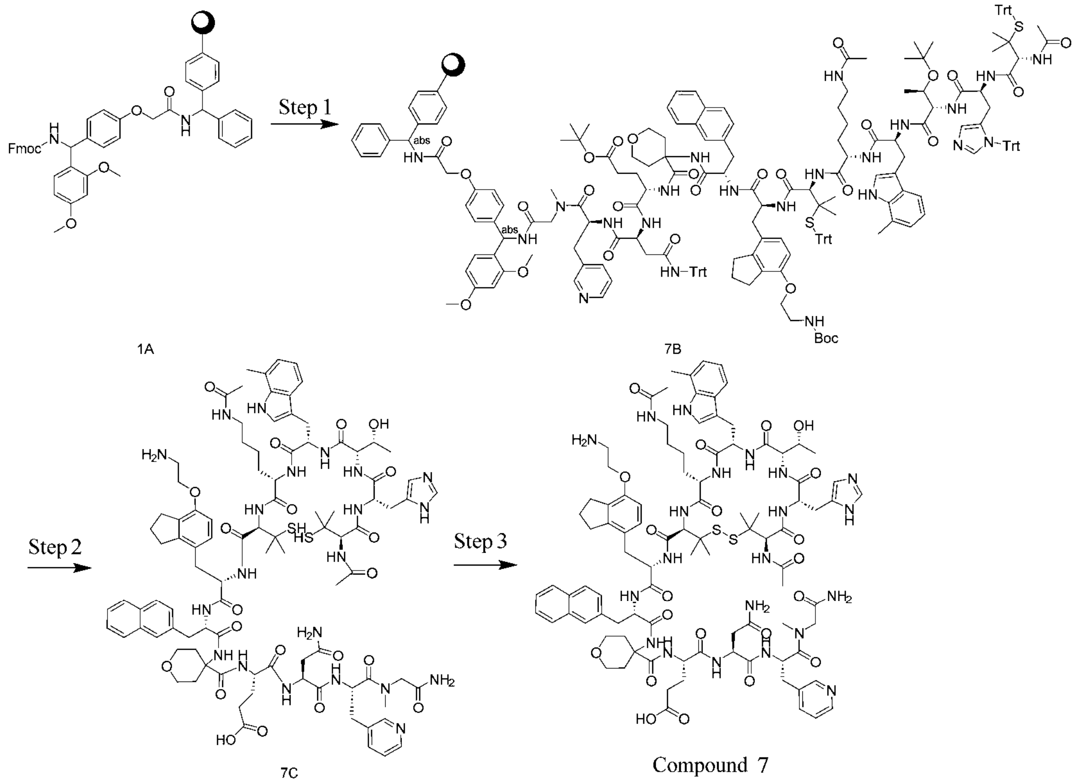

1A
7B
7C
Compound 7

Step 1: **7B** was synthesised using standard Fmoc chemistry.

1. Rink Amide MBHA Resin (1 mmol, 1.4 g, sub: 0.7 mmol/g) and dichloromethane as the solvent were added to a reactor, and the resin was swollen for 30 min. 20% piperidine/DMF was added, and the mixture was mixed for 30 minutes.
2. The solvent was pumped off, and the resin was rinsed with DMF five times.
3. Fmoc-Sar-OH-protected amino acid solution was added, and the mixture was mixed for 30 seconds. Then, a coupling reagent was added, and the mixture was bubbled with nitrogen for 1.5 hours. The reaction was monitored with ninhydrin.
4. The solvent was pumped off, and the resin was rinsed with DMF three times.
5. 20% piperidine/DMF was added, and the mixture was mixed for 30 minutes.
6. The solvent was pumped off, and the resin was rinsed with DMF five times.
7. Fmoc-protected amino acid solution was added, and the mixture was mixed for 30 seconds. Then, a coupling reagent was added, and the mixture was bubbled with nitrogen for 1.5 hours. The reaction was monitored with ninhydrin.
8. The solvent was pumped off, and the resin was rinsed with DMF three times.
9. Steps 5-8 were repeated for the coupling of the subsequent amino acid.
10. In the last step, the resin was rinsed with MeOH twice, DCM once, and MeOH twice. The solvent was pumped off under vacuum to give the peptide resin **7B** (6 g), which was used directly in the next reaction.

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

Step 2: 50 ml of cleavage solution (91% trifluoroacetic acid + 4% triisopropylsilane + 3% 1,2-ethanedithiol + 2% water) was added to a 100 ml reaction flask, and stirred evenly. Then, the peptide resin 7B (6 g) was added, and the mixture was stirred at room temperature for 2 hours. The resin was filtered to obtain the filtrate, which was added to 300 ml of methyl tert-butyl ether (which was cooled to 0°C in advance). White floccules were precipitated, and centrifuged (3 min at 3000 rpm). The white precipitate was washed with methyl tert-butyl ether three times, and dried in vacuo to give the crude peptide 7C as an off-white solid (2.2 g), which was used directly in the next reaction.
LCMS m/z =655.2 $[M/3+H]^+$,982.1$[M/2+H]^+$

Step 3: To a 2 L reaction flask, water (750 ml), acetonitrile (250 ml), and 7C (2 g, 1.02 mmol) were added successively, and stirred evenly. Then, iodine/acetonitrile solution (0.1 mol/L) was slowly added dropwise until the reaction solution turned light yellow. Ascorbic acid was added to quench the reaction, and the resultant was purified by preparative HPLC. Separation method: 1. Instrument: Waters 2767 (preparative liquid chromatographic instrument); and chromatography column: SunFire@PrepC18 (19 mm × 250 mm). 2. The sample was filtered with a 0.45 μm filter head to prepare a sample solution. 3. Preparative chromatography conditions: a. composition of mobile phases A and B: A: 0.1% trifluoroacetic acid/$H_2O$, and B: $CH_3CN$; b. gradient elution: mobile phase = 5%-45%; c. flow rate: 12 ml/min; d. elution time: 30 min; and retention time: 16 min. **Compound 7** (50 mg, purity: 98%) was obtained.

LCMS m/z =981.0 $[M/2+H]^+$.

Example 8:

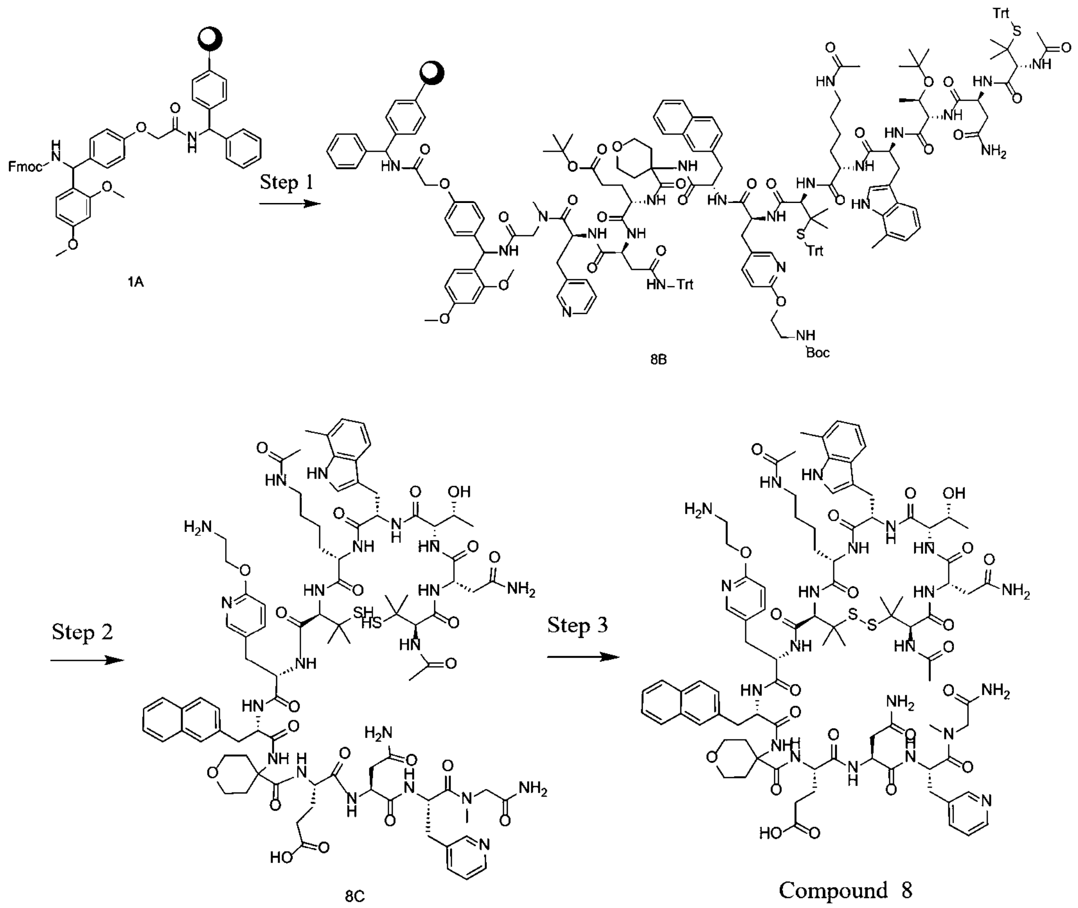


1A

8B

8C

Compound 8

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 4** (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-Asn(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 8** (50 mg, purity: 99%) was obtained by using the materials in the above table as starting materials

and adopting a method for **Compound 1.**

LCMS m/z =950.3 $[M/2+H]^+$.

Example 9:

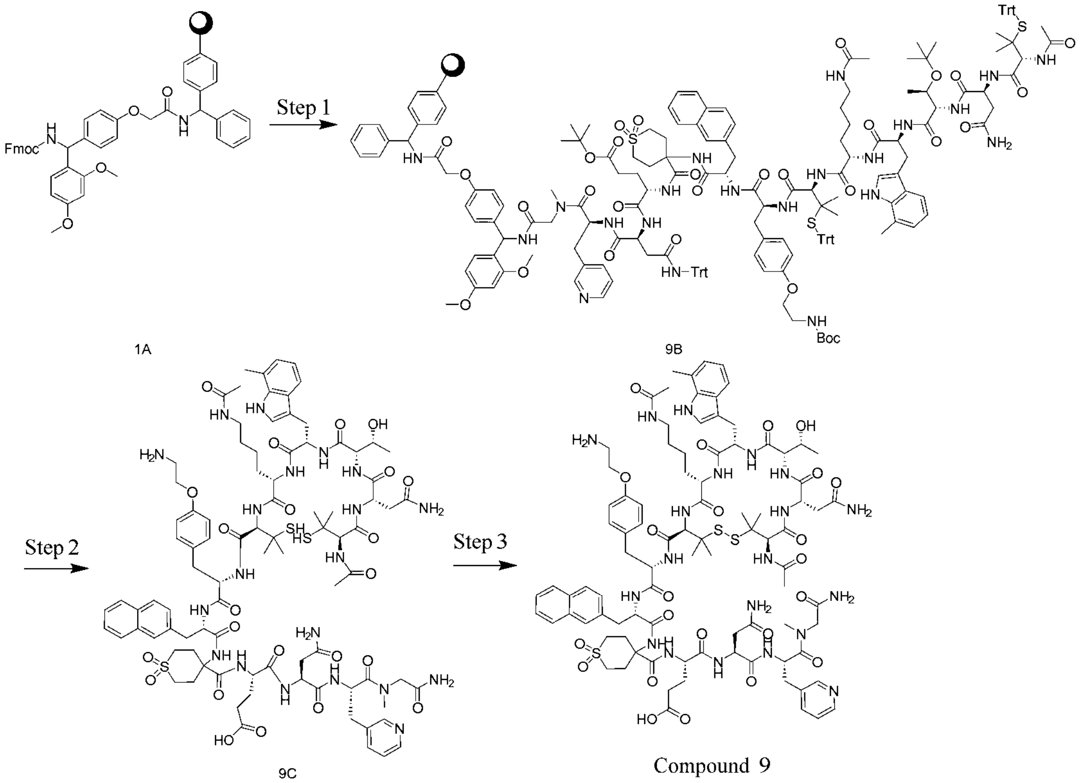


1A

9B

9C

Compound 9

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-p henylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-Asn(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 9** (25 mg, purity: 97%) was obtained by using the materials in the above table as starting materials and adopting a method for **Compound 1.**

LCMS m/z =973.6 [M/2+H]$^+$.

Example 10:

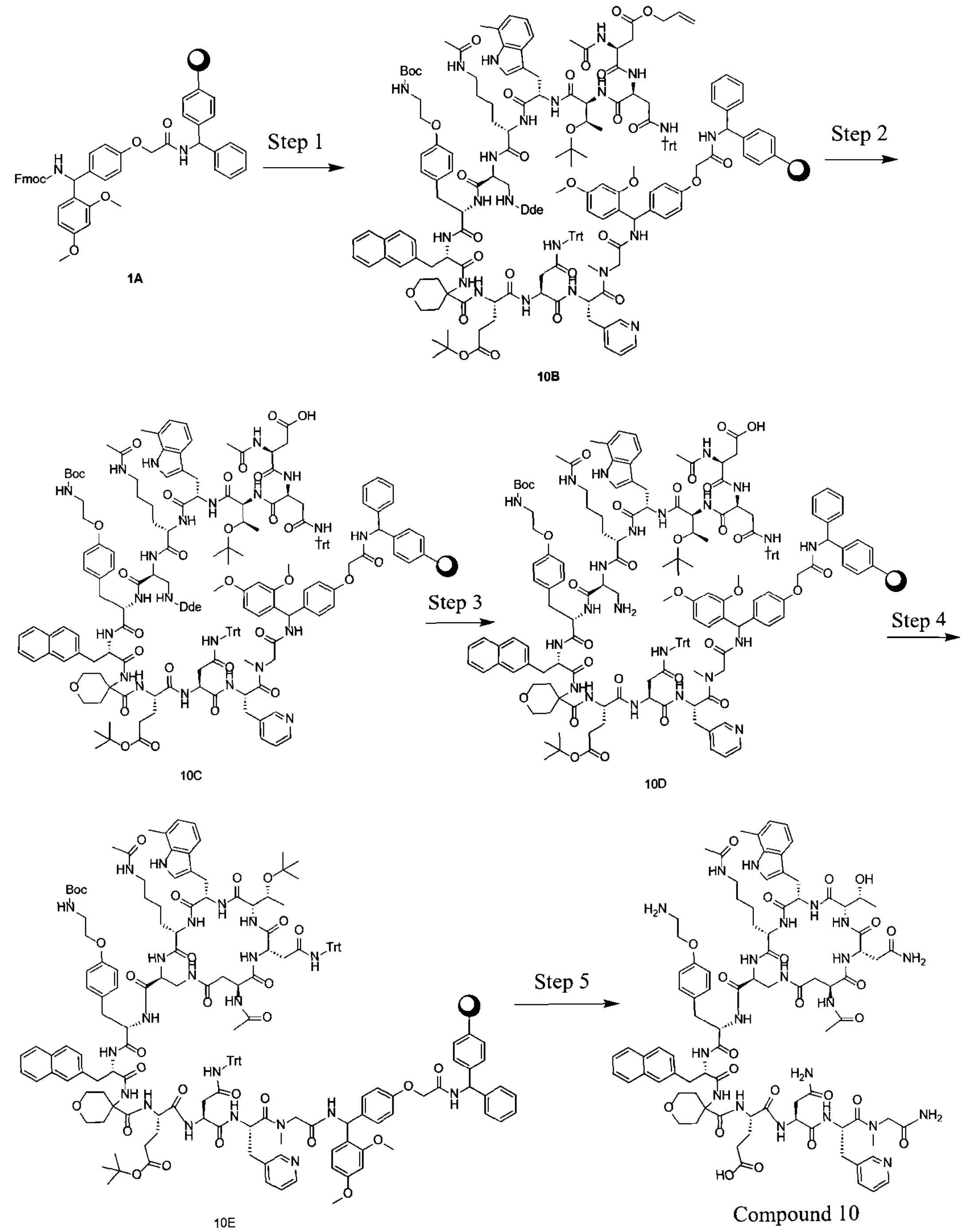


1A

10B

10C

10D

10E

Compound 10

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |

(continued)

| Serial No. | Starting material | Coupling reagent |
|---|---|---|
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amino-ethoxy)]-L-phenylalanine (3.0 eq.) | DIC (3 eq.) and HOBT (3 eq.) |
| 8 | Fmoc-Dap(Dde) -OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-Asn(Trt)-OH (3.0 eq.) | DIC (3 eq.) and HOBT (3 eq.) |
| 13 | Fmoc-Asp(OAll)-OH (2.0 eq.) | DIC (2 eq.) and HOBT (2 eq.) |
| 14 | Acetic anhydride | DIEA |

Step 1: **Compound 10B** was obtained by using the materials in the above table as starting materials and adopting a method for **Compound 1.**

Step 2: 30 ml of dichloromethane was added to a solid-phase synthesis reactor, followed by phenylsilane (1 g) and tetrakis(triphenylphosphine)palladium (462 mg) successively. The mixture was bubbled with nitrogen, and reacted for 8 hours. The resin was washed with dichloromethane 5 times, N,N-dimethylformamide 5 times, 0.5% solution of sodium diethyldithiocarbamate in N,N-dimethylformamide for 15 min + 15 min, and then N,N-dimethylformamide three times to give the peptide resin **10C.**

Step 3: 2% solution of hydrazine hydrate in N,N-dimethylformamide was added to the reactor, and the mixture was reacted for 30 min. The resultant was washed with N,N-dimethylformamide solution 5 times to give the peptide resin **10D.**

Step 4: N-methylpyrrolidone (20 ml), benzotriazole-N,N,N',N'-tetramethylurea hexafluorophosphate (417.2 mg, 1.1 mmol), and DIEA (0.19 ml, 1.1 mmol) were added to the solid-phase synthesis reactor, and the mixture was reacted at room temperature for 16 h. The reaction was monitored with ninhydrin until it was colourless. The solvent was pumped off, and the resin was washed with N,N-dimethylformamide three times, methanol twice, dichloromethane once, and methanol twice. The solvent was pumped off under vacuum to give the peptide resin **10E** (5.2 g).

Step 5: 60 ml of cleavage solution (91% trifluoroacetic acid + 4% triisopropylsilane + 3% 1,2-ethanedithiol + 2% water) was added to a 100 ml reaction flask, and stirred evenly. Then, the peptide resin **10E** (5.2 g) was added, and the mixture was stirred at room temperature for 2 hours. The resin was filtered to obtain the filtrate, which was added to 500 ml of methyl tert-butyl ether (which was cooled to 0°C in advance). White floccules were precipitated, and centrifuged (3 min at 3000 rpm). The white precipitate was washed with methyl tert-butyl ether three times, and dried in vacuo to give the crude peptide **Compound 10** as an off-white solid (1.8 g), which was purified by preparative HPLC. Separation method: 1. Instrument: Waters 2767 (preparative liquid chromatographic instrument); and chromatography column: SunFire@-PrepC18 (19 mm × 250 mm). 2. The sample was filtered with a 0.45 μm filter head to prepare a sample solution. 3. Preparative chromatography conditions: a. composition of mobile phases A and B: A: 0.1% trifluoroacetic acid/$H_2O$, and B: $CH_3CN$; b. gradient elution: mobile phase = 5%-45%; c. flow rate: 12 ml/min; d. elution time: 30 min; and retention time: 17 min. Lyophilization was carried out to afford **Compound 10** (25 mg, purity: 91.9%) as a white solid.

LCMS m/z =1821.3$[M+H]^+$.

Example 11:

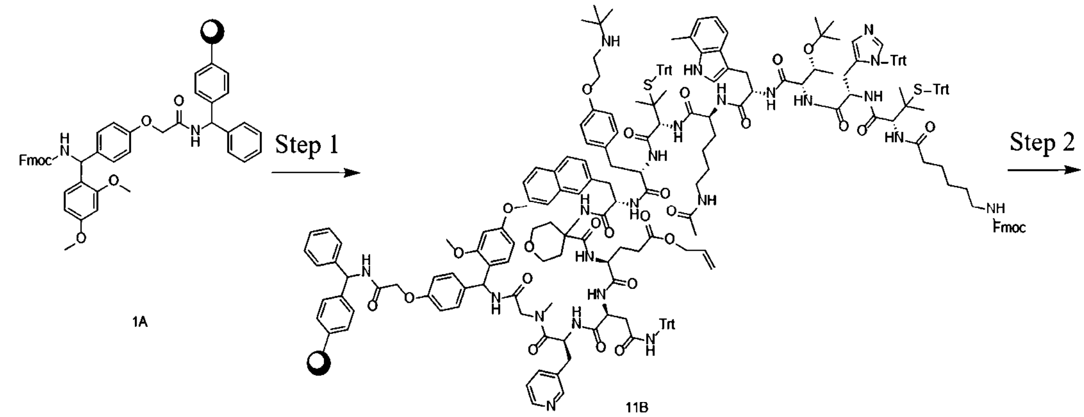


1A

11B

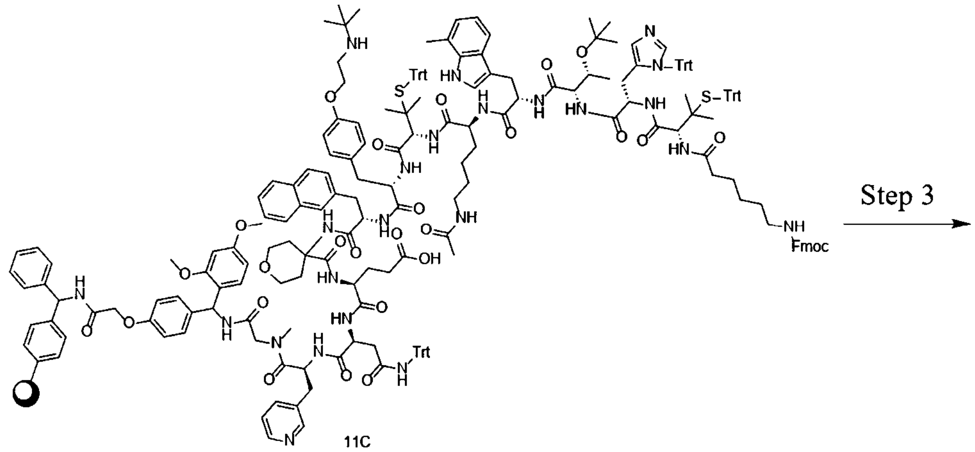


11C

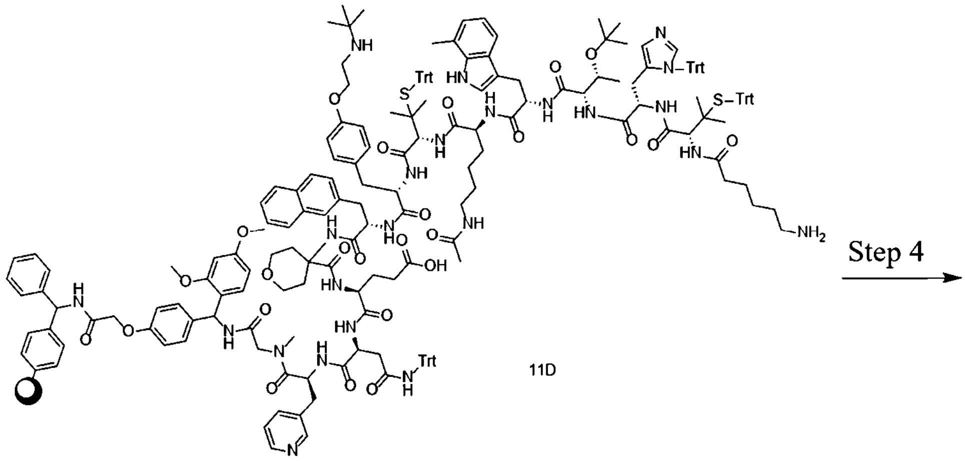


11D

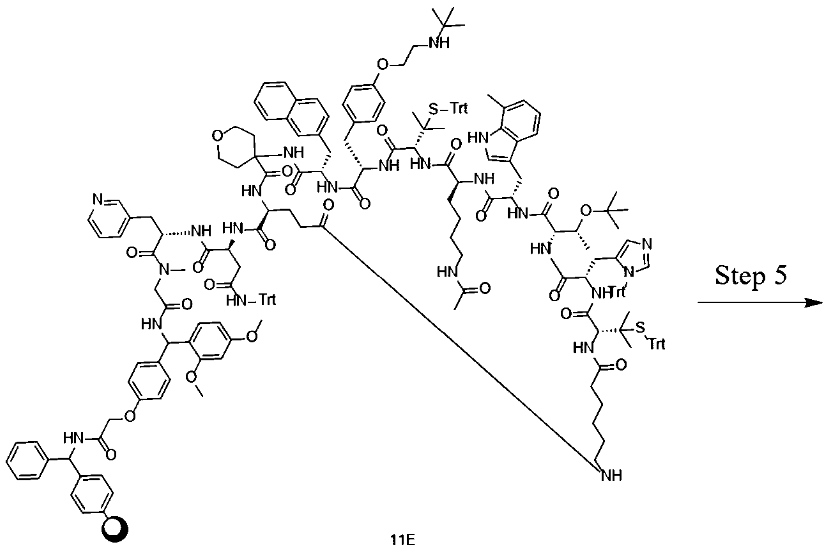


11E

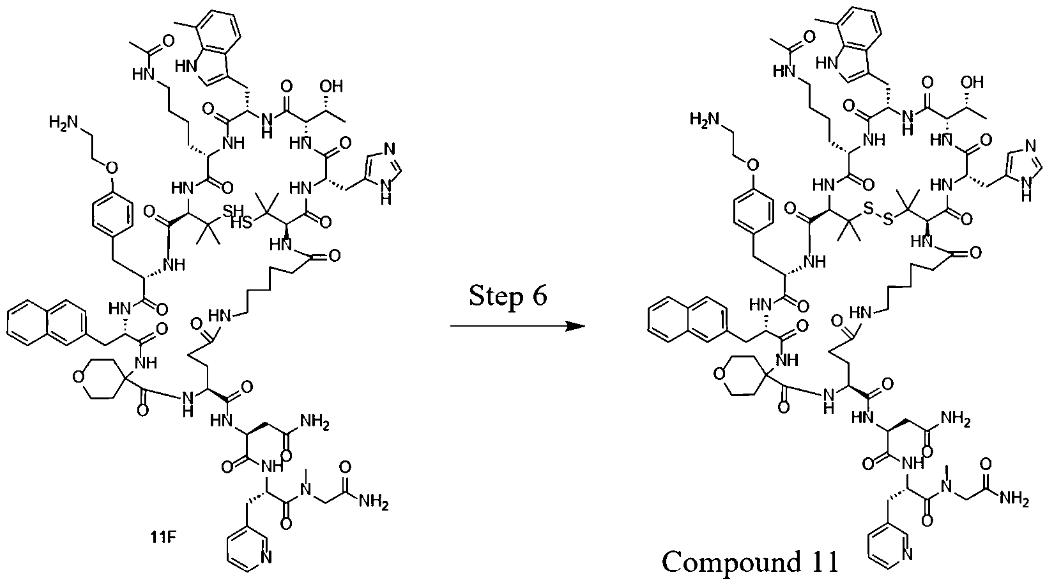


11F

Compound 11

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |

(continued)

| Serial No. | Starting material | Coupling reagent |
|---|---|---|
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-p henylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Fmoc-6-aminohexanoic acid (3.0 eq.) | DIC (3 eq.) and HOBT (3 eq.) |

Step 1: **Compound 11B** was obtained by using the materials in the above table as starting materials and adopting a method for **Compound 1.**

Step 2: 30 ml of dichloromethane was added to a solid-phase synthesis reactor, followed by phenylsilane (1 g) and tetrakis(triphenylphosphine)palladium (462 mg) successively. The mixture was bubbled with nitrogen, and reacted for 8 hours. The resin was washed with dichloromethane 5 times, N,N-dimethylformamide 5 times, 0.5% solution of sodium diethyldithiocarbamate in N,N-dimethylformamide for 15 min + 15 min, and then N,N-dimethylformamide three times to give the peptide resin **11C.**

Step 3: 20% solution of piperidine in N,N-dimethylformamide was added to the reactor, and the mixture was reacted for 30 min. The resultant was washed with N,N-dimethylformamide solution 5 times to give the peptide resin **11D.**

Step 4: N-methylpyrrolidone (20 ml), benzotriazole-N,N,N',N'-tetramethylurea hexafluorophosphate (417.2 mg, 1.1 mmol), and DIEA (0.19 ml, 1.1 mmol) were added to the solid-phase synthesis reactor, and the mixture was reacted at room temperature for 16 h. The reaction was monitored with ninhydrin until it was colourless. The solvent was pumped off, and the resin was washed with N,N-dimethylformamide three times, methanol twice, dichloromethane once, and methanol twice. The solvent was pumped off under vacuum to give the peptide resin **11E.**

Step 5: 50 ml of cleavage solution (91% trifluoroacetic acid + 4% triisopropylsilane + 3% 1,2-ethanedithiol + 2% water) was added to a 100 ml reaction flask, and stirred evenly. Then, the peptide resin **11E** (5 g) was added, and the mixture was stirred at room temperature for 2 hours. The resin was filtered to obtain the filtrate, which was added to 500 ml of methyl tert-butyl ether (which was cooled to 0°C in advance). White floccules were precipitated, and centrifuged (3 min at 3000 rpm). The white precipitate was washed with methyl tert-butyl ether three times, and dried in vacuo to give the crude peptide **11F** as an off-white solid (1.5 g).

Step 6: To a 2 L reaction flask, water (1050 ml), acetonitrile (450 ml), and **11F** (1.5 g, 0.76 mmol) were added successively, and stirred evenly. Then, iodine/acetonitrile solution (0.1 mol/L) was slowly added dropwise until the reaction solution turned light yellow. Ascorbic acid was added to quench the reaction, and the resultant was purified by preparative HPLC. Separation method: 1. Instrument: Waters 2767 (preparative liquid chromatographic instrument); and Chromatographic column: SunFire@PrepC18 (19 mm × 250 mm). 2. The sample was filtered with a 0.45 μm filter head to prepare a sample solution. 3. Preparative chromatography conditions: a. composition of mobile phases A and B: A: 0.1% trifluoroacetic acid/$H_2O$, and B: $CH_3CN$; b. gradient elution: mobile phase = 5%-45%; c. flow rate: 12 ml/min; d. elution time: 30 min; and retention time: 16 min. Lyophilization was carried out to afford Compound **11** (28 mg, purity: 99%) as a white solid.

LCMS m/z =987.5$[M/2+H]^+$ 658.8$[M/3+H]^+$.

Example 12:

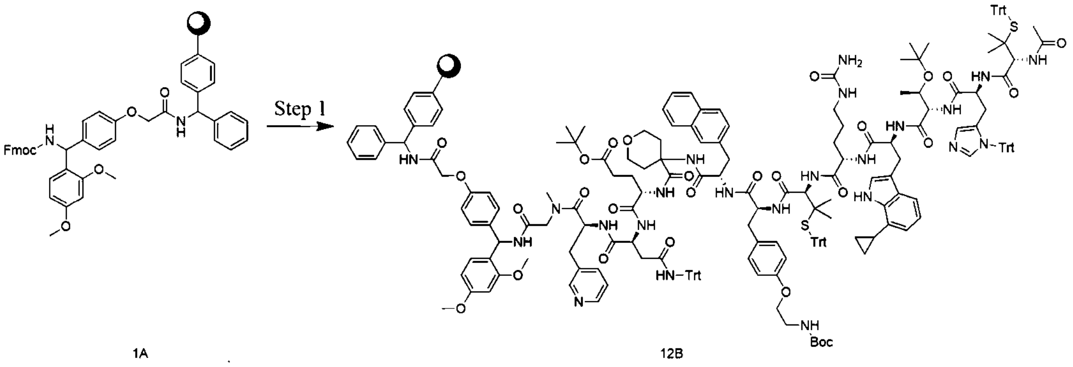


1A 12B

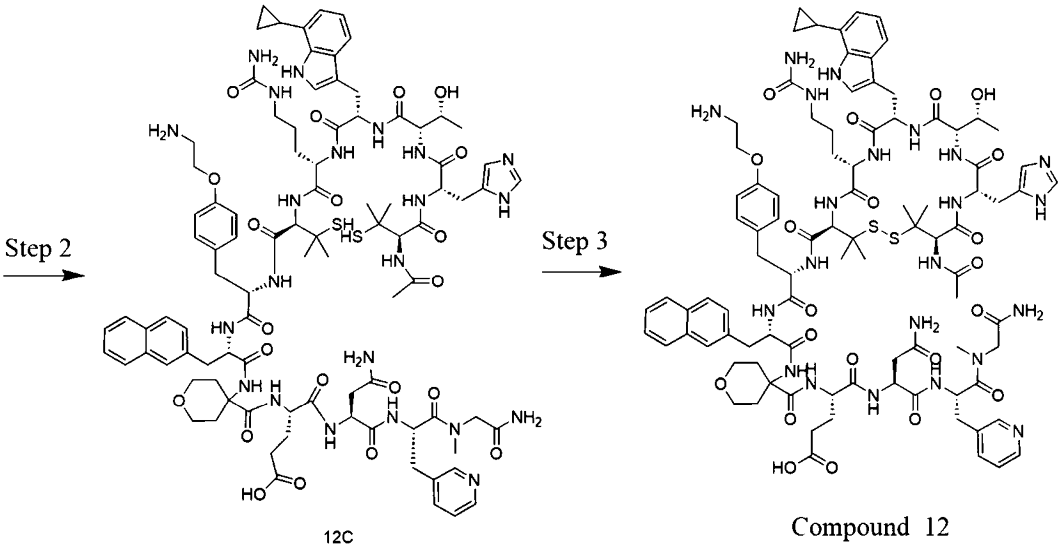


12C Compound 12

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Cit-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 12** (300 mg, purity: 98%) was obtained by using the materials in the above table as starting materials and adopting a method for **Compound 1.**

LCMS m/z =967.5[M/2+H]$^+$ 645.5[M/3+H]$^+$.

Example 13:

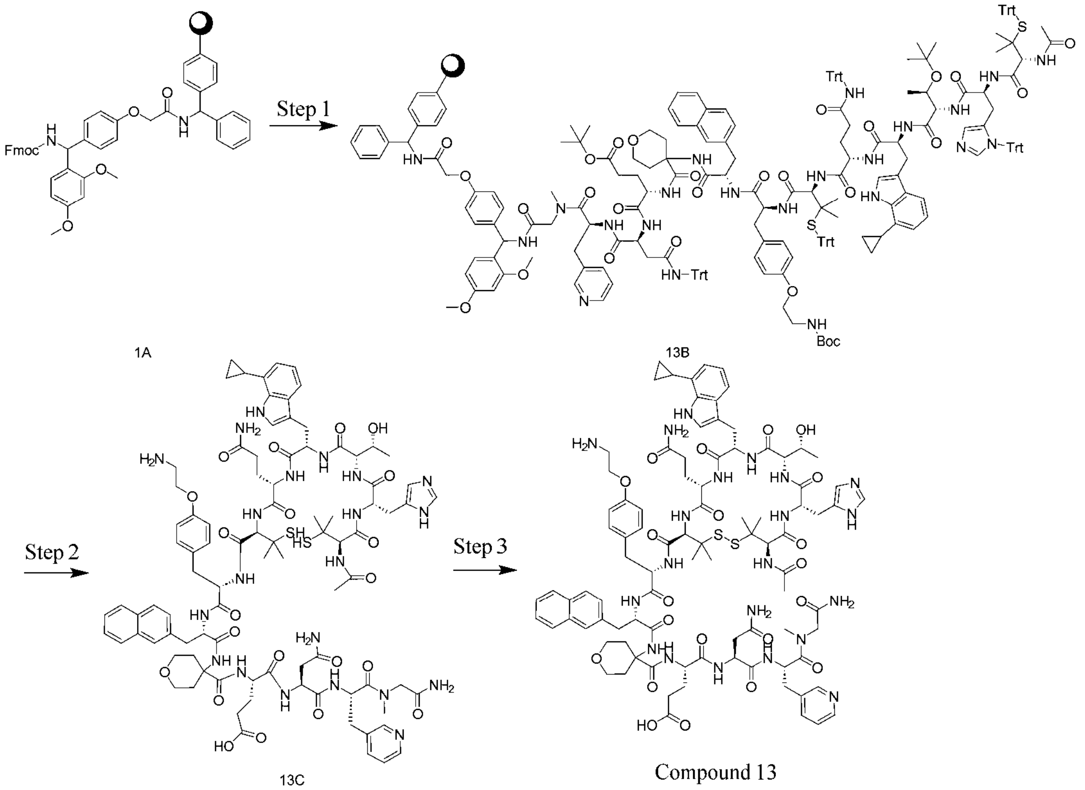


1A

13B

13C

Compound 13

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 13** (350 mg, purity: 98%) was obtained by using the materials in the above table as starting materials and adopting a method for **Compound 1.**

LCMS m/z =953.1[M/2+H]$^+$ 635.9[M/3+H]$^+$.

Example 14:

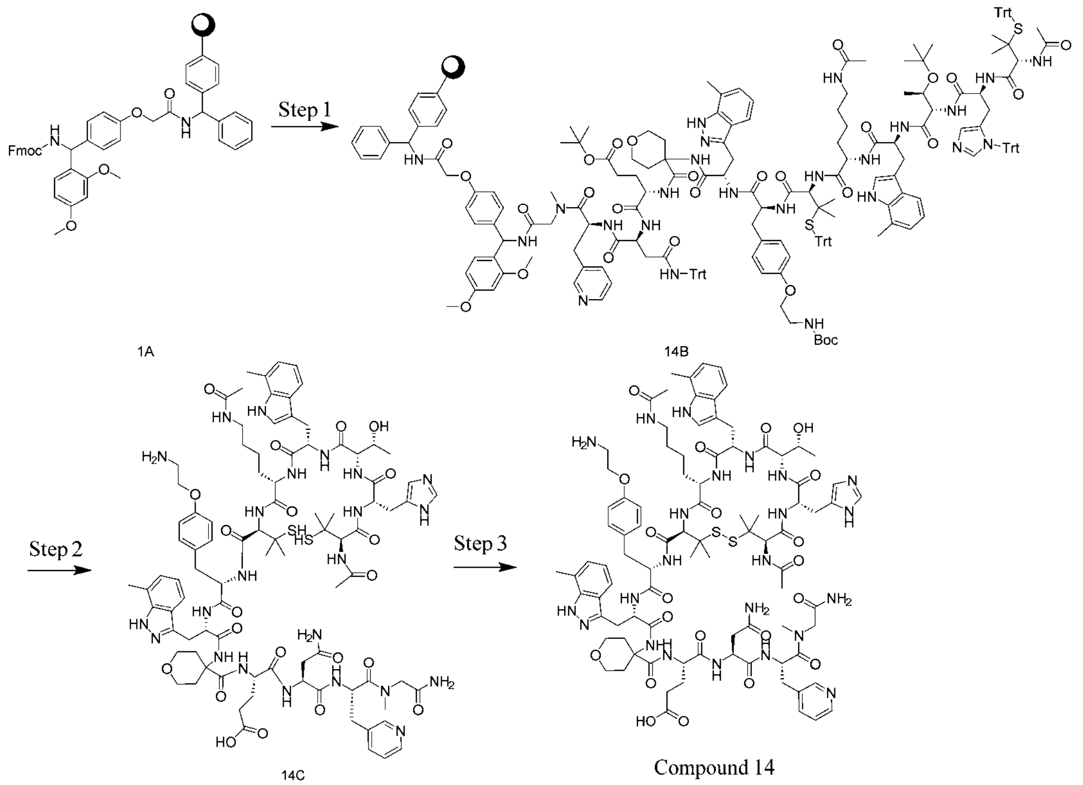


1A 14B 14C Compound 14

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | **Intermediate 1** (2.0 eq.) | DIC (2 eq.) and HOBT (2 eq.) |
| 7 | Fmoc-4-[2-(Boc-amido-ethoxy)]-L-p henylalanine (3.0 eq.) | DIC (3 eq.) and HOBT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 14** (8 mg, purity: 95%) was obtained by using the materials in the above table as starting materials and adopting a synthetic method for **Compound 1.**

LCMS m/z = 963.4 $[M/2+H]^+$ 642.7$[M/3+H]^+$.

Example 15:

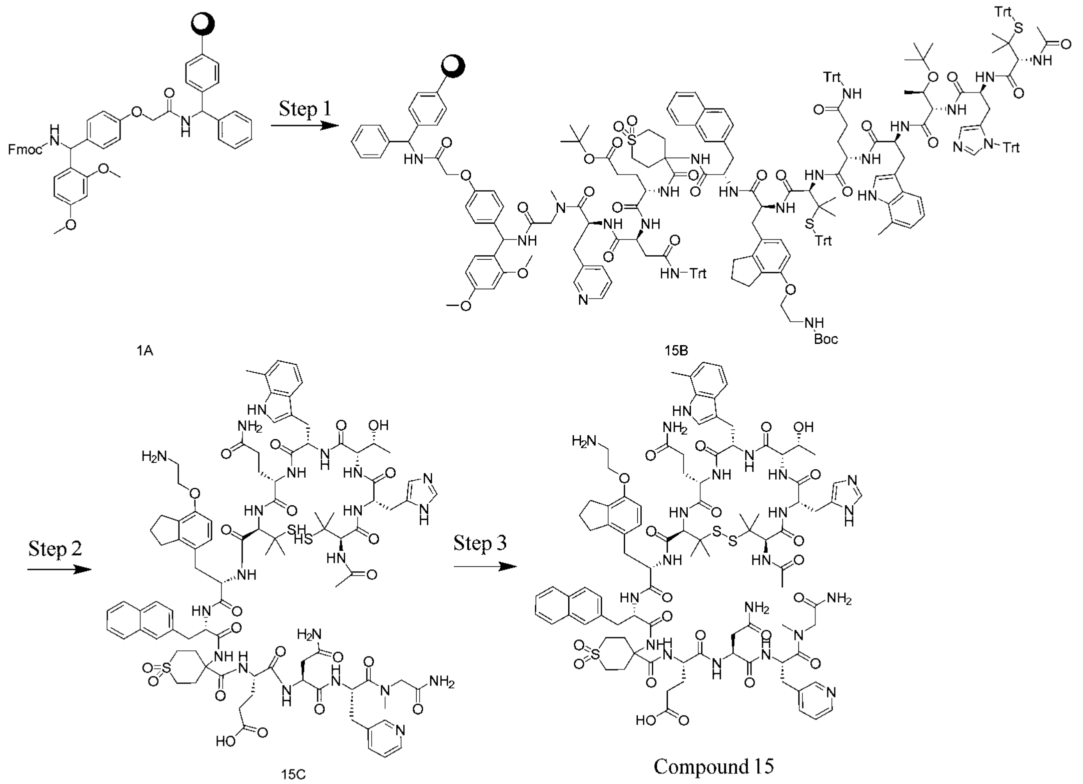


1A

15B

15C

Compound 15

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 15** (130 mg, purity: 98%) was obtained by using the materials in the above table as starting materials and adopting a method for **Compound 1.**

LCMS m/z =984.2 [M/2+H]$^+$, 656.3 [M/3+H]$^+$.

Example 16:

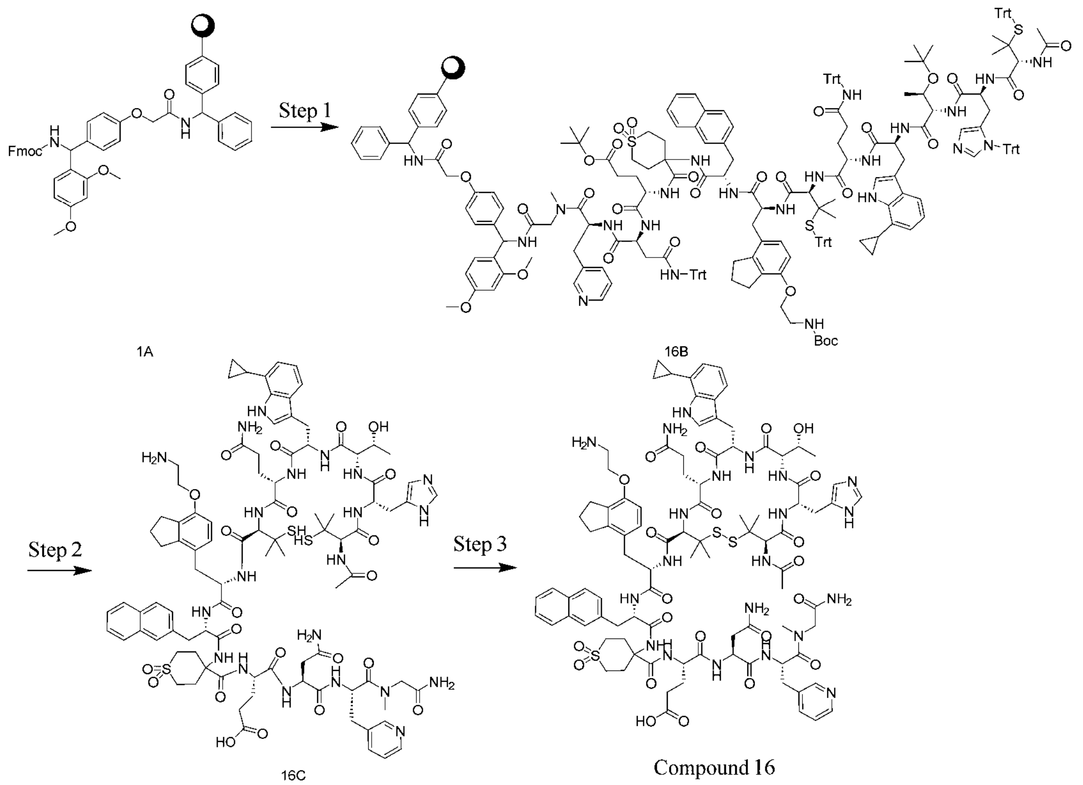


1A

16B

16C

Compound 16

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 16** (400 mg, purity: 94%) was obtained by using the materials in the above table as starting materials and adopting a method for **Compound 1.**

LCMS m/z =997.4 $[M/2+H]^+$, 665.2 $[M/3+H]^+$.

Example 17:

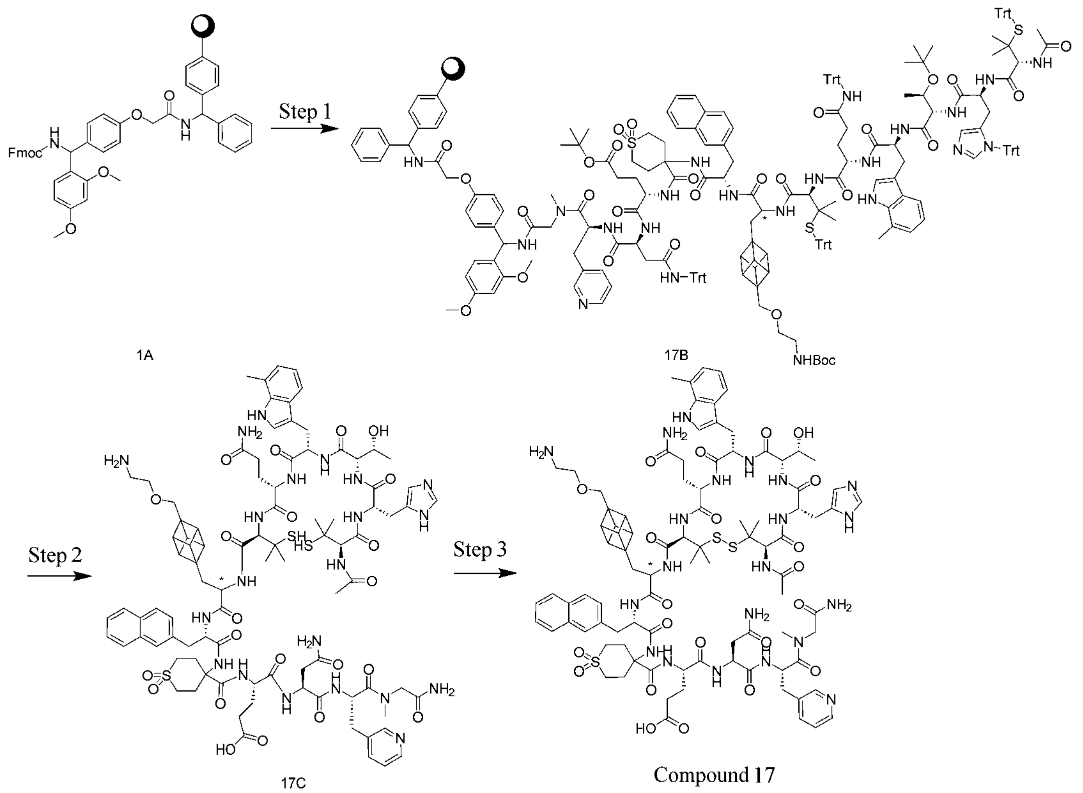


1A

17B

17C

Compound 17

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 7** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 17** (40 mg, purity: 93%) was obtained by using the materials in the above table as starting materials and adopting a method for **Compound 1.**

LCMS m/z =984.0 $[M/2+H]^+$, 656.5 $[M/3+H]^+$.

Example 18:

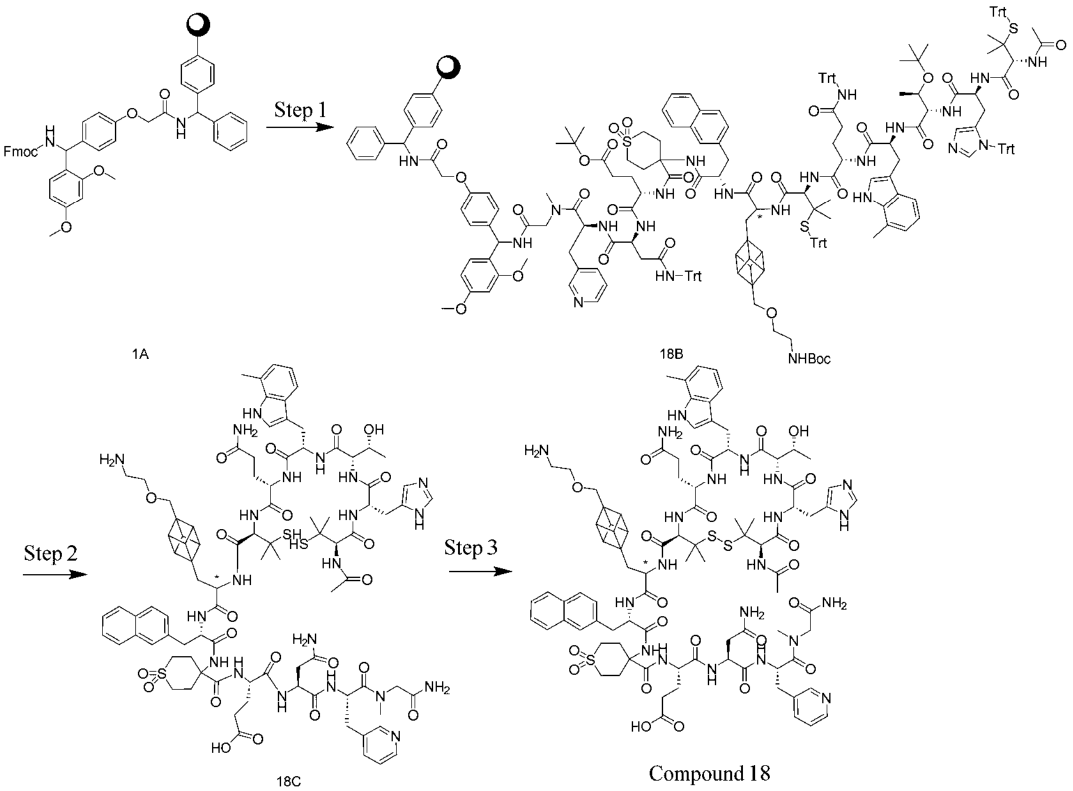


1A 18B 18C Compound 18

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 8** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |

**Compound 18** (40 mg, purity: 94%) was obtained by using the materials in the above table as starting materials and adopting a method for **Compound 1.**

LCMS m/z =984.0 $[M/2+H]^+$, 656.5 $[M/3+H]^+$.

Example 19:

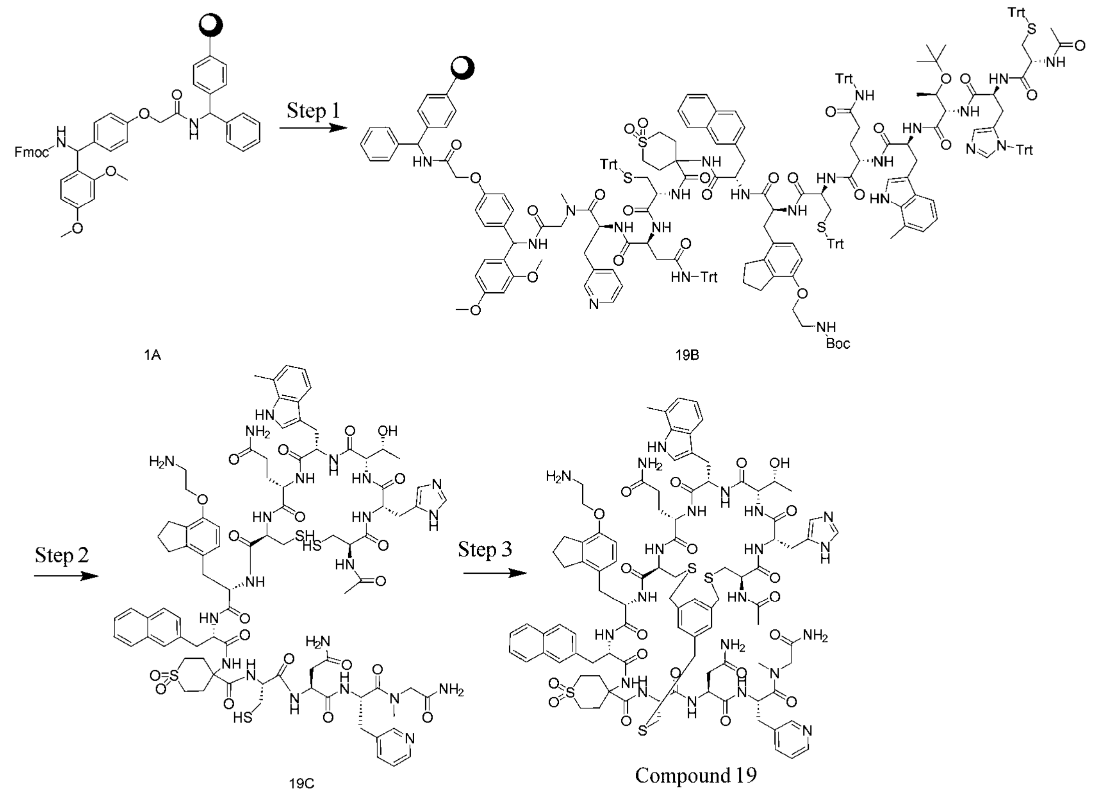


1A 19B 19C Compound 19

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Cys(Trt)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Cys(Trt) -OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Cys(Trt)-OH (2.0 eq.) | DIC (2 eq.) and HOBT (2 eq.) |
| 14 | Acetic anhydride | DIEA |

**Compound 19C** (1.5 g) was obtained by using the materials in the above table as starting materials and adopting a method for **Compound 1.**

Step 3: To a 3 L reaction flask, water (1050 ml), acetonitrile (450 ml), and **19C** (1.5 g, 0.8 mmol) were added successively, and stirred evenly. Then, 1,3,5-tri(bromomethyl)benzene (0.26 g, 0.8 mmol) was added. The pH was adjusted to 8 with ammonium bicarbonate. The mixture was reacted for 16 h and purified by preparative HPLC. Separation

method: 1. Instrument: Waters 2767 (preparative liquid chromatographic instrument); and Chromatographic column: SunFire@PrepC18 (19 mm×250 mm). 2. The sample was filtered with a 0.45 μm filter head to prepare a sample solution. 3. Preparative chromatography conditions: a. composition of mobile phases A and B: A: 0.1% trifluoroacetic acid/$H_2O$, and B: $CH_3CN$; b. gradient elution: mobile phase = 5%-45%; c. flow rate: 12 ml/min; d. elution time: 30 min; and retention time: 17 min. Lyophilization was carried out to afford **Compound 19** (50 mg, purity: 97%) as a white solid.

LCMS m/z =1001.5 [M/2+H]$^+$, 667.8 [M/3+H]$^+$.

Example 20:

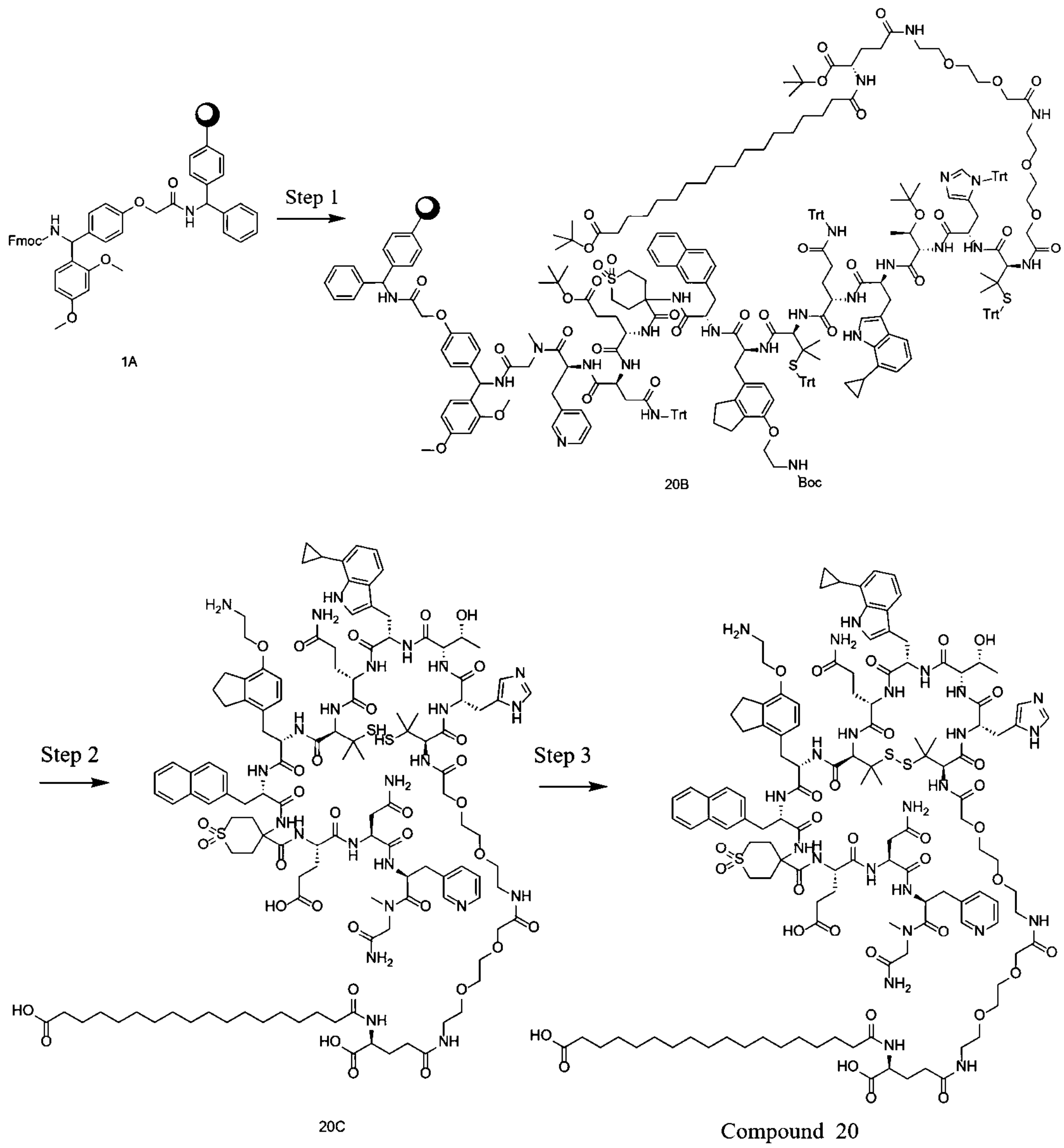


| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |

(continued)

| Serial No. | Starting material | Coupling reagent |
|---|---|---|
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Fmoc-AEEA-OH (3.0 eq.) | DIC (3 eq.) and HOBT (3 eq.) |
| 15 | Fmoc-AEEA-OH (3.0 eq.) | DIC (3 eq.) and HOBT (3 eq.) |
| 16 | Fmoc-Glu-OtBu (3.0 eq.) | DIC (3 eq.) and HOBT (3 eq.) |
| 17 | Mono-tert-butyl octadecanedioate (3.0 eq.) | DIC (3 eq.) and HOBT (3 eq.) |

**Compound 20** (40 mg, purity: 96%) was obtained by using the materials in the above table as starting materials and adopting a method for **Compound 1.**

LCMS m/z = 890.1 $[M/3+H]^+$.

Example 21:

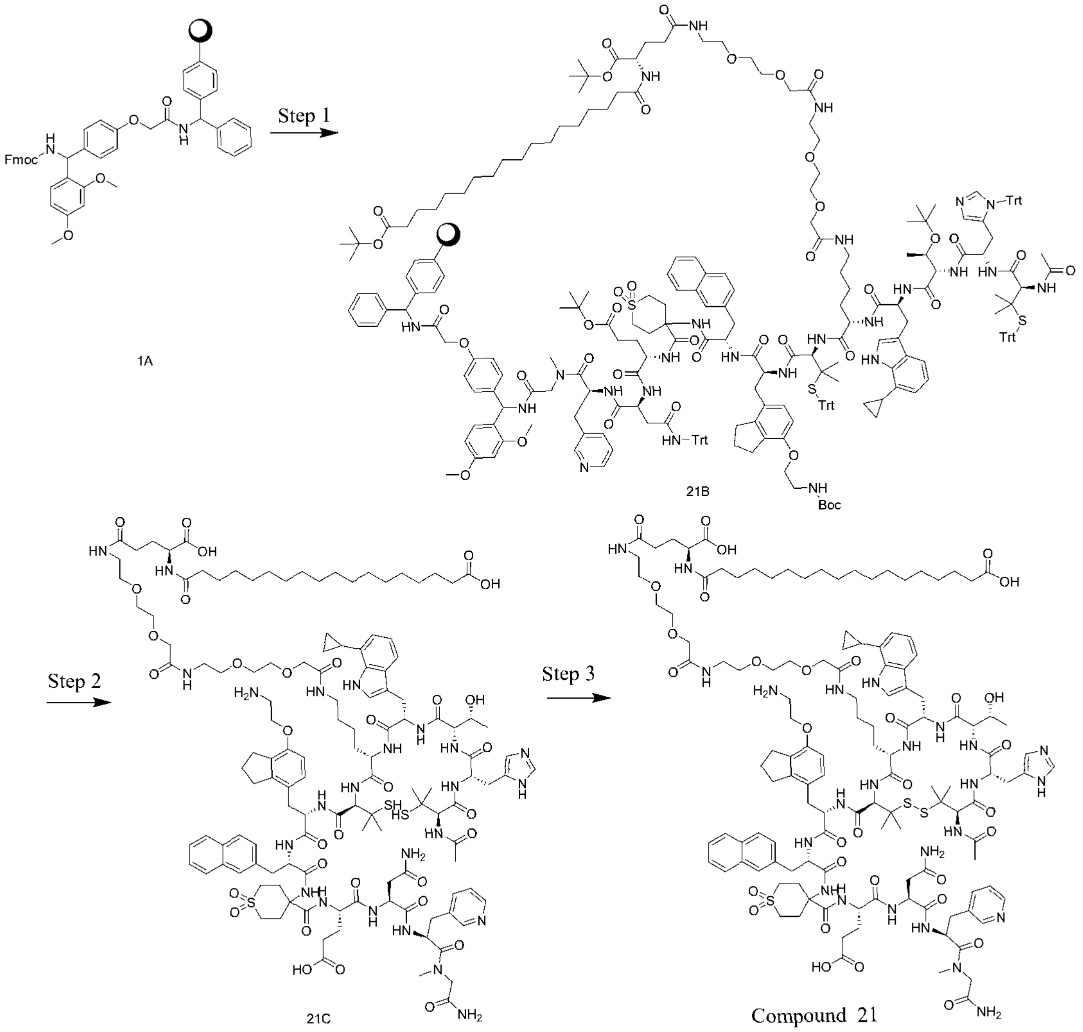


1A

21B

21C

Compound 21

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Lys(Dde)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |
| 15 | Fmoc-AEEA-OH (3.0 eq.) | DIC (3 eq.) and HOBT (3 eq.) |
| 16 | Fmoc-AEEA-OH (3.0 eq.) | DIC (3 eq.) and HOBT (3 eq.) |

(continued)

| Serial No. | Starting material | Coupling reagent |
|---|---|---|
| 17 | Fmoc-Glu-OtBu (3.0 eq.) | DIC (3 eq.) and HOBT (3 eq.) |
| 18 | Mono-tert-butyl octadecanedioate (3.0 eq.) | DIC (3 eq.) and HOBT (3 eq.) |

Dde was removed with 2% hydrazine hydrate/DMF after solid-phase synthesis was carried out to Step 14 by using the materials in the above table as starting materials and adopting a method for **Compound 1.** Then, the synthesis was continued to be carried out using a method for **Compound 1** to give **Compound 21**(30 mg, purity: 97%).

LCMS m/z = 904.0 $[M/3+H]^+$.

Example 22:

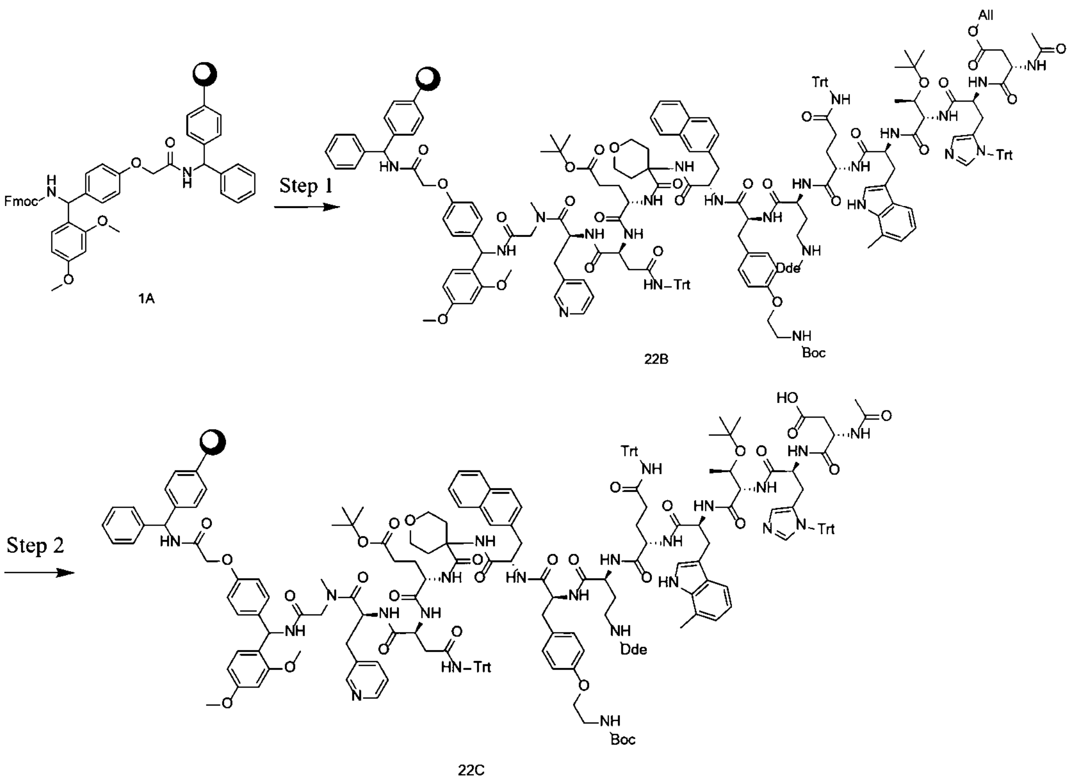


1A

22B

22C

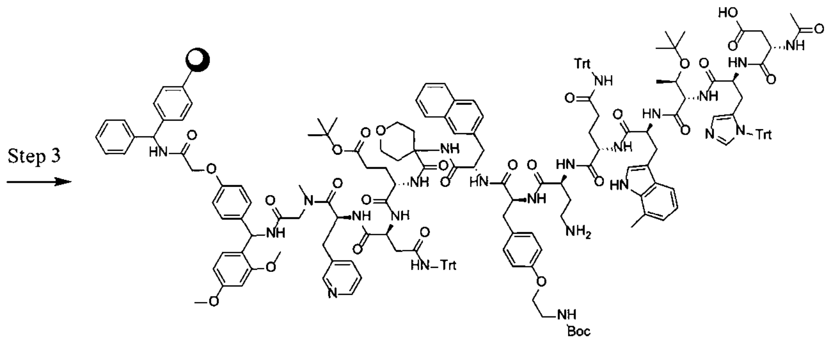


22D

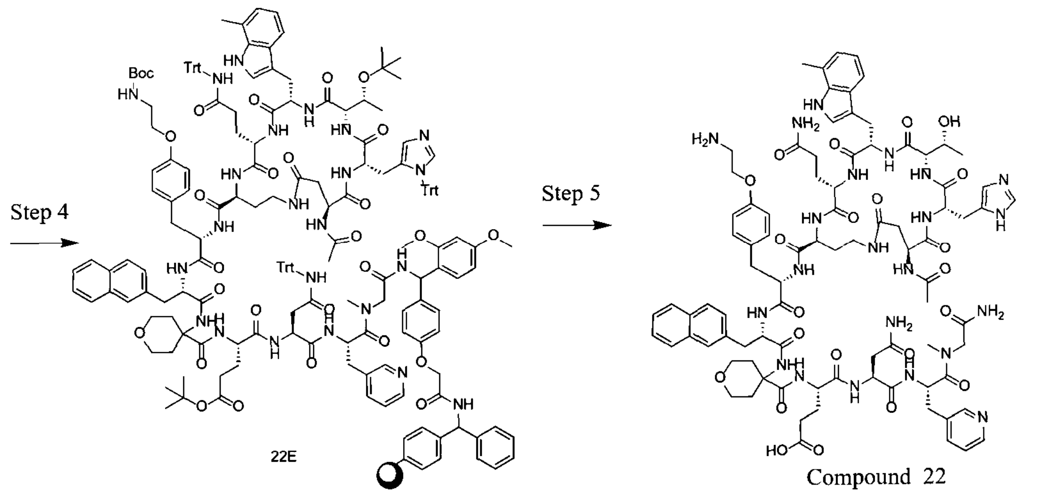


22E

Compound 22

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOBT (3 eq.) |
| 8 | Fmoc-Dab(Dde) -OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Asp(OAll)-OH (2.0 eq.) | DIC (2 eq.) and HOBT (2 eq.) |
| 14 | Acetic anhydride | DIEA |

**Compound** 22 (15 mg, purity: 87%) was obtained by using the materials in the above table as starting materials and adopting a method for **Compound 10.**

LCMS m/z = 908.7[M/2+H]$^+$.

Example 23:

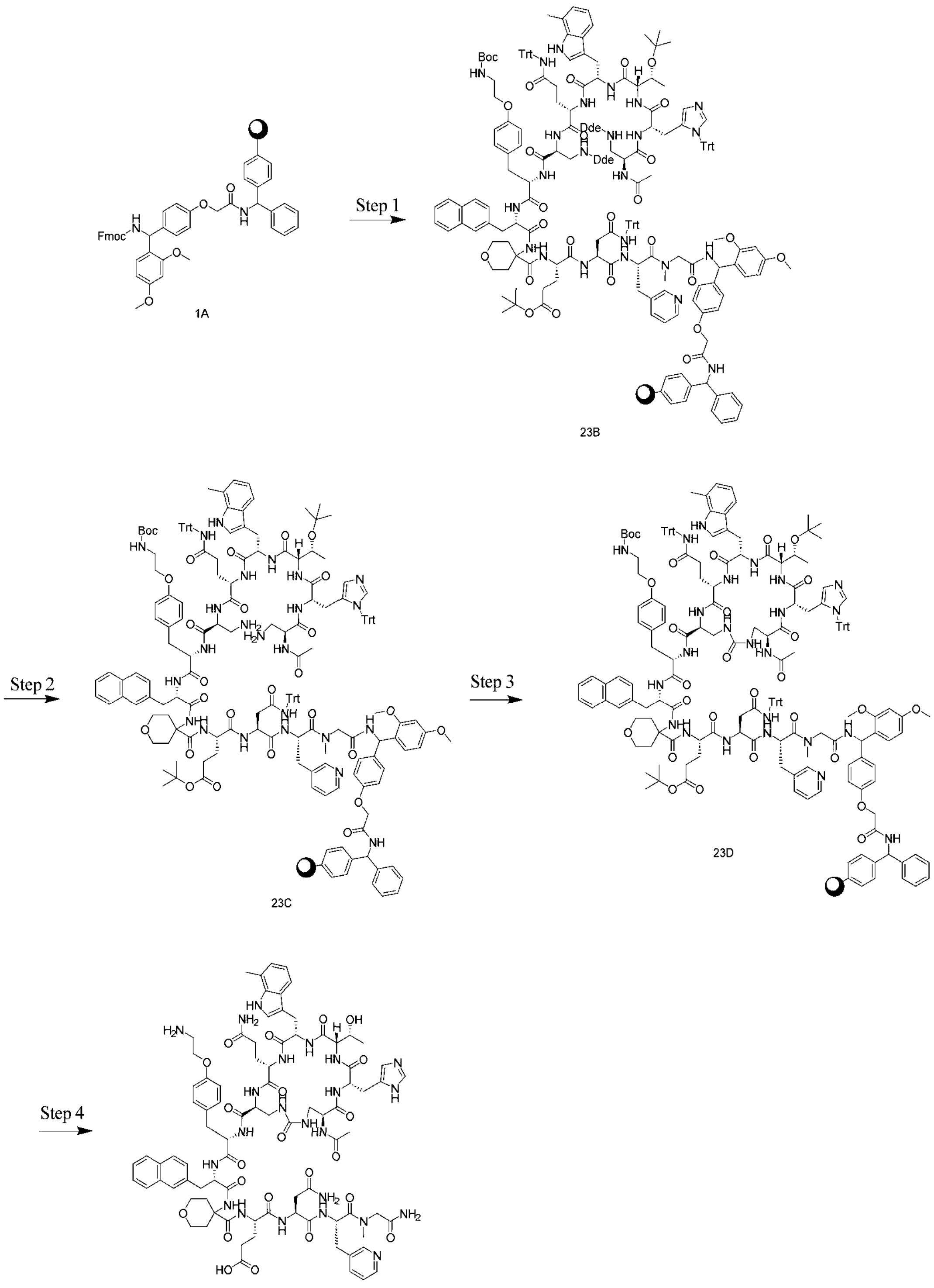


1A

23B

23C

23D

Compound 23

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |

(continued)

| Serial No. | Starting material | Coupling reagent |
|---|---|---|
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOBT (3 eq.) |
| 8 | Fmoc-Dap(Dde) -OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Dap(Dde) -OH (2.0 eq.) | DIC (2 eq.) and HOBT (2 eq.) |
| 14 | Acetic anhydride | DIEA |
| 15 | N,N'-carbonyldiimidazole (1.1 eq.) | DIEA (2.0 eq.) |

Dde was removed with 2% hydrazine hydrate/DMF after solid-phase synthesis was carried out to Step 14 by using the materials in the above table as starting materials and adopting a method for **Compound 1.** Then, the synthesis was continued to be carried out using a method for **Compound 1** to give **Compound 23**(25 mg, purity: 95%).

LCMS m/z =909.2 [M/2+H]$^+$.

Example 24:

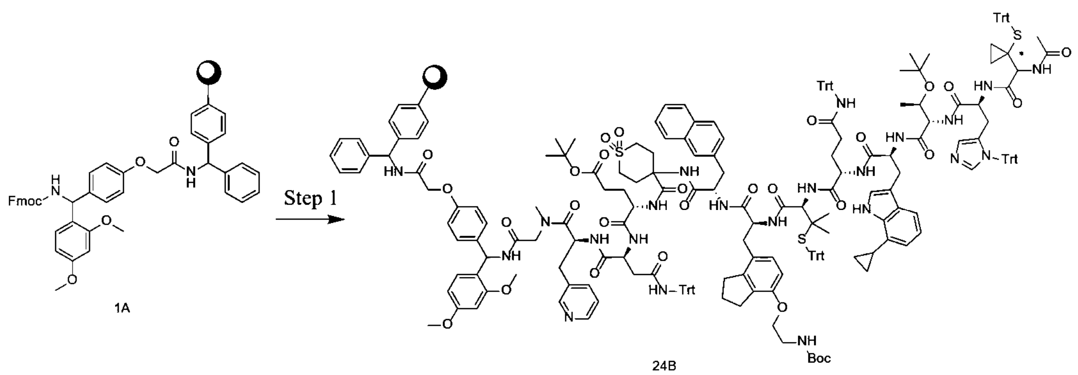


1A

24B

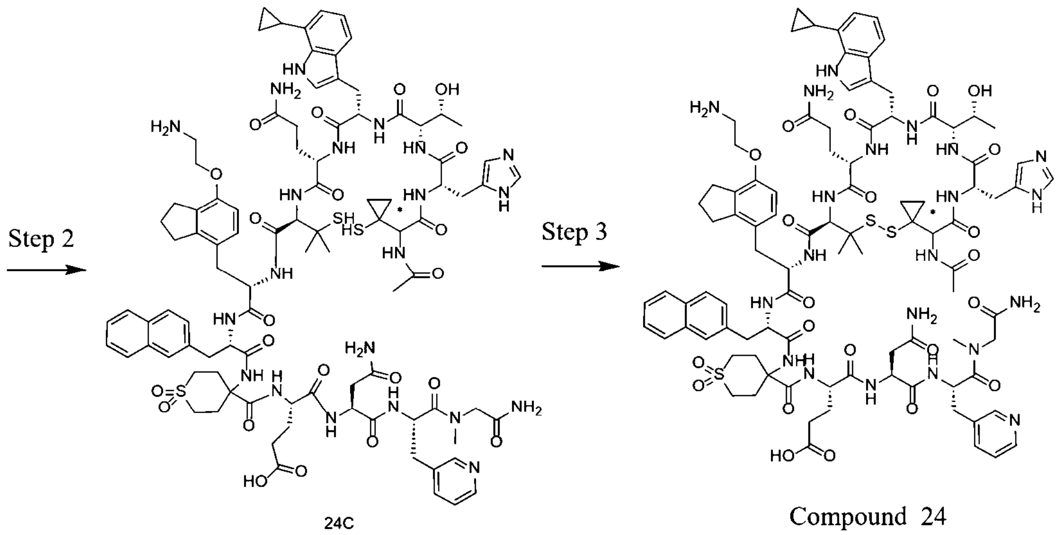


24C

Compound 24

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOAT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOAT (2 eq.) |
| 8 | Fmoc-Pen(Trt)-OH (2.0 eq.) | DIC (2 eq.) and HOAT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOAT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | **Intermediate 9** (2.0 eq.) | DIC (2 eq.) and HOAT (2 eq.) |
| 14 | Acetic anhydride | DIEA |

**Compound 24** (180 mg, purity: 96%) was obtained by using the materials in the above table as starting materials and adopting a method for **Compound 1.**

LCMS m/z =996.2 $[M/2+H]^+$, 664.6 $[M/3+H]^+$.

Example 25:

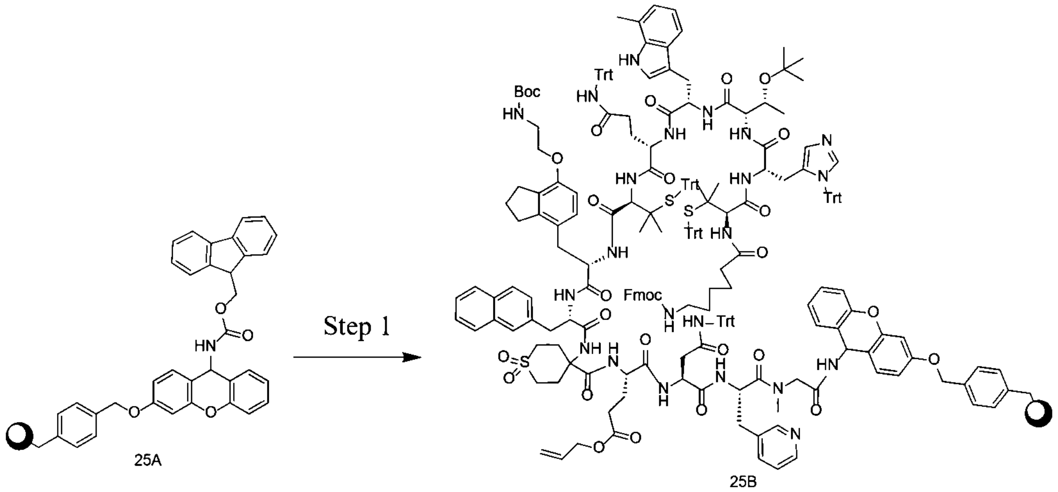


25A 25B

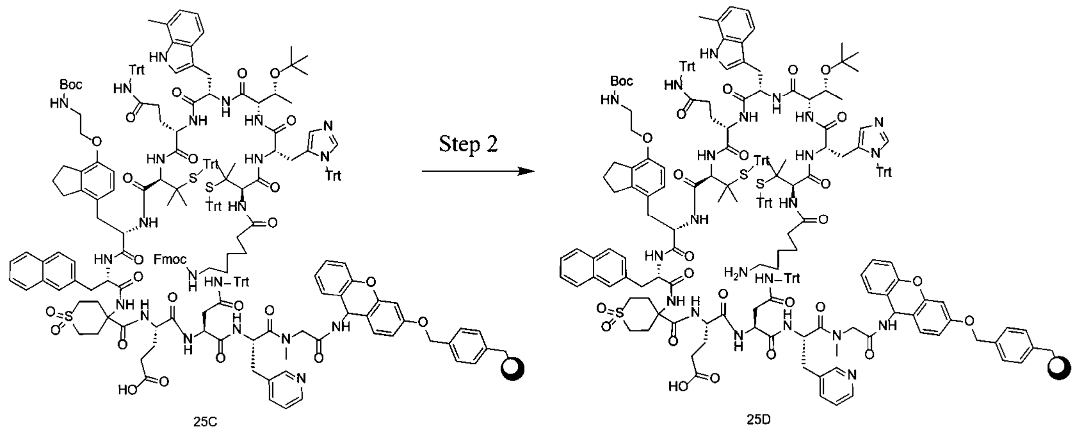


25C 25D

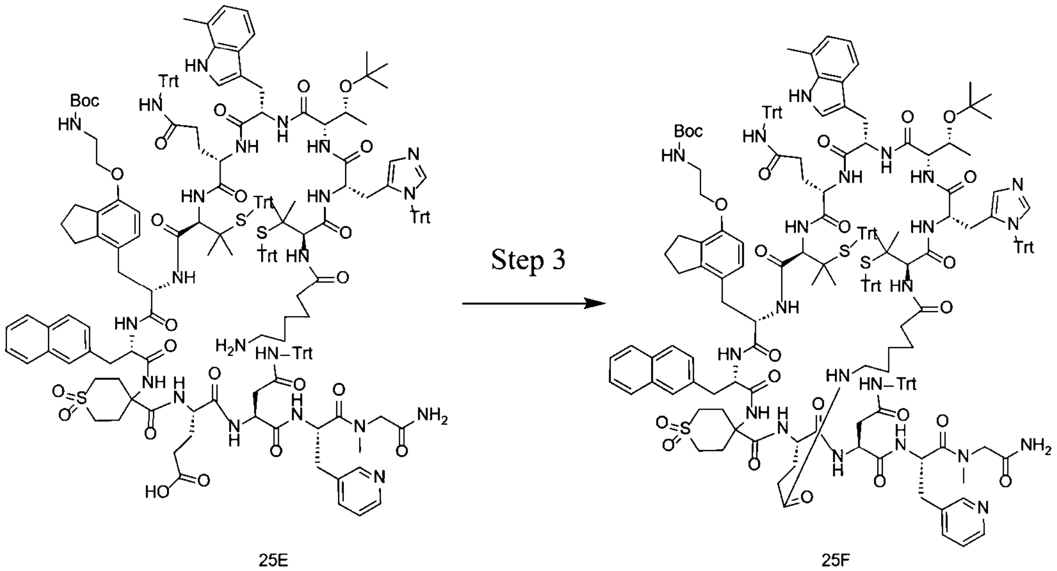


25E 25F

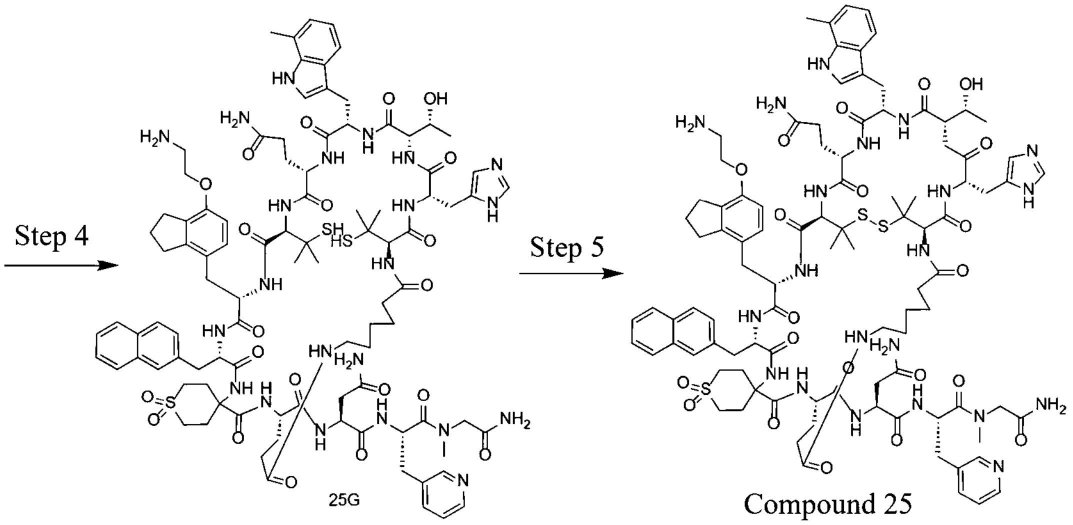


25G

Compound 25

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | **Intermediate 5** (2.0 eq.) | DIC (2 eq.) and HOAT (2 eq.) |
| 6 | Fmoc-2-Nal-OH (3.0 eq.) | DIC (3 eq.) and HOBT (3 eq.) |
| 7 | **Intermediate 3** (3.0 eq.) | DIC (3 eq.) and HOAT (3 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOAT (2 eq.) |
| 14 | Fmoc-6-aminohexanoic acid (3.0 eq.) | DIC (3 eq.) and HOBT (3 eq.) |

Step 1: **Compound 25B** was obtained by using the materials in the above table as starting materials and adopting a method for **Compound 1.**

Step 2: 30 ml of dichloromethane was added to a solid-phase synthesis reactor, followed by phenylsilane (1 g) and tetrakis(triphenylphosphine)palladium (462 mg) successively. The mixture was bubbled with nitrogen, and reacted for 8 hours. The resin was washed with dichloromethane 5 times, N,N-dimethylformamide 5 times, 0.5% solution of sodium diethyldithiocarbamate in N,N-dimethylformamide for 15 min + 15 min, and then N,N-dimethylformamide three times to give the peptide resin **25C.**

Step 3: 20% solution of piperidine in N,N-dimethylformamide was added to the reactor, and the mixture was reacted for 30 min, washed with N,N-dimethylformamide solution 5 times, and washed with methanol three times. The solvent was pumped off under vacuum to give the peptide resin **25D** (5 g).

Step 4: 50 ml of cleavage solution (2% trifluoroacetic acid + 98% dichloromethane) was added to a 100 ml reaction flask, and stirred evenly. Then, the peptide resin **25D** (5 g) was added, and the mixture was stirred at room temperature for 0.5 hours. The resin was filtered to obtain the filtrate. The peptide resin was cleaved again according to the above cleavage method, and the filtrates obtained in the two filtrations were collected, washed twice with water, washed once with saturated brine, and dried over anhydrous sodium sulphate. The solvent was pumped off under vacuum to give **Compound 25E** (2.3 g).

Step 5: To a 2 L flask, dichloromethane (1.2 L), **Compound 25E** (2.3 g, 0.68 mmol), benzotriazole-N,N,N',N'-tetramethylurea hexafluorophosphate (417.2 mg, 1.1 mmol) and DIEA (0.19 ml, 1.1 mmol) were added. The mixture was reacted at room temperature for 16 h. When the reaction was basically completed as shown by MS, the reaction mixture was washed twice with water, washed once with saturated brine, and dried over anhydrous sodium sulphate. The solvent was pumped off under vacuum to give **Compound 25F** (2.1 g).

Step 6: 60 ml of cleavage solution (91% trifluoroacetic acid + 4% triisopropylsilane + 3% 1,2-ethanedithiol + 2% water) was added to a 100 ml reaction flask, and stirred evenly. Then, **Compound 25F** (2.1 g) was added, and the mixture was stirred at room temperature for 2 hours. The resin was filtered to obtain the filtrate, which was added to 600 ml of methyl tert-butyl ether (which was cooled to 0°C in advance). White floccules were precipitated, and centrifuged (3 min at 3000 rpm). The white precipitate was washed with methyl tert-butyl ether three times, and dried in vacuo to give the crude peptide **25G** as an off-white solid (1.2 g).

Step 7: To a 2 L reaction flask, water (1050 ml), acetonitrile (450 ml), and **25G** (1.2 g, 0.6 mmol) were added successively, and stirred evenly. Then, iodine/acetonitrile solution (0.1 mol/L) was slowly added dropwise until the reaction solution turned light yellow. Ascorbic acid was added to quench the reaction, and the resultant was purified by preparative HPLC. Separation method: 1. Instrument: Waters 2767 (preparative liquid chromatographic instrument); and Chromatographic column: SunFire@PrepC18 (19 mm×250 mm). 2. The sample was filtered with a 0.45 μm filter head to prepare a sample solution. 3. Preparative chromatography conditions: a. composition of mobile phases A and B: A: 0.1% trifluoroacetic acid/$H_2O$, and B: $CH_3CN$; b. gradient elution: mobile phase = 5%-45%; c. flow rate: 12 ml/min; d. elution time: 30 min; and retention time: 16 min. Lyophilization was carried out to afford **Compound 25** (80 mg, purity: 95%) as a white solid.

LCMS m/z =1010.9[M/2+H]$^+$ 674.3[M/3+H]$^+$.

Example 26:

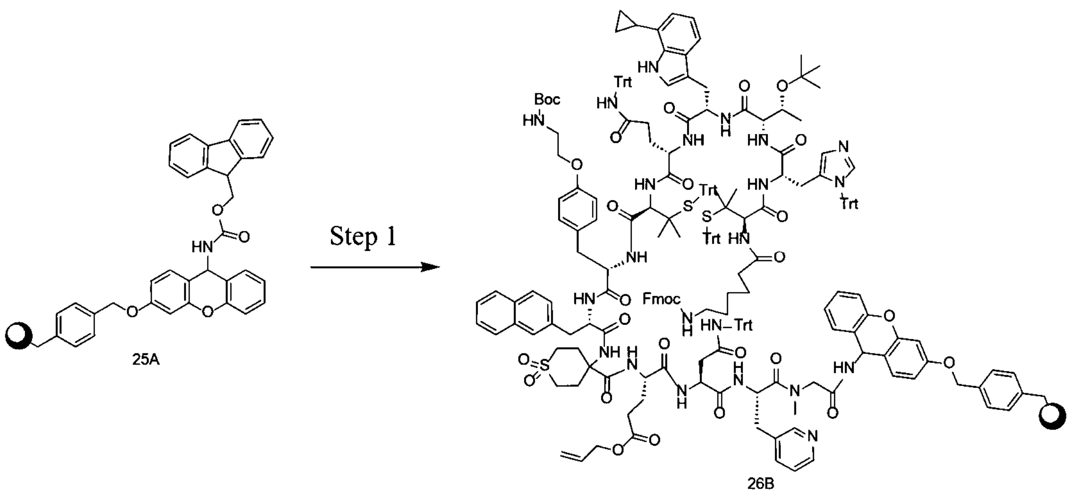


25A

26B

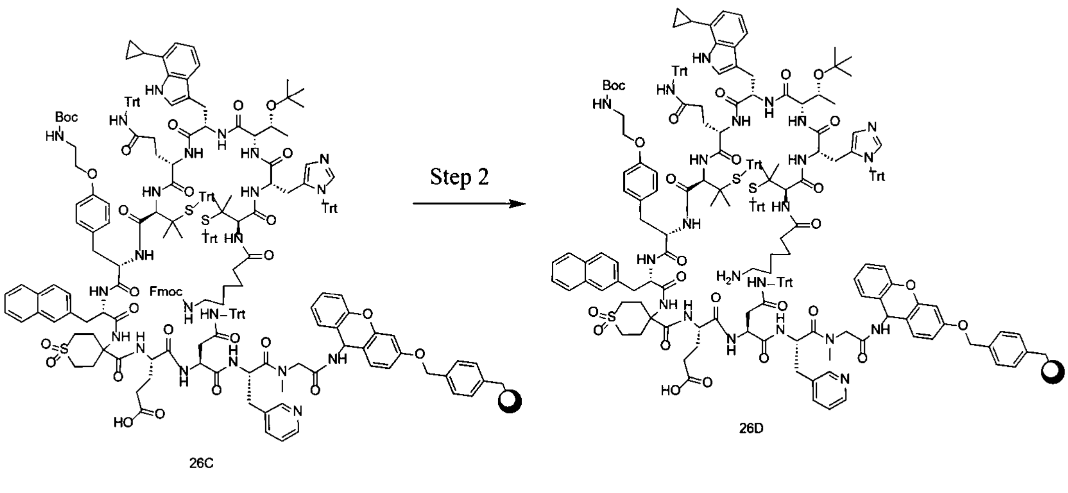


26C

26D

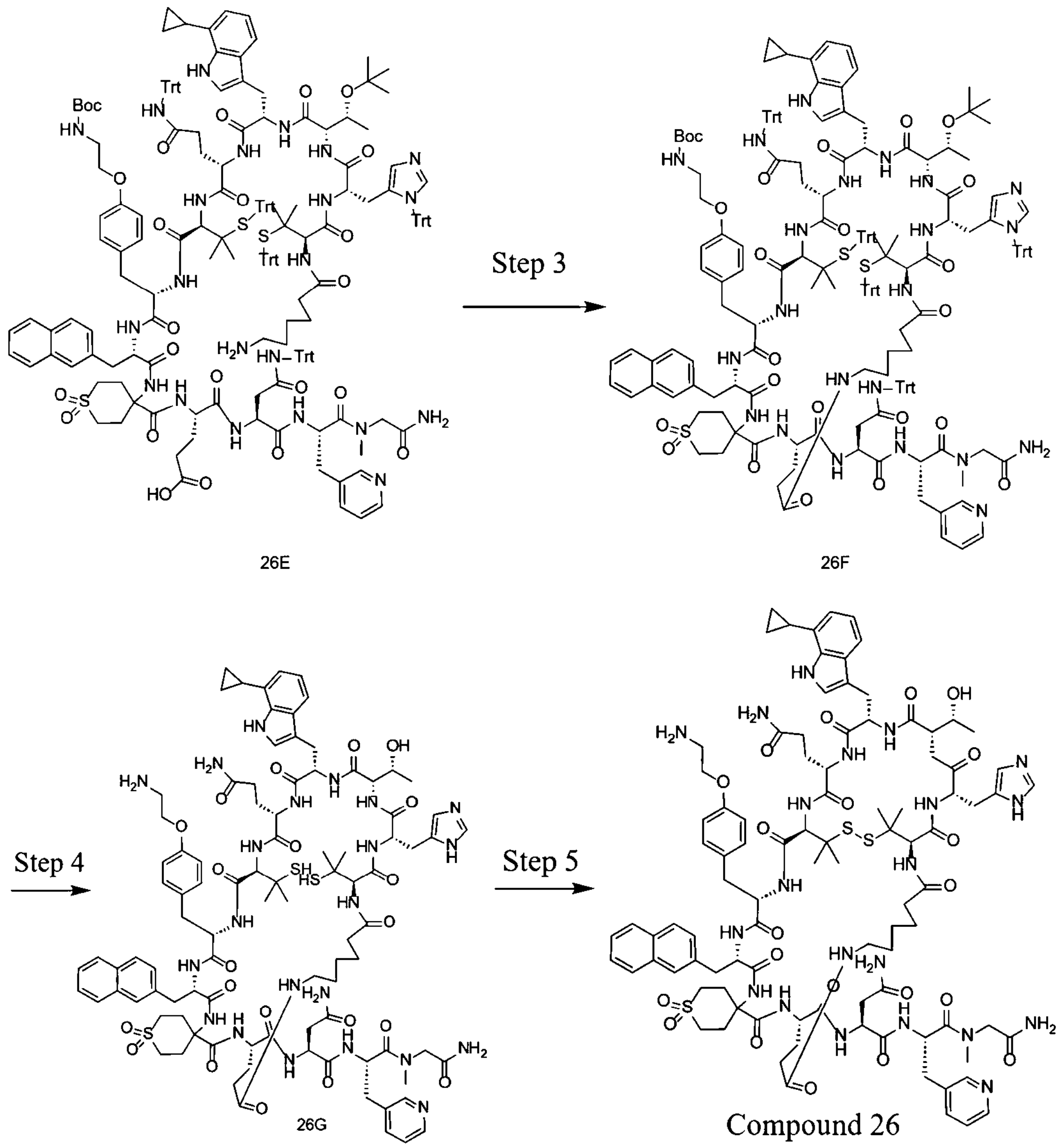


26E

26F

26G

Compound 26

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | **Intermediate 5** (2.0 eq.) | DIC (2 eq.) and HOAT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-p henylalanine (3.0 eq.) | DIC (3 eq.) and HOAT (3 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOAT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOAT (2 eq.) |

(continued)

| Serial No. | Starting material | Coupling reagent |
|---|---|---|
| 14 | Fmoc-6-aminohexanoic acid (3.0 eq.) | DIC (3 eq.) and HOBT (3 eq.) |

**Compound 26** (50 mg, purity: 98%) was obtained by using the materials in the above table as starting materials and adopting a method for **Compound 25.**

LCMS m/z =1003.7 [M/2+H]$^+$, 669.7 [M/3+H]$^+$.

Example 27:

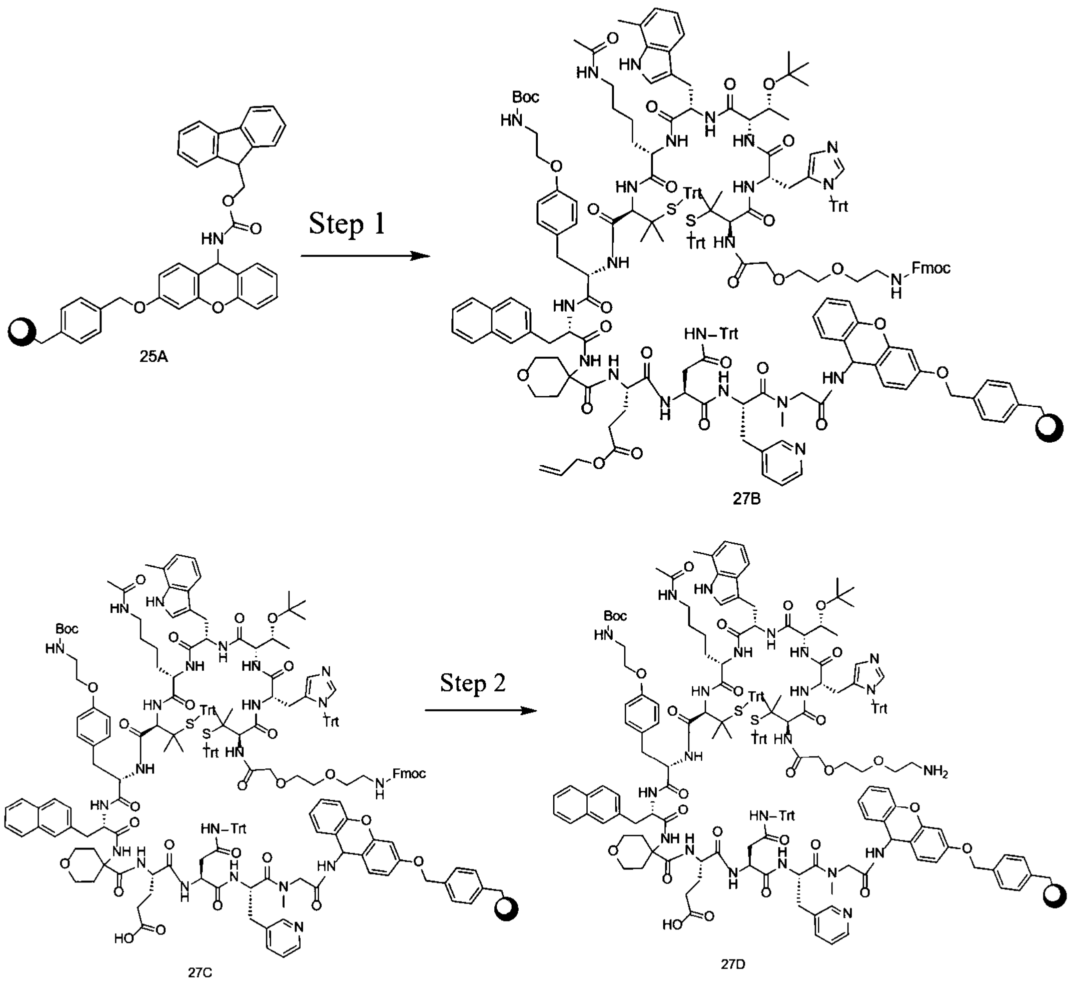


25A, 27B, 27C, 27D

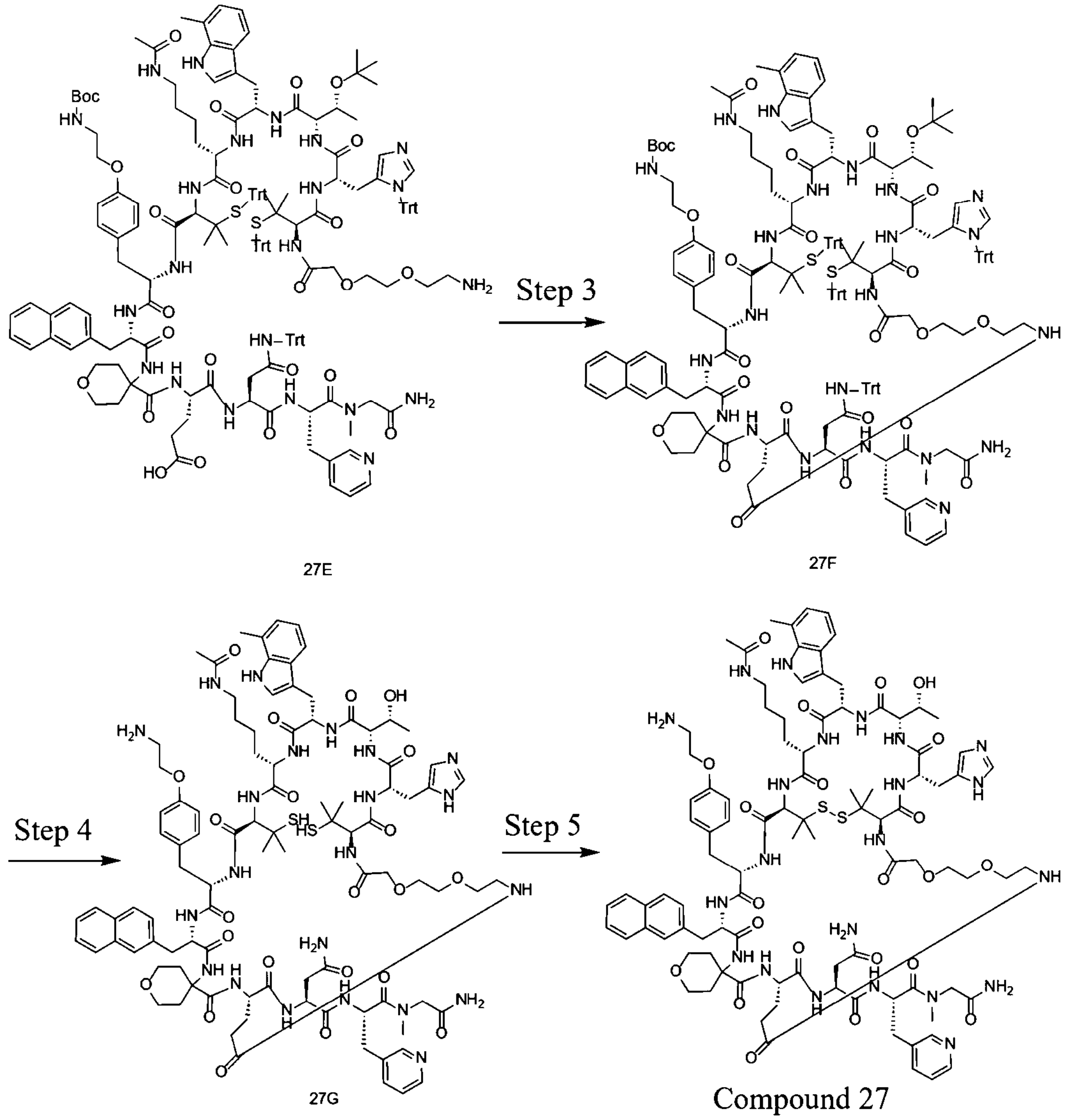


27E

27F

27G

Compound 27

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-p henylalanine (3.0 eq.) | DIC (3 eq.) and HOAT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOAT (2 eq.) |

(continued)

| Serial No. | Starting material | Coupling reagent |
|---|---|---|
| 14 | Fmoc-AEEA-OH (3.0 eq.) | DIC (3 eq.) and HOBT (3 eq.) |

**Compound 27** (65 mg, purity: 99%) was obtained by using the materials in the above table as starting materials and adopting a method for **Compound 25.**

LCMS m/z =1003.8 [M/2+H]$^+$.

Example 28:

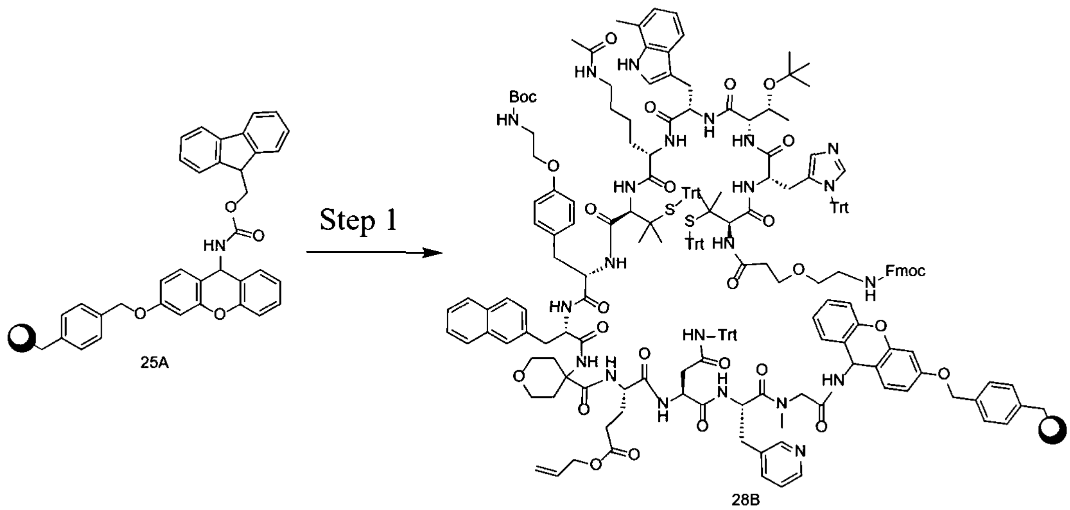


25A

28B

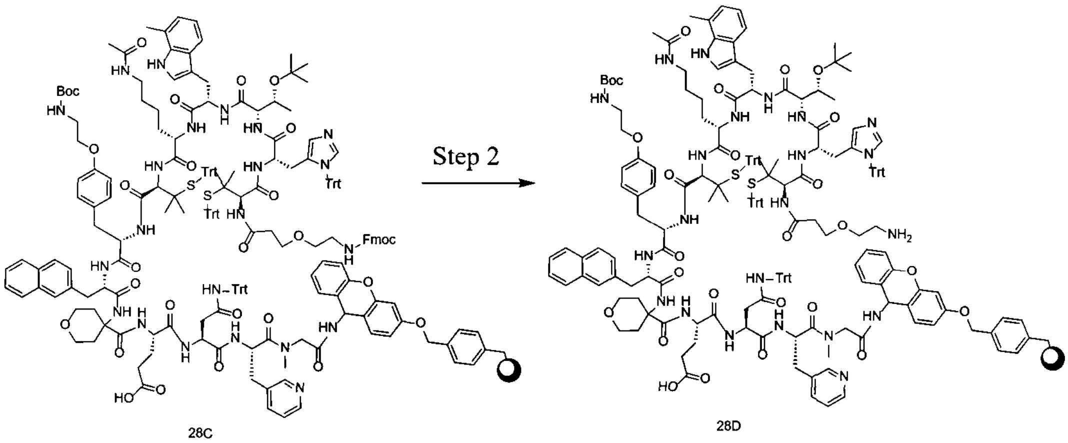


28C

28D

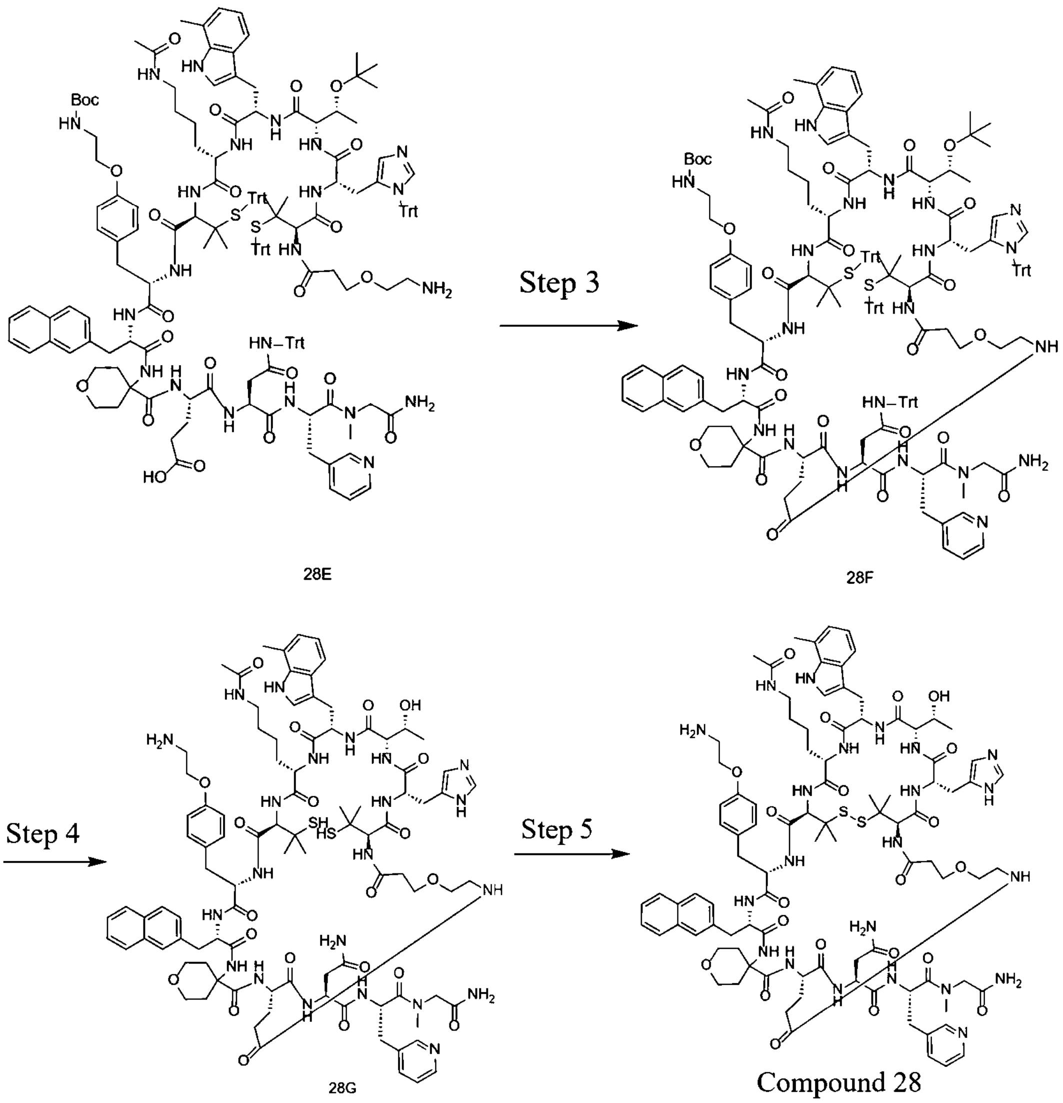


28E

28F

28G

Compound 28

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-phenylalanine (3.0 eq.) | DIC (3 eq.) and HOAT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOAT (2 eq.) |
| 14 | N-fluorenylmethoxycarbonyl-ethylene glycol-carboxylic acid (3.0 eq.) | DIC (3 eq.) and HOBT (3 eq.) |

**Compound 28** (48 mg, purity: 95%) was obtained by using the materials in the above table as starting materials and adopting a method for **Compound 25.**

LCMS m/z =988.8 $[M/2+H]^+$.

Example 29:

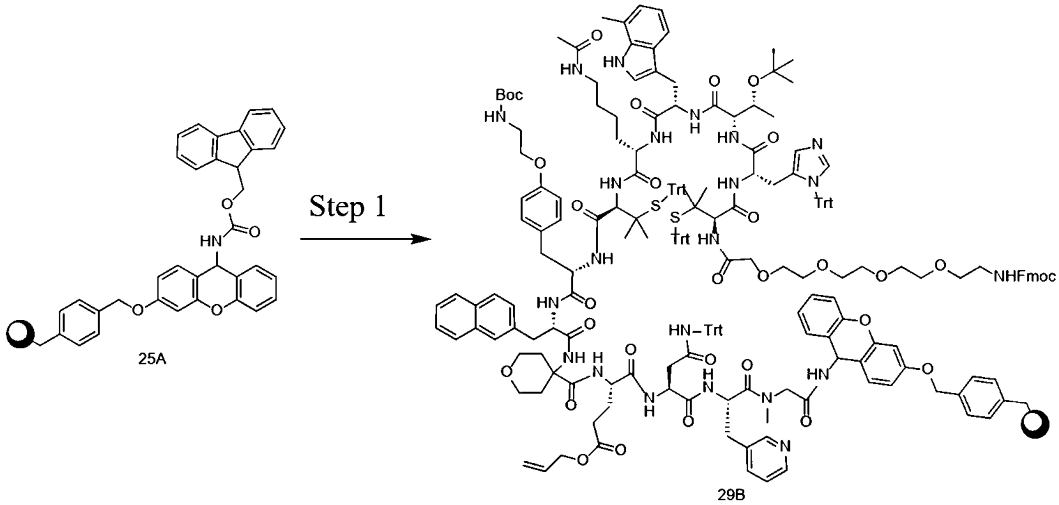


25A

29B

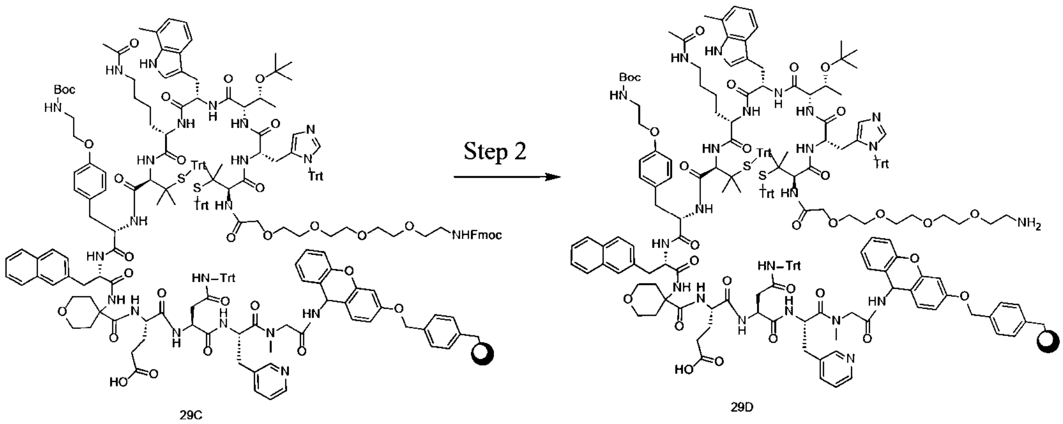


29C

29D

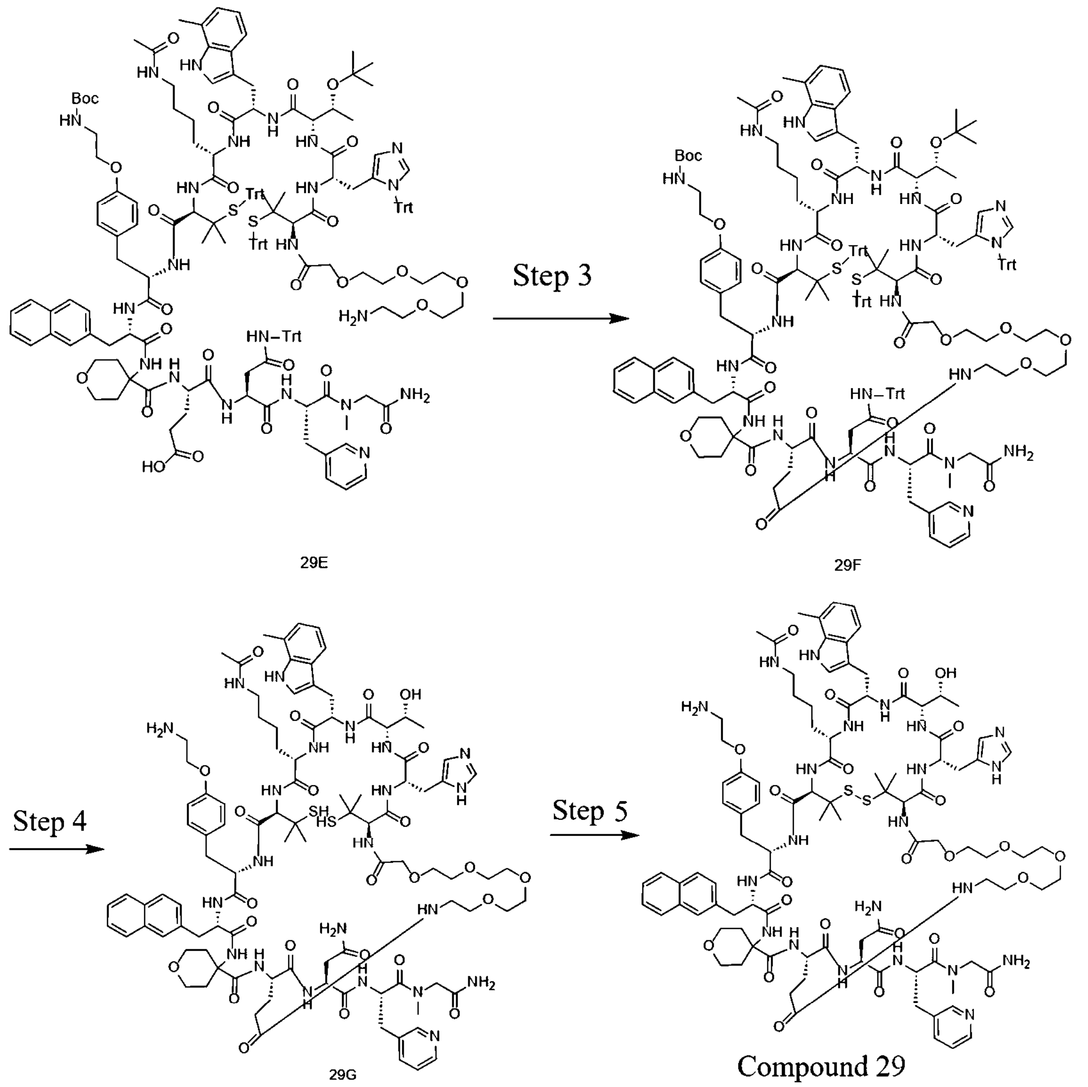


29E 29F 29G Compound 29

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-p henylalanine (3.0 eq.) | DIC (3 eq.) and HOAT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOAT (2 eq.) |
| 14 | N-fluorenylmethoxycarbonyl-tetraethylene glycol-acetic acid (3.0 eq.) | DIC (3 eq.) and HOBT (3 eq.) |

**Compound 29** (140 mg, purity: 99%) was obtained by using the materials in the above table as starting materials and adopting a method for **Compound 25.**

LCMS m/z =1047.9 [M/2+H]$^+$.

Example 30:

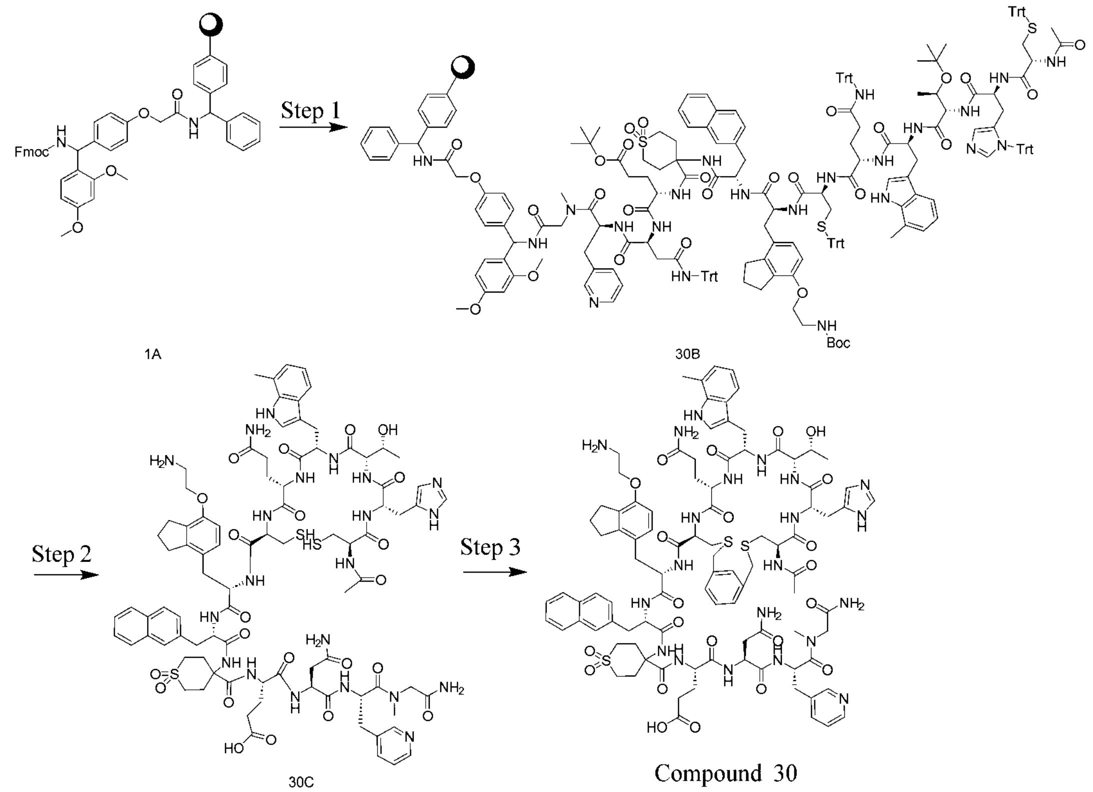


1A 30B 30C Compound 30

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOAT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOAT (2 eq.) |
| 8 | Fmoc-Cys(Trt) -OH (3.0 eq.) | DIC (3 eq.) and HOBT (3 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOAT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Cys(Trt) -OH (3.0 eq.) | DIC (3 eq.) and HOBT (3 eq.) |
| 14 | Acetic anhydride | DIEA |

**Compound 30** (180 mg, purity: 92%) was obtained by using the materials in the above table as starting materials

and adopting a method for **Compound 19.**

LCMS m/z =1008.5 [M/2+H]$^+$, 672.8 [M/3+H]$^+$.

Example 31:

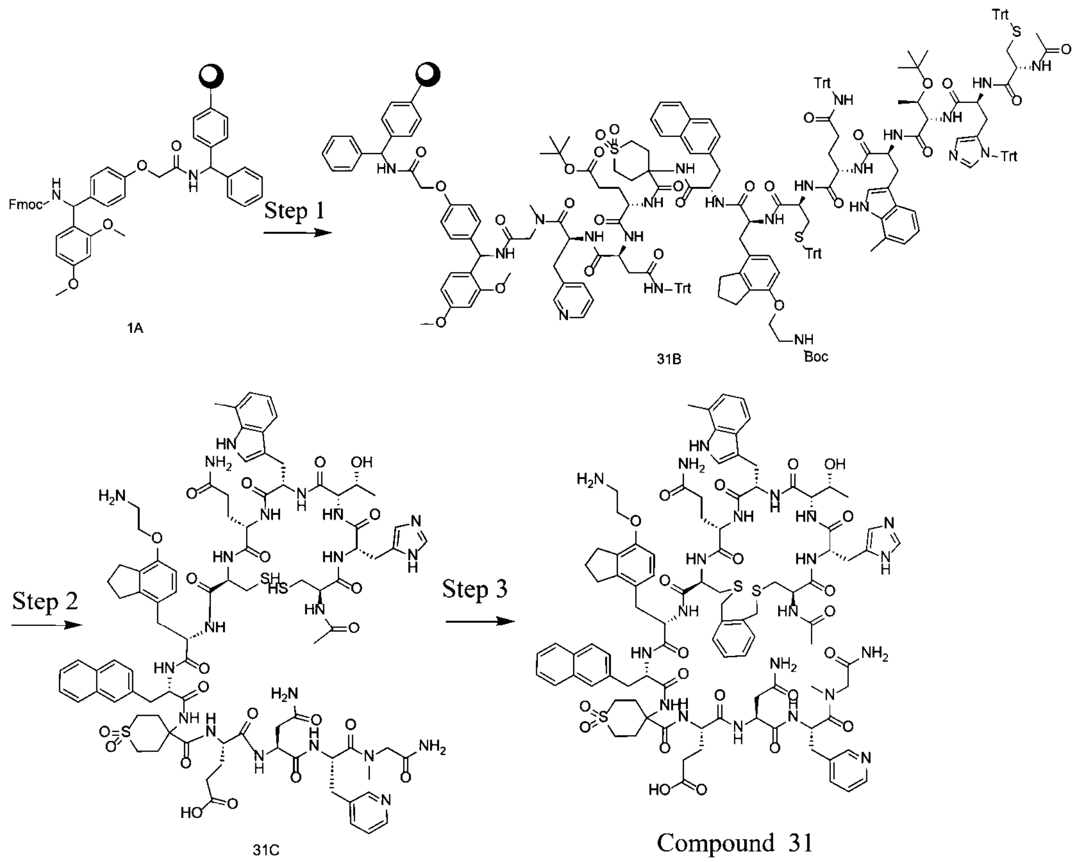


1A

31B

31C

Compound 31

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOAT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOAT (2 eq.) |
| 8 | Fmoc-Cys(Trt) -OH (3.0 eq.) | DIC (3 eq.) and HOBT (3 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOAT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Cys(Trt) -OH (3.0 eq.) | DIC (3 eq.) and HOBT (3 eq.) |
| 14 | Acetic anhydride | DIEA |

**Compound 31** (279 mg, purity: 94%) was obtained by using the materials in the above table as starting materials and adopting a method for **Compound 19.**

LCMS m/z =1008.4 [M/2+H]$^+$, 672.9 [M/3+H]$^+$.

Example 32:

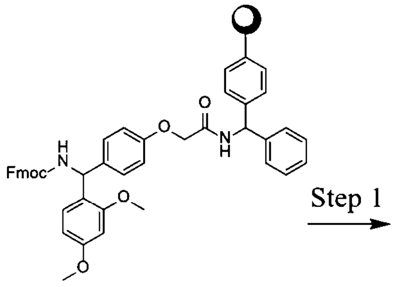


1A

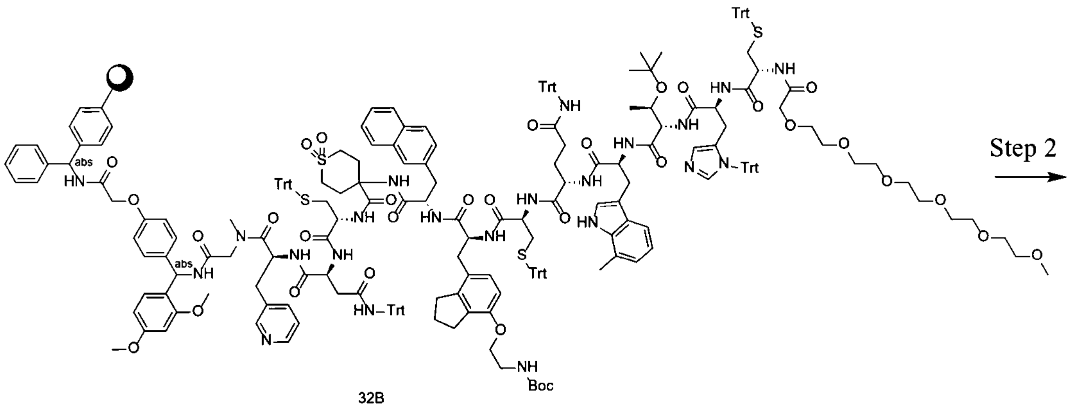


32B

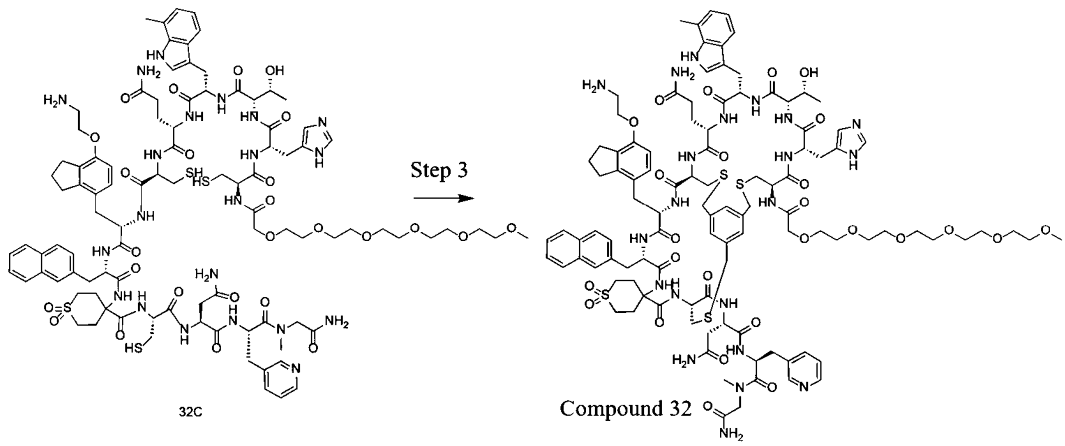


32C

Compound 32

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Cys(Trt)-OH (3.0 eq.) | DIC (3 eq.) and HOBT (3 eq.) |
| 5 | **Intermediate 5** (2.0 eq.) | DIC (2 eq.) and HOBT (2 eq.) |

(continued)

| Serial No. | Starting material | Coupling reagent |
|---|---|---|
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Cys(Trt) -OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Cys(Trt)-OH (2.0 eq.) | DIC (2 eq.) and HOBT (2 eq.) |
| 14 | m-PEG5-CH2COOH (2.0 eq.) | DIC (2 eq.) and HOBT (2 eq.) |

**Compound 32** (200 mg, 99%) was obtained by using the materials in the above table as starting materials and adopting a method for **Compound 19.**

LCMS m/z =1126.6 $[M/2+H]^+$, 751.6 $[M/3+H]^+$.

Example 33:

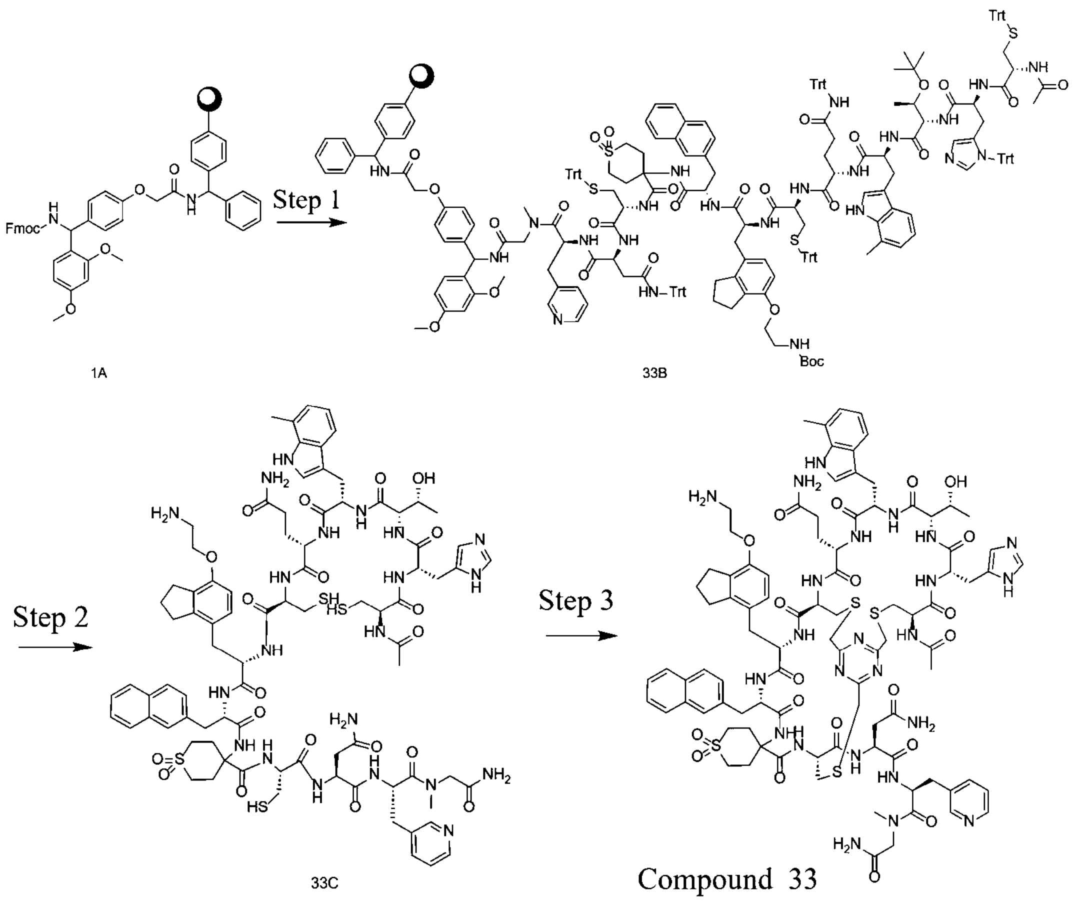


1A

33B

33C

Compound 33

| Serial No. | Starting material | Coupling reagent |
|---|---|---|
| 1 | Fmoc-Sar-OH (3.0 eq.) | DIC (3 eq.) and HOBT (3 eq.) |

(continued)

| Serial No. | Starting material | Coupling reagent |
|---|---|---|
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Cys(Trt)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Cys(Trt) -OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Cys(Trt)-OH (2.0 eq.) | DIC (2 eq.) and HOBT (2 eq.) |
| 14 | Acetic anhydride | DIEA |

**Compound 33** (190 mg, 97%) was obtained by using the materials in the above table as starting materials and adopting a method for **Compound 19.**

LCMS m/z =1002.9 $[M/2+H]^+$, 669.3 $[M/3+H]^+$.

Example 34:

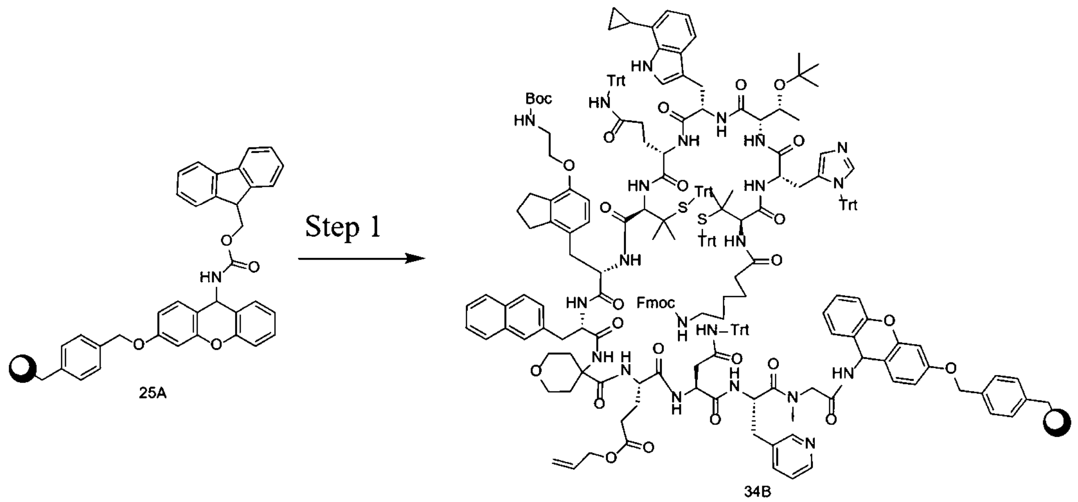


25A

34B

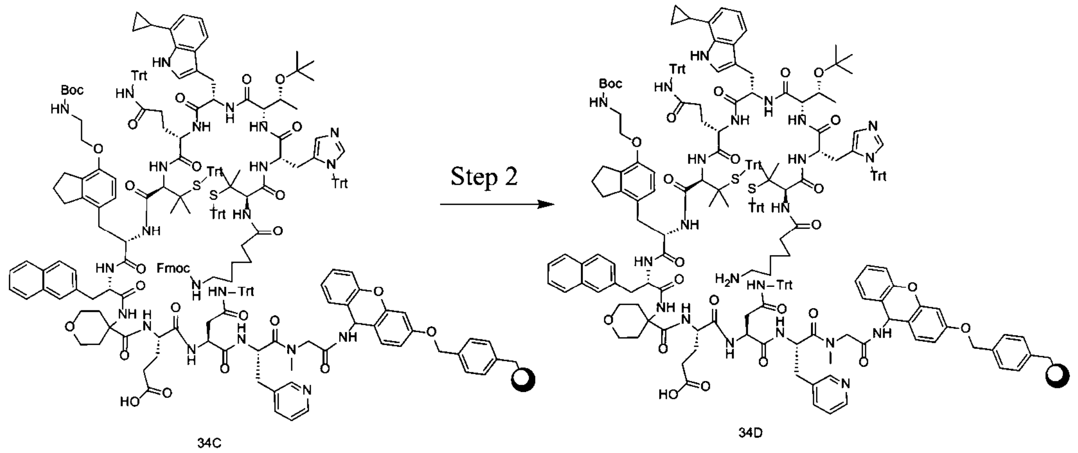


34C

34D

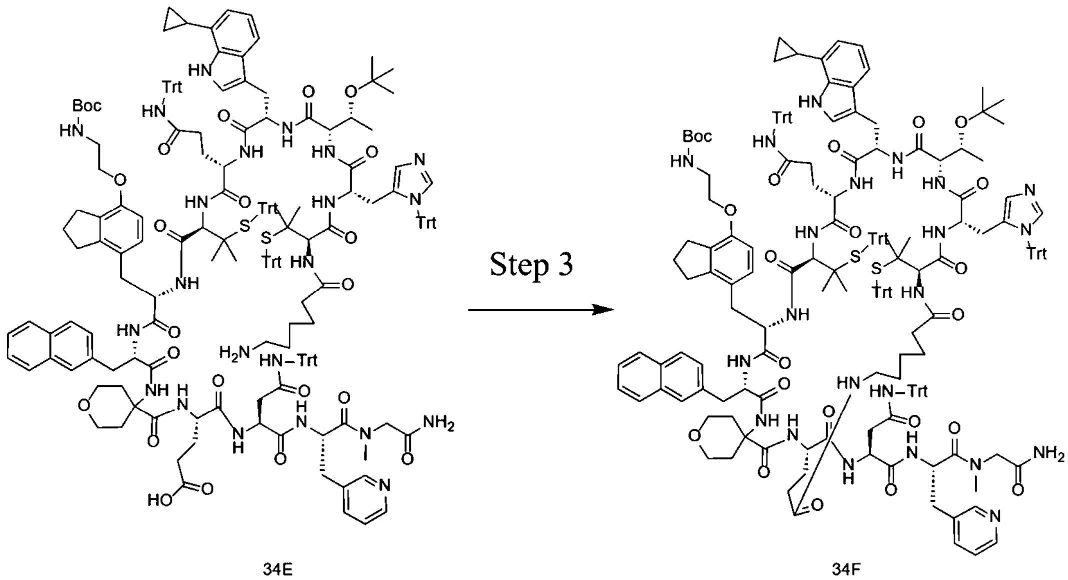


34E

34F

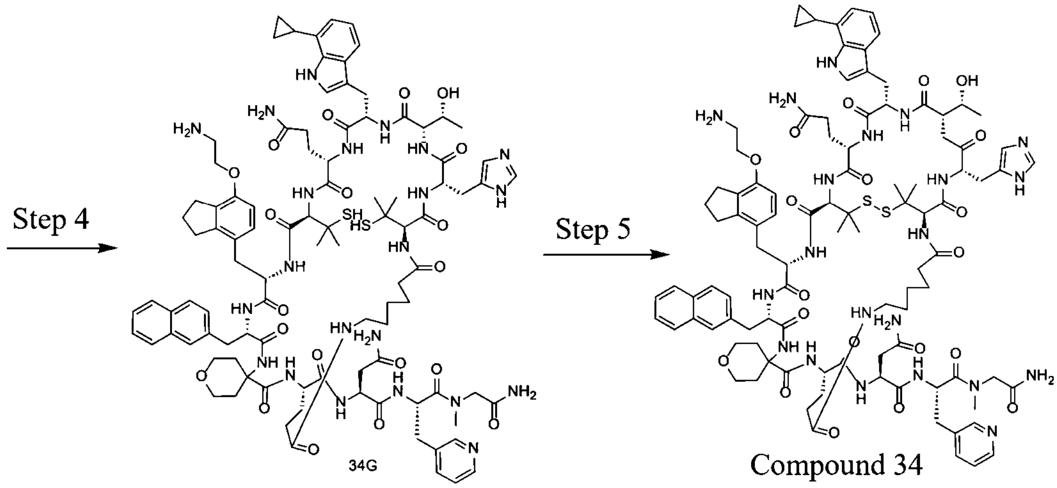


34G

Compound 34

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | Fmoc-ThpGly-OH (2.0 eq.) | DIC (2 eq.) and HOAT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOAT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOAT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOAT (2 eq.) |
| 14 | Fmoc-6-aminohexanoic acid (3.0 eq.) | DIC (3 eq.) and HOBT (3 eq.) |

**Compound 34** (130 mg, purity: 94%) was obtained by using the materials in the above table as starting materials and adopting a method for **Compound 25.**

LCMS m/z =1000.0 $[M/2+H]^+$, 667.2 $[M/3+H]^+$.

Example 35:

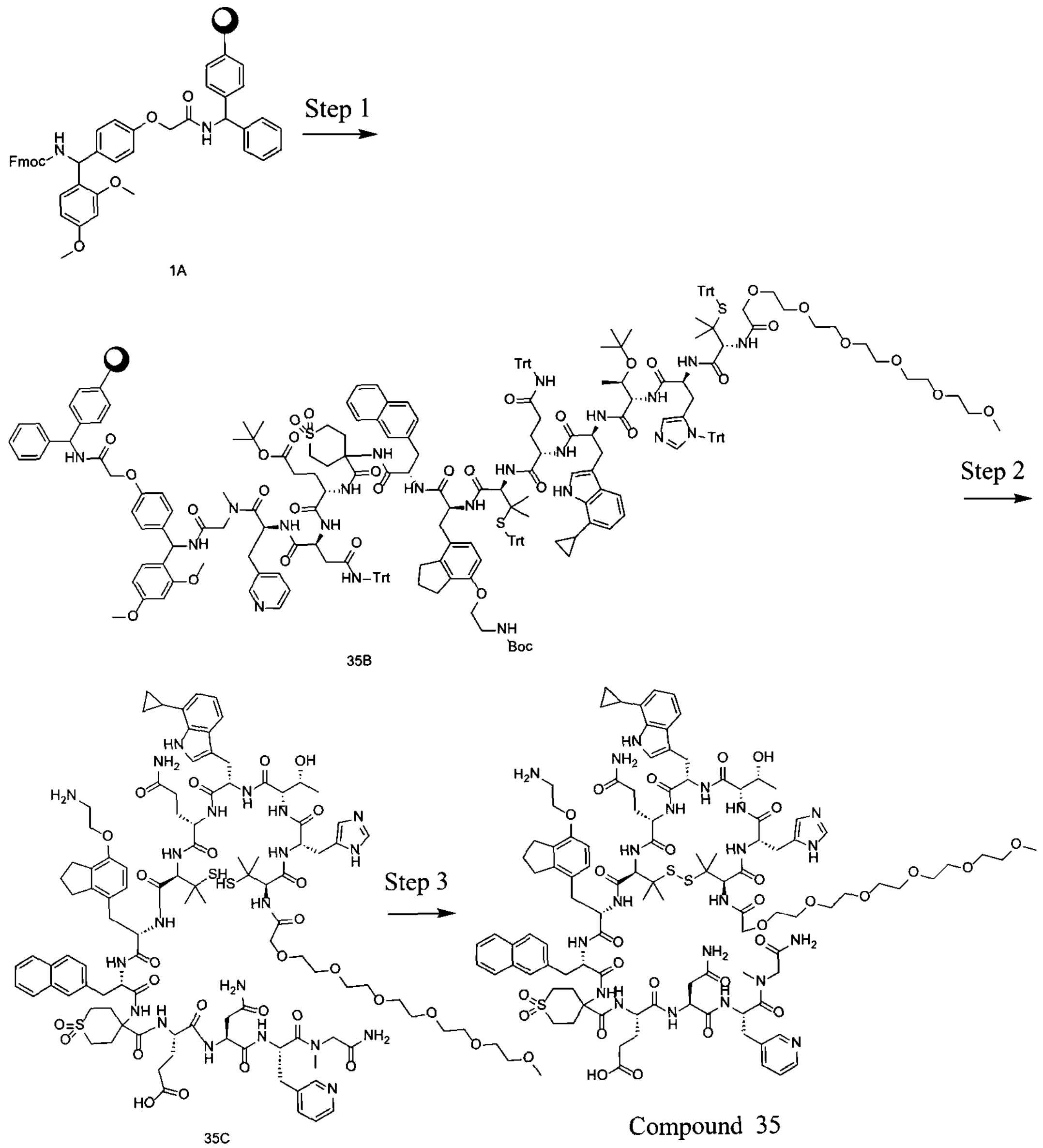


1A

35B

35C

Compound 35

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |

(continued)

| Serial No. | Starting material | Coupling reagent |
|---|---|---|
| 13 | Fmoc-Pen(Trt)-OH (2.0 eq.) | DIC (2 eq.) and HOBT (2 eq.) |
| 14 | m-PEG5-CH2COOH (2.0 eq.) | DIC (2 eq.) and HOBT (2 eq.) |

**Compound 35** (150 mg, purity: 94%) was obtained by using the materials in the above table as starting materials and adopting a method for **Compound 1.**

LCMS m/z =1122.3 $[M/2+H]^+$, 748.5 $[M/3+H]^+$.

Example 36:

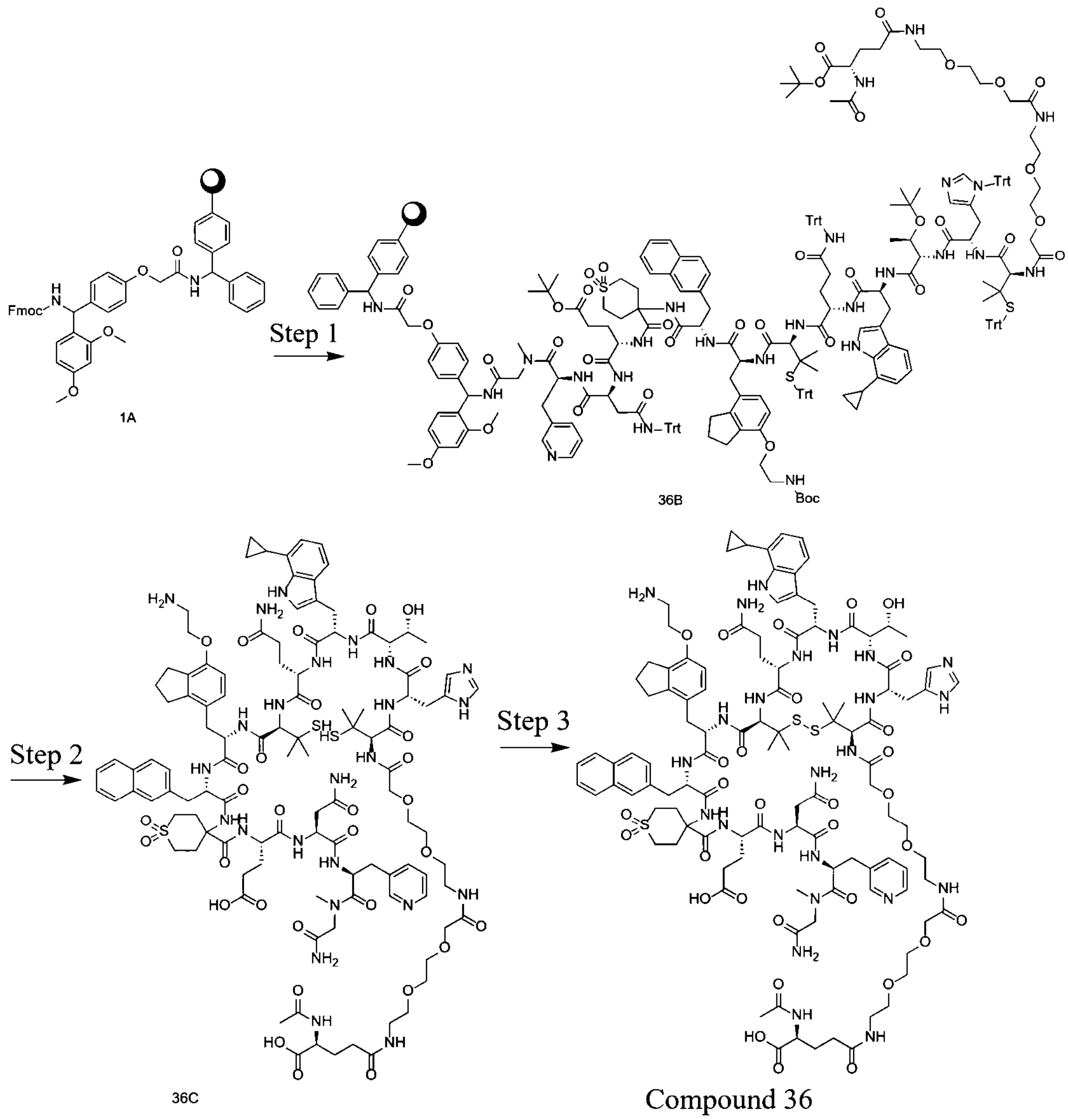


1A

36B

36C

Compound 36

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |

(continued)

| Serial No. | Starting material | Coupling reagent |
|---|---|---|
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Fmoc-AEEA-OH (3.0 eq.) | DIC (3 eq.) and HOBT (3 eq.) |
| 15 | Fmoc-AEEA-OH (3.0 eq.) | DIC (3 eq.) and HOBT (3 eq.) |
| 16 | Fmoc-Glu-OtBu (3.0 eq.) | DIC (3 eq.) and HOBT (3 eq.) |
| 17 | Acetic anhydride | DIEA |

**Compound 36** (300 mg, purity: 96%) was obtained by using the materials in the above table as starting materials and adopting a method for **Compound 1.**

LCMS m/z = 805.0 $[M/3+H]^+$.

Example 37:

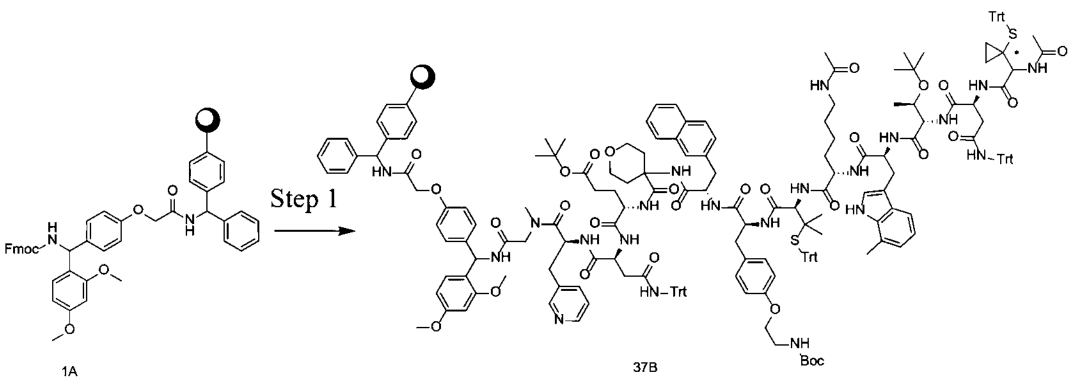


1A 37B

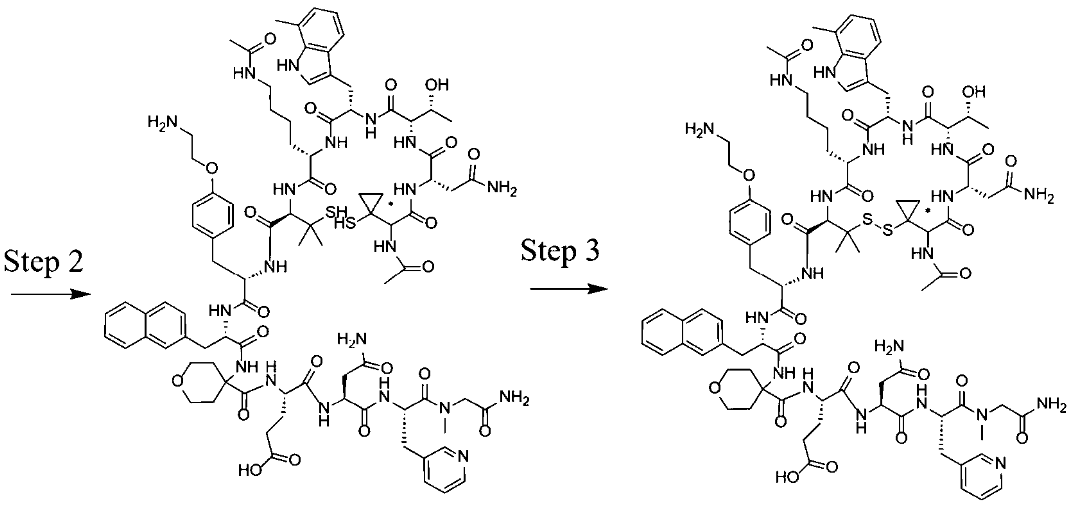


37C

**Compound 37**

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (3.0 eq.) | DIC (3 eq.) and HOBT (3 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-p henylalanine (3.0 eq.) | DIC (3 eq.) and HOBT (3 eq.) |
| 8 | Fmoc-Pen(Trt)-OH (3.0 eq.) | DIC (3 eq.) and HOBT (3 eq.) |
| 9 | Fmoc-Lys(Ac)-OH (3.0 eq.) | DIC (3 eq.) and HOBT (3 eq.) |
| 10 | Fmoc-7MeTrp-OH (3.0 eq.) | DIC (3 eq.) and HOBT (3 eq.) |
| 11 | Fmoc-Thr(tBu)-OH (3.0 eq.) | DIC (3 eq.) and HOBT (3 eq.) |
| 12 | Fmoc-Asn(Trt)-OH (3.0 eq.) | DIC (3 eq.) and HOBT (3 eq.) |
| 13 | **Intermediate 9**(3.0 eq.) | DIC (3 eq.) and HOBT (3 eq.) |
| 14 | Acetic anhydride | DIEA |

**Compound 37** (100 mg, purity: 95%) was obtained by using the materials in the above table as starting materials and adopting a synthetic method for **Compound 1.**
LCMS m/z = 948.5 $[M/2+H]^+$

Example 38:

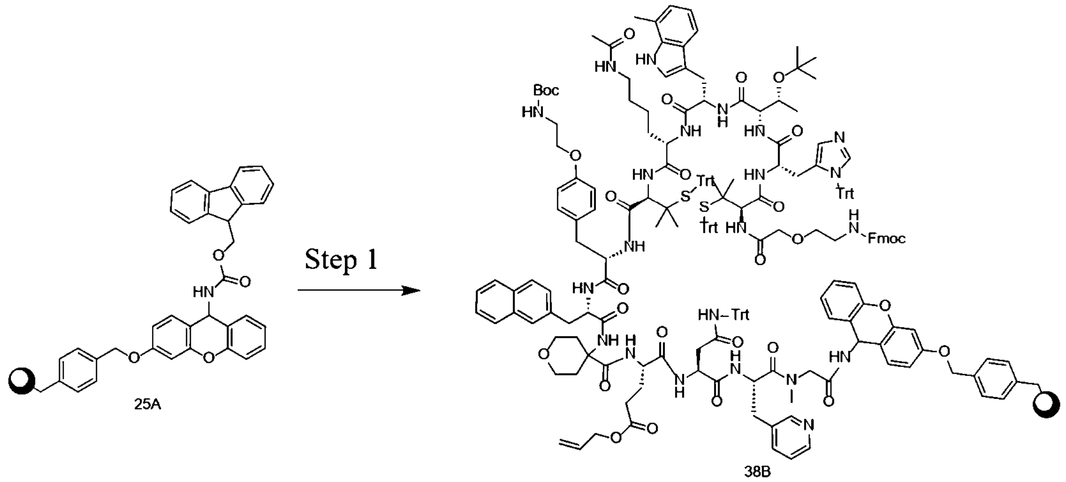


25A

38B

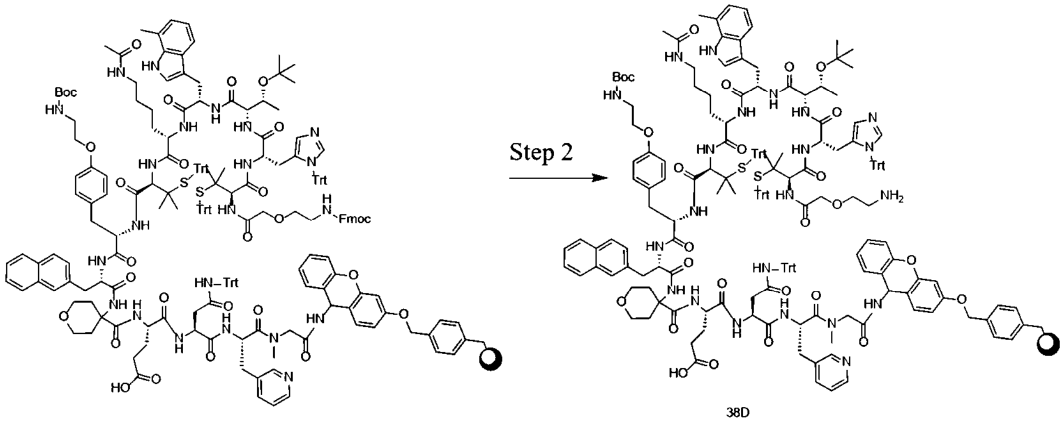


38C

38D

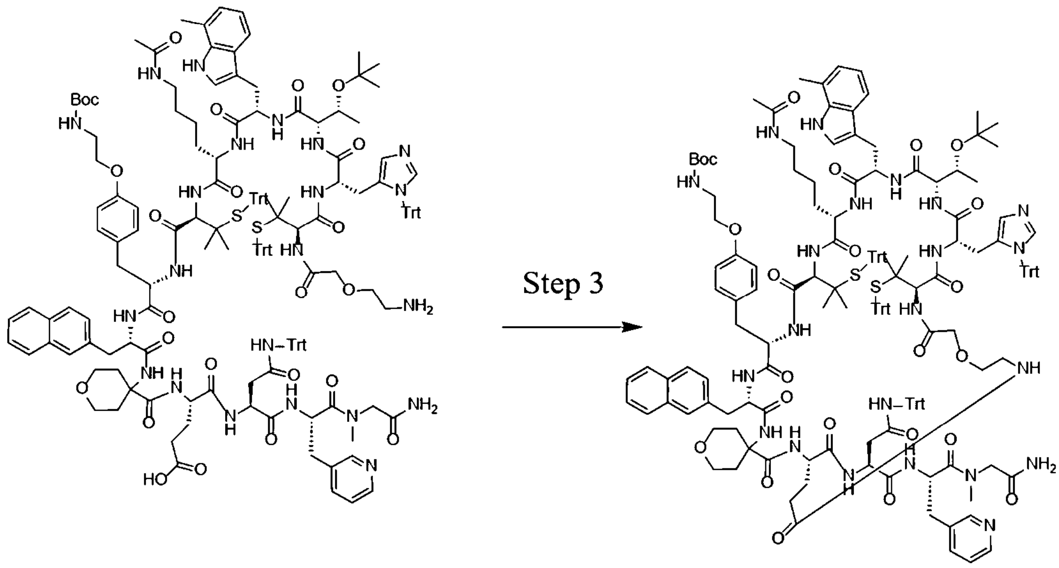


38E

38F

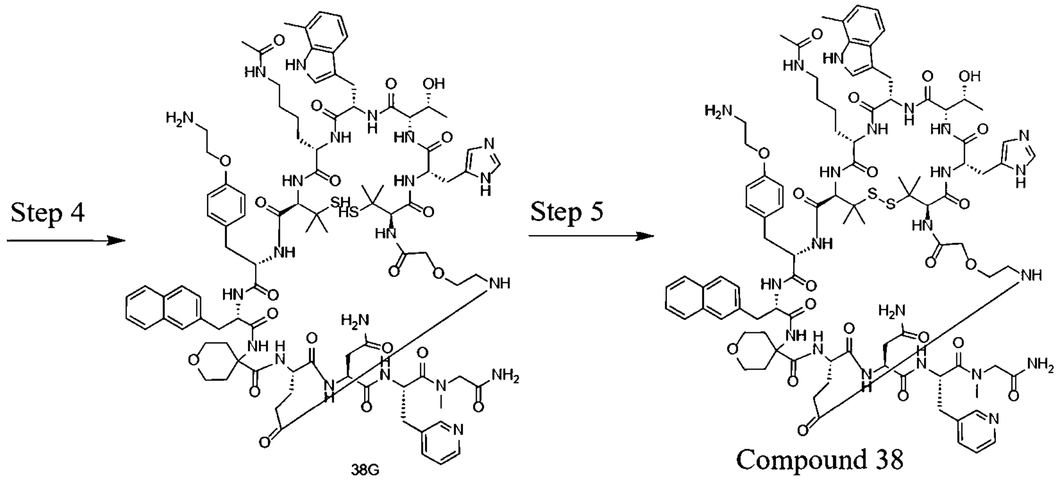


38G

Compound 38

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-p henylalanine (3.0 eq.) | DIC (3 eq.) and HOAT (3 eq.) |
| 8 | Fmoc-Pen(Trt)-OH (2.0 eq.) | DIC (2 eq.) and HOBT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOAT (2 eq.) |
| 14 | Fmoc-5-amino-3-oxopentanoic acid (3.0 eq.) | DIC (3 eq.) and HOBT (3 eq.) |

**Compound 38** (45 mg, purity: 95%) was obtained by using the materials in the above table as starting materials and adopting a method for **Compound 25.**

LCMS m/z =981.7 $[M/2+H]^+$.

Example 39:

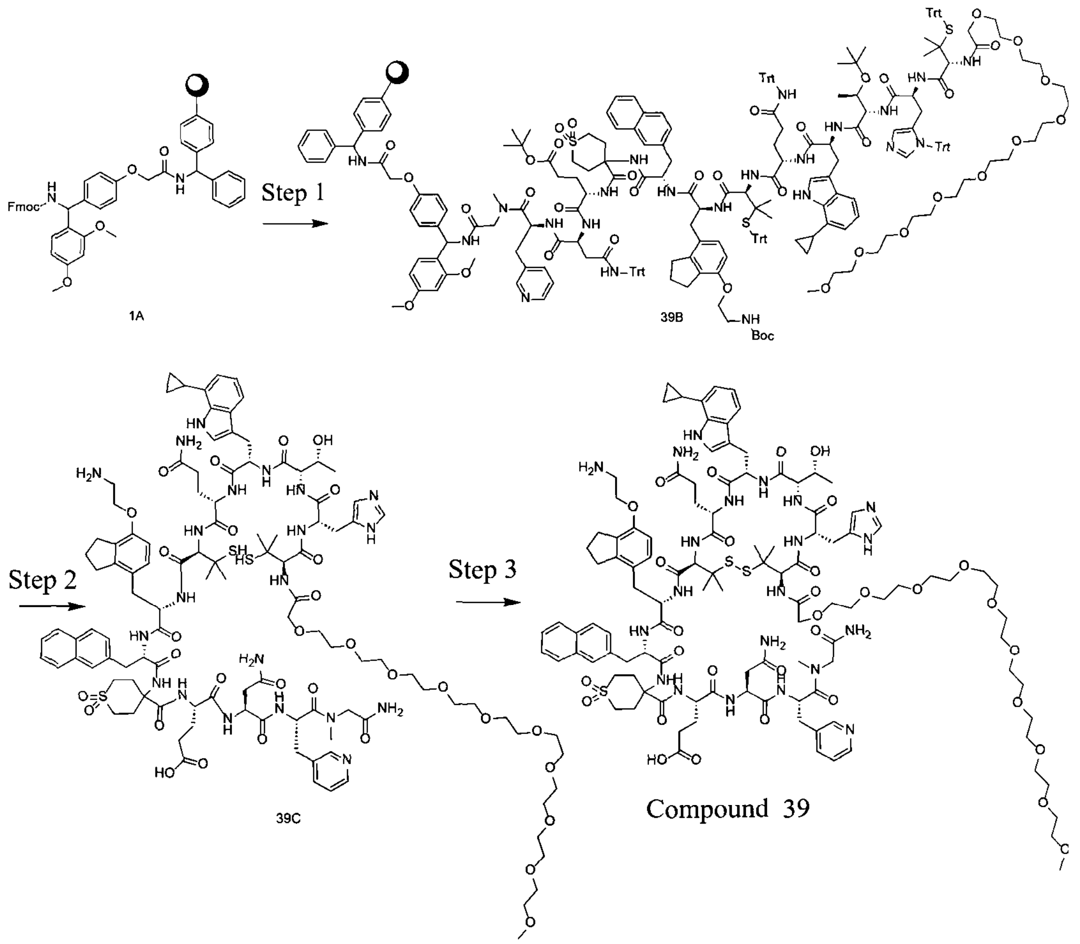


1A

39B

39C

Compound 39

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | m-PEG9-CH2COOH (2.0 eq.) | DIC (2 eq.) and HOBT (2 eq.) |

**Compound 39** (110 mg, purity: 97%) was obtained by using the materials in the above table as starting materials and adopting a method for **Compound 1.**

LCMS m/z = 807.5 $[M/3+H]^+$.

Example 40:

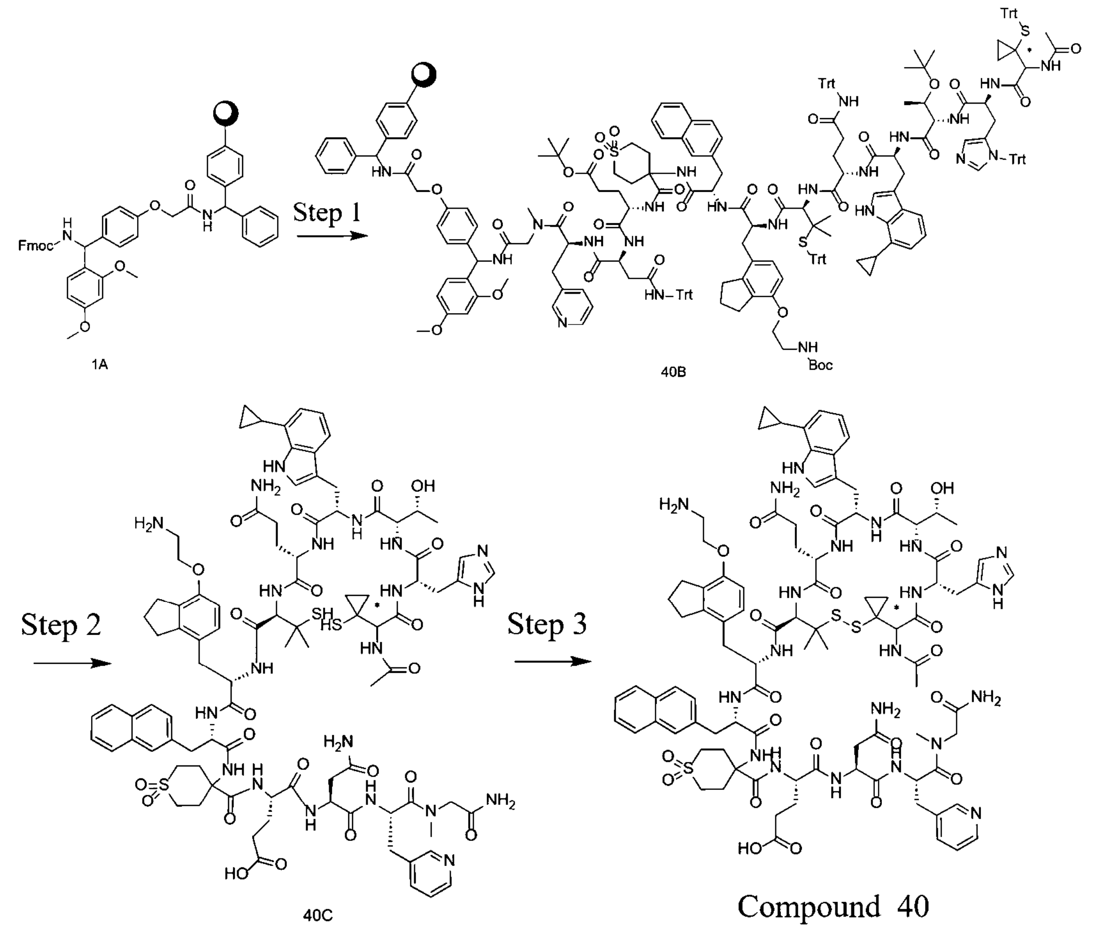


1A 40B 40C Compound 40

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOAT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOAT (2 eq.) |
| 8 | Fmoc-Pen(Trt)-OH (2.0 eq.) | DIC (2 eq.) and HOAT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOAT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | **Intermediate 10** (2.0 eq.) | DIC (2 eq.) and HOAT (2 eq.) |
| 14 | Acetic anhydride | DIEA |

**Compound 40** (150 mg, purity: 94%) was obtained by using the materials in the above table as starting materials and adopting a method for **Compound 1.**

LCMS m/z =996.4 [M/2+H]$^+$, 664.7 [M/3+H]$^+$.

Example 41:

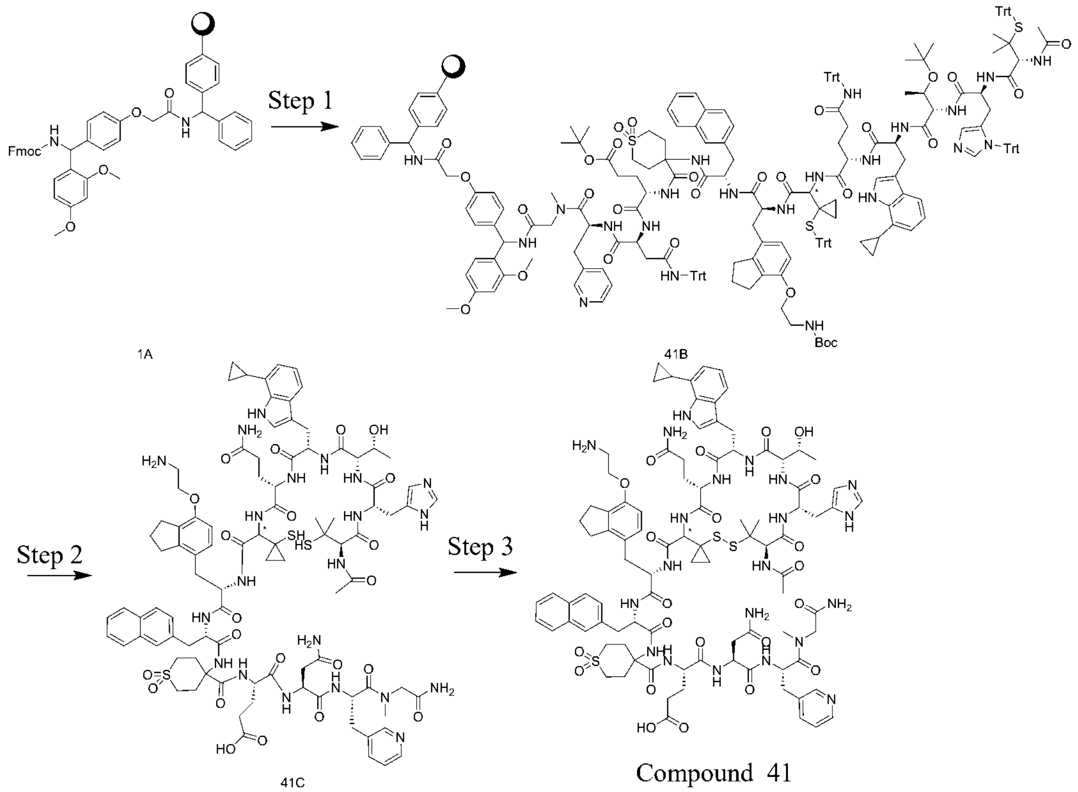


41C

Compound 41

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOAT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOAT (2 eq.) |
| 8 | **Intermediate 10** (2.0 eq.) | DIC (2 eq.) and HOAT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOAT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOAT (2 eq.) |
| 14 | Acetic anhydride | DIEA |

**Compound 41** (150 mg, purity: 97%) was obtained by using the materials in the above table as starting materials

and adopting a method for **Compound 1.**

LCMS m/z =996.7 [M/2+H]+, 664.8 [M/3+H]+.

Example 42:

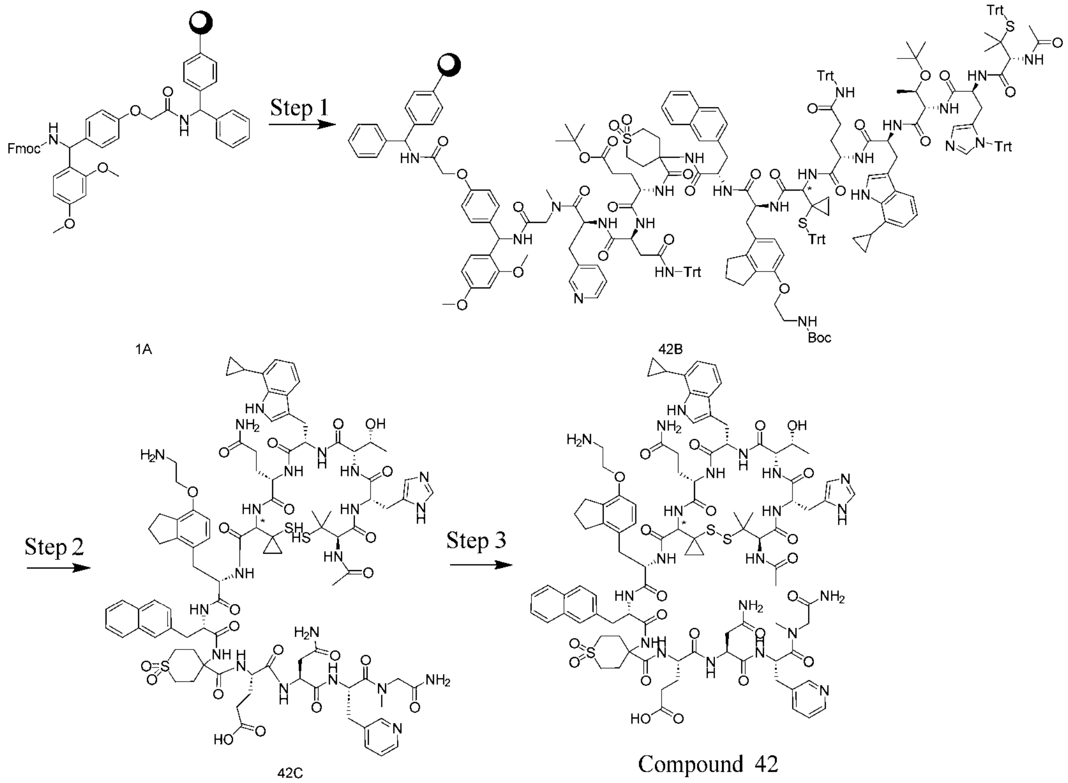


1A

42B

42C

Compound 42

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOAT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOAT (2 eq.) |
| 8 | **Intermediate 9** (2.0 eq.) | DIC (2 eq.) and HOAT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOAT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOAT (2 eq.) |
| 14 | Acetic anhydride | DIEA |

**Compound 42** (35 mg, purity: 98%) was obtained by using the materials in the above table as starting materials and adopting a method for **Compound 1.**

LCMS m/z =996.2[M/2+H]$^+$, 664.5 [M/3+H]$^+$.

Example 43:

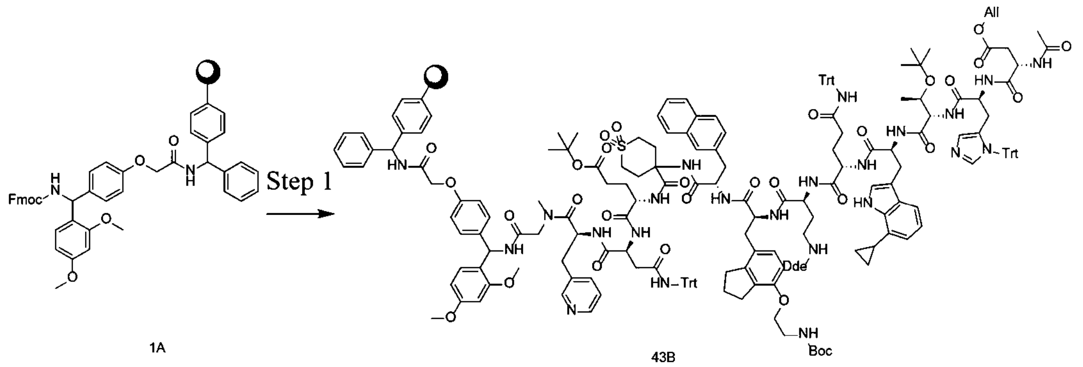


1A 43B

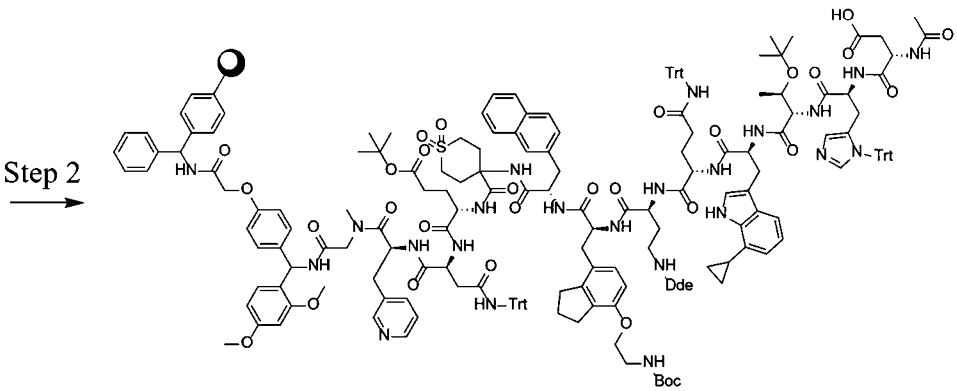


43C

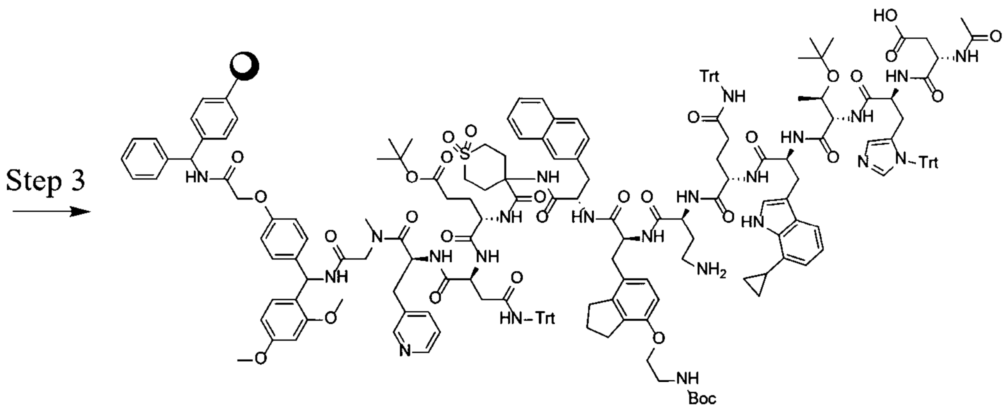


43D

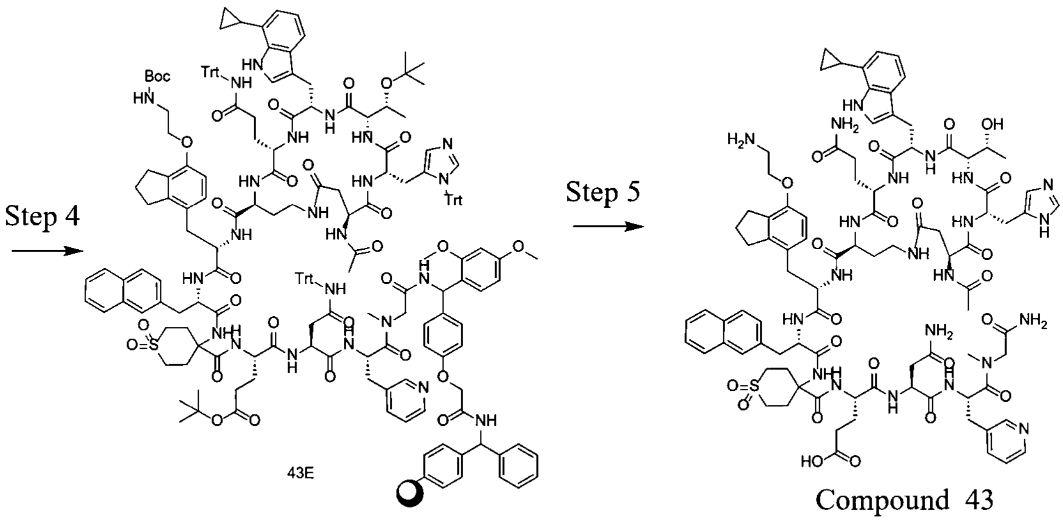


43E

Compound 43

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (3.0 eq.) | DIC (3 eq.) and HOBT (3 eq.) |
| 8 | Fmoc-Dab(Dde) -OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Asp(OAll)-OH (2.0 eq.) | DIC (2 eq.) and HOBT (2 eq.) |
| 14 | Acetic anhydride | DIEA |

**Compound 43** (95 mg, purity: 97%) was obtained by using the materials in the above table as starting materials and adopting a method for **Compound 10.**

LCMS m/z = 965.9$[M/2+H]^+$, 644.4$[M/3+H]^+$.

Example 44

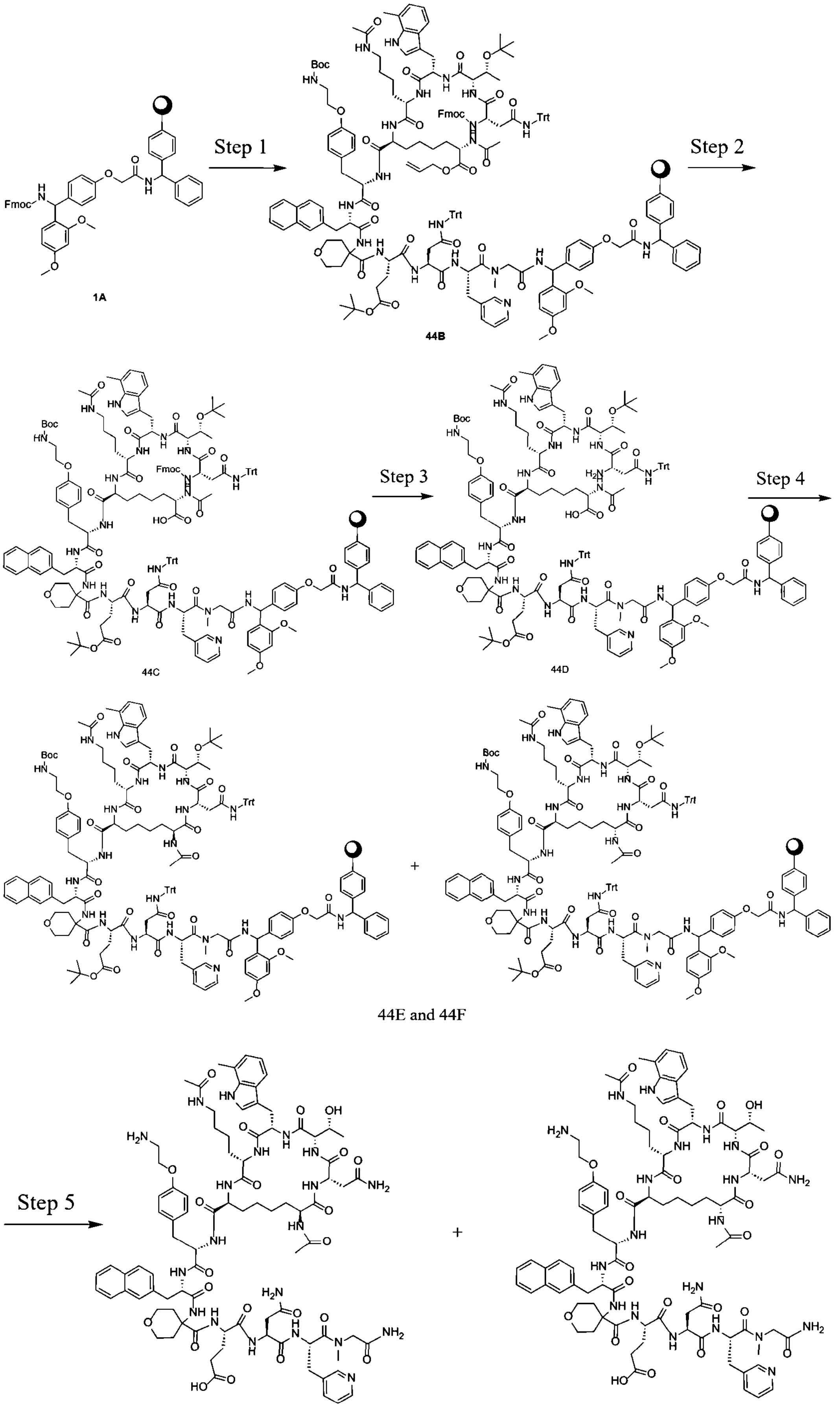


1A

44B

44C

44D

44E and 44F

Compound 44-1 and Compound 44-2

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amino-ethoxy)]-L-phenylalanine (3.0 eq.) | DIC (3 eq.) and HOBT (3 eq.) |
| 8 | **Intermediate 11** (2.0 eq.) | DIC (2 eq.) and HOAT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-Asn(Trt)-OH (3.0 eq.) | DIC (3 eq.) and HOBT (3 eq.) |

Step 1: **Compound 44B** was obtained by using the materials in the above table as starting materials and adopting a method for **Compound 1.**

Step 2: 30 ml of dichloromethane was added to a solid-phase synthesis reactor, followed by phenylsilane (1 g) and tetrakis(triphenylphosphine)palladium (462 mg) successively. The mixture was bubbled with nitrogen, and reacted for 8 hours. The resin was washed with dichloromethane 5 times, N,N-dimethylformamide 5 times, 0.5% solution of sodium diethyldithiocarbamate in N,N-dimethylformamide for 15 min + 15 min, and then N,N-dimethylformamide three times to give the peptide resin **44C.**

Step 3: 20% solution of piperidine in N,N-dimethylformamide was added to the reactor, and the mixture was reacted for 30 min. The resultant was washed with N,N-dimethylformamide solution 5 times to give the peptide resin **44D.**

Step 4: N-methylpyrrolidone (20 ml), benzotriazole-N,N,N',N'-tetramethylurea hexafluorophosphate (417.2 mg, 1.1 mmol), and DIEA (0.19 ml, 1.1 mmol) were added to the solid-phase synthesis reactor, and the mixture was reacted at room temperature for 24 h. The reaction was monitored with ninhydrin until it was colourless. The solvent was pumped off, and the resin was washed with N,N-dimethylformamide three times, methanol twice, dichloromethane once, and methanol twice. The solvent was pumped off under vacuum to give the peptide resin **44E/F** (5 g).

Step 5: 40 ml of cleavage solution (91% trifluoroacetic acid + 4% triisopropylsilane + 3% 1,2-ethanedithiol + 2% water) was added to a 100 ml reaction flask, and stirred evenly. Then, the peptide resin **44E/F** (5 g) was added, and the mixture was stirred at room temperature for 2 hours. The resin was filtered to obtain the filtrate, which was added to 300 ml of methyl tert-butyl ether (which was cooled to 0°C in advance). White floccules were precipitated, and centrifuged (3 min at 3000 rpm). The white precipitate was washed with methyl tert-butyl ether three times, and dried in vacuo to give the crude peptide **Compound 44E/F** as an off-white solid (1.5 g), which was purified by preparative HPLC. Separation method: 1. Instrument: Waters 2767 (preparative liquid chromatographic instrument); and chromatography column: SunFire@-PrepC18 (19 mm × 250 mm). 2. The sample was filtered with a 0.45 μm filter head to prepare a sample solution. 3. Preparative chromatography conditions: a. composition of mobile phases A and B: A: 5 mmol/L ammonium acetate/$H_2O$, and B: $CH_3CN$; b. gradient elution: mobile phase = 5%-45%; c. flow rate: 12 ml/min; d. elution time: 30 min. Lyophilization was carried out to afford **Compound 44-1** (77 mg, purity: 94%, retention time: 13.198 min) and **Compound 44-2** (44 mg, purity: 98%, retention time: 14.509 min) as white solids.

**44-1.** LCMS m/z =903.8$[M/2+H]^+$.603.0$[M/3+H]^+$.

**44-2.** LCMS m/z =904.1$[M/2+H]^+$,

Example 45:

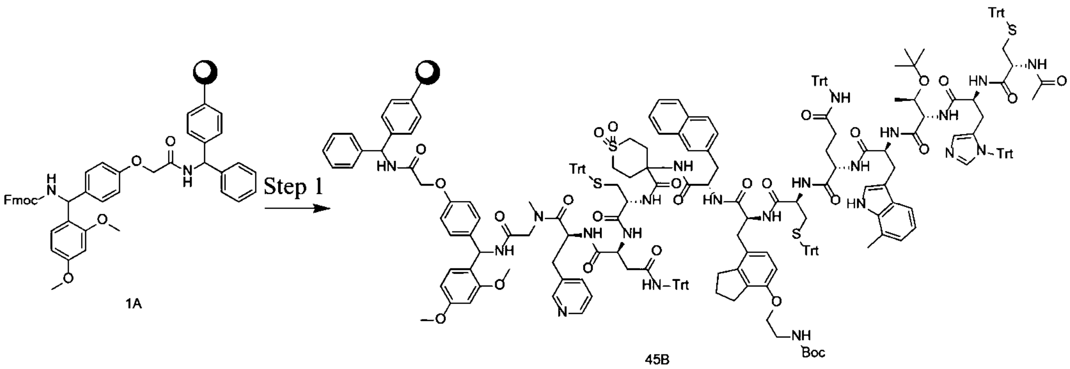


1A

45B

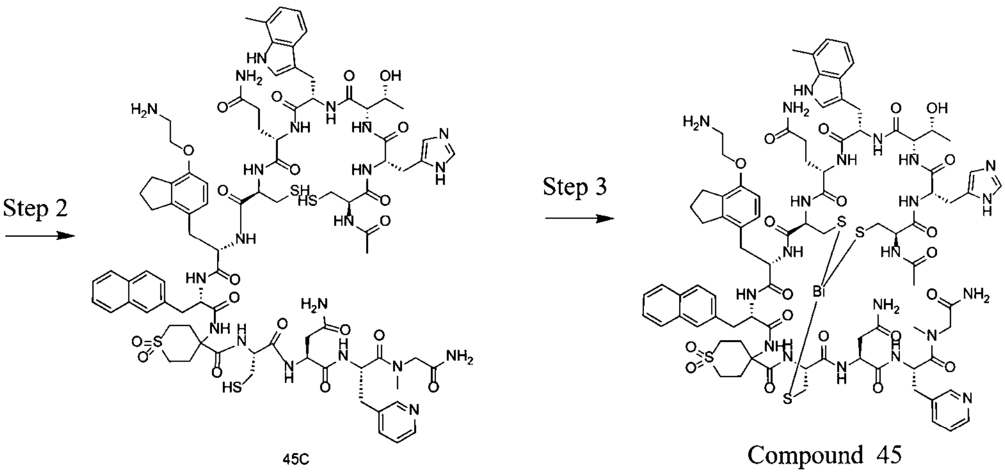


45C

Compound 45

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Cys(Trt)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Cys(Trt) -OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Cys(Trt)-OH (2.0 eq.) | DIC (2 eq.) and HOBT (2 eq.) |
| 14 | Acetic anhydride | DIEA |

**Compound 45C** (1.5 g) was obtained by using the materials in the above table as starting materials and adopting a method for **Compound 1.**

Step 3: To a 3 L reaction flask, water (750 ml), acetonitrile (750 ml), and **45C** (1.5 g, 0.8 mmol) were added successively, and stirred evenly. Then, bismuth bromide (0.36 g, 0.8 mmol) was added. The pH was adjusted to 8 with ammonium bicarbonate. The mixture was reacted for 16 h and purified by preparative HPLC. Separation method: 1. Instrument: Waters 2767 (preparative liquid chromatographic instrument); and Chromatographic column: SunFire@-

PrepC18 (19 mm × 250 mm). 2. The sample was filtered with a 0.45 μm filter head to prepare a sample solution. 3. Preparative chromatography conditions: a. composition of mobile phases A and B: A: 0.1% trifluoroacetic acid/$H_2O$, and B: $CH_3CN$; b. gradient elution: mobile phase = 5%-45%; c. flow rate: 12 ml/min; d. elution time: 30 min; and retention time: 17 min. Lyophilization was carried out to afford **Compound 45** (57 mg, purity: 90%) as a white solid.

LCMS m/z =1047.1 [M/2+H]$^+$, 698.5 [M/3+H]$^+$.

Example 46:

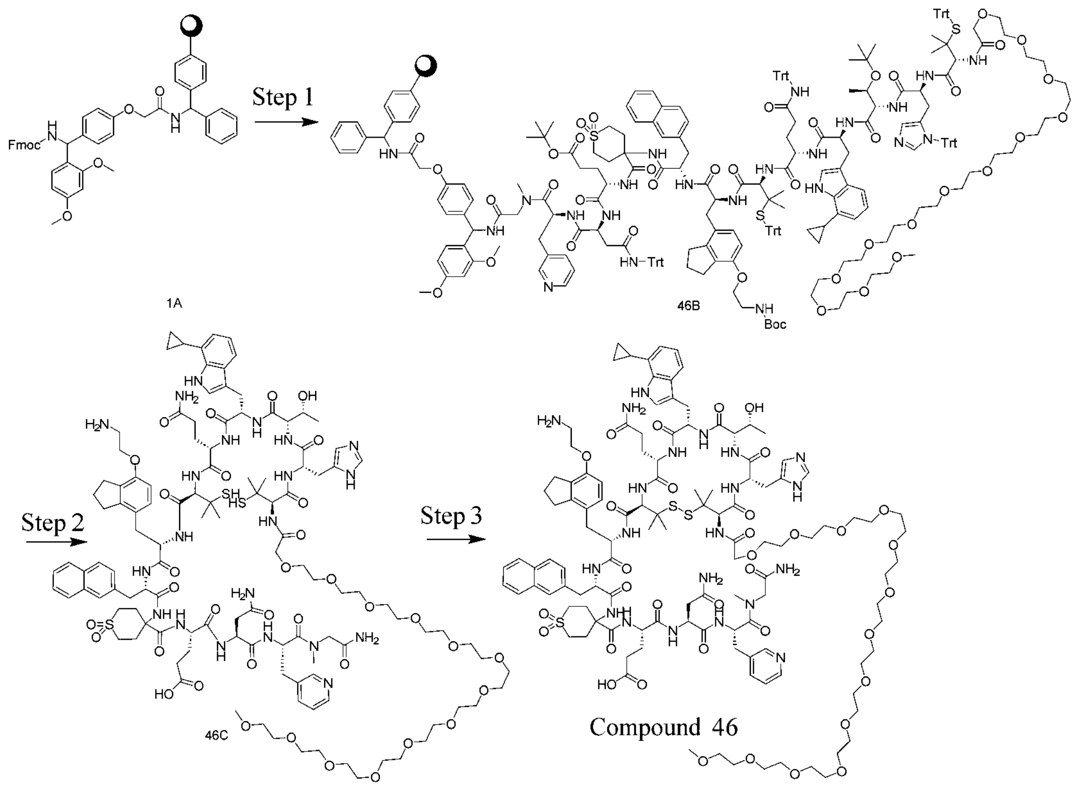


1A

46B

46C

Compound 46

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | m-PEG12-CH2COOH (2.0 eq.) | DIC (2 eq.) and HOBT (2 eq.) |

**Compound 46** (105 mg, purity: 92%) was obtained by using the materials in the above table as starting materials and adopting a method for **Compound 1.**

LCMS m/z = 851.7 [M/3+H]$^+$.

Example 47:

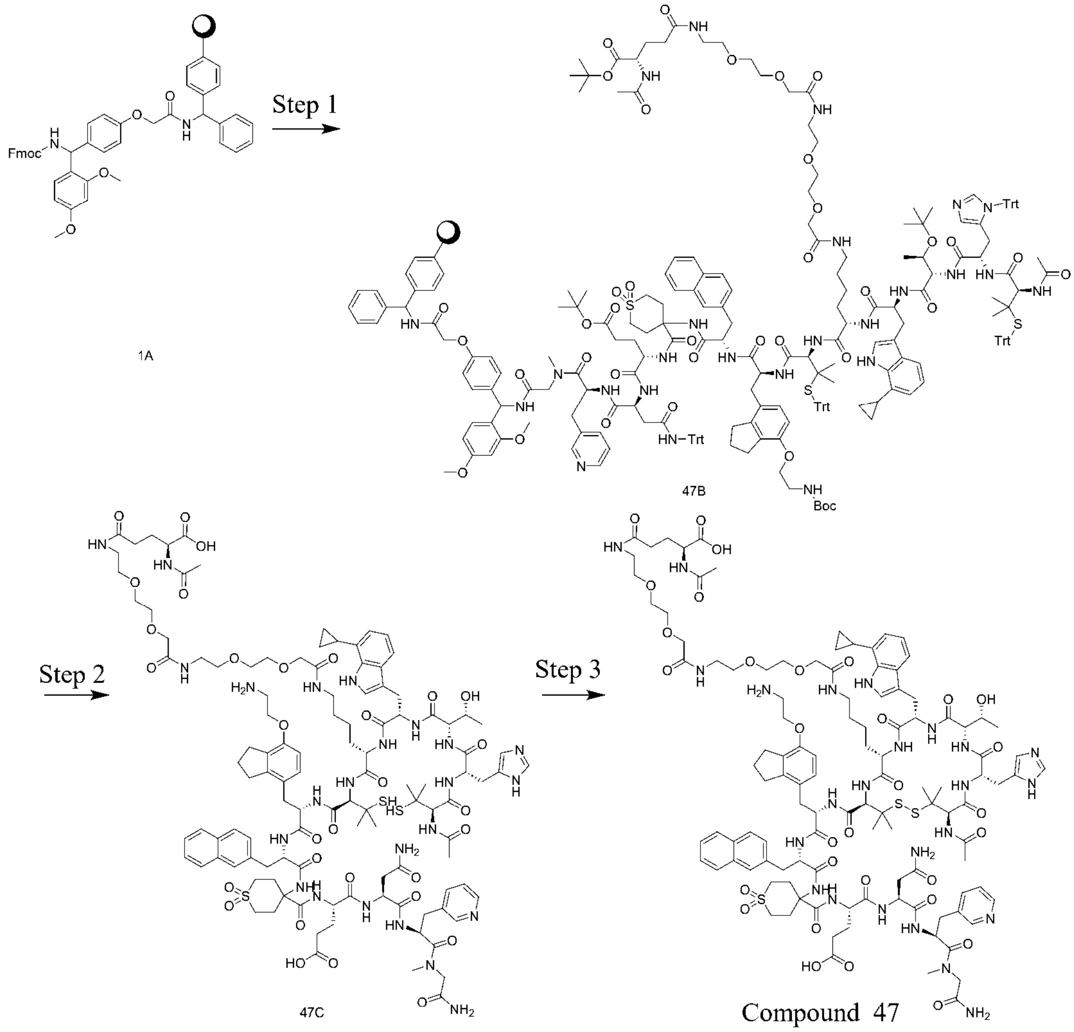


1A

47B

47C

Compound 47

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Lys(Dde)-OH (3.0 eq.) | DIC (3 eq.) and HOBT (3 eq.) |
| 10 | **Intermediate 6** (2.0 eq.) | DIC (2 eq.) and HOBT (2 eq.) |

(continued)

| Serial No. | Starting material | Coupling reagent |
|---|---|---|
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |
| 15 | Fmoc-AEEA-OH (3.0 eq.) | DIC (3 eq.) and HOBT (3 eq.) |
| 16 | Fmoc-AEEA-OH (3.0 eq.) | DIC (3 eq.) and HOBT (3 eq.) |
| 17 | Fmoc-Glu-OtBu (3.0 eq.) | DIC (3 eq.) and HOBT (3 eq.) |
| 18 | Acetic anhydride | DIEA |

**Compound 47** (320 mg, purity: 95%) was obtained by using the materials in the above table as starting materials and adopting a method for **Compound 21.**

LCMS m/z = 818.9 $[M/3+H]^+$, 614.5 $[M/4+H]^+$.

Example 48:

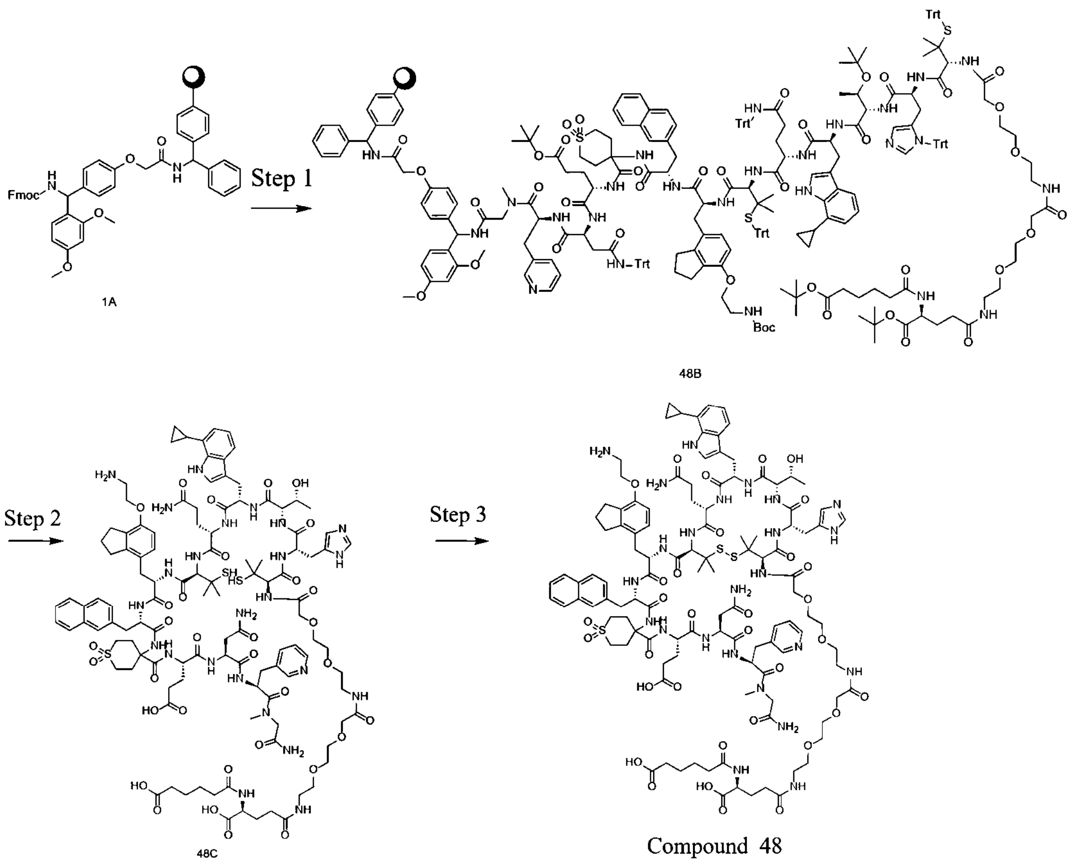


1A

48B

48C

Compound 48

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |

(continued)

| Serial No. | Starting material | Coupling reagent |
|---|---|---|
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Fmoc-AEEA-OH | DIC (3 eq.) and HOBT (3 eq.) |
| 15 | Fmoc-AEEA-OH | DIC (3 eq.) and HOBT (3 eq.) |
| 16 | Fmoc-Glu-OtBu | DIC (3 eq.) and HOBT (3 eq.) |
| 17 | 6-(tert-butoxy)-6-oxohexanoic acid | DIC (3 eq.) and HOBT (3 eq.) |

**Compound 48** (200 mg, purity: 97%) was obtained by using the materials in the above table as starting materials and adopting a method for **Compound 1.**

LCMS m/z = 1049.7 [M/2+H]$^+$, 833.7 [M/3+H]$^+$.

Example 49:

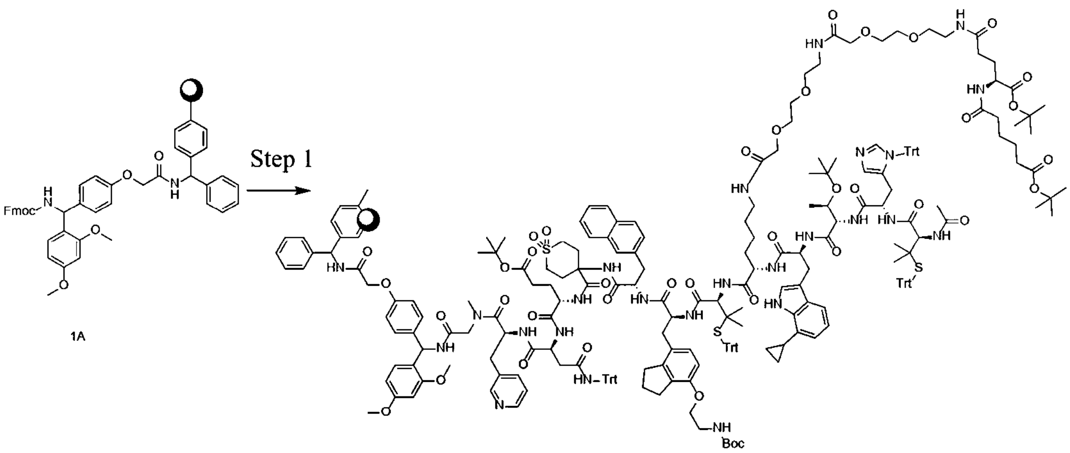


1A

49B

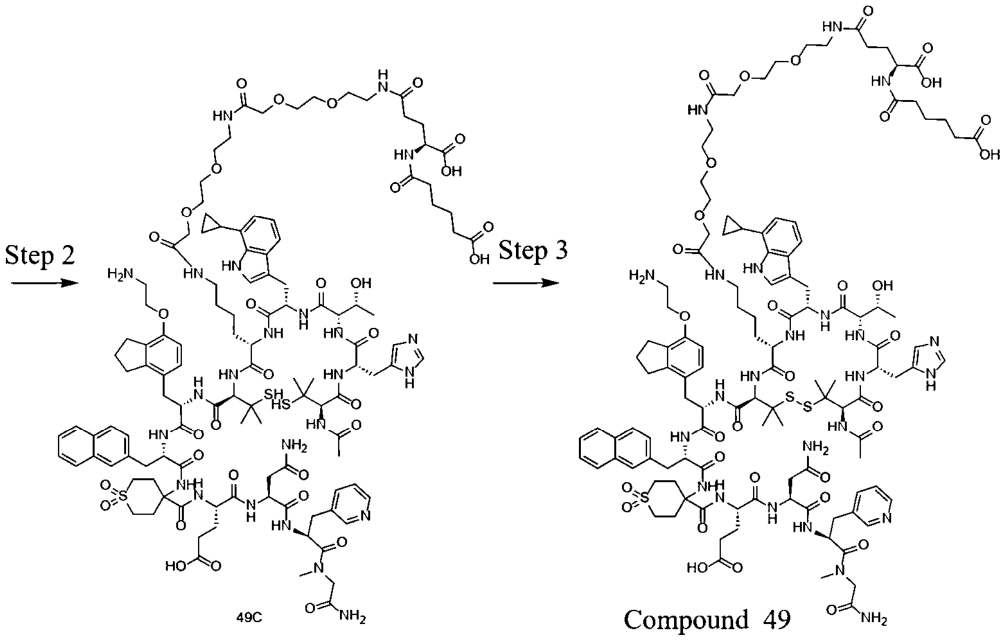


49C

Compound 49

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (2.0 eq.) | DIC (2 eq.) and HOBT (2 eq.) |
| 8 | Fmoc-Pen(Trt)-OH (2.0 eq.) | DIC (2 eq.) and HOBT (2 eq.) |
| 9 | Fmoc-Lys(Dde)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | Fmoc-Pen(Trt)-OH (2.0 eq.) | DIC (2 eq.) and HOBT (2 eq.) |
| 14 | Acetic anhydride | DIEA |
| 15 | Fmoc-AEEA-OH (3.0 eq.) | DIC (3 eq.) and HOBT (3 eq.) |
| 16 | Fmoc-AEEA-OH (3.0 eq.) | DIC (3 eq.) and HOBT (3 eq.) |
| 17 | Fmoc-Glu-OtBu (3.0 eq.) | DIC (3 eq.) and HOBT (3 eq.) |
| 18 | 6-(tert-butoxy)-6-oxohexanoic acid | DIC (3 eq.) and HOBT (3 eq.) |

**Compound 49** (220 mg, purity: 95%) was obtained by using the materials in the above table as starting materials and adopting a method for **Compound 21.**

LCMS m/z = 1271.0 [M/2+H]$^+$, 847.7 [M/3+H]$^+$.

Example 50:

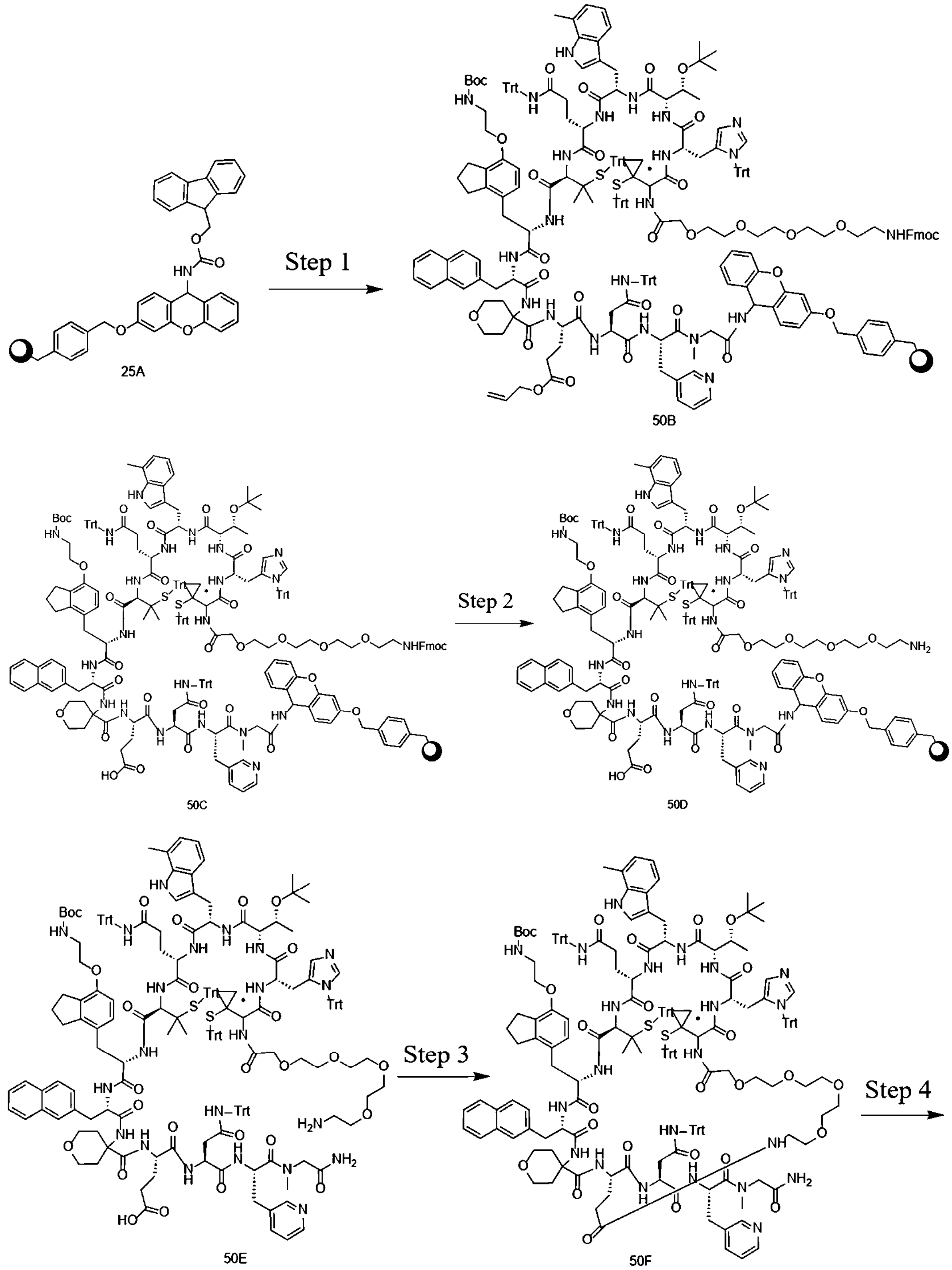


25A

50B

50C

50D

50E

50F

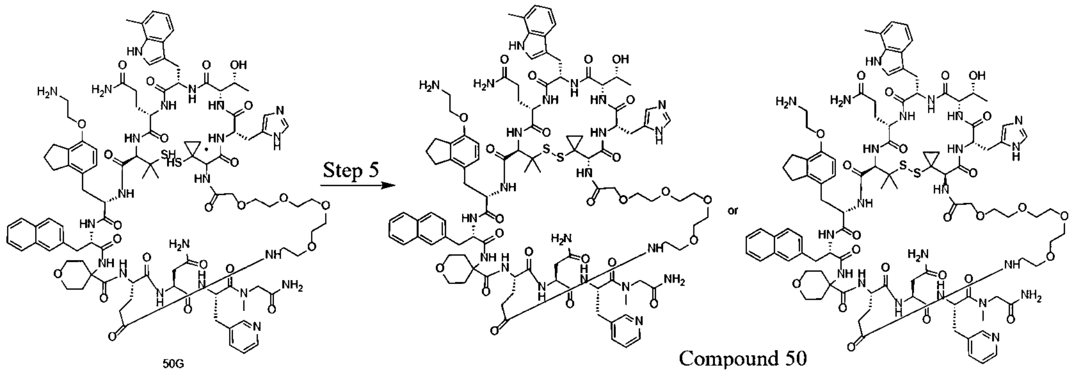


50G

or

Compound 50

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (3.0 eq.) | DIC (3 eq.) and HOAT (3 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | **Intermediate 9** (2.0 eq.) | DIC (2 eq.) and HOAT (2 eq.) |
| 14 | N-fluorenylmethoxycarbonyl-tetraethylene glycol-acetic acid (3.0 eq.) | DIC (3 eq.) and HOBT (3 eq.) |

**Compound 50** (100 mg) was obtained by using the materials in the above table as starting materials and adopting a method for **Compound 25.**

LCMS m/z =1046.0 $[M/2+H]^+$.

Example 51:

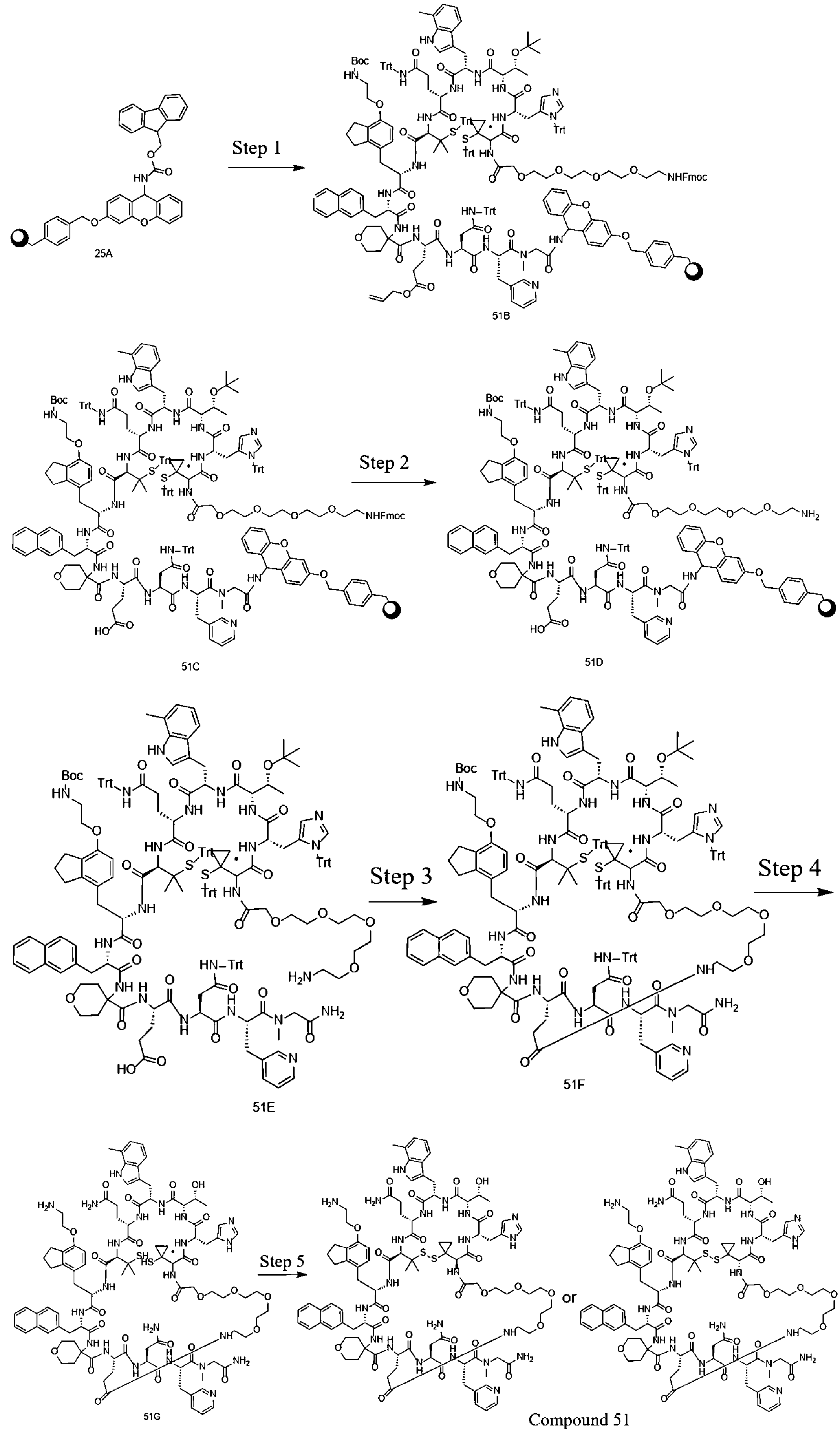


25A

51B

51C

51D

51E

51F

51G

or

Compound 51

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Intermediate 3 (3.0 eq.) | DIC (3 eq.) and HOAT (3 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | **Intermediate 10** (2.0 eq.) | DIC (2 eq.) and HOAT (2 eq.) |
| 14 | N-fluorenylmethoxycarbonyl-tetraethylene glycol-acetic acid (3.0 eq.) | DIC (3 eq.) and HOBT (3 eq.) |

**Compound 51** (75 mg) was obtained by using the materials in the above table as starting materials and adopting a method for **Compound 25.**

LCMS m/z =1046.0 [M/2+H]+.

Example 52:

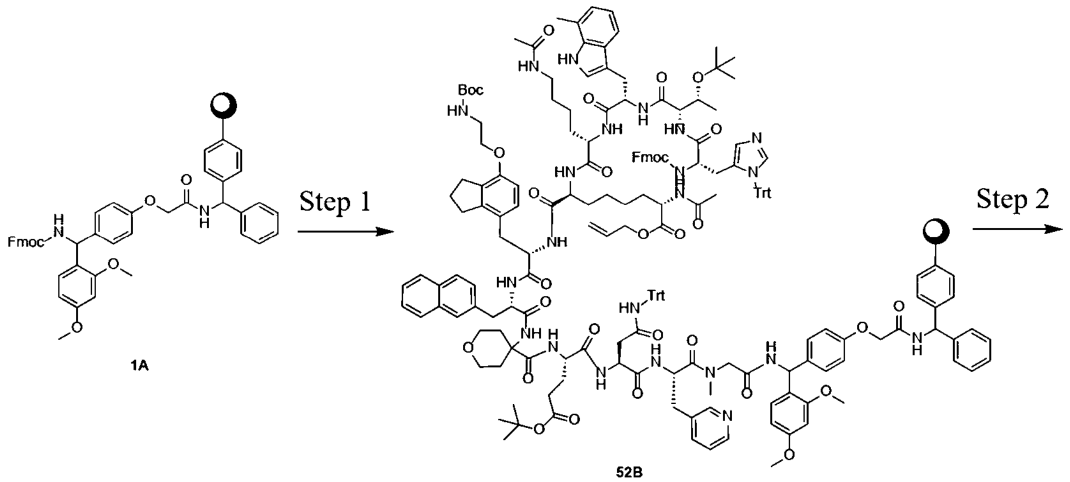


1A

52B

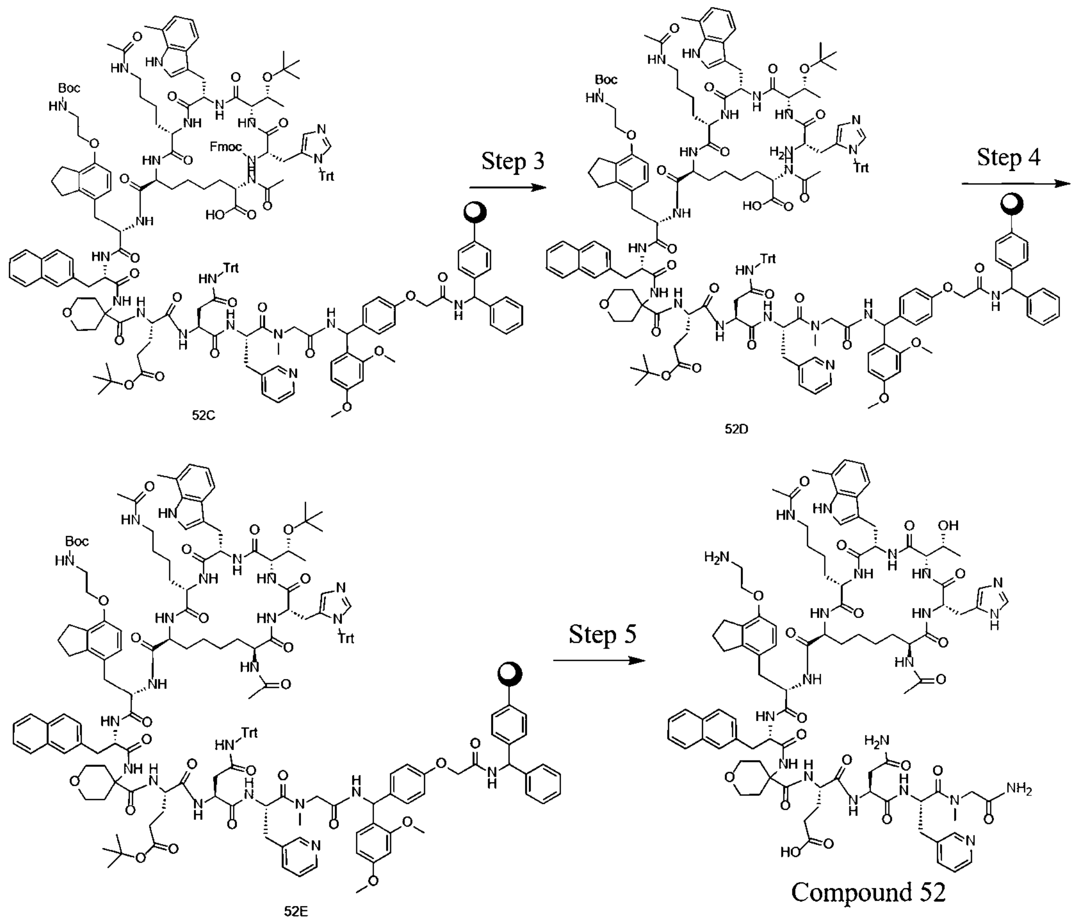


52C 52D 52E Compound 52

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | Fmoc-ThpGly-OH (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (3.0 eq.) | DIC (3 eq.) and HOBT (3 eq.) |
| 8 | **Intermediate 11** (2.0 eq.) | DIC (2 eq.) and HOAT (2 eq.) |
| 9 | Fmoc-Lys(Ac)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |

**Compound 52** (400 mg) was obtained by using the materials in the above table as starting materials and adopting a method for **Compound 44.**

LCMS m/z =935.5[M/2+H]$^+$, 623.9[M/3+H]$^+$.

Example 53:

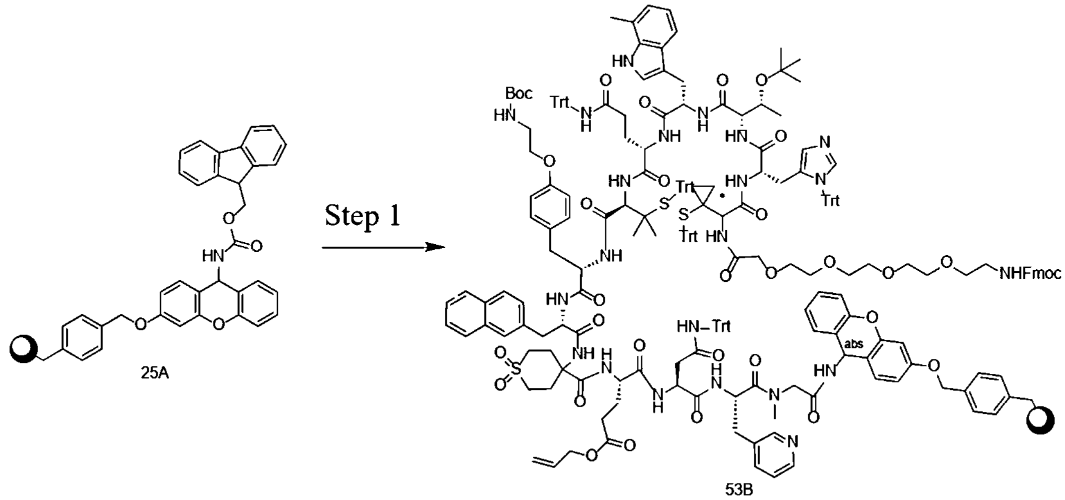


25A

53B

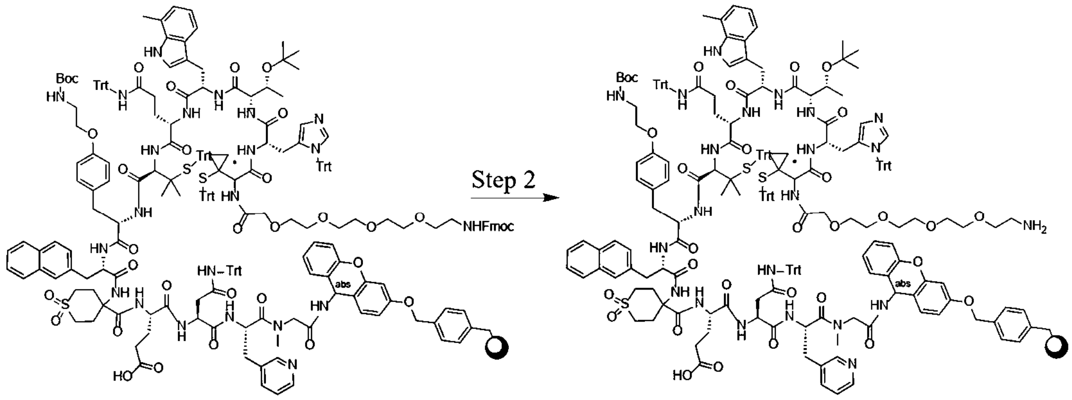


53C

53D

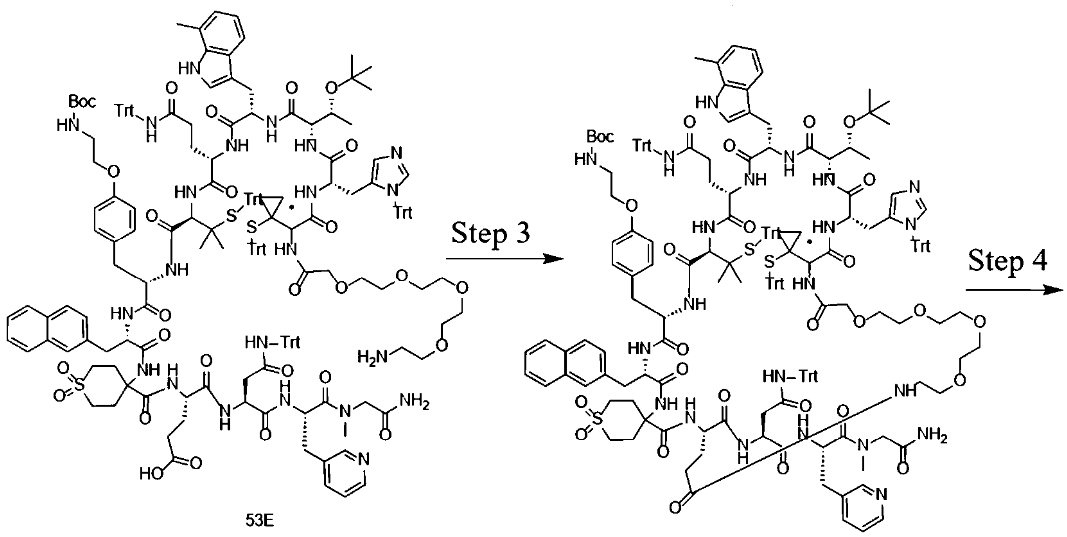


53E

53F

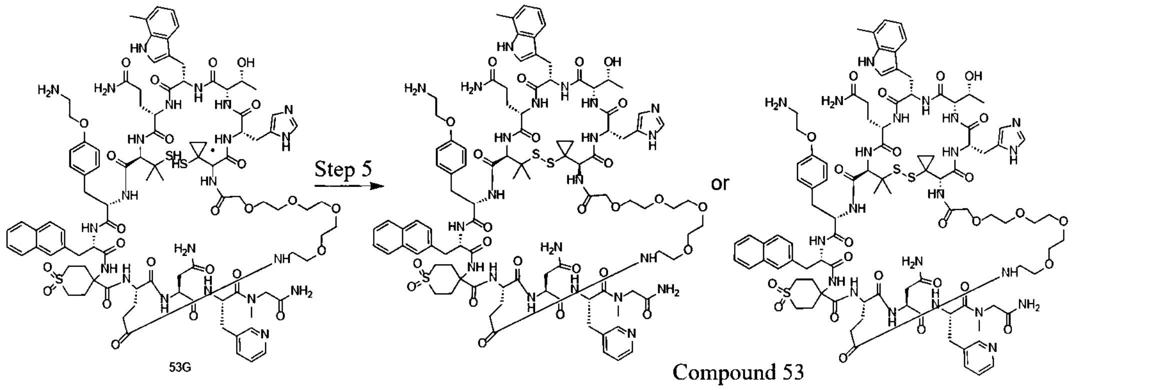


53G

or

Compound 53

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OAll)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | Fmoc-4-[2-(Boc-amido-ethoxy)]-L-ph enylalanine (3.0 eq.) | DIC (3 eq.) and HOAT (3 eq.) |
| 8 | Fmoc-Pen(Trt)-OH (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | **Intermediate 10** (2.0 eq.) | DIC (2 eq.) and HOAT (2 eq.) |
| 14 | N-fluorenylmethoxycarbonyl-tetraethylene glycol-acetic acid (3.0 eq.) | DIC (3 eq.) and HOBT (3 eq.) |

**Compound 53** (72 mg) was obtained by using the materials in the above table as starting materials and adopting a method for **Compound 25.**

LCMS m/z =1049.6 $[M/2+H]^+$,700.2 $[M/3+H]^+$.

Example 54:

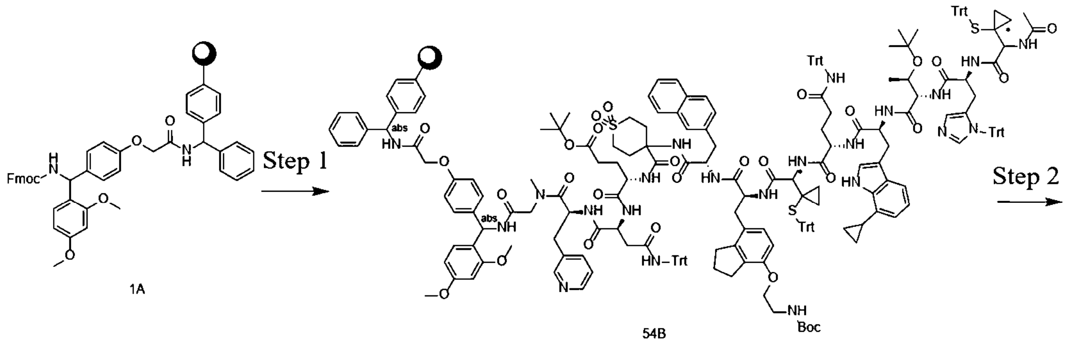


1A

54B

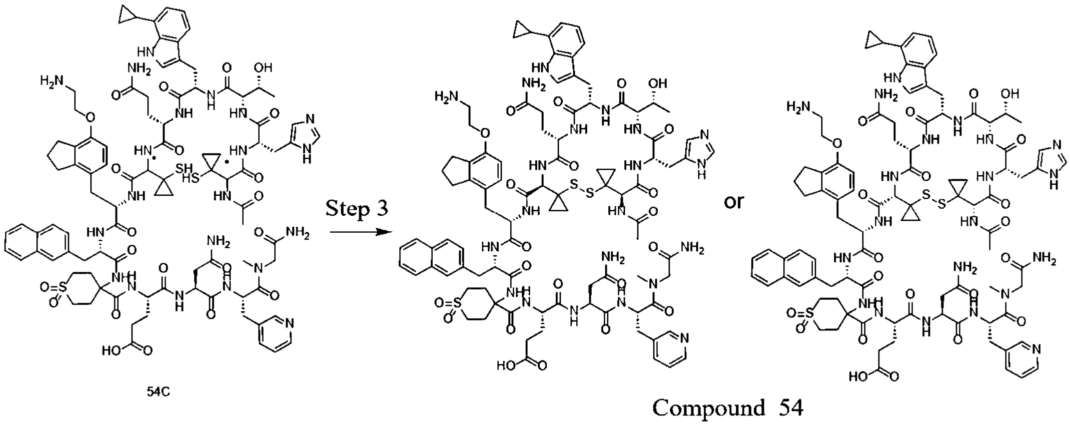


54C

Compound 54

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (3.0 eq.) | DIC (3 eq.) and HOBT (3 eq.) |
| 8 | **Intermediate 10** (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | **Intermediate 6** (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | **Intermediate 10** (2.0 eq.) | DIC (2 eq.) and HOBT (2 eq.) |
| 14 | Acetic anhydride | DIEA |

**Compound 54** (400 mg) was obtained by using the materials in the above table as starting materials and adopting a method for **Compound 1.**

LCMS m/z = 995.0[M/2+H]$^+$.

Example 55:

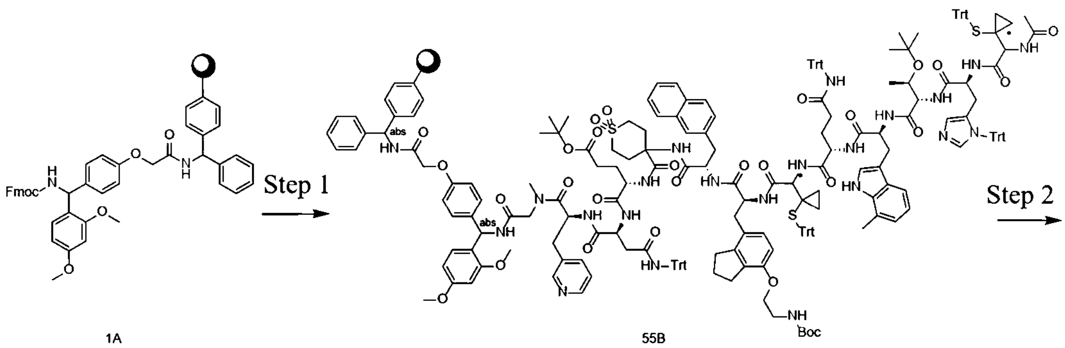


1A 55B

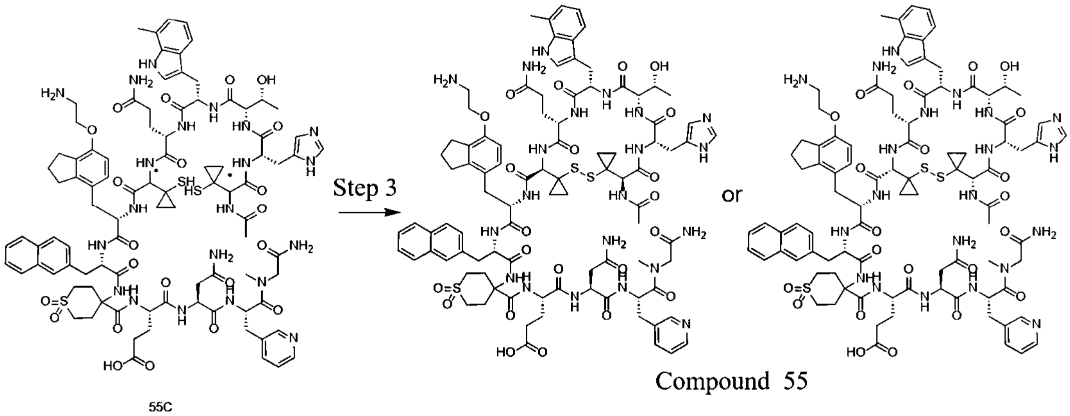


Compound 55

55C

| Serial No. | Starting material | Coupling reagent |
|---|---|---|
| 1 | Fmoc-Sar-OH (3.0 eq.) | DIC (3 eq.) and HOBT (3 eq.) |
| 2 | Fmoc-3Pal-OH (3.0 eq.) | DIC (3 eq.) and HOBT (3 eq.) |
| 3 | Fmoc-Asn(Trt)-OH (3.0 eq.) | DIC (3 eq.) and HOBT (3 eq.) |
| 4 | Fmoc-Glu(OtBu)-OH (3.0 eq.) | DIC (3 eq.) and HOBT (3 eq.) |
| 5 | **Intermediate 5** (2.0 eq.) | DIC (2 eq.) and HOBT (2 eq.) |
| 6 | Fmoc-2-Nal-OH (3.0 eq.) | DIC (3 eq.) and HOBT (3 eq.) |
| 7 | **Intermediate 3** (3.0 eq.) | DIC (3 eq.) and HOBT (3 eq.) |
| 8 | **Intermediate 10** (2.0 eq.) | DIC (2 eq.) and HOBT (2 eq.) |
| 9 | Fmoc-Gln(Trt)-OH (3.0 eq.) | DIC (3 eq.) and HOBT (3 eq.) |
| 10 | Fmoc-7MeTrp-OH (2.0 eq.) | DIC (2 eq.) and HOBT (2 eq.) |
| 11 | Fmoc-Thr(tBu)-OH (3.0 eq.) | DIC (3 eq.) and HOBT (3 eq.) |
| 12 | Fmoc-His(Trt)-OH (3.0 eq.) | DIC (3 eq.) and HOBT (3 eq.) |
| 13 | **Intermediate 10** (2.0 eq.) | DIC (2 eq.) and HOBT (2 eq.) |
| 14 | Acetic anhydride | DIEA |

**Compound 55** (410 mg) was obtained by using the materials in the above table as starting materials and adopting a method for **Compound 1.**

LCMS m/z = 982.0[M/2+H]$^+$.

**Bioassay methods**

**1. IL-23α/IL-12β and IL-23R binding assay**

Compounds were tested for their inhibition on the binding of IL-23α/IL-12β to IL-23R by the TR-FRET method. Solutions of proteins IL-23α/IL-12β (ACRO, Cat# ILB-H52W5) and IL-23R (ACRO, Cat# ILR-H82F3) were prepared in reaction buffer PPI (PerkinElmer, Cat# 61DB10RDF). The final concentrations of IL-23α/IL-12β and IL-23R in the reaction mixture were both 0.3 nM. Positive reference Guselkumab was 3-fold diluted from an initial concentration of 30 nM, with 10 doses. 0.1 μL of the positive reference diluted in the reaction buffer was delivered to a 384-well plate (Grenier, Cat# 784075) by the acoustic liquid delivery technique (Echo 655), and the plate was centrifuged at 1000 rpm for 1 minute. 2.5 μL of IL-23α/IL-12β solution was transferred into the 384-well reaction plate, and the plate was centrifuged at 1000 rpm for 1 minute, and incubated at 25°C for 60 minutes. 2.5 μL of IL-23R solution was transferred into the 384-well reaction plate, and the plate was centrifuged at 1000 rpm for 1 minute. 5 μL of Streptavidin-Tb cryptate and Anti 6HIS-d2 detection mixture was transferred into the 384-well reaction plate, and the plate was centrifuged at 1000 rpm for 1 minute, and incubated at 25°C for 60 minutes. Finally, the HTRF signal (Ratio 665/620 nm) was read with a BMG high-throughput drug screening multifunctional microplate reader. $IC_{50}$ values were obtained by nonlinear regression curve fitting using GraphPad Prism software.

Table 1

| Compound | $IC_{50}$ (nM) | Compound | $IC_{50}$ (nM) |
|---|---|---|---|
| **Compound 1** | 0.0642 | **Compound 33** | 0.395 |
| **Compound 2** | 0.0768 | **Compound 35** | 0.063 |
| **Compound 3** | 0.1738 | **Compound 36** | 0.042 |
| **Compound 6** | 0.1456 | **Compound 37** | 0.050 |
| **Compound 7** | 0.05387 | **Compound 38** | 0.212 |
| **Compound 8** | 0.06909 | **Compound 39** | 0.057 |
| **Compound 9** | 0.05596 | **Compound 40** | 0.064 |
| **Compound 11** | 0.1236 | **Compound 41** | 0.069 |
| **Compound 12** | 0.0417 | **Compound 44-1** | 0.145 |
| **Compound 13** | 0.0384 | **Compound 44-2** | 0.134 |
| **Compound 15** | 0.075 | **Compound 45** | 0.192 |
| **Compound 16** | 0.063 | **Compound 46** | 0.095 |
| **Compound 19** | 0.248 | **Compound 47** | 0.093 |
| **Compound 20** | 0.116 | **Compound 48** | 0.058 |
| **Compound 21** | 0.248 | **Compound 49** | 0.068 |
| **Compound 22** | 0.129 | **Compound 50** | 0.073 |
| **Compound 23** | 0.161 | **Compound 51** | 0.075 |
| **Compound 24** | 0.063 | **Compound 52** | 0.101 |
| **Compound 27** | 0.114 | **Compound 53** | 0.065 |
| **Compound 28** | 0.122 | **Compound 54** | 0.091 |
| **Compound 29** | 0.089 | **Compound 55** | 0.068 |
| **Compound 30** | 0.460 | | |
| **Compound 31** | 0.258 | | |
| Conclusion: The compounds of the present disclosure, such as the compounds in the Examples, are able to significantly inhibit the binding of IL-23α/IL-12β to IL-23R. | | | |

**2. IL-23R reporter gene assay**

The purpose of this assay was to assess the ability of compounds to inhibit the binding of IL23p19 to IL23R in a

reporter gene system. The HEK-blue IL23 reporter gene cell line (Invivogen, hkb-il23) was cultured in DMEM + 10% FBS + 100 ug/mL Normocin medium. When the cell density reached 80%-90%, 5000 cells/well were plated into a 384-well plate, and the plate was incubated overnight at 37°C with 5% CO2. Then, the compound stock solution was diluted in DMSO, and 40 nL of the dilution was transferred into the 384-well culture plate by Echo. The plate was incubated at 37°C with 5% $CO_2$ for 0.5 hours. 40 nL/well of rhIL23 (R&D, 1290-IL) was added to the 384-well cell culture plate at a final concentration of 1 ng/mL, and the plate was incubated at 37°C with 5% $CO_2$ for 24 hours. 18 μL of Quanti-Blue™ solution was added to a new 384-well plate. 2 μL/well of cell culture supernatant was transferred into the 384-well plate prepared in Step 6, and the plate was incubated at 37°C with 5% $CO_2$ for 1 hour. Absorbance values at 620-655 nM were read on BMG. The binding ability of the compounds was assessed using the following formula, and $IC_{50}$ values were obtained by fitting with Graphpad.

Calculation formula of inhibition rate:

$$\% \text{ inhibition} = [\overline{Data}_{\text{negative}} - Data_{\text{sample}}]/[\overline{Data}_{\text{negative}} - \overline{Data}_{\text{positive}}] \times 100\ \%$$

where *Data* $_{\text{negative}}$: mean of DMSO control group; and
*Data* $_{\text{positive}}$: mean of non-IL23-stimulated group

The calculation formula of $IC_{50}$ was as follows:

$$Y=Bottom + (Top-Bottom)/(1+10\^{}((LogIC50 -X)*HillSlope))$$

where X: concentration of compound; and
Y: inhibition rate of compound

Table 2. Inhibition of IL-23R by test compounds

| Compound | $IC_{50}$ (nM) | Compound | $IC_{50}$ (nM) |
|---|---|---|---|
| **Compound 1** | 1.1 | **Compound 13** | 3.12 |
| **Compound 2** | 1.21 | **Compound 15** | 5.26 |
| **Compound 3** | 1.36 | **Compound 16** | 6.07 |
| **Compound 7** | 6.38 | **Compound 24** | 9.38 |
| **Compound 9** | 2.56 | **Compound 27** | 1.37 |
| **Compound 11** | 7.47 | **Compound 28** | 6.27 |
| **Compound 12** | 3.58 | **Compound 29** | 1.72 |
| Conclusion: The compounds of the present disclosure, such as the compounds in the Examples, are able to significantly inhibit the binding of IL23p19 to IL23R. | | | |

**3. IL-23-stimulated pSTAT3 detection assay in PBMCs**

Cryopreserved human PBMCs were thawed, and seeded onto a plate previously covered with an anti-CD3 antibody at 1 × 10^6 cells/well. Then, an anti-CD28 antibody was added to the plate, and the cells were incubated at 37°C with 5% $CO_2$ for 5 days. On Day 5, the cells were seeded onto a 96-well plate at a density of 100K cells/well after 4 hours of stimulation by FBS starvation. The diluted Compound was transferred into the 96-well cell culture plate, and the plate was incubated at 37°C with 5% $CO_2$ for 1 hour. rhIL23 (R&D, 1290-IL) was added to the cell culture plate, and the plate was incubated at 37°C with 5% $CO_2$ for 30 minutes. The cells in the wells were lysed on ice with a lysis buffer containing 1× PHOSstop solution for 30 minutes, and the plate was centrifuged at 1000 rpm for 1 minute. Then, the supernatant was transferred into a 96-well ELISA plate, and pSTAT3 ELISA assay was performed according to the instructions of the kit (CST, 7300CA). Absorbance values at 450 nM were read on PHERAstar FSX (BMG LRBTECH). The inhibition rate of the compounds was assessed using the following formula, and $IC_{50}$ values were obtained by fitting with Graphpad.

Calculation formula of inhibition rate:

$$\% \text{ inhibition} = [\overline{Data}_{\text{negative}} - Data_{\text{sample}}]/[\overline{Data}_{\text{negative}} - \overline{Data}_{\text{positive}}] \times 100\ \%$$

where $Data_{\text{negative}}$: mean of DMSO control group; and
$Data_{\text{positive}}$: mean of non-IL23-stimulated group

The calculation formula of $IC_{50}$ was as follows:

$$Y=Bottom + (Top-Bottom)/(1+10^{\wedge}((LogIC50 - X)*HillSlope))$$

where X: concentration of compound; and
Y: inhibition rate of compound.

Table 3. pSTAT3 inhibition

| Compound | pSTAT3 inhibition/$IC_{50}$ (nM) |
|---|---|
| **Compound 1** | 0.0071 |
| **Compound 2** | 0.0068 |
| **Compound 3** | 0.0066 |
| **Compound 7** | 0.0014 |
| **Compound 9** | 0.0006 |
| **Compound 12** | 0.0009 |
| **Compound 13** | 0.0010 |
| **Compound 15** | 0.0062 |
| **Compound 16** | 0.0069 |
| **Compound 22** | 0.0157 |
| Conclusion: The compounds of the present disclosure, such as the compounds in the Examples, have a significant inhibitory effect on STAT3 phosphorylation. | |

**4. Pharmacokinetic test in mice**

**4.1. Experimental animals:** Male Balb/c mice (20-25 g, 18 mice/compound) were purchased from Chengdu Dossy Experimental Animals Co., Ltd.

**4.2. Experimental design:** On the day of the experiment, the Balb/c mice were randomly grouped by body weight. The animals were fasted with water available for 12-14 h one day before the administration, and were fed 4 h after the administration.

Table 4. Administration information

| Group | Number | Administration information | | | | | |
|---|---|---|---|---|---|---|---|
| | Male | | Adminis tration dosage (mg/kg) | Administr ation concentrat ion (mg/mL) | Administr ation volume (mL/kg) | Collected sample | Administration mode |
| G1 | 3 | **Compound 7** | 1 | 0.2 | 5 | Plasma | Intravenous administration |
| G2 | 3 | | 10 | 1 | 10 | Plasma | Intragastric ad-ministration |

(continued)

| Group | Number |  | Administration information | | | | |
|---|---|---|---|---|---|---|---|
| | Male | | Adminis tration dosage (mg/kg) | Administr ation concentrat ion (mg/mL) | Administr ation volume (mL/kg) | Collected sample | Administration mode |
| G3 | 3 | **Compound 16** | 1 | 0.2 | 5 | Plasma | Intravenous administration |
| G4 | 3 | | 10 | 1 | 10 | Plasma | Intragastric administration |
| G5 | 3 | **Compound 24** | 1 | 0.2 | 5 | Plasma | Intravenous administration |
| G6 | 3 | | 10 | 1 | 10 | Plasma | Intragastric administration |
| Note: Vehicle for intravenous administration: PBS; and vehicle for intragastric administration: PBS | | | | | | | |

Before and after administration, 0.06 mL of blood was taken from the orbit under isoflurane anaesthesia, and placed in an EDTAK2 centrifuge tube. The tube was centrifuged at 5000 rpm at 4°C for 10 min, and plasma was collected. The blood collection time points for the intravenous group and intragastric group were both 0 min, 5 min, 15 min, 30 min, 1 h, 2 h, 4 h, 6 h, 8 h, and 24 h. Before analysis and detection, all samples were stored at -80°C, and the samples were quantitatively analysed by LC-MS/MS.

Table 5. Pharmacokinetic parameters of test compounds in mouse plasma

| Test compound | Administration mode | CL (mL/min/kg) | $Vd_{ss}$ (L/kg) | $AUC_{0-t}$ (hr*ng/mL) | F (%) |
|---|---|---|---|---|---|
| **Compound 7** | i.v. (1 mg/kg) | 3.52±0.38 | 0.261±0.010 | 4717±512 | - |
| | i.g. (10 mg/kg) | - | - | 227±100 | 0.482±0.21 |
| **Compound 16** | i.v. (1 mg/kg) | 3.06±0.13 | 0.378±0.022 | 5438±220 | - |
| | i.g. (10 mg/kg) | - | - | 143±13 | 0.263±0.023 |
| **Compound 24** | i.v. (1 mg/kg) | 2.42±0.064 | 0.225±0.017 | 6773±183 | - |
| | i.g. (10 mg/kg) | - | - | 227±139 | 0.335±0.20 |
| -: not applicable.<br>Conclusion: The compounds of the present disclosure, such as the compounds in the Examples, have good pharmacokinetic characteristics in mice, for example, **Compound 7, Compound 16, and Compound 24** have excellent pharmacokinetic characteristics in mice. | | | | | |

**5. Test of stability in plasma**

In this experiment, plasmas of five species, including human, monkey, dog, rat, and mouse, were used to evaluate the stability in plasma of **Compound 7.**

A plasma sample was prepared at a concentration level of 1000 ng/mL, and dispensed into EP tubes at time points 0 h and 6 h. Acetonitrile solution containing an internal standard was added directly to the 0 h sample; and the 6 h sample was placed at 37°C for the corresponding time, and then added with an acetonitrile solution containing internal standard. The concentration of the test compound in the sample was measured by the LC-MS/MS method, and the residual rate was calculated from the ratio of the peak areas of the test compound to the internal standard in the time point sample and the 0 h sample.

**Experimental results:** At the concentration level of 1000 ng/mL under the test conditions, the residual rates of the test Compounds were shown in Table 6 below.

Table 6. Residual rate% at 37°C for 6 h

| Test compound | Human | Monkey | Dog | Rat | Mouse |
|---|---|---|---|---|---|
| **Compound 7** | >99 | >99 | >99 | 98.3 | >99 |

**6. Test of stability in gastrointestinal fluid**

1. Solution preparation

1.1. Preparation of dilute hydrochloric acid: 23.4 ml of hydrochloric acid was diluted with water to 1000 ml.

1.2. Preparation of simulated gastric fluid: 1.64 ml of dilute hydrochloric acid and 1 g of pepsin were added to about 80 ml of water. After being shaken well, the mixture was diluted with water to 100 ml.

1.3. Preparation of 0.1 mol/L NaOH: 0.4 g of NaOH was dissolved in 100 ml of water.

1.4. Preparation of simulated intestinal fluid: 0.68 g of potassium dihydrogen phosphate was dissolved in 50 ml of water, and the pH value was adjusted to 6.8 with 0.1 mol/L sodium hydroxide solution. 1 g of trypsin was dissolved in an appropriate amount of water. After the two solutions were mixed, the mixture was diluted with water to 1000 ml.

2. Sample preparation

About 12.5 mg of the sample was added to a 25 ml volumetric flask and dissolved in the simulated gastric fluid (or simulated intestinal fluid), and the mixture was diluted to the mark.

3. Analytical method

Instrument model: Agilent 1260 Infinity; mobile phase A: 10 mmol/L $K_2HPO_4$, and mobile phase B: acetonitrile; column: Phenomenex Gemini@ 3 um C18 110Å 150 * 4.6 mm; wavelength: 224 nm; column temperature: 30°C; sample tray temperature: 37°C; injection time: 35 min; injection volume: 10 ul; injection mode: gradient injection; and gradient method. The gradient mobile phase was shown in Table 7.

Table 7. Gradient mobile phase

| Time (min) | 0 | 20 | 25 | 27 | 35 |
|---|---|---|---|---|---|
| A (%) | 80 | 50 | 10 | 80 | 80 |
| B (%) | 20 | 50 | 90 | 20 | 20 |
| Sample concentration: 0.5 mg/ml | | | | | |

4. Sampling and testing and results

Sampling and testing: Firstly, the blank solution (simulated gastric fluid or simulated intestinal fluid) was tested. Then, the prepared sample (newly prepared for use) was placed in the injection tray for immediate injection. Then, a needle of sample was injected at 4.5 h, 9.5 h, and 18 h, respectively.

The test results were shown in Table 8.

Table 8. Stability in gastrointestinal fluid

| | Test time | 0 h | 4.5 h | 9.5 h | 18 h | RSD (%) |
|---|---|---|---|---|---|---|
| Peak area% | **Compound 7** (simulated gastric fluid) | 95.281 | 94.678 | 96.304 | 93.661 | 1.2 |
| | **Compound** 7 (simulated intestinal fluid) | 94.095 | 93.552 | 93.575 | 94.831 | 0.7 |
| Conclusion: **Compound 7** has good stability in simulated gastrointestinal fluid. | | | | | | |

## Claims

1. A cyclic peptide compound, or a stereoisomer, a pharmaceutically acceptable salt, a solvate or a dimer thereof, **characterized in that** the peptide compound has an amino acid sequence of formula (I):

$$Xa_1\text{-}Xa_2\text{-}Xa_3\text{-}Xa_4\text{-}Xa_5\text{-}Xa_6\text{-}Xa_7\text{-}Xa_8\text{-}Xa_9\text{-}Xa_{10}\text{-}Xa_{11}\text{-}Xa_{12}\text{-}Xa_{13} \quad (I)$$

in which $Xa_1$ and $Xa_6$ are each independently selected from Pen, Pcn, Asn, Ala, Ala(3-amino), Ala(2-ethyne), Ala(3-azido), Ala(2-ethene), Val(2-ethene), Asp, 2,4-diaminobutyric acid, Ser, Cys, Hcys, or Glu, and the residues of $Xa_1$ and $Xa_6$ react to form a peptide ring or form a cyclic peptide via $L_1$;
$Xa_2$ is selected from Asn, His, or an analogue of Asn and His;
$Xa_3$ is selected from Thr or an analogue of Thr;
$Xa_4$ is selected from Trp or an analogue of Trp;
$Xa_5$ is selected from Lys, Gln, Arg, Cit, or an analogue of Lys, Gln, Cit and Arg;
$Xa_7$ is selected from Phe or an analogue of Phe;
$Xa_8$ is selected from Phe, Trp, 2-Nal, or an analogue of Phe, Trp and 2-Nal;
$Xa_9$ is selected from Thp or an analogue of Thp;
$Xa_{10}$ is selected from Glu, Cys, or an analogue of Glu and Cys;
$Xa_{11}$ is selected from Asn, Lys, or an analogue of Asn and Lys;
$Xa_{12}$ is selected from 3-Pal, Phe, Asp, or an analogue of 3-Pal, Phe and Asp;
$Xa_{13}$ is selected from Sarc or an analogue of Sarc;
alternatively, the residues of any amino acid in $Xa_1$, $Xa_2$, $Xa_3$, $Xa_4$, $Xa_5$, $Xa_7$, $Xa_8$, $Xa_9$, $Xa_{10}$, $Xa_{11}$, $Xa_{12}$, and $Xa_{13}$ are directly condensed or are linked via $L_1$ to form one or more peptide rings;
$L_1$ is $W_1$-$R^L$-$W_2$;
$R^L$ is selected from a bond, $C_{1-6}$ alkylene, $C_{2-4}$ alkenylene, $C_{2-4}$ alkynylene, 3-to 6-membered cycloalkyl, 4- to 6-membered heterocycloalkyl, 5- to 6-membered heteroaryl, 6- to 10-membered aryl, or $-(OCH_2CH_2)_a-$, wherein the alkylene, alkenylene, alkynylene, cycloalkyl, heterocycloalkyl, heteroaryl, or aryl is optionally further substituted with 1-4 $R^{L1}$;
a is any integer selected from 0-10; each $R^{L1}$ is independently selected from halogen, =O, $C_{1-4}$ alkyl, $C_{2-4}$ alkenyl, $C_{1-4}$ alkoxy, 3- to 6-membered cycloalkyl, COOH, $NH_2$, or $-NH-C(=O)-C_{1-4}$ alkyl, wherein the alkyl, alkoxy, or cycloalkyl is optionally further substituted with 1-4 substituents selected from halogen, CN, OH, and $NH_2$;
$W_1$ and $W_2$ are each independently selected from a bond, $C_{1-6}$ alkylene, -O-, - S-, $-NR^{W1}-$, $-CONR^{W1}-$, $-NR^{W1}CO-$, -C(=O)O-, or -OC(=O)-, wherein one or more $-CH_2-$ in the alkylene are optionally replaced by 1-4 groups selected from -O-, -S-, - $NR^{W1}$-, or -CO-, and the alkylene is optionally further substituted with 1-4 substituents selected from halogen, =O, $C_{1-4}$ alkyl, halo$C_{1-4}$ alkyl, CN, OH, and $NH_2$;
$R^{W1}$ is selected from H, $C_{1-4}$ alkyl, or halogen;
and the peptide compound is optionally linked to a protecting group;
the protecting group is selected from Ac, glutaryl, succinyl, $NH_2$, or OH;
alternatively, a polypeptide is separated by covalent bonds formed between reactive groups $Xa_1$, $Xa_6$ and $Xa_{10}$ and a molecular scaffold to form a polypeptide loop containing at least two rings;
alternatively, the peptide compound is optionally conjugated to a modifying group at $Xa_1$, $Xa_5$, or $Xa_7$;
provided that: the peptide compound is not of the following structure: (Ac)Pen-Asn-Thr-Trp($CH_3$)-Lys(Ac)-Pen-Phe[4-(2-aminoethoxy)]-[2-Nal]-Thp-Glu-Asn-[3-Pal]-Sarc($NH_2$), wherein a disulphide bond is formed between Pen and Pen.

2. The peptide compound, or the stereoisomer, the pharmaceutically acceptable salt, the solvate or the dimer thereof according to claim 1, **characterized in that** the peptide compound has an amino acid sequence of formula (II) or formula (II-1):

$$(Ac)Xa_1\text{-}Xa_2\text{-Thr-}Xa_4\text{-}Xa_5\text{-}Xa_6\text{-}Xa_7\text{-}Xa_8\text{-}Xa_9\text{-}Xa_{10}\text{-Asn-(3-Pal)-Sarc}(NH_2)$$

(II)

$$(Ac)Xa_1\text{-}Xa_2\text{-Thr-}Xa_4\text{-Lys(Ac)-}Xa_6\text{-}Xa_7\text{-}Xa_8\text{-}Xa_9\text{-Glu-Asn-(3-Pal)-Sarc}(NH_2)$$

(II-1) (SEQ ID NO.1)

wherein the residues of $Xa_1$ and $Xa_6$ react to form a peptide ring, or are linked via $L_1$ to form a peptide ring.

**3.** The peptide compound, or the stereoisomer, the pharmaceutically acceptable salt, the solvate or the dimer thereof according to claim 1, **characterized in that** the peptide compound has an amino acid sequence of formula (III) or formula (III-1):

(Ac)$Xa_1$-$Xa_2$-Thr-Trp($R_1$)-$Xa_5$-$Xa_6$-$Xa_7$-$Xa_8$-$Xa_9$-$Xa_{10}$-Asn-(3-Pal)-Sarc($NH_2$)

(III)

(Ac)$Xa_1$-$Xa_2$-Thr-Trp($CH_3$)-Lys(Ac)-$Xa_6$-$Xa_7$-$Xa_8$-$Xa_9$-Glu-Asn-(3-Pal)-Sarc($NH_2$)

(III-1) (SEQ ID NO.2)

wherein the residues of $Xa_1$ and $Xa_6$ react to form a peptide ring or form a cyclic peptide via Li;
and the residues of Trp($R_1$) and $Xa_5$ are directly condensed or are linked via $L_1$ to form a peptide ring;
or the residues of Trp($CH_3$) and Lys(Ac) are directly condensed or are linked via $L_1$ to form a peptide ring;
or $Xa_1$, $Xa_6$ and $Xa_{10}$ and the molecular scaffold form a bicyclic peptide;
$R_1$ is selected from H, $C_{1-4}$ alkyl, $C_{3-6}$ cycloalkyl or 4- to 8-membered heterocycloalkyl, wherein the alkyl, cycloalkyl or heterocycloalkyl is optionally further substituted with 1-4 substituents selected from halogen, =O, $C_{1-4}$ alkyl, halo$C_{1-4}$ alkyl, CN, OH and $NH_2$.

**4.** The peptide compound, or the stereoisomer, the pharmaceutically acceptable salt, the solvate or the dimer thereof according to claim 1, **characterized in that** the peptide compound has an amino acid sequence of formula (IV), formula (IV-1) or formula (V):

(N-terminus)$Xa_1$-Asn-Thr-Trp($R_1$)-$Xa_5$-$Xa_6$-$Xa_7$-(2-Nal)-$Xa_9$-$Xa_{10}$-$Xa_{11}$-$Xa_{12}$-Sarc(C-terminus)

(IV)

(N-terminus)$Xa_1$-Asn-Thr-Trp($CH_3$)-Lys(Ac)-$Xa_6$-$Xa_7$-(2-Nal)-$Xa_9$-Glu-$Xa_{11}$-$Xa_{12}$-Sarc(C-terminus)

(IV-1) (SEQ ID NO.3)

(N-terminus)$Xa_1$-His-Thr-Trp($R_1$)-$Xa_5$-$Xa_6$-$Xa_7$-(2-Nal)-$Xa_9$-$Xa_{10}$-$Xa_{11}$-$Xa_{12}$-Sarc(C-terminus) (V)
wherein the residues of $Xa_1$ and $Xa_6$ react to form a peptide ring, or are linked via $L_1$ to form a peptide ring;
at the N-terminus and the C-terminus, a protecting group is present or absent;
or the N-terminus is conjugated to a modifying group;
the residues of 2-Nal and $Xa_{12}$ are linked via $L_1$ to form a peptide ring; and/or
the residues of $Xa_9$ and $Xa_{12}$ are linked via $L_1$ to form a peptide ring; and/or
the residues of Lys(Ac) and $Xa_7$ are linked via $L_1$ to form a peptide ring; and/or
the residues of $Xa_7$ and $Xa_{11}$ are linked via $L_1$ to form a peptide ring; and/or
the residues of 2-Nal and Glu are linked via $L_1$ to form a peptide ring; and/or
the residues of $Xa_1$ and Glu are linked via $L_1$ to form a peptide ring; and/or
the residues of $Xa_1$ and $Xa_{11}$ are linked via $L_1$ to form a peptide ring; and/or
the residues of $Xa_1$ and $Xa_{10}$ are linked via $L_1$ to form a peptide ring; and/or
the residues of 2-Nal and Sarc are linked via $L_1$ to form a peptide ring; and/or
the residues of $Xa_9$ and Sarc are linked via $L_1$ to form a peptide ring; and/or
the residues of $Xa_{11}$ and Sarc are linked via $L_1$ to form a peptide ring; and/or
the residues of $Xa_1$, Sarc and Glu are linked via $L_1$ to form a peptide ring; and/or
or $Xa_1$, $Xa_6$ and $Xa_{10}$ and the molecular scaffold form a bicyclic peptide;
$R_1$ is selected from $C_{1-2}$ alkyl, cyclopropyl, cyclobutyl, cyclopentyl or cyclohexyl, wherein the alkyl, cyclopropyl, cyclobutyl, cyclopentyl or cyclohexyl is optionally further substituted with 1-4 substituents selected from F, Cl, Br, =O, methyl, ethyl, $-CH_2CH_2F$, $-CH_2CHF_2$, $-CH_2CF_3$, $-CH_2F$, $-CHF_2$, $-CF_3$, CN, OH and $NH_2$;
provided that: (1) when $Xa_1$ at the N-terminus is linked to the residues of other amino acids via $L_1$, a protecting group at the N-terminus is absent;
(2) when Sarc at the C-terminus is linked to the residues of other amino acids via $L_1$, a protecting group at the C-terminus is absent.

**5.** The peptide compound, or the stereoisomer, the pharmaceutically acceptable salt, the solvate or the dimer thereof according to claim 1, **characterized in that**

the residues of $Xa_1$ and $Xa_6$ react to form a peptide ring or form a cyclic peptide via Li; and the residues of one or two groups of $Xa_4$ and $Xa_5$, $Xa_1$ and $Xa_{10}$, $Xa_8$ and $Xa_{10}$, $Xa_8$ and $Xa_{12}$, $Xa_8$ and $Xa_{13}$, $Xa_9$ and $Xa_{13}$, $Xa_5$ and $Xa_7$, $Xa_{11}$ and $Xa_{13}$, $Xa_1$ and $Xa_{11}$, $Xa_7$ and $Xa_{11}$ are directly condensed or are linked via $L_1$ to form a peptide ring; or $Xa_1$, $Xa_6$ and $Xa_{10}$ and the molecular scaffold form a bicyclic peptide.

**6.** The peptide compound, or the stereoisomer, the pharmaceutically acceptable salt, the solvate or the dimer thereof according to claim 1, **characterized in that** the peptide compound is a dimer compound, and the dimer compound is formed by linking the peptide compound to amino acid residues in the peptide compound via a polyethylene glycol chain; the polyethylene glycol chain is

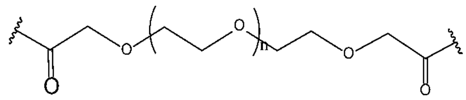,

n is any integer selected from 0-99.

**7.** The peptide compound, or the stereoisomer, the pharmaceutically acceptable salt, the solvate or the dimer thereof according to claim 1, **characterized in that** the modifying group is

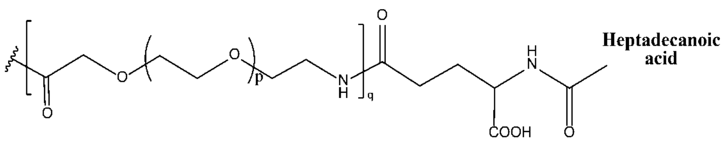,

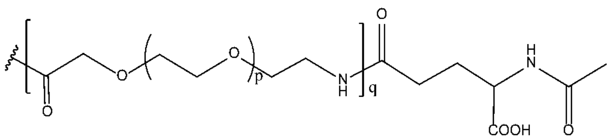,

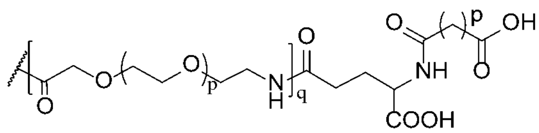 or 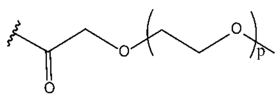,

in which p is any integer selected from 0-50, and q is any integer selected from 0-50.

**8.** The peptide compound, or the stereoisomer, the pharmaceutically acceptable salt, the solvate or the dimer thereof according to any one of claims 1-7, **characterized in that**

the residues of $Xa_1$ and $Xa_6$ react to form the following structure:

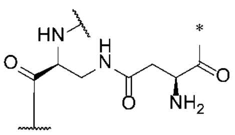, 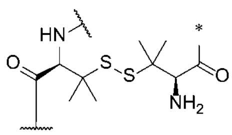, 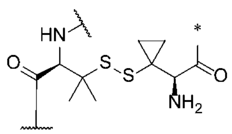, 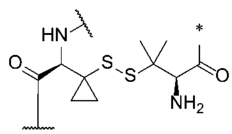,

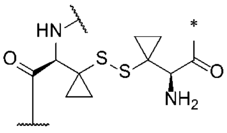, 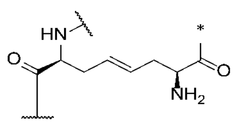, 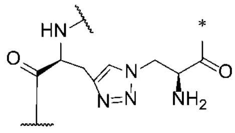, 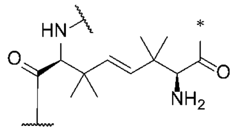,

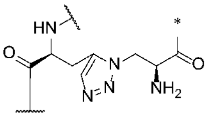, 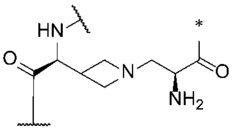, 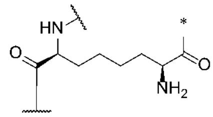, 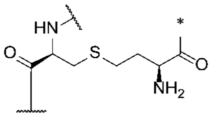,

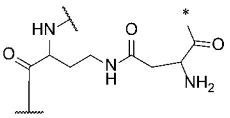, 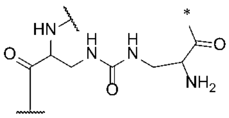, 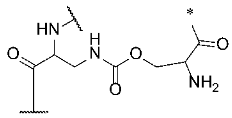, 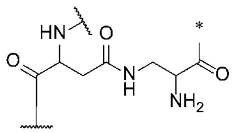,

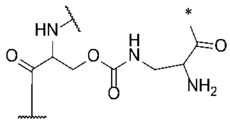, 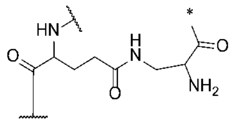 or 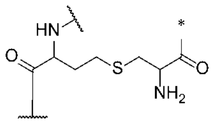,

in which the * end is an end of $Xa_1$, $Xa_1$ is linked to $Xa_2$ via the * position, and the $NH_2$ terminal end is linked to a protecting group or the $NH_2$ terminal end is conjugated to a modifying group;
$Xa_2$ is selected from Asn, His, or an analogue of His, and the analogue of His is selected from

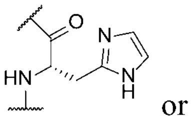 or 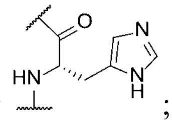;

$Xa_3$ is Thr;
$Xa_4$ is an analogue of Trp, and the analogue of Trp is selected from

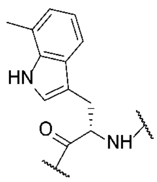

, 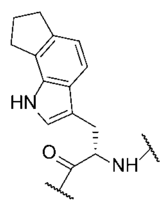, 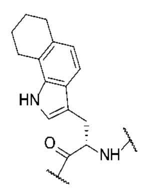, 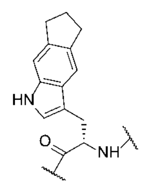, 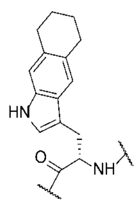, 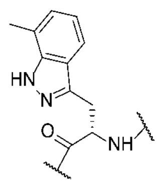, 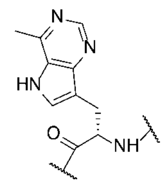,

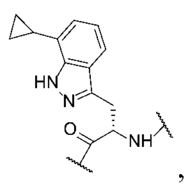, or 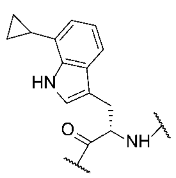;

$Xa_5$ is selected from Lys, Gln, Arg, Cit, or an analogue Arg and Lys, and the analogue of Arg and Lys is selected

from

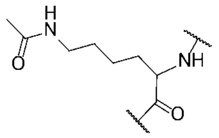 or 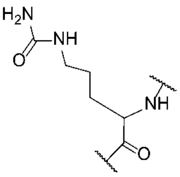;

alternatively, a residue of $Xa_5$ is conjugated to a modifying group;

$Xa_7$ is selected from Phe or an analogue of Phe, and the analogue of Phe is selected from

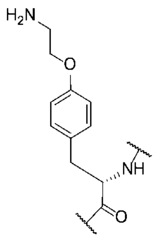, 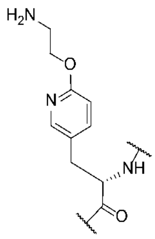, 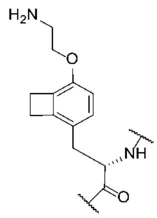, 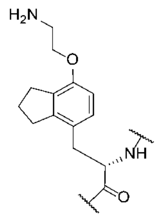, 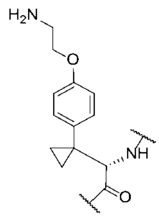,

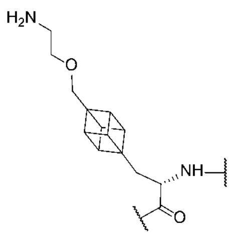, or 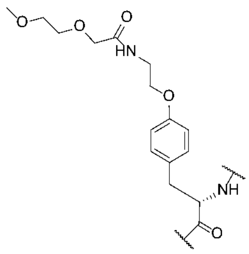;

alternatively, a residue of $Xa_7$ is conjugated to a modifying group;

$Xa_8$ is selected from Phe, Trp, 2-Nal, or an analogue of Phe, Trp, and 2-Nal, and the analogue of Phe, Trp, and 2-Nal is selected from

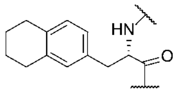, 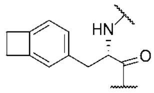

, 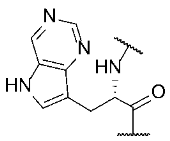, 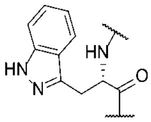, 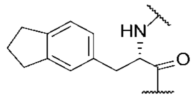, 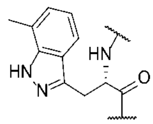, or 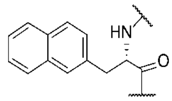;

$Xa_9$ is selected from Thp or an analogue of Thp, and the analogue of Thp is selected from

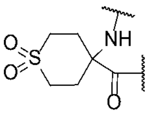, 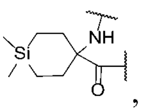, 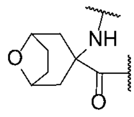, or 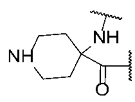;

$Xa_{10}$ is selected from Glu or Cys;

$Xa_{11}$ is selected from Asn or Lys;

$Xa_{12}$ is selected from 3-Pal or Phe;

the molecular scaffold is selected from:

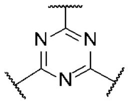, 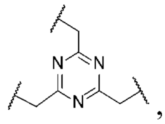, 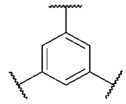,

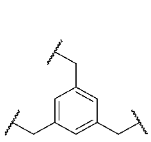, 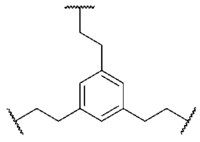, 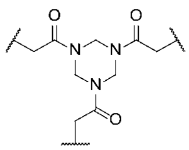, 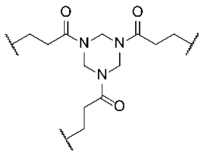 or 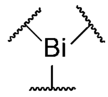.

**9.** The peptide compound, or the stereoisomer, the pharmaceutically acceptable salt, the solvate or the dimer thereof according to claim 1, **characterized in that**

$L_1$ is selected from a bond, vinyl, propenyl, butenyl, -O-(CH2)$_r$-O-(CH2)$_r$-NH- C(=O)-, -O-$(CH_2)_r$-O-$(CH_2)_r$-, -O-$(CH_2)_r$-O-$(CH_2)_r$-NH-, -C(=O)-$(CH_2)_r$-O-$(CH_2)_r$- O-$(CH_2)_r$-, -C(=O)-$(CH_2)_r$-O-$(CH_2)_r$-O-$(CH_2)_r$-NH-, -C(=O)-$(CH_2)_r$-O-$(CH_2)_r$-NH- C(=O)-, -NH-C(=O)-, -C(=O)-$(CH_2)_r$-O-$(CH_2)_r$-, -O-$(CH_2)_r$-NH-C(=O)-$(CH_2)_r$-, -(CH2)r-O-(CH2)r-, -O-$(CH_2)_r$-NH-, $C_{1-6}$ alkylene, -C(=O)-, -C(=O)-$(CH_2)_r$-NH-,

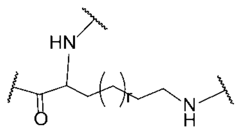, 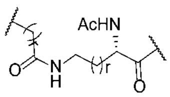,

-$(CH_2)_r$-O-$(CH_2)_r$-NH-, -O-$(CH_2)_r$-O-$(CH_2)_r$-O-$(CH_2)_r$-O-$(CH_2)_r$-NH-, -$(CH_2)_r$-NH-, -C(=O)-$(CH_2)_r$-O-$(CH_2)_r$-NH-, -$(CH_2)_r$-NH-C(=O)-$(CH_2)_r$-, or -C(=O)-$(CH_2)$-$(OCH_2CH_2)_a$-NH-;
r is selected from 0, 1, 2, 3, 4, 5, 6, 7, 8, 9, or 10; and
a is selected from 3, 4, 5, or 6.

**10.** The peptide compound, or the stereoisomer, the pharmaceutically acceptable salt, the solvate or the dimer thereof according to claim 1, **characterized in that** the peptide compound is of a structure selected from one of the structures in Table 1.

**11.** A pharmaceutical composition, **characterized by** comprising the peptide compound or the pharmaceutically acceptable salt thereof according to any one of claims 1 to 10, and a pharmaceutically acceptable carrier and/or excipient.

**12.** Use of the peptide compound or the pharmaceutically acceptable salt thereof according to any one of claims 1 to 10, or the pharmaceutical composition according to claim 11 in the preparation of a medicament for preventing and treating an IL-23-overexpressing disease or condition in a diseased tissue of a subject.

**13.** The use according to claim 12, **characterized in that** the IL-23-overexpressing disease or condition includes inflammatory bowel disease, Crohn's disease and psoriasis.

**14.** A pharmaceutical composition or a pharmaceutical preparation, **characterized by** comprising 1-1500 mg of the peptide compound or the pharmaceutically acceptable salt thereof according to any one of claims 1 to 10, and a pharmaceutically acceptable carrier and/or excipient.

**15.** A method for treating a disease in a mammal or a human, **characterized in that** the method comprises administering to a subject a therapeutically effective amount of the peptide compound or the pharmaceutically acceptable salt thereof according to any one of claims 1 to 10, wherein the therapeutically effective amount is preferably 1-1500 mg, and the disease is preferably inflammatory bowel disease, Crohn's disease and psoriasis.

**INTERNATIONAL SEARCH REPORT**

International application No.
**PCT/CN2024/090789**

**A. CLASSIFICATION OF SUBJECT MATTER**

C07K7/08(2006.01)i; C07K7/04(2006.01)i; C07K7/02(2006.01)i; A61K38/00(2006.01)i

According to International Patent Classification (IPC) or to both national classification and IPC

**B. FIELDS SEARCHED**

Minimum documentation searched (classification system followed by classification symbols)

IPC:C07K,A61K

Documentation searched other than minimum documentation to the extent that such documents are included in the fields searched

Electronic data base consulted during the international search (name of data base and, where practicable, search terms used)

CNTXT, ENTXTC, VCN, VEN, PUBMED, NCBI, CNKI, BAIDU, BING, STN: 白介素-23受体, IL-23, IL-23R, 抑制剂, inhibitor, 肽, peptide, 环肽, cyclic peptide, pen, sarc, 化合物1-55, compounds 1-55

**C. DOCUMENTS CONSIDERED TO BE RELEVANT**

| Category* | Citation of document, with indication, where appropriate, of the relevant passages | Relevant to claim No. |
|---|---|---|
| X | CN 115298196 A (JANSSEN BIOTECH, INC. et al.) 04 November 2022 (2022-11-04) claims 162-186, and description, paragraphs 681 and 1549 | 1-15 |
| A | CN 115279782 A (JANSSEN BIOTECH, INC. et al.) 01 November 2022 (2022-11-01) entire document | 1-15 |
| A | CN 107206254 A (PROTAGONIST THERAPEUTICS, INC.) 26 September 2017 (2017-09-26) entire document | 1-15 |
| A | CN 108348580 A (PROTAGONIST THERAPEUTICS, INC.) 31 July 2018 (2018-07-31) entire document | 1-15 |
| A | CN 113563423 A (PROTAGONIST THERAPEUTICS, INC.) 29 October 2021 (2021-10-29) entire document | 1-15 |
| A | CN 114341161 A (PROTAGONIST THERAPEUTICS, INC.) 12 April 2022 (2022-04-12) entire document | 1-15 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **18 July 2024** | **25 July 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2024/090789**

**C. DOCUMENTS CONSIDERED TO BE RELEVANT**

| Category* | Citation of document, with indication, where appropriate, of the relevant passages | Relevant to claim No. |
|---|---|---|
| A | US 2020040037 A1 (PROTAGONIST THERAPEUTICS, INC.) 06 February 2020 (2020-02-06)<br>entire document | 1-15 |
| A | US 2023129095 A1 (PROTAGONIST THERAPEUTICS, INC.) 27 April 2023 (2023-04-27)<br>entire document | 1-15 |

Form PCT/ISA/210 (second sheet) (July 2022)

**INTERNATIONAL SEARCH REPORT**

International application No.
**PCT/CN2024/090789**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/090789**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

Claim 15 relates to a method for treating a disease in a mammal or a human, which falls within methods for treatment of diseases as defined in PCT Rule 39.1(iv). The present report is provided on the basis of a corresponding pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/090789**

| Patent document cited in search report | Publication date (day/month/year) | Patent family member(s) | Publication date (day/month/year) |
|---|---|---|---|
| CN 115298196 A | 04 November 2022 | BR 112022013957 A2 | 11 October 2022 |
| | | AR 121070 A1 | 13 April 2022 |
| | | PE 20230370 A1 | 06 March 2023 |
| | | KR 20220141807 A | 20 October 2022 |
| | | AU 2021207653 A1 | 04 August 2022 |
| | | JP 2023139158 A | 03 October 2023 |
| | | MX 2022008741 A | 03 October 2022 |
| | | TW 202140517 A | 01 November 2021 |
| | | WO 2021146441 A1 | 22 July 2021 |
| | | CL 2022001893 A1 | 03 March 2023 |
| | | US 2024150405 A1 | 09 May 2024 |
| | | CO 2022009931 A2 | 21 October 2022 |
| | | JP 2023511552 A | 20 March 2023 |
| | | JP 7441955 B2 | 01 March 2024 |
| | | CA 3167751 A1 | 22 July 2021 |
| | | DOP 2022000147 A | 15 January 2023 |
| | | EP 4090670 A1 | 23 November 2022 |
| | | JOP 20220169 A1 | 30 January 2023 |
| | | IL 294680 A | 01 September 2022 |
| | | US 2021261622 A1 | 26 August 2021 |
| | | US 11845808 B2 | 19 December 2023 |
| | | ECSP 22063238 A | 30 September 2022 |
| | | CR 20220332 A | 28 November 2022 |
| CN 115279782 A | 01 November 2022 | EP 4090669 A1 | 23 November 2022 |
| | | JP 2023511551 A | 20 March 2023 |
| | | WO 2021146454 A1 | 22 July 2021 |
| | | US 2022402983 A1 | 22 December 2022 |
| | | US 12018057 B2 | 25 June 2024 |
| | | KR 20220141808 A | 20 October 2022 |
| | | BR 112022014011 A2 | 20 December 2022 |
| | | AU 2021209086 A1 | 04 August 2022 |
| | | MX 2022008740 A | 23 September 2022 |
| | | CA 3168135 A1 | 22 July 2021 |
| CN 107206254 A | 26 September 2017 | JP 2017530090 A | 12 October 2017 |
| | | US 2021363185 A1 | 25 November 2021 |
| | | US 11884748 B2 | 30 January 2024 |
| | | SG 10201810154 WA | 28 December 2018 |
| | | US 2018079783 A1 | 22 March 2018 |
| | | US 10023614 B2 | 17 July 2018 |
| | | CA 2955460 A1 | 21 January 2016 |
| | | DK 3169403 T3 | 18 March 2024 |
| | | BR 112017001010 A2 | 14 November 2017 |
| | | US 2018022778 A1 | 25 January 2018 |
| | | US 10196424 B2 | 05 February 2019 |
| | | US 2018079782 A1 | 22 March 2018 |
| | | US 10035824 B2 | 31 July 2018 |
| | | SG 11201700327 WA | 27 February 2017 |
| | | KR 20170033372 A | 24 March 2017 |
| | | KR 102482790 B1 | 29 December 2022 |
| | | US 2016145306 A1 | 26 May 2016 |

Form PCT/ISA/210 (patent family annex) (July 2022)

**INTERNATIONAL SEARCH REPORT**
**Information on patent family members**

International application No.
**PCT/CN2024/090789**

| Patent document cited in search report | Publication date (day/month/year) | Patent family member(s) | Publication date (day/month/year) |
|---|---|---|---|
| | | US 9624268 B2 | 18 April 2017 |
| | | WO 2016011208 A1 | 21 January 2016 |
| | | US 2024199700 A1 | 20 June 2024 |
| | | JP 2020128443 A | 27 August 2020 |
| | | JP 6995933 B2 | 21 February 2022 |
| | | US 2019337983 A1 | 07 November 2019 |
| | | US 10941183 B2 | 09 March 2021 |
| | | IL 285614 A | 30 September 2021 |
| | | AU 2015289642 A1 | 02 February 2017 |
| | | AU 2015289642 B2 | 25 February 2021 |
| | | EP 4378536 A2 | 05 June 2024 |
| | | RU 2017105115 A | 17 August 2018 |
| | | RU 2017105115 A3 | 21 January 2019 |
| | | RU 2736637 C2 | 19 November 2020 |
| | | RU 2736637 C9 | 08 February 2021 |
| | | EP 3169403 A1 | 24 May 2017 |
| | | EP 3169403 A4 | 24 January 2018 |
| | | EP 3169403 B1 | 14 February 2024 |
| | | EP 3169403 B9 | 03 July 2024 |
| | | IL 250111 A0 | 30 March 2017 |
| | | IL 250111 B | 31 August 2021 |
| CN 108348580 A | 31 July 2018 | EP 3341011 A2 | 04 July 2018 |
| | | EP 3341011 A4 | 20 February 2019 |
| | | CR 20180029 A | 05 June 2018 |
| | | PH 12018500086 A1 | 30 July 2018 |
| | | JP 2018522008 A | 09 August 2018 |
| | | JP 6858174 B2 | 14 April 2021 |
| | | IL 256827 A | 29 March 2018 |
| | | UA 123772 C2 | 02 June 2021 |
| | | MX 2018000542 A | 27 June 2018 |
| | | KR 20180030663 A | 23 March 2018 |
| | | KR 102513978 B1 | 27 March 2023 |
| | | CO 2018000349 A2 | 10 July 2018 |
| | | AU 2016293619 A1 | 01 February 2018 |
| | | AU 2016293619 B2 | 25 February 2021 |
| | | CL 2018000128 A1 | 15 June 2018 |
| | | CL 2018003322 A1 | 18 January 2019 |
| | | SV 2018005614 A | 22 May 2018 |
| | | EA 201890325 A1 | 29 June 2018 |
| | | EA 035733 B1 | 31 July 2020 |
| | | EA 035733 B9 | 14 January 2021 |
| | | BR 112018000691 A2 | 18 September 2018 |
| | | NI 201800008 A | 29 June 2018 |
| | | CA 2991984 A1 | 19 January 2017 |
| | | HK 1259149 A1 | 29 November 2019 |
| | | PE 20180571 A1 | 04 April 2018 |
| | | HK 1257747 A1 | 25 October 2019 |
| | | ECSP 18002929 A | 31 March 2018 |
| | | CL 2021000343 A1 | 06 August 2021 |
| | | DOP 2018000010 A | 30 March 2018 |

Form PCT/ISA/210 (patent family annex) (July 2022)

**INTERNATIONAL SEARCH REPORT**
**Information on patent family members**

International application No.
**PCT/CN2024/090789**

| Patent document cited in search report | Publication date (day/month/year) | Patent family member(s) | Publication date (day/month/year) |
|---|---|---|---|
| | | WO 2017011820 A2 | 19 January 2017 |
| | | WO 2017011820 A3 | 23 February 2017 |
| | | SG 10201912066 SA | 27 February 2020 |
| CN 113563423 A | 29 October 2021 | None | |
| CN 114341161 A | 12 April 2022 | TW 202116793 A | 01 May 2021 |
| | | WO 2021007433 A1 | 14 January 2021 |
| | | AR 119389 A1 | 15 December 2021 |
| US 2020040037 A1 | 06 February 2020 | TW 202019948 A | 01 June 2020 |
| | | EP 3820883 A1 | 19 May 2021 |
| | | EP 3820883 A4 | 20 April 2022 |
| | | CA 3104418 A1 | 16 January 2020 |
| | | JP 2021530477 A | 11 November 2021 |
| | | WO 2020014646 A1 | 16 January 2020 |
| | | US 2022251142 A1 | 11 August 2022 |
| US 2023129095 A1 | 27 April 2023 | WO 2021146458 A1 | 22 July 2021 |

Form PCT/ISA/210 (patent family annex) (July 2022)